(19) Europäisches Patentamt
European Patent Office
Office européen des brevets

(11) EP 4 556 017 A1

(12) **EUROPEAN PATENT APPLICATION**
published in accordance with Art. 153(4) EPC

(43) Date of publication:
**21.05.2025 Bulletin 2025/21**

(21) Application number: **23838915.9**

(22) Date of filing: **11.07.2023**

(51) International Patent Classification (IPC):
***A61K 38/16*** *(2006.01)* ***A61P 1/16*** *(2006.01)*
***A61P 31/20*** *(2006.01)* ***C07K 14/705*** *(2006.01)*

(52) Cooperative Patent Classification (CPC):
**A61K 38/16; A61P 1/16; A61P 31/20; C07K 14/705**

(86) International application number:
**PCT/CN2023/106678**

(87) International publication number:
**WO 2024/012423 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.07.2022 CN 202210853740**

(71) Applicant: **Shanghai Hep Pharmaceutical Co., Ltd.**
**Pudong New Area, Shanghai 201203 (CN)**

(72) Inventor: **LIU, Hongli**
**Shanghai 201203 (CN)**

(74) Representative: **V.O.**
**P.O. Box 87930**
**2508 DH Den Haag (NL)**

Remarks:
The complete document including Reference Table(s) and the Sequence Listing(s) can be downloaded from the EPO website

(54) **HEPATITIS B VIRUS RECEPTOR OATP AND USE THEREOF**

(57) Provided in the present invention are a hepatitis B virus receptor OATP and the use thereof. Specifically, provided in the present invention is the use of a substance in the preparation of an agent for preventing and/or treating hepatitis B virus (HBV) and/or hepatitis D virus (HDV) infections in an animal or diseases related to said infections, wherein the substance prevents or reduces the interaction between HBV and/or HDV and an organic anion transporting polypeptide (OATP) in the animal, and/or prevents or reduces the expression and/or function of OATP in the animal. Further provided in the present invention are a cell model and an animal model having susceptibility to HBV and/or HDV that are obtained by means of transferring an exogenous SLCO gene into a cell or expressing an exogenous OATP protein, and a method for establishing the models; a method for delivering a drug by means of targeting OATP, and a drug delivery vehicle targeting OATP; and an OATP-targeted delivery polypeptide vehicle derived from a hepadnaviridae surface antigen.



Processed by Luminess, 75001 PARIS (FR)

## Description

FIELD OF THE INVENTION

The present invention relates to HBV receptor OATP and use thereof, specifically to the use of HBV receptor OATP in the treatment and prevention of HBV and/or HDV infection and related diseases, HBV and/or HDV cell and animal infection models and drug screening. The present invention also relates to the use of polypeptides derived from the surface antigen of hepadnavirus in the treatment and prevention of HBV and/or HDV infection and related diseases, targeting OATP in drug delivery vehicles and inhibiting OATP transport function.

BACKGROUND OF THE INVENTION

About 350 million people worldwide have been infected with hepatitis B virus (HBV), and about 200 million people suffer from chronic hepatitis B. These people have a higher chance of progressing to cirrhosis, liver failure, and liver cancer. HBV belongs to the family Hepadnaviridae, which is an ancient pathogen that naturally infects mammals (Orthohepadnavirus, OHV), birds (Avihepadnavirus, AHV), fish (Metahepadnavirus) and amphibians (Herpetohepadnavirus). Among mammals, rodent prohepadnaviruses have been found in only three species of the Sciuridae family, namely the arctic ground squirrel, the ground squirrel, and the woodchuck. In primates, orthohepadnavirus have been isolated from hominoid species, including human, chimpanzee, gorilla, gibbon, and orangutan. Natural orthohepadnavirus infections have been reported in three New World monkey species, including Woolly monkeys, capuchin monkeys, and Squirrel monkeys. Natural infection of bat orthohepadnavirus infects multiple species in at least 5 clades [Jacquet S, et al. J Virol. 2019;93(5):e01738-18.].

The human HBV genome is a partially double-stranded circular DNA, approximately 3.2 kb in length. The four highly overlapping gene regions encode at least 8 proteins, namely the large (L), middle (M) and small (Small, S) surface antigens HBs encoded by the S region gene; pre-C, HBc, HBe encoded by the C gene; polymerase encoded by P gene and HBx encoded by X gene. There are three main forms of related particles produced by HBV, including Dane particles with a diameter of 42-47nm, 20nm spherical particles that are 10,000-1,000,000 times more abundant than Dane particles, and a small number of filamentous fibers with a diameter of 20nm and varying lengths. Among them, only Dane particles are composed of genomic DNA, viral DNA polymerase, nucleocapsid and envelope, and are the only viral particles of HBV with infectious activity. The HBV viral envelope contains three surface antigen proteins: large (L), medium (M), and small (S). These proteins are encoded by a single open reading frame of the S region gene with three different translation start sites, namely L (Pre-S1+Pre S2+S), M(Pre S2+S) and S(S). The expression levels of the three surface proteins vary greatly, and their distribution patterns are significantly different. The S protein is highly expressed and is the main protein of HBs in the circulating blood of HBV-infected patients, while the M and L proteins are very small, accounting for only 5-15% and 1-2% of the total HBs protein respectively. The envelope proteins of 20nm spherical particles and filamentous fibers are mainly composed of S protein and varying amounts of M protein, with almost no L protein, while the envelope proteins of Dane particles are mainly L protein.

Hepatitis delta virus (HDV) is an RNA-deficient virus, a satellite virus of HBV, and a spherical particle with a diameter of about 36 nm. The inner part is the nucleocapsid, which is composed of 1.7kb circular RNA and about 200 copies of hepatitis D antigen (HDAg); the outer envelope contains HBV surface antigen and the host cell plasma membrane. HDV can only be passed down in the presence of HBV and is rarely infected alone. Co-infection of HBV and HDV usually leads to severe clinical prognosis. An estimated 15 million people worldwide are chronically infected with HDV. Its incidence of liver cirrhosis and liver cancer are three times and nine times that of chronic hepatitis B, respectively. It is considered the most serious viral hepatitis. Since HDV borrows the HBV envelope, the infection receptors of HBV and HDV are considered to be very similar [Hughes et al., Lancet 378, 73 (2011); Sureau, Curr Top Microbiol Immunol 307, 113 (2006); Barrera et al., J Virol 78, 5233 (2004)].

The surface antigen Pre-S1 region of HBV contains key amino acid sequences that bind to hepatocyte-specific receptors [Le Seyec J et al. J Virol. 1999; 73(3): 2052-7.], in which the N-terminal amino acid sequence and myristic acid modification is required for HBV infection [Bruss V et al. Virology. 1996; 218: 396-399]. The human liver cell line HepG2 can support HBV and HDV infection after transfection with human sodium taurocholate cotransporting polypeptide (hNTCP), and the HBVPreS1 region polypeptide with N-terminal myristate modification can interact with hNTCP binds, so hNTCP is considered to be a functional receptor for HBV and HDV infection [Yan H et al. Elife.2012; 1: e00049.; Ni Y et al. Gastroenterology. 2014; 146(4): 1070-83.]. However, HepG2 cells transfected with hNTCP (HepG2-hNTCP) need to be infected by HBV in the presence of polyethylene glycol (PEG) [Yan H et al. Elife. 2012; 1: e00049.], and the infection is not a natural process, and the infection efficiency is low [Herrscher C et al. Cells.2020Jun18;9(6):1486]. Moreover, even if human cell lines of non-liver origin or non-human hepatocytes (such as hepatocytes of rats, mice, dog, etc.) are transfected with hNTCP, they do not support HBV infection [Lempp FA et al. Hepatology.2017; 66(3) :703-716]; hNTCP transgenic mice also do not support HBV infection [He W et al. J Virol. 2016 Sep 12; 90 (19): 8866-74]. It can be seen that hNTCP is a

necessary but not sufficient condition for efficient HBV infection, and other components are involved in receptor composition [Herrscher C et al. Cells. 2020 Jun 18;9(6):1486]. Except for humans, tree shrews, chimpanzees, and their primary liver cells that are naturally susceptible to HBV or HDV, cell and animal models that can be naturally infected with HBV are currently unavailable. Therefore, it is necessary to further study other components of the HBV receptor and develop effective methods for the treatment and prevention of HBV and/or HDV infection and related diseases. It is also necessary to develop in vitro cell infection systems and transgenic animal models for screening drugs that can treat and prevent HBV and/or HDV, the mechanism of HBV infection of liver cells can also be used to develop liver-targeted drug delivery vehicles.

Organic anion transporting polypeptide (OATP) is encoded by the Solute Carrier Organic Anion Transporter Family Member (SLCO) gene. Currently, more than 300 OATP (SLCO) transporter superfamilies have been found in more than 40 species, of which at least 11 OATP (SLCO) transporter superfamilies are expressed in humans. OATPs are usually divided into families and subfamilies based on the boundaries of 40% and 60% amino acid sequence homology. Six families (OATP1-6) and 13 subfamilies (such as OATP1A, OATP1B, OATP1C, etc.) are formed in humans, monkeys, dogs, and rodents (Hagenbuch B, et al. Mol Aspects Med. 2013Apr-Jun; 34 (2-3):396-412). OATP is predicted to be a 12-transmembrane protein, a sodium ion-independent transporter, with a wide range of endogenous and exogenous transport substrates, mainly amphipathic organic anions.

SUMMARY OF THE INVENTION

The present invention provides a method and a medicament for preventing and/or treating hepatitis B virus (HBV) and/or hepatitis D virus (HDV) infection or diseases related to the infection in animals. The method includes administering to an animal in need of such prevention and/or treatment an effective amount of an agent that prevents or reduces the interaction between HBV and/or HDV and the organic anion transport polypeptide OATP in said animal, and /or can prevent or reduce the expression and/or function of OATP in said animal.

In one or more embodiments, the animal is preferably mammals and birds, further preferably humans, chimpanzees, bonobos, tree shrews, ducks, marmots, arctic ground squirrels, ground squirrels, bats, woolly monkeys, capuchin monkeys and squirrel monkeys. The animal is more preferably humans.

In one or more embodiments, said OATP include, but are not limited to, mammalian, bird, fish, and amphibian OATP, preferably mammalian and bird OATP, further preferably mammalian and bird OATP1B subfamily, including OATP1B3 and/or OATP1B1 and/or OATP1B2, more preferably mammalian OATP1B3 and/or or OATP1B1 and/or OATP1B2, and further preferably OATP1B3 and/or OATP1B1 and/or OATP1B2 derived from humans, chimpanzees, bonobos, tree shrews, marmots, arctic ground squirrels, ground squirrels, bats, woolly monkeys, capuchin monkeys and squirrel monkeys. Even further preferably, said OATP is human OATP1B3 and/or human OATP1B1.

In one or more embodiments, said agent that prevents or reduces the interaction between HBV and/or HDV and OATP can act on HBV and/or HDV, or on OATP, or on HBV and/or HDV and OATP; preferably, said agent acts on HBV and/or HDV, or on OATP; further preferably, said agent acts on human OATP; even further preferably, said agent acts on human OATP1B3 and/or human OATP1B1, that is, targets human OATP1B3 and/or human OATP1B1 for action.

In one or more embodiments, said agent that prevents or reduces the interaction between HBV and/or HDV and OATP is preferably polypeptides, antibodies, proteins, small molecules and polynucleosides that can interact with OATP acids and other agents. It is further preferred that the said agents are small molecules, proteins, antibodies or polypeptides that can interact with human OATP1B3 and/or human OATP1B1, more preferably are antibodies or polypeptides that can interact with human OATP1B3 and/or human OATP1B1, more preferably are polypeptides that can act with human OATP1B3 and/or human OATP1B1. Even further preferably, said agent is a polypeptide derived from the amino acid sequence of the HBVPre-S1 region with a fatty acid modification at its N-terminal. Even further preferably, said agent is a polypeptide derived from the amino acid sequence 13-59 of the Pre-S1 region of human HBV genotype C with a fatty acid modification at its N-terminal. More preferably, said agent is the polypeptide shown in SEQ ID NO: 67 and SEQ ID NO: 68, whose N-terminus is modified with myristic acid and whose C-terminus is amidated.

In one or more embodiments, said agent that can prevent or reduce the expression and/or function of OATP in the animal is preferably that can act on SLCO gene, OATP mRNA, OATP protein translation, expression, modification, transport or degrade. Further preferably, said agent is a reagent that acts on gene knockout, gene insertion, homologous recombination, gene editing and transcription inhibition of the SLCO gene, or is an antisense nucleic acid or RNA interference that acts on OATP mRNA, or is an agent that acts on OATP mRNA. OATP protein ubiquitination or protein degradation targeting chimera (PROTAC). Further preferably, the agent is a gene editing agent targeting the SLCO gene, antisense nucleic acids targeting OATP mRNAs or PROTAC agent for RNA interference and targeting OATP protein. Further preferably, said agents is polynucleotide and oligonucleotide, further preferably DNA or RNA sequence that enable gene editing, DNA or RNA sequence that enable RNA interference, DNA or RNA sequence that enable antisense nucleic acids, and more preferably small interfering RNA (siRNA) that enable RNA interference.

In one or more embodiments, said methods and agents for treating and/or preventing HBV and/or HDV infection

or diseases related to said infections can be used alone or in combination with agents that can prevent or reduce the interaction between HBV and/or HDV and NTCP and/or agents that can prevent or reduce NTCP expression and/or function.

The present invention also provides cells or animal models susceptible to HBV and/or HDV and the method of establishment thereof, wherein, transfect the exogenous SLCO gene into the cell or animal, and/or express the exogenous OATP protein in the cell or animal, and the animals, cells or animals that are originally not susceptible to HBV and/or HDV or are not easily infected, acquire susceptibility to HBV and/or HDV after being transfected with the SLCO gene and/or expressing exogenous OATP protein. The present invention also includes drugs and lead compounds obtained by screening using the above-mentioned HBV and/or HDV infected cells and/or animal models and the drugs can be used to treat and/or prevent HBV and/or HDV infection.

In one or more embodiments, said SLCO gene include, but are not limited to, SLCO gene from mammals, birds, fish, and amphibians, preferably mammalian and bird SLCO gene, and further preferably mammalian and bird SLCO1B subfamilies, including SLCO1B3 and/or SLCO1B1 and/or SLCO1B2. More preferably, said SLCO gene is mammalian SLCO1B3 and/or SLCO1B1 and/or SLCO1B2, and further preferably SLCO1B3 and/or SLCO1B1 and/or SLCO1B2 derived from humans, impanzees, bonobos, tree shrews, marmots, arctic ground squirrels, ground squirrels, bats, woolly monkeys, capuchin monkeys or squirrel monkeys. Even further preferably, said SLCO gene is human (for example, shown in SEQ ID NO: 3), chimpanzee, tree shrew SLCO1B3 and/or human, chimpanzee, tree shrew SLCO1B1. Still further preferably, the SLCO gene is human SLCO1B3 and/or human SLCO1B1 (for example, shown in SEQ ID NO: 3, 6).

In one or more embodiments, said OATP protein include, but are not limited to, OATP protein from mammals, birds, fish, and amphibians, preferably mammalian and bird OATP protein, and further preferably mammalian and bird OATP1B subfamilies, including OATP1B3 and/or OATP1B1 and/or OATP1B2. More preferably, said OATP protein is mammalian OATP1B3 and/or OATP1B1 and/or OATP1B2, and further preferably OATP1B3 and/or OATP1B1 and/or OATP1B2 derived from humans, chimpanzees, bonobos, tree shrews, marmots, arctic ground squirrels, ground squirrels, bats, woolly monkeys, capuchin monkeys or squirrel monkeys. More preferably, the OATP protein is human, chimpanzee or tree shrew OATP1B3, and/or human, chimpanzee or tree shrew OATP1B1. Still further preferably, the OATP protein is human OATP1B3 and/or human OATP1B1 (for example, shown in SEQ ID NO: 1, 4).

In one or more embodiments, in said cells or animal models susceptible to HBV and/or HDV and the method of establishment thereof, the SLCO gene can be transfected alone, or the exogenous SLC10A1 gene can be transfected at the same time; or the exogenous OATP protein can be expressed alone, or the exogenous NTCP protein can be expressed at the same time. Said SLC10A1 gene include, but are not limited to, mammalian, bird, fish and amphibian SLC10A1 gene, preferably mammalian and bird SLC10A1 gene, and further preferably derived from humans, chimpanzees, bonobos, tree shrews, marmots, arctic ground squirrels, ground squirrels, bats, woolly monkeys, capuchin monkeys, squirrel monkeys and ducks. Even further preferably, said SLC10A1 gene is human (for example, shown in SEQ ID NO: 79), chimpanzee, tree shrew SLC10A1. Still further preferably, said SLC10A1 gene is human SLC10A1 (for example, shown in SEQ ID NO: 79). Said NTCP protein include, but are not limited to, mammalian, bird, fish and amphibian NTCP protein, preferably mammalian and bird NTCP protein, and further preferably derived from humans, chimpanzees, bonobos, tree shrews, marmots, arctic ground squirrels, ground squirrels, bats, woolly monkeys, capuchin monkeys, squirrel monkeys and ducks. Even further preferably, said NTCP protein is human (for example, shown in SEQ ID NO: 7), chimpanzee, tree shrew NTCP protein. Still further preferably, said NTCP protein is human NTCP protein (for example, shown in SEQ ID NO: 7).

In one or more embodiments, said cells transfected with exogenous SLCO gene and/or expressing OATP protein include, but are not limited to, human liver cell lines, mice liver cell lines, rat liver cell lines, and primary liver cell lines or liver cell lines of other animal origins. Human liver cell lines include, but are not limited to, HepG2, Huh7, Hep RG, and other human liver-derived cell lines. mice-derived liver lines include, but are not limited to, Hepa1-6, Hep56.1D, and other mice-derived hepatocytes. rats-derived liver lines include, but are not limited to, BRL 3A, TC5123, and other rats-derived hepatocytes. Preferably, said cells are human liver cell lines HepG2, Huh7, Hep RG, mice liver cell lines Hepa1-6, Hep56.1D, or rats liver cell line TC5123. Further preferably, the cells are human liver cell lines HepG2 or Huh7.

In one or more embodiments, the animals transfected with exogenous SLCO gene and/or expressing OATP protein include, but are not limited to, non-human mammals, birds and other animals. Non-human mammals include, but are not limited to, mice, rats, dogs, monkeys, tree shrews, pigs, rabbits, bonobos, tree shrews and other mammals. Birds include, but are not limited to, ducks, herons, and other birds. Other animals include bats and other animals. Said animal are preferably mice, rats, dogs and monkeys. Further preferably, said animal are mice and rats.

The present invention also includes drugs and lead compounds for the treatment and/or prevention of HBV and/or HDV infections and related diseases obtained by screening said HBV and/or HDV susceptible cells and/or animal models, said drugs and lead compounds include, but are not limited to, small molecules, proteins, peptides, antibodies, poly RNA, poly DNA and other drugs. Said treatment and/or prevention of HBV and/or HDV infection and related diseases include but are not limited to preventing or inhibiting HBV and/or HDV infection, preventing or inhibiting HBV and/or HDV replication, and clearing HBV and/or HDV Infect, preventing or inhibiting HBV and/or HDV release, activating and/or modulating HBV-specific and/or HDV-specific immune responses.

The present invention also provides a method and vehicles for drug delivery by targeting OATP, which vehicles at least retains the biological activity of binding to OATP and delivering the carried drug into cells.

In one or more embodiments, said OATP protein include, but are not limited to, OATP protein from mammals, birds, fish, and amphibians, preferably mammalian and bird OATP protein, and further preferably mammalian and bird OATP1B subfamilies, including OATP1B3 and/or OATP1B1 and/or OATP1B2. More preferably, said OATP protein is mammalian OATP1B3 and/or OATP1B1 and/or OATP1B2, and further preferably OATP1B3 and/or OATP1B1 and/or OATP1B2 derived from humans, chimpanzees, bonobos, tree shrews, marmots, arctic ground squirrels, ground squirrels, bats, woolly monkeys, capuchin monkeys or squirrel monkeys. Still further preferably, the OATP protein is human OATP1B3 and/or human OATP1B1 (for example, shown in SEQ ID NO: 1, 4).

In one or more embodiments, said vehicle can bind OATP alone, or can bind OATP and NTCP simultaneously. Said NTCP protein include, but are not limited to, mammalian, bird, fish and amphibian NTCP protein, preferably mammalian and bird NTCP protein, and further preferably derived from humans, chimpanzees, bonobos, tree shrews, marmots, arctic ground squirrels, ground squirrels, bats, woolly monkeys, capuchin monkeys, squirrel monkeys and ducks. Even further preferably, said NTCP protein is a human, chimpanzee or tree shrew NTCP protein. Even further preferably, said NTCP protein is derived from humans.

In one or more embodiments, said backbone of the vehicle includes, but is not limited to, polypeptides, small molecule drugs, proteins, antibodies, nucleic acids, and other forms that can bind to OATP and deliver the carried drug or group into cells and/or organs. Preferably, said backbone can bind to OATP1B3 and/or OATP1B1 and can deliver the carried drug or group into hepatocytes and/or liver; further preferably, said backbone can bind to human OATP1B3 and/or human OATP1B1 and can deliver the carried drug or group into human hepatocytes and/or human liver.

In one or more embodiments, said drug or group contained in the vehicle includes, but is not limited to, small molecule drugs, nucleic acids, proteins, antibodies, polypeptides, or other drugs or groups, and said drug or group may be linked to the rest of said vehicle (the backbone) by covalent and/or non-covalent bonds. In said OATP-targeted delivery vehicles, the drug or group may be linked to the rest of the vehicle either directly or through a linker. Said small molecule drugs include, but are not limited to, fluorescent groups, toxins, chemotherapeutic agents, signal modulating molecules, cell signaling agonists, cell signaling inhibitors, and other small molecule drugs or groups. Said nucleic acids include, but are not limited to, DNA, RNA, and other nucleic acid molecules, preferably contained nucleic acids are protein coding DNA or RNA, antisense nucleic acids, nucleic acids used for RNA interference, nucleic acids used for gene editing, and viral vehicles carrying sequences of these nucleotides.

In one or more embodiments of the methods and vehicles, the drugs or groups are delivered for use in species including, but not limited to, mammals, birds, fish and amphibians, preferably mammals and birds, further preferred for use in humans, chimpanzees, bonobos, tree shrews, ducks, marmots, arctic ground squirrels, ground squirrels, bats, woolly monkeys, capuchin monkeys and squirrel monkeys, and still further preferred for use in humans.

In one or more embodiments, in said methods and vehicles, the systems into which the delivered drugs or groups enter include, but are not limited to, the digestive system, the respiratory system, the circulatory system, the nervous system, the motor system, the urinary system, and the reproductive system, endocrine system, and other systems; the organs and tissues into which the delivered drugs or groups enter include, but are not limited to, the liver, the kidneys, the lungs, the heart, the muscle, the fat, the brain nerves, intestines, pancreas, and other organs or tissues; the cells into which the drugs or groups are delivered include, but are not limited to, hepatocytes, lung cells, kidney cells, nerve cells, muscle cells, fat cells, blood cells, lymphocytes, and other cells. The delivered drugs or groups preferably enter the digestive system, the delivered drugs or groups preferably enter the liver, and the delivered drugs or groups preferably enter hepatocytes.

In some embodiments of the present invention, the backbone of said OATP-targeted delivery vector provided herein comprises, but is not limited to, an OATP-targeted delivery polypeptide derived from a hepadnaviridae surface antigen that retains at least the biological activity of binding to the OATP and delivering the drugs or groups hosted therein into the cell. The said OATP targeted delivery polypeptide derived from hepadnavirus surface antigen is preferably derived from an orthohepadnavirus or Avihepadnavirus virus surface antigen. The OATP targeted delivery polypeptide is further preferably derived from human hepatitis B virus (HBV), tree shrew HBV, woolly monkey hepatitis B virus (WMHBV), woodchuck hepatitis B virus (WHV), duck hepatitis B virus (DHBV), bat HBV and other hepatitis B virus surface antigens. More preferably, the polypeptide is derived from the human HBVPre-S1 region and retains at least the biological activity of binding to OATP and delivering the carried drugs or groups into cells.

In some embodiments of the invention, said OATP targeted delivery vehicle derived from hepadnavirus surface antigen is a polypeptide having the structure of the following formula (I):

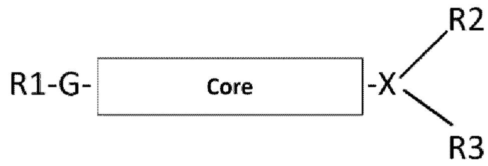


(I)

among them, G is glycine, the core sequence is the amino acid sequence derived from the surface antigen of hepadnavirus, X is an amino acid residue with two amino groups or two carboxyl groups or other groups available for linkage reaction, and R1 is a N-terminal modification of the vehicle, R2 does not exist, or is a flanking sequence or other group, R3 is a carried pharmacophore group or a linking group that can interact with a drug or group.

In one or more embodiments, the core sequence is preferably derived from orthohepadnavirus or Avihepadnavirus virus surface antigen, further preferably derived from human hepatitis B virus (HBV), tree shrew HBV, woolly monkey hepatitis B virus (WMHBV), prairie dog hepatitis B virus (WHV), duck B Hepatitis B virus (DHBV), bat HBV and other hepatitis B virus surface antigens. And their variants are further preferably derived from the human HBV surface antigen Pre-S1 region and its variants, further preferably derived from the surface amino acids 14-48 of the antigen Pre-S1 region of human HBV genotype C (the example sequence is shown in SEQ ID NO: 97) and its variants.

In one or more embodiments, R1 is preferably an N-terminal essential modifier group, which may be selected from hydrophobic or other groups, said hydrophobic group may be selected from, but is not limited to, myristic acid, palmitic acid, stearic acid, oleic acid, linoleic acid, cholesterol, polyethylene glycol, arachidonic acid, and other hydrophobic groups; further preferred are fatty acid groups, which may be selected from, but are not limited to, myristic acid, palmitic acid, stearic acid, and other fatty acids; further preferred are myristic acid, palmitic acid, stearic acid groups, and still preferred are myristic acid groups.

In one or more embodiments, R2 does not exist, or is a flanking sequence. R2 may or may not have a C-terminal modification. Further preferably, R2 is a flanking sequence derived from the Pre-S1 region of human HBV surface antigen, with a C-terminal stabilizing modification; further preferably, it is a flanking sequence derived from the surface antigen of Pre-S1 region of human HBV genotype C (the example sequence is shown in SEQ ID NO: 141) and its variants, with C-terminal amidation modification.

In one or more embodiments, R3 is a pharmacophore group, or a linking group that can interact with a drug or group. The pharmacophore group or linking group is directly or indirectly connected to the backbone of the vehicle through covalent bonds and/or non-covalent bonds or otherwise. There is or is not a linker between the pharmacophore or linking group and the backbone of the vehicle.

In one or more embodiments, the delivery vehicle shown in formula (I) contains pharmacophores, drugs or groups including, but not limited to, small molecule drugs, nucleic acids, proteins, antibodies, peptides, viruses or other drugs or groups. In some embodiments, R3 is selected from, but is not limited to, small molecule groups or linking groups that can interact with it, which can be, but is not limited to, fluorescent groups, toxins, chemotherapeutic drugs, signal regulatory molecules, cell signal agonists, cell signal Inhibitors and other small molecule drugs or groups, or linking groups that can interact with them. In some embodiments, R3 is preferably, but not limited to, fluorescent groups. In some embodiments, R3 is selected from, but not limited to, nucleic acids and can be, but is not limited to, DNA, RNA, and other nucleic acid molecules or linking groups that can interact with them for the purpose of transducing exogenous genes, protein coding, up-regulating, or down-regulating the expression of target genes; preferably, R3 is selected from but not limited to protein-coding DNA or RNA, antisense nucleic acids, nucleic acids for RNA interference, nucleic acids for gene editing, or a linking groups that can interact with it. In some embodiments of the invention, the nucleic acids contained in said vehicles also comprise viral vehicles carrying the sequences of these nucleic acids, or linker groups that can interact with them. In some embodiments of the present invention, R3 can be selected from, but is not limited to, small molecule pharmacophore groups with cell regulatory activity, active proteins, coding nucleic acids encoding active proteins, interference or antisense active protein mRNA, gene-coding nucleic acids and biologically active nucleic acids such as viral vehicles, or linking groups that can interact with the small molecule pharmacophore groups, active proteins, coding nucleic acids or biologically active nucleic acids.

In some preferred embodiments of the present invention, the core sequence of the OATP targeted delivery vehicle derived from hepadnavirus surface antigen represented by formula (I) is derived from amino acids 14-48 of surface antigen of Pre-S1 region of human HBV genotype C or a variant thereof ; R1 is preferably a cardamomate, palmitate, stearic acid, cholesterol, polyethylene glycol group; the flanking sequence of R2 is preferably derived from amino acids 50-59 of surface antigen of Pre-S1 region of human HBV genotype C with an amidation modification at the C-terminus; preferably, the amino acid sequence of the vehicle is as shown in any one of SEQ ID NO: 159-164. In some preferred embodiments of the present invention, the core sequence of the OATP targeted delivery vehicle derived from hepadnavirus surface antigen represented by formula (I) is derived from amino acids 14-56 of the Pre-S1 region of human HBV

genotype C surface antigen or a variant thereof ; R1 is preferably a cardamomate, palmitate, stearic acid, cholesterol, polyethylene glycol group; R2 flanking sequence is preferably derived from amino acids 58-59 of human HBV genotype C surface antigen with an amidation modification at the C-terminus; preferably, the amino acid sequence of the vehicle is as shown in any one of SEQ ID NO: 165-164. In some preferred embodiments of the present invention, the core sequence of the OATP targeted delivery vehicle derived from hepadnavirus surface antigen represented by formula (I) is derived from amino acids 14-48 of the human HBV genotype C surface antigen Pre-S1 region or a variant thereof ; R1 is preferably a cardamomate, palmitate, stearic acid, cholesterol, polyethylene glycol group; R2 flanking sequence is preferably derived from amino acids 50-59 of human HBV genotype C surface antigen with an amidation modification at the C-terminus; preferably, the amino acid sequence of the vehicle is as shown in any one of SEQ ID NO: 159-164. In one embodiment of the present invention, the core sequence of said OATP-targeted delivery peptide vector derived from hepatophilic DNA viral surface antigen shown in formula (I) is selected from the amino acid sequence derived from amino acids 14-48 in the surface antigen of Pre-S1 region of human HBV genotype C; R1 is a cardamom acid group; R2 flanking sequence selected from amino acids 50-59 of surface antigen of Pre-S1 region of human HBV genotype C with amidation modification at the C-terminus; R3 is a FITC fluorescent example motif.

The present invention also provides a hepadnavirus surface antigen-derived peptide that binds to OATP and inhibits its transport function and the peptide inhibiting the transport function of OATP by binding OATP. In one or more embodiments, said OATP protein include, but are not limited to, OATP protein from mammals, birds, fish, and amphibians, preferably mammalian and bird OATP protein, and further preferably mammalian and bird OATP1B subfamilies, including OATP1B3 and/or OATP1B1 and/or OATP1B2. More preferably, said OATP protein is mammalian OATP1B3 and/or OATP1B1 and/or OATP1B2, and further preferably OATP1B3 and/or OATP1B1 and/or OATP1B2 derived from humans, chimpanzees, bonobos, tree shrews, marmots, arctic ground squirrels, ground squirrels, bats, woolly monkeys, capuchin monkeys or squirrel monkeys. Still further preferably, said OATP is human OATP1B3 and/or human OATP1B1.

Preferably, the amino acid sequence of the polypeptide is derived from a hepadnavirus surface antigen. Preferably, the amino acid sequence of the polypeptide is derived from HBV virus surface antigens. These HBVs include but are not limited to human hepatitis B virus (HBV), tree shrew HBV, woolly monkey hepatitis B virus (WMHBV), woodchuck hepatitis B virus (WHV), Duck hepatitis B virus (DHBV), bat HBV and other hepatitis B viruses. More preferably, the amino acid sequence of the polypeptide is derived from human hepatitis B virus (HBV) surface antigen (HBsAg). The human hepatitis B virus (HBV) includes, but is not limited to, HBV of genotypes A, B, C, D, E, F, G, H, and I. Further preferably, the amino acid sequence of said polypeptide is derived from the human hepatitis B virus (HBV) surface antigen (HBsAg) Pre-S1 region, further preferably from amino acids 2-77 (D genotype) or amino acids 13-88 (C genotype) of the human hepatitis B virus (HBV) surface antigen (HBsAg) Pre-S1 region, more preferably from the amino acids 13-59 (C genotype) of the HBsAg Pre-S1 region. Preferably, said polypeptide with a fatty acid or other modification at the N-terminus, further preferably with a myristic acid modification at the N-terminus. More preferably, the amino acid sequence of the polypeptide is as shown in SEQ ID NO: 67 or 68, and the N-terminus of the polypeptide is modified with myristic acid.

Preferably, the transport function of the OATP includes but is not limited to the transport of statins drugs, sartans drugs, tinibs drugs, anti-tumor drugs such as platinum drugs, paclitaxel drugs and other drugs.

DESCRIPTION OF DRAWINGS

Figure 1: map of recombinant lentivirus WL1142. hHEPALK3 in the figure represents hOATP1B3.
Figure 2: map of recombinant lentivirus WL1143. hGBIO in the figure represents hNTCP.
Figure 3: structure of L47-FITC (A) HPLC identification diagram of L47-FITC (B) and mass spectrometry identification diagram of L47-FITC (C).
Figure 4: structure of L47 (A) and mass spectrometry identification diagram of L47 (B).
Figure 5: signal fluorescence intensity obtained from PBS-incubated Hela cells as a negative control for FITC and PI staining and from paraformaldehyde-fixed Hela cells incubated with L47-FITC and PI, respectively, as a positive control for FITC and PI staining.
Figure 6: flow cytometry results of blank BLANK group.
Figure 7: flow cytometry results of single-staining group.
Figure 8: flow cytometry results of double-staining group.
Figure 9: comparison of FITC signaling in blank PBS-stained Hela cells, L47-FITC/PI double-stained Hela, Hela-hNTCP, Hela-hOATP1 B3, and Hela-hNTCP/ hOATP1B3 cells.
Figure 10: fluorescence intensity of blank PBS-stained Hela cells, L47-FITC/PI double-stained Hela, Hela-hNTCP, Hela-hOATP1B3, and Hela-hNTCP/ hOATP1B3 cells.
Figure 11: fluorescence signals after administration of PBS or L47-FITC to different cells under irradiation of Leica LED8 cyan 475±14nm wavelength excitation light.

Figure 12: inhibitory effect of L47 on E217βG uptake activity by OATP1B1.
Figure 13: inhibitory effect of L47 on E217βG uptake activity by OATP1B3.
Figure 14: inhibitory effect of Hepalatide on Valsartan uptake activity by OATP1B1.
Figure 15: inhibitory effect of Hepalatide on Valsartan uptake activity by OATP1B3.
Figure 16: inhibitory effect of Hepalatide on Atorvastatin uptake activity by OATP1B1.
Figure 17: Map of recombinant lentivirus WL1281. hHEPA LK1 and WL1281 in the figure both represent hOATP1B1.
Figure 18: culture supernatant HBsAg and HBeAg concentrations on day 9 (day9) post-infection of Example 6 Test 1.
Figure 19: culture supernatant HBsAg and HBeAg concentrations on day 9 (day9) post-infection of Example 6 Test 2.

## DETAILED DESCRIPTION

The present invention found that both human NTCP and human OATP1B3 can bind to polypeptides derived from HBVPre-S1, but the binding of hNTCP to the HBVPre-S1 derived polypeptides is limited to the cell membrane and does not mediate the entry of the polypeptide into cells. While, the binding of HBVPre-S1 derived polypeptide to OATP1B3 can mediate the entry of the polypeptide into cells. Furthermore, co-expression of OATP1B3 and NTCP can enhance the binding of HBVPre-S1-derived polypeptide to cells. It is well known in the art that the HBVPre-S1 region is a key region in mediating HBV infection, and this region binds to the HBV infection functional receptor NTCP (Yan H et al. Elife. 2012;1:e00049.; Ni Y et al. Gastroenterology. 2014;146(4):1070-83.); OATP1B3 is highly homologous to OATP1B1(König J, et al. J Biol Chem. 2000; 275(30):23161-8.), both of which can form a heterodimer with NTCP (Zhang Y, et al. PLoS One. 2017;12(6):e0180257.). Combining the findings of the present invention and what is known in the field, it can be reasonably inferred that: 1) human OATP1B3 is an HBV infection receptor, and HBV can bind to human OATP1B3 or human OATP1B1 through its Pre-S1 region to mediate HBV entry into cells; 2) human OATP1B3 or human OATP1B1 forms co-receptors for HBV infection with NTCP. Given that hepadnavirus infection and homologous OATP molecules also exist in other animals, this infection mechanism can be extended to hepadnavirus infection of other animals.

Therefore, this present invention proposes:

1) Infections and related diseases caused by HBV and/or HDV can be treated and/or prevented by preventing or reducing the interaction of HBV and/or HDV with OATP, or by preventing or reducing OATP expression and/or function;
2) For cells or animals that are not susceptible to infection by HBV or HDV, transfect SLCO exogenous genes, or transfect SLCO and SLC10A1 exogenous genes to express exogenous OATP proteins, or jointly express OATP and NTCP proteins to obtain HBV and/or HDV-susceptible cells and animal models which can be screened to obtain drugs or lead compounds for the treatment and/or prevention of HBV and/or HDV infections;
3) Drugs, exogenous genes, and expression regulatory substances can be delivered into cells by targeting OATP for the prevention and/or treatment of related diseases;
4) The HBVPre-S1 derived peptide binds to OATP to inhibit the OATP transport function and regulate the pharmacokinetics of drugs that use OATP as a transporter to improve the efficacy and safety of these drugs.

### Terms and Definitions

It should be understood that within the scope of the present description, the above-mentioned technical features of the present description and the technical features specifically described in the following (such as the examples) can be combined with each other to form a preferred technical solution. Unless defined otherwise, all technical and scientific terms used herein have the meaning commonly understood by one of ordinary skill in the art to which this invention belongs. For the purposes of this disclosure, the following terms are as defined below. The article "a" refers to one or more than one (i.e. at least one) grammatical object of the article. For example, "an element" refers to one element or more than one element. Unless the context clearly indicates otherwise, the term "or" is used interchangeably with the term "and/or". To the extent that the terms "contains," "includes," "has," or grammatical variants thereof are used in this disclosure or in the claims, these terms may be used in a similar manner as explained when the term "contains" is used as a transition word in the claims. The term "includes" or its grammatical variations means "including, but not limited to" and may be used interchangeably with "including, but not limited to". The term "about" means a certain amount, level, value, value, frequency, percentage, size, size, quantity, weight or length that is compared to a reference amount, level, value, value, frequency, percentage, size, size, quantity, weight or length with almost 30%, 25%, 20%, 15%, 10%, 9%, 8%, 7%, 6%, 5%, 4%, 3%, 2%, or 1% variation. When the term "about" is used with a numerical range, the term modifies the range by extending its limits above or below the numerical value. Generally, the term "about" modifying a numerical value means within 10% above and within 10% below that numerical value.

As used herein, the terms "polypeptide," "peptide," and "protein" are used interchangeably and include full-length proteins and fragments thereof, as well as variants of full-length proteins and fragments thereof.

As used herein, a "variant" in connection with a polypeptide described herein refers to a polypeptide that differs in

amino acid sequence from a given polypeptide but retains one or more biological activities of the given polypeptide as described herein. Biological functions described herein include interacting with OATP, binding to OATP, delivering drugs into cells, and inhibiting OATP transport function. Polypeptide variants described herein may have one or more amino acid additions (e.g., insertions), deletions, or substitutions relative to a given polypeptide. In some embodiments, polypeptide variants described herein may have 1-30, 1-20, 1-10, 1-8, 1-5, or 1-3 (including within these ranges) amino acid additions (such as insertions), deletions or substitutions relative to a given polypeptide. For example, the amino acid sequence of a variant may contain conservative substitutions of amino acids. Conservative substitution of amino acids, that is, replacing one amino acid with a different amino acid with similar properties (such as hydrophilicity and degree of charge and distribution of charged regions).

The terms "conservative amino acid substitution" and "conservative substitution" are used interchangeably herein to refer to a specified amino acid exchange within a set of amino acid ranges in which one amino acid is exchanged with a different amino acid of similar size, structure, charge and/or polarity exchange. Since conservative substitutions usually involve relatively small changes, they do not significantly alter the biological activity of the peptide. These small changes can be identified in part by considering the hydropathic index of an amino acid based on its hydrophobicity and charge. Amino acids with similar hydrophilic index and hydrophilicity values can be substituted for each other and still retain protein functions. The hydropathic index and hydrophilicity value of an amino acid are affected by the specific side chains of that amino acid. Consistent with this observation, amino acid substitutions that match biological function depend on the relative similarity of the amino acids, particularly those of their side chains, in terms of hydrophobicity, hydrophilicity, chargeability, size, and other properties.

Families of amino acid residues with similar side chains are well known in the art, including a basic side chain (e.g., lysine, arginine, histidine), an acidic side chain (e.g., aspartic acid, glutamic acid), a uncharged polar side chain (e.g., glycine, asparagine, glutamine, serine, threonine, tyrosine, cysteine, tryptophan), a non-polar side chains (e.g., alanine, valine, leucine, isoleucine, proline, phenylalanine, methionine), a β-branched side chain (e.g., threonine, valine, isoleucine) and an aromatic side chain (e.g., tyrosine, phenylalanine, tryptophan, histidine). Thus, in some embodiments, the amino acid residues in the polypeptide can be used Substitution of another amino acid residue from the same side chain family. In other embodiments, a stretch of amino acid sequence may be replaced by a stretch of amino acid sequence that is structurally similar but differs in the order and/or composition of the side chain family members. In other embodiments, mutations may be introduced randomly throughout the entire sequence or part of the polypeptide sequence. Examples of conservative amino acids substitutions include substitutions of one of the aliphatic or hydrophobic amino acids Ala, Val, Leu and Ile for another amino acid in the group of four; substitutions between the hydroxyl-containing residues Ser and Thr; substitutions between acidic residues Asp and Glu; substitutions between the amide residues Asn and Gln; substitutions between basic residues Lys, Arg and His; substitutions between the aromatic residues Phe, Tyr and Trp; and substitutions between the small amino acids Ala, Ser, Thr, Met and Gly. It is reasonably foreseeable that conservative substitutions, such as replacing a conservative amino acid with a similar, structurally related amino acid, will not have a substantial impact on the biological activity of the polypeptide.

"Variant" polypeptides also include polypeptides that share some identity with a given polypeptide, such as polypeptides that are at least about 30%, 40%, 50%, 60%, 70%, 80%, 85%, 90%, 95%, 96%, 97%, 98% or 99% identical to a given polypeptide. As used herein, "variants" of a polypeptide also include polypeptides that contain a portion of the given polypeptide corresponding to the native sequence of the protein. "Variant" may also refer to fusion or chimeric proteins, containing polypeptides derived from proteins from two or more different sources. Non-limiting examples of fusion proteins described herein may include, for example, fusion proteins of one polypeptide derived from a hepadnavirus and another polypeptide derived from a non-hepadnavirus protein, fusion protein of two polypeptides derived from different hepadnavirus genotypes, and fusion protein of two polypeptides derived from different regions of the surface antigen of any hepadnavirus genotype or two polypeptides derived from different sequences within the surface antigenic region of any hepadnavirus genotype.

The term "sequence identity" (e.g., 50% identical to a sequence) refers to the degree to which sequences are identical when compared on an amino acid-for-amino-acid basis within a comparison window. In some embodiments, a polypeptide described herein may contain an amino acid sequence that is at least about 30%, 40%, 50%, 60%, 70%, 80%, 85%, 90%, 95%, 96%, 97%, 98%, or 99% identical to the sequence of a given polypeptide and still retains one or more biological activities of the given polypeptide. By optimally arranging two sequences in the comparison window, determine the number of positions where the same amino acid appears in the two sequences to obtain the number of matching positions, and divide the number of matching positions by the total number of positions in the comparison window, and multiply the result by 100 to get the percent sequence identity and calculate "percent identity" or "% identity". The optimal sequence alignment for alignment in a comparison window may be performed by employing a computer to execute an algorithm known in the art, such as a program of the BLAST® family, or by visual observation, employing either method selected to produce the best alignment (i.e., to produce the highest percentage of homology in the comparison window). For sequence comparison, one sequence is used as a reference sequence and test sequences are compared to this reference sequence. When using a sequence comparison algorithm, test sequences and reference sequences are

entered into the computer, and if it's necessary, subsequence coordinates are designed and sequence algorithm program parameters are set. The sequence comparison algorithm then calculates the current sequence identity of the test sequence relative to the reference sequence based on the set program parameters. The setting of the parameters of the sequence algorithm program is well known in the art. For example, the comparison window can be set to cover the entire length of either or both comparison sequences, such as the entire length of the reference sequence, while allowing for differences of up to 5% of the total amino acid number of the reference sequence.

The term "variant" also includes proteins that contain the same amino acid sequence as a given polypeptide, retain one or more biological activities of the given polypeptide, but is chemically and/or post-translationally modified in a manner different from the given polypeptide. "Variant" may also be used to describe a polypeptide that has been differentially processed, such as proteolytically, phosphorylated, or otherwise post-translationally modified, but which retains one or more biological activities of a given polypeptide. "Variant" may include natural and/or non-natural amino acid residues. These terms also include post-translationally modified polypeptides, including, for example, glycosylated, sialylated, acetylated, and/or phosphorylated proteins. The term "variant" also includes polypeptides that have been chemically modified at one or more amino acid residues.

As used herein, unless otherwise stated, a "variant" of a polypeptide includes fragments of the variant.

The term "fragment" refers to a polypeptide consisting only of a contiguous portion of the complete full-length polypeptide sequence and structure. The fragment may comprise a C-terminal deletion and/or an N-terminal deletion of that natural polypeptide. The fragments often share some functionality of the full-length sequence. Depending on the full-length sequence, the length of the fragment with the desired function may vary, for example, generally at least 10 consecutive amino acid residues in length.

The term "coding sequence" or a sequence "encoding" a selected polypeptide means that a nucleic acid molecule is transcribed (for DNA) and translated (for mRNA) into a polypeptide in vitro or in vivo when under the control of appropriate regulatory sequences. The boundaries of the coding sequence can be determined by a start codon at the 5' (amino) terminus and a translation stop codon at the 3' (carboxy) terminus. The transcription termination sequence may be located 3' to the coding sequence.

The term "variant" as used herein with reference to a gene or coding sequence includes, but is not limited to, changes in the sequence resulting from base changes in the DNA or RNA encoding nucleic acid sequence, such as base substitutions, insertions, deletions or duplications. Herein, the terms "nucleic acid," "nucleotide," and "base" are used interchangeably. As used herein, a "variant" related to a coding sequence described herein refers to one that differs in base sequence from a given code but retains one or more biological activities of the expressed protein, including the entire coding sequence and fragments thereof, and variants of the full-length coding sequence and fragments thereof. As used herein, unless otherwise stated, a "variant" of a gene or coding sequence includes fragments of the variant. Coding sequences variants described herein may have one or more amino acid additions (e.g., insertions), deletions, or substitutions relative to a given polypeptide. In some embodiments, coding sequence variants described herein may have 1-100, 1-20, 1-10, 1-8, 1-5, or 1-3 (including within these ranges) amino acid additions (such as insertions), deletions or substitutions relative to a given coding sequence. For example, the coding sequence may contain synonymous mutations of bases, and due to the degeneracy of biological codons, after a certain base in the coding sequence is changed, the position of the original amino acid is translated into the same amino acid. Base substitution does not cause amino acid changes, also known as neutral mutation or silent mutation. For example, UCU, UCC, UCA, UCG, AGU, and AGC can all encode serine (Ser), and the codes of these serines can be replaced with each other. For example, codons encoding amino acids are replaced with codons encoding different amino acids with similar properties (such as hydrophilicity, degree of charge, and distribution of charged regions) to achieve conservative substitutions of amino acids in the expressed protein, and does not significantly change the biological activity of the expressed protein. Families of amino acid residues with similar side chains are well known in the art, including a basic side chain (e.g., lysine, arginine, histidine), an acidic side chain (e.g., aspartic acid, glutamic acid), a uncharged polar side chain (e.g., glycine, asparagine, glutamine, serine, threonine, tyrosine, cysteine, tryptophan), a non-polar side chains (e.g., alanine, valine, leucine, isoleucine, proline, phenylalanine, methionine), a β-branched side chain (e.g., threonine, valine, isoleucine) and an aromatic side chain (e.g., tyrosine, phenylalanine, tryptophan, histidine). Thus, in some embodiments, a codon in a coding series encoding an amino acid in a protein may be replaced with a codon encoding another amino acid of the same side chain family. For example, the codons UCU, UCC, UCA, UCG, AGU, and AGC encoding serine in the coding sequence can be replaced by the codons ACU, ACC, ACA, and ACG encoding threonine. Examples of conservative amino acid codons substitutions include substitutions of one of the aliphatic or hydrophobic amino acids codons Ala, Val, Leu and Ile for another amino acid codon in the group of four; substitutions between the hydroxyl-containing residues Ser and Thr codons; substitutions between acidic residues Asp and Glu codons; substitutions between the amide residues Asn and Gln codons; substitutions between basic residues Lys, Arg and His codons; substitutions between the aromatic residues Phe, Tyr and Trp codons; and substitutions between the small amino acids Ala, Ser, Thr, Met and Gly codons. It is reasonably foreseeable that conservative amino acid codon substitution, such as replacing a conserved amino acid codon with a codon for a similar, structurally related amino acid, will not have a substantial impact on the biological activity of the protein

expressed in the coding sequence.

The term "variant" also encompasses deletions or insertions of one or more bases, in particular deletions or insertions of a multiple of three bases, in a given coding sequence. For example, the deletion or insertion of 3, 6, 9, 15, 30, 60, 90, and 300 bases will not cause changes in a series of downstream codons, changes in the reading frame, or frame shift mutations.

The term "variant" also includes various chemical modifications on DNA, RNA bases and ribose of a given coding sequence and variants thereof. This includes but not limited to phosphorylation modification, methylation modification, PEG modification, histone modification, etc. It also contains couplings of a given coding sequence and variants thereof with proteins, peptides, and other chemical molecules. It also includes capping a given coding sequence and its variants, such as m7GTP capping; or tailing a given coding sequence and its variants, such as adding a poly-A tail.

The term "variant" also includes a variant that has some identity to a given coding sequence, such as at least about 30%, 40%, 50%, 60%, 70%, 80%, 85%, 90%, 95%, 96%, 97%, 98% or 99% identity to a given coding sequence. The term "sequence identity" (e.g., 50% identical to a sequence) refers to the degree to which sequences are identical when compared on a base-for-base basis within a comparison window. In some embodiments, a coding sequence described herein may contain a base sequence that is at least about 30%, 40%, 50%, 60%, 70%, 80%, 85%, 90%, 95%, 96%, 97%, 98%, or 99% identical to the base sequence of a given coding sequence and still retains one or more biological activities of the proteins expressed by given coding sequences. By optimally arranging two sequences in the comparison window, determine the number of positions where the same base appears in the two sequences to obtain the number of matching positions, and divide the number of matching positions by the total number of positions in the comparison window, and multiply the result by 100 to get the percent sequence identity and calculate "percent identity" or "% identity". The optimal sequence alignment for alignment in a comparison window may be performed by employing a computer to execute an algorithm known in the art, such as a program of the BLAST® family, or by visual observation, employing either method selected to produce the best alignment (i.e., to produce the highest percentage of homology in the comparison window). For sequence comparison, one sequence is used as a reference sequence and test sequences are compared to this reference sequence. When using a sequence comparison algorithm, test sequences and reference sequences are entered into the computer, and if it's necessary, subsequence coordinates are designed and sequence algorithm program parameters are set. The sequence comparison algorithm then calculates the current sequence identity of the test sequence relative to the reference sequence based on the set program parameters. The setting of the parameters of the sequence algorithm program is well known in the art. For example, the comparison window can be set to cover the entire length of either or both comparison sequences, such as the entire length of the reference sequence, while allowing for differences of up to 5% of the total base number of the reference sequence.

The term "transformation" as used herein refers to the insertion of an exogenous polynucleotide into a host cell, regardless of the method of insertion: e.g., transformation by direct uptake, transfection, infection, etc. Specific transfection methods are described further below. The exogenous polynucleotide can be maintained as an unintegrated vehicle, such as an episome, or can be integrated into the host genome.

The term "host cell" is a cell that has been transformed, or is capable of being transformed by a foreign DNA sequence.

Methods and agents for treating or preventing HBV and/or HDV infection and related diseases

The present invention provides a method and agent for preventing and/or treating animal hepadnavirus and/or HDV infections or related diseases by preventing or reducing the interaction between hepadnavirus and/or HDV and OATP, and/or preventing or reducing the expression and/or function of OATP. In another aspect, the present invention provides applications of substances capable of preventing or reducing the interaction between hepadnavirus and/or HDV and OATP and/or preventing or reducing the expression and/or function of OATP in the manufacture of medicaments for the prevention and/or treatment of hepatophilic DNA viral and/or HDV infections in animals, or of related diseases. In another aspect, the present invention provides substances for use in the prevention and/or treatment of hepadnavirus and/or HDV infections in animals or related diseases by preventing or reducing the interaction between hepadnavirus and/or HDV and OATP and/or preventing or reducing the expression and/or function of OATP.

As used herein, the animals include, but are not limited to, mammals, birds, fishes, amphibians and other animals.

The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid animals. The New World monkeys include, but are not limited to, Woolly monkeys, capuchin monkeys, Squirrel monkeys and other New World monkeys. The old-world monkeys include, but are not limited to, Macaca fascicularis and other old-world monkeys. The rodents include, but are not limited to, animals of the family Sciuridae,

myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. Animals described herein include in particular humans, primates, tree shrews, rodents, birds and bats, especially humans, chimpanzees, bonobos, tree shrews, ducks, marmots, arctic ground squirrels, ground squirrels, bats, woolly monkeys, capuchin monkeys and squirrel monkeys. In particularly preferred embodiments, the animal described herein is a human.

The hepadnaviruses include, but are not limited to, Orthohepadnavirus (OHV), Avihepadnavirus, Metahepadnavirus, Herpetohepadnavirus and other hepadnaviruses. The OHV includes but is not limited to the orthohepadnavirus infecting humans (HBV), woolly monkeys (Woolly monkey HBV, WMHBV), capuchin monkeys (Capuchin monkey HBV, CMHBV), squirrel monkey, woodchuck (Woodchuck hepatitis virus, WHV), arctic ground squirrel, ground squirrel (ground squirrel hepatitis virus, GSHV), tree shrew, chimpanzee, bat (Bat hepatitis virus, BHV) and other animals, and example genome sequences can be found in GenBank accession numbers including but not limited to AF046996 (WMHBV), NC_043528 (CMHBV), NC_004107 (WHV), NC_001484 (GSHV), JX941468 (BHV). The Avihepadnavirus includes, but is not limited to, the Avihepadnavirus infecting ducks (duck HBV, DHBV), herons (heron HBV, HHBV) and other birds, and example genome sequences can be found in, but are not limited to, GenBank accession numbers NC_001344 (DHBV), NC_001486 (HHBV).

Orthohepadnavirus that infect humans (HBV) described herein include, but are not limited to, HBV genotype A, B, C, D, E, F, G, H, and I, and example genome sequences can be found in, but are not limited to, GenBank accession numbers KC875260 (genotype A), AY220704 (genotype B), AF461363 (genotype C), AY 796030 (genotype D), AB205129 (genotype E), DQ823095 (genotype F), HE981176 (genotype G) and AB179747 (genotype H).

The HDV described herein includes, but is not limited to, HDV genotype 1, 2, 3, 4, 5, 6, 7 and 8, and example genome sequences can be found in, but are not limited to, GenBank accession numbers MG711803 (genotype 1), MK234593 (genotype 2), LT604954 (genotype 3), MK248835 (genotype 4), JX888107 (genotype 5), MG711780 (genotype 6), MG711804 (genotype 7) and LT604973 (Genotype 8).

As described herein, the OATP include, but are not limited to, superfamily derived from mammals, birds, fishes, amphibians, and other animals. In some embodiments, the OATP superfamily includes, but is not limited to, OATP1, OATP2, OATP3, OATP4, OATP5, OATP6, OATP7, OATP8, OATP9, OATP10, OATP11 and other OATP families. In some embodiments, the OATP family includes, but is not limited to, the OATP family expressed in hepatocytes. In some embodiments, the OATP includes, but is not limited to, the OATP1B subfamily. In some preferred embodiments, the OATP includes, but is not limited to, the OATP1B subfamily expressed in hepatocytes. In some preferred embodiments, the OATPs include, but are not limited to, OATP1B3, OATP1B1, OATP1B2, and other OATP1B subfamily members.

The OATP1B3 protein and SLCO1B3 gene described herein can be derived from mammals, birds, fishes, amphibians and other animals. The mammals include, but are not limited to, primates, tree shrews (tupaia, tree threw), rodents, bats, Artiodactyla, Carnivora, and other infectious species mammals which can be infected with hepadnavirus or can be infected with hepadnavirus after hepatocytes are transfected with exogenous SLC10A1 gene or express exogenous NTCP protein. The birds include, but are not limited to, ducks, herons and other birds that can be infected with Avihepadnavirus or can be infected with hepadnavirus after liver cells are transfected with exogenous SLC10A1 gene or express exogenous NTCP protein. The primates include, but are not limited to, hominoid species, New World monkeys, Old World monkeys, and other primates that can be infected with hepadnaviruses or can be infected with hepadnavirus after hepatocytes are transfected with exogenous SLC10A1 gene or express exogenous NTCP protein. The hominoid species includes, but is not limited to, humans, chimpanzees, gorillas, gibbons, orangutans, and other hominoid species that can be infected with hepadnaviruses or can be infected with hepadnavirus after hepatocytes are transfected with exogenous SLC10A1 gene or express exogenous NTCP protein. The New World monkeys include, but are not limited to, Woolly monkeys, capuchin monkeys, Squirrel monkeys and other New World monkeys that can be infected with hepadnaviruses or can be infected with hepadnavirus after hepatocytes are transfected with exogenous SLC10A1 gene or express exogenous NTCP protein. The old-World monkeys include, but are not limited to, Macaca fascicularis, and other old-World monkeys that can be infected with hepadnaviruses or can be infected with hepadnavirus after hepatocytes are transfected with exogenous SLC10A1 gene or express exogenous NTCP protein. The rodents include, but are not limited to, animals of the Sciuridae family, myomorpha, and other rodents that can be infected with hepadnaviruses or can be infected with hepadnavirus after hepatocytes are transfected with exogenous SLC10A1 gene or express exogenous NTCP protein. The Sciuridae animals include, but are not limited to, arctic ground squirrel, ground squirrel, woodchuck and other Sciuridae animals that can be infected with hepadnaviruses or can be infected with hepadnavirus after hepatocytes are transfected with exogenous SLC10A1 gene or express exogenous NTCP protein. The myomorpha animals include, but are not limited to, rats, mice and other myomorpha animals that can be infected with hepadnaviruses or can be infected with hepadnavirus after hepatocytes are transfected with exogenous SLC10A1 gene or express exogenous NTCP protein. The artiodactyla animals include but are not limited to pigs and other artiodactyla animals that can be infected with hepadnaviruses or can be infected with hepadnavirus after hepatocytes are transfected with exogenous SLC10A1 gene or

express exogenous NTCP protein. The Carnivora animals include but are not limited to dogs and other Carnivora animals that can be infected with hepadnaviruses or can be infected with hepadnavirus after hepatocytes are transfected with exogenous SLC10A1 gene or express exogenous NTCP protein.

The OATP1B3 amino acid sequence described herein includes, but is not limited to, the OATP1B3 amino acid sequence derived from humans (SEQ ID NO: 1, NCBI Reference Sequence: NM_019844), the OATP1B3 amino acid sequence derived from chimpanzees (NCBI Reference Sequence: XM_016923821), the OATP1B3 amino acid sequence derived from tree shrews (NCBI Reference Sequence: XM_006155085), the OATP1B3 amino acid sequence derived from Macaca fascicularis (NCBI Reference Sequence: NM_001283191), the OATP1B3 amino acid sequence derived from arctic ground squirrel (NCBI Reference Sequence: XM_026391932), the OATP1B3 amino acid sequence derived from rats (NCBI Reference Sequence: XM_032905480), the OATP1B3 amino acid sequence derived from pigs (NCBI Reference Sequence: NM_001315607), the OATP1B3 amino acid sequence derived from dogs (NCBI Reference Sequence: NM_001166047), and the OATP1B3 amino acid sequences derived from the other animals mentioned above.

The OATP1B3 coding sequence (CDS) described herein includes, but is not limited to, the OATP1B3 coding sequence derived from humans (SEQ ID NO: 2, NCBI Reference Sequence: NM_019844), the OATP1B3 coding sequence derived from chimpanzees (NCBI Reference Sequence: XM_016923821), the OATP1B3 coding sequence derived from tree shrews (NCBI Reference Sequence: XM_006155085), the OATP1B3 coding sequence derived from Macaca fascicularis (NCBI Reference Sequence: NM_001283191), the OATP1B3 coding sequence derived from arctic ground squirrels (NCBI Reference Sequence: XM_026391932), the OATP1B3 coding sequence derived from rats (NCBI Reference Sequence: XM_032905480), the OATP1B3 coding sequence derived from pigs (NCBI Reference Sequence: NM_001315607), the OATP1B3 coding sequence derived from dogs (NCBI Reference Sequence: NM_001166047), and the OATP1B3 coding sequence derived from the other animals mentioned above.

The SLCO1B3 gene sequences described herein include, but are not limited to, the SLCO1B3 gene sequence derived from humans (SEQ ID NO:3, NCBI Reference Sequence: NM_019844), the SLCO1B3 gene sequence derived from chimpanzees (genebank Gene ID: 749254), the SLCO1B3 gene sequence derived from tree shrews (genebank Gene ID: 102476084), the SLCO1B3 gene sequence derived from Macaca fascicularis (genebank Gene ID: 102136593), the SLCO1B3 gene sequence derived from arctic ground squirrels (genebank Gene ID: 113185010), the SLCO1B3 gene sequence derived from rats (genebank Gene ID:116903127), the SLCO1B3 gene sequence derived from pigs (genebank Gene ID:100620829), the SLCO1B3 gene sequence derived from dogs (genebank Gene ID:403519). And the SLCO1B3 gene sequences derived from the other animals mentioned above, including but not limited to gene bank NC_050564, NC_013676, NW_017869963, NW_006501111, NW_022196903, NW_003613944, NW_004801640, NW_004949097, NW_015351262, NW_024404945, NW_020539983, NW_023144717, NW_006711288, NW_019154091, NW_004569192, NC_044309, NW_020339853, NC_018729, NW_017619867, NW_023365217, NC_051831, NW_020656135, NW_022631003, NC_045619, NW_004451484, NC_043709, NW_020356453, NC_048233, NW_020312893, NW_020998839, NW_006383153, NW_023270504, NC_032654, NC_010447, NW_006781351, NW_023601653, NW_011889225, NC_009149, NW_007675154, NW_023458025, NW_006159939, NW_023666069, NW_012009027, NC_022282, NC_041764, NW_012010756, NC_044984, NC_037679, NW_012109719, NW_022681456, NW_016809129, NC_044558, NC_045443, NC_044614, NC_048251, NC_036891, NC_000012, NC_036915, NC_044403, NW_022611650, NW_024100944, NC_033666, NW_006408616 and other SLCO1B3 gene sequences.

The present invention also contains variants and homologous sequences of the above-mentioned OATP1B3 amino acid sequence, nucleic acid coding sequence and SLCO1B3 gene sequence.

The OATP1B1 protein and SLCO1B1 gene described herein can be derived from mammals, birds, fishes, amphibians and other animals. The mammals include, but are not limited to, primates, tree shrews (tupaia, tree threw), rodents, bats, Artiodactyla, Carnivora, and other infectious species mammals which can be infected with hepadnavirus or can be infected with hepadnavirus after hepatocytes are transfected with exogenous SLC10A1 gene or express exogenous NTCP protein. The birds include, but are not limited to, ducks, herons and other birds that can be infected with Avihepadnavirus or can be infected with hepadnavirus after liver cells are transfected with exogenous SLC10A1 gene or express exogenous NTCP protein. The primates include, but are not limited to, hominoid species, New World monkeys, Old World monkeys, and other primates that can be infected with hepadnaviruses or can be infected with hepadnavirus after hepatocytes are transfected with exogenous SLC10A1 gene or express exogenous NTCP protein. The hominoid species includes, but is not limited to, humans, chimpanzees, gorillas, gibbons, orangutans, and other hominoid species that can be infected with hepadnaviruses or can be infected with hepadnavirus after hepatocytes are transfected with exogenous SLC10A1 gene or express exogenous NTCP protein. The New World monkeys include, but are not limited to, Woolly monkeys, capuchin monkeys, Squirrel monkeys and other New World monkeys that can be infected with hepadnaviruses or can be infected with hepadnavirus after hepatocytes are transfected with exogenous SLC10A1 gene or express exogenous NTCP protein. The old-World monkeys include, but are not limited to, Macaca fascicularis, and other New World monkeys that can be infected with hepadnaviruses or can be infected with hepadnavirus after hepatocytes are transfected with exogenous SLC10A1 gene or express exogenous NTCP protein. The rodents include,

but are not limited to, animals of the Sciuridae family, myomorpha, and other rodents that can be infected with hepadnaviruses or can be infected with hepadnavirus after hepatocytes are transfected with exogenous SLC10A1 gene or express exogenous NTCP protein. The Sciuridae animals include, but are not limited to, arctic ground squirrel, ground squirrel, woodchuck and other Sciuridae animals that can be infected with hepadnaviruses or can be infected with hepadnavirus after hepatocytes are transfected with exogenous SLC10A1 gene or express exogenous NTCP protein. The myomorpha animals include, but are not limited to, rats, mice and other myomorpha animals that can be infected with hepadnaviruses or can be infected with hepadnavirus after hepatocytes are transfected with exogenous SLC10A1 gene or express exogenous NTCP protein. The artiodactyla animals include but are not limited to pigs and other artiodactyla animals that can be infected with hepadnaviruses or can be infected with hepadnavirus after hepatocytes are transfected with exogenous SLC10A1 gene or express exogenous NTCP protein. The Carnivora animals include but are not limited to dogs and other Carnivora animals that can be infected with hepadnaviruses or can be infected with hepadnavirus after hepatocytes are transfected with exogenous SLC10A1 gene or express exogenous NTCP protein.

The OATP1B1 amino acid sequence described herein includes, but is not limited to, the OATP1B1 amino acid sequence derived from humans (SEQ ID NO: 4, NCBI Reference Sequence: NM_006446), the OATP1B1 amino acid sequence derived from bonobos (NCBI Reference Sequence: XM _003828901), the OATP1B1 amino acid sequence derived from Macaca fascicularis (NCBI Reference Sequence: NM_ 001284540) and the OATP1B1 amino acid sequences derived from other animals mentioned above.

The OATP1B1 nucleic acid coding sequence described herein includes, but is not limited to, the OATP1B1 nucleic acid coding sequence derived from humans (SEQ ID NO: 5, NCBI Reference Sequence: NM_006446), the OATP1B1 nucleic acid coding sequence derived from bonobos (NCBI Reference Sequence: XM _003828901), the OATP1B1 nucleic acid coding sequence derived from Macaca fascicularis (NCBI Reference Sequence: NM_ 001284540) and the OATP1B1 nucleic acid coding sequence derived from other animals mentioned above.

The SLCO1B1 gene sequence described herein includes, but is not limited to, the SLCO1B1 gene sequence derived from humans (SEQ ID NO: 6, genebank Gene ID: 10599), the SLCO1B1 gene sequence derived from bonobos (genebank Gene ID: 100976318), the SLCO1B1 gene sequence derived from Macaca fascicularis (genebank GeneID: 102137347) and the SLCO1B1 gene sequence derived from other animals mentioned above, including but not limited to gene bank NC_000012, NC_041764, NC_036891, NC_022282, NC_048391, NW_012009027, NW_012014011, NC_037679, NW_012109719, NC_044614, NC_048251, NC_036915, NW_006399825, NW_023666069, NW_016809129, NC_044558, NC_044403, NW_022611650, NW_012112030, NC_045443, NW_006399825 and other SLCO1B1 gene sequences.

The present invention also contains variants and homologous sequences of the above-mentioned OATP1B1 amino acid sequence, nucleic acid coding sequence and SLCO1B1 gene sequence.

In some animals, there is the OATP1B2 protein that is homologous to OATP1B3, and the SLCO1B2 gene that is homologous to SLCO1B3.

The OATP1B2 protein and SLCO1B2 gene described herein can be derived from mammals, birds, fishes, amphibians and other animals. The mammals include, but are not limited to, primates, tree shrews (tupaia, tree threw), rodents, bats, Artiodactyla, Carnivora, and other infectious species mammals which can be infected with hepadnavirus or can be infected with hepadnavirus after hepatocytes are transfected with exogenous SLC10A1 gene or express exogenous NTCP protein; the birds include, but are not limited to, ducks, herons and other birds that can be infected with Avihepadnavirus or can be infected with hepadnavirus after liver cells are transfected with exogenous SLC10A1 gene or express exogenous NTCP protein. The primates include, but are not limited to, hominoid species, New World monkeys, Old World monkeys, and other primates that can be infected with hepadnaviruses or can be infected with hepadnavirus after hepatocytes are transfected with exogenous SLC10A1 gene or express exogenous NTCP protein. The hominoid species includes, but is not limited to, humans, chimpanzees, gorillas, gibbons, orangutans, and other hominoid species that can be infected with hepadnaviruses or can be infected with hepadnavirus after hepatocytes are transfected with exogenous SLC10A1 gene or express exogenous NTCP protein. The New World monkeys include, but are not limited to, Woolly monkeys, capuchin monkeys, Squirrel monkeys and other New World monkeys that can be infected with hepadnaviruses or can be infected with hepadnavirus after hepatocytes are transfected with exogenous SLC10A1 gene or express exogenous NTCP protein. The old World monkeys include, but are not limited to, Macaca fascicularis, and other New World monkeys that can be infected with hepadnaviruses or can be infected with hepadnavirus after hepatocytes are transfected with exogenous SLC10A1 gene or express exogenous NTCP protein. The rodents include, but are not limited to, animals of the Sciuridae family, myomorpha, and other rodents that can be infected with hepadnaviruses or can be infected with hepadnavirus after hepatocytes are transfected with exogenous SLC10A1 gene or express exogenous NTCP protein. The Sciuridae animals include, but are not limited to, arctic ground squirrel, ground squirrel, woodchuck and other Sciuridae animals that can be infected with hepadnaviruses or can be infected with hepadnavirus after hepatocytes are transfected with exogenous SLC10A1 gene or express exogenous NTCP protein. The myomorpha animals include, but are not limited to, rats, mice and other myomorpha animals that can be infected with hepadnaviruses or can be infected with hepadnavirus after hepatocytes are transfected with exogenous SLC10A1 gene or express exogenous NTCP

protein. The artiodactyla animals include but are not limited to pigs and other artiodactyla animals that can be infected with hepadnaviruses or can be infected with hepadnavirus after hepatocytes are transfected with exogenous SLC10A1 gene or express exogenous NTCP protein. The Carnivora animals include but are not limited to dogs and other Carnivora animals that can be infected with hepadnaviruses or can be infected with hepadnavirus after hepatocytes are transfected with exogenous SLC10A1 gene or express exogenous NTCP protein.

The OATP1B2 amino acid sequence described herein includes, but is not limited to, the OATP1B2 amino acid sequence derived from rats (NCBI Reference Sequence: NM_031650), the OATP1B2 amino acid sequence derived from mice (NCBI Reference Sequence: NM_020495), and the OATP1B2 amino acid sequence derived from other animals mentioned above. The OATP1B2 nucleic acid coding sequence described herein includes, but is not limited to, the OATP1B2 nucleic acid coding sequence derived from rats (NCBI Reference Sequence: NM_031650), the OATP1B2 nucleic acid coding sequence derived from mice (NCBI Reference Sequence: NM_020495), and the OATP1B2 nucleic acid coding sequence derived from other animals mentioned above.

The SLCO1B2 gene sequence described herein includes, but is not limited to, the SLCO1B2 gene sequence derived from rats (genebank Gene ID: 58978), the SLCO1B2 gene sequence derived from mice (genebank Gene ID: 28253), and the SLCO1B2 gene sequence derived from other animals mentioned above including but not limited to gene bank NC_000072, NC_051339 and other SLCO1B2 gene sequences. The

present invention also contains variants and homologous sequences of the above-mentioned OATP1B2 amino acid sequence, nucleic acid coding sequence and SLCO1B2 gene sequence. Herein, the agent or substance used to prevent or reduce the interaction between hepadnavirus and/or HDV and OATP at least retains the activity of binding or acting on OATP or the activity of acting on the interaction region between HBV or HDV and OATP, to prevent or reduce HBV and/or HDV infection. The agents or substances having the activity of binding or acting on OATP include, but are not limited to, polypeptides derived from hepadnavirus surface antigens; peptides, antibodies, proteins, small molecule drugs, nucleotides and other agents that can bind or interact with OATP; and agents currently known to act on OATP. The method for preventing or reducing the interaction between hepadnavirus and/or HDV and OATP includes administering to a subject in need thereof the agent or substance, including but not limited to polypeptides derived from hepadnavirus surface antigens; peptides, antibodies, proteins, small molecule drugs, nucleotides and other agents that can bind or interact with OATP; and agents currently known to act on OATP.

The agents and substances described herein can bind or act with OATP to prevent or reduce the interaction of HBV and/or HDV with OATP. The invention specifically includes agents and substances that bind or act on OATP1B1 and/or OATP1B3, including but not limited to polypeptides derived from hepadnavirus surface antigens; peptides, antibodies, proteins, small molecule drugs, nucleotides and other agents that can bind or interact with OATP1B1 and/or OATP1B3; and agents currently known to act on OATP1B1 and/or OATP1B3. The agents or substances can bind or act on OATP1B1 and/or OATP1B3 to prevent or reduce the interaction of HBV and/or HDV with OATP1B1 and/or OATP1B3. The invention specifically includes agents and substances that bind or act on human OATP1B1 and/or human OATP1B3, including but not limited to polypeptides derived from hepadnavirus surface antigens; peptides, antibodies, proteins, small molecule drugs, nucleotides and other agents that can bind or interact with human OATP1B1 and/or human OATP1B3; and agents currently known to act on human OATP1B1 and/or human OATP1B3. The agents or substances can bind or act on human OATP1B1 and/or human OATP1B3 to prevent or reduce the interaction of HBV and/or HDV with human OATP1B1 and/or human OATP1B3.

The peptides described herein that can bind or interact with OATP include, but are not limited to, peptides or peptide modifiers obtained by the techniques including but not limited to peptide library techniques (random peptide libraries, combinatorial peptide libraries, synthetic peptide libraries and other peptide library techniques), phage presentation techniques, yeast presentation techniques, viral presentation techniques, and other peptide screening techniques that target OATP. In particular, the peptides described herein that can bind or interact with OATP1B1 and/or OATP1B3 include, but are not limited to, peptides or peptide modifiers obtained by the techniques including but not limited to peptide library techniques (random peptide libraries, combinatorial peptide libraries, synthetic peptide libraries and other peptide library techniques), phage presentation techniques, yeast presentation techniques, viral presentation techniques, and other peptide screening techniques that target OATP1B1 and/or OATP1B3. In particular, the peptides described herein that can bind or interact with human OATP1B1 and/or human OATP1B3 include, but are not limited to, peptides or peptide modifiers obtained by the techniques including but not limited to peptide library techniques (random peptide libraries, combinatorial peptide libraries, synthetic peptide libraries and other peptide library techniques), phage presentation techniques, yeast presentation techniques, viral presentation techniques, and other peptide screening techniques that target human OATP1B1 and/or human OATP1B3.

The antibodies described herein that bind or interact with OATP include, but are not limited to, anti-OATP antibody sera, polyclonal antibodies, monoclonal antibodies or antibody fragments or antibody modifiers obtained by, including but not limited to, immunizing an animal with OATP as a target via OATP protein or protein fragment or fusion protein, or polyclonal antibodies, monoclonal antibodies, humanized antibodies, fully-humanized antibodies, antibody fragments, fusion antibodies, bispecific antibodies, multispecific antibodies and other antibodies obtained by hybridoma

technology, phage presentation technology, yeast presentation technology, immunolibrary technology, ribosome display and mRNA presentation screening techniques, nanobody (Nb) screening methods, transgenic mice fully human antibody screening techniques, single-cell photoconduction techniques, flow cytometry techniques and other antibody screening techniques, or antibody fragments, or antibody modifiers. The above-mentioned antibodies or antibody serum are characterized by being able to bind to OATP and prevent or reduce the interaction between HBV and/or HDV and OATP. In particular, the present invention includes antibody sera, polyclonal antibodies, monoclonal antibodies, humanized antibodies, fully humanized antibodies, antibody fragments, fusion antibodies, bispecific antibodies, multispecific antibodies, other antibodies, or antibody fragments, or antibody modifiers that can bind or interact with OATP1B1 and/or OATP1B3, and are characterized by being able to bind to OATP1B1 and/or OATP1B3 and prevent or reduce the interaction of HBV and/or HDV with OATP1B1 and/or OATP1B3. In particular, the present invention includes antibody sera, polyclonal antibodies, monoclonal antibodies, humanized antibodies, fully humanized antibodies, antibody fragments, fusion antibodies, bispecific antibodies, multispecific antibodies, other antibodies, or antibody fragments, or antibody modifiers that can bind or interact with human OATP1B1 and/or human OATP1B3, and are characterized by being able to bind to human OATP1B1 and/or human OATP1B3 and prevent or reduce the interaction of HBV and/or HDV with human OATP1B1 and/or human OATP1B3. The present invention also contains antibodies, antibody fragments or antibody modifications directed against the interaction region between HBV or HDV and OATP.

The proteins described herein that can bind or interact with OATP include, but are not limited to, proteins, amino acid fragments, or protein modifiers obtained by phage display technology, yeast display technology, cell display technology, random library and other protein-protein interaction screening technology using OATP as a target. The invention particularly includes proteins that can bind or interact with OATP1B1 and/or OATP1B3 include, but are not limited to, proteins, amino acid fragments, or protein modifiers obtained by phage display technology, yeast display technology, cell display technology, random library and other protein-protein interaction screening technology using OATP1B1 and/or OATP1B3 as targets. In particular, the present invention includes proteins that can bind or interact with human OATP1B1 and/or human OATP1B3 include, but are not limited to, proteins, amino acid fragments, or protein modifiers obtained by phage display technology, yeast display technology, cell display technology, random library and other protein-protein interaction screening technology using human OATP1B1 and/or human OATP1B3 as targets.

The nucleotides described herein that can bind or interact with OATP include, but are not limited to, PolyDNA, oligoDNA, polyRNA, oligoRNA, deoxyribonucleic acid protein complex (DNP) or nucleic acid modification obtained by screening with OATP as a target. The invention particularly includes nucleotides that can bind or interact with OATP1B1 and/or OATP1B3 include, but are not limited to, PolyDNA, oligoDNA, polyRNA, oligoRNA, deoxyribonucleic acid protein complex (DNP) or nucleic acid modification obtained by screening with OATP1B1 and/or OATP1B3 as targets. In particular, the present invention includes nucleotides that can bind or interact with human OATP1B1 and/or human OATP1B3 include, but are not limited to, PolyDNA, oligoDNA, polyRNA, oligoRNA, deoxyribonucleic acid protein complex (DNP) or nucleic acid modification obtained by screening with human OATP1B1 and/or human OATP1B3 as targets.

The small molecule compounds described herein that can bind to or interact with OATP include, but are not limited to, small molecule compounds that bind to or interact with OATP obtained through design or compound screening. The invention particularly includes small molecule compounds that can bind to or interact with OATP1B1 and/or OATP1B3 include, but are not limited to, small molecule compounds that bind to or interact with OATP1B1 and/or OATP1B3 obtained through design or compound screening. In particular, the present invention includes small molecule compounds that can bind to or interact with human OATP1B1 and/or human OATP1B3 include, but are not limited to, small molecule compounds that bind to or interact with human OATP1B1 and/or human OATP1B3 obtained through design or compound screening.

The agents and substances known to act on OATP described herein include, but are not limited to, agents and substances known to act on OATP1B1 and/or OATP1B3, and particularly include, but are not limited to, agents and substances known to act on human OATP1B1 and/or human OATP1B3, and the agents and substances including but not limited to statins (including but not limited to atorvastatin, pitavastatin, rosuvastatin, simvastatin, pravastatin and other statins), sartans (including but not limited to valsartan, olmesartan, telmisartan and other sartans), bosentan, docetaxel, fexofenadine, glecaprevir, glyburide (glibenclamide), grazoprevir, nateglinide, paclitaxel, paritaprevir, repaglinide, simeprevir, voxilaprevir, steroid hormone metabolites, thyroid hormones metabolites, inflammatory mediators, bile acids, bilirubin, Bromosulfophthalein, Estradiol-17β-glucuronide, Cholecystokinin octapeptide, Estrone-3-sulfate, liver function markers, mushroom toxins, antibiotics, antiviral drugs, anticancer drugs and other agents and substances known to interact with OATP1B1 and/or OATP1B3.

In preventing or reducing the expression and/or function of OATP as described herein, the agents or substances used is at least retained the effect on the OATP-encoding gene SLCO, or on the OATP mRNA, or on OATP protein expression, and/or modification, and/or transport, and/or degradation, to prevent or reduce OATP protein expression, or reducing the biological function of the expressed OATP protein, or enhancing the degradation of OATP protein. Preventing or reducing OATP expression and/or function as described herein specifically includes, but is not limited to, preventing or reducing OATP1B1 and/or OATP1B3 expression and/or function, at least retaining the effect on OATP1B1 and/or

OATP1B3 encoding genes SLCO1B3 or SLCO1B1, or on OATP1B1 and/or OATP1B3 mRNA, or on OATP1B1 and/or OATP1B3 protein expression, and/or modification, and/or transport, and/or degradation, to prevent or reduce OATP1B1 and/or OATP1B3 protein expression, or reducing the biological function of expressed OATP1B1 and/or OATP1B3 proteins, or enhancing OATP1B1 and/or OATP1B3 protein degradation. Preventing or reducing OATP1B1 and/or OATP1B3 expression and/or function as described herein specifically includes, but is not limited to, preventing or reducing human OATP1B1 and/or human OATP1B3 expression and/or function, at least retaining the effect on human OATP1B1 and/or human OATP1B3 encoding genes human SLCO1B3 or human SLCO1B1, or on human OATP1B1 and/or human OATP1B3 mRNA, or on human OATP1B1 and/or human OATP1B3 protein expression, and/or modification, and/or transport, and/or degradation, to prevent or reduce human OATP1B1 and/or human OATP1B3 protein expression, or reducing the biological function of expressed human OATP1B1 and/or human OATP1B3 proteins, or enhancing human OATP1B1 and/or human OATP1B3 protein degradation.

The methods described herein that act on the SLCO gene include, but are not limited to, gene knockout, gene insertion, homologous recombination, gene editing, and other methods that inhibit gene transcription activity. The methods described herein that act on the SLCO gene specifically include, but are not limited to, methods that act on the SLCO1B3 or SLCO1B1 gene, especially include but are not limited to methods that act on the human SLCO1B3 or human SLCO1B1 gene, including but not limited to gene knockout, gene insertion, homologous recombination, gene editing, and other methods to inhibit gene transcriptional activity. The methods described herein that act on OATP mRNA include, but are not limited to, antisense nucleic acids, RNA interference, and other methods that cleave and degrade the mRNA, destabilize the mRNA, or prevent the mRNA translation. The methods described herein that act on the OATP mRNA specifically include, but are not limited to, methods that act on the OATP1B1 and/or OATP1B3 mRNA, especially include but are not limited to methods that act on the human OATP1B1 and/or human OATP1B3 mRNA, including but not limited to antisense nucleic acids, RNA interference, and other methods that cleave and degrade the mRNA, destabilize the mRNA, or prevent the mRNA translation.

The methods described herein to reduce the biological function of the expressed OATP protein include but are not limited to inhibiting post-translational modifications such as protein glycosylation to reduce the biological function of the OATP protein. The methods described herein that reduce the biological function of the expressed OATP protein specifically include, but are not limited to, methods that reduce the biological function of the expressed OATP1B1 and/or OATP1B3 protein, especially include but are not limited to methods that reduce the biological function of the expressed human OATP1B1 and/or human OATP1B protein, including but not limited to inhibiting post-translational modifications such as protein glycosylation to reduce the biological function of the human OATP1B1 and/or human OATP1B protein. The methods that act on OATP protein transport include, but are not limited to, methods such as inhibiting protein sorting and Golgi apparatus to reduce the OATP protein present on the cell membrane surface, or methods such as enhancing the endocytosis of the OATP protein on the cell membrane surface to reduce the OATP protein on the cell membrane surface.

The methods described herein that act on OATP protein transport specifically include, but are not limited to, methods that act on OATP1B1 and/or OATP1B protein transport, especially include but are not limited to methods that that act on human OATP1B1 and/or human OATP1B protein transport, including but not limited to such as inhibiting protein sorting and Golgi apparatus to reduce the human OATP1B1 and/or human OATP1B protein present on the cell membrane surface, or methods such as enhancing the endocytosis of the human OATP1B1 and/or human OATP1B protein on the cell membrane surface to reduce the human OATP1B1 and/or human OATP1B protein on the cell membrane surface.

The methods for enhancing OATP protein degradation described herein particularly include, but are not limited to, methods for enhancing OATP1B1 and/or OATP1B3 protein degradation, particularly including but are not limited to methods for enhancing human OATP1B1 and/or human OATP1B3 protein degradation, including but not limited to increasing protein ubiquitination, protein degradation targeting chimeras (PROTAC) and other methods that destabilize proteins and/or enhance protein degradation.

The methods and agents or substances that prevent or reduce the expression and/or function of OATP1B1 and/or OATP1B3 described herein further include, but are not limited to, gene editing targeting the SLCO gene, antisense nucleic acids, ribozymes, DNAzymes that targeting OATP mRNA, or methods, reagents and substances for RNA Interference PROTAC or PROTAC targeting OATP protein. The gene editing described herein is also called Genome Editing, including but not limited to the use of Cre-lox-mediated recombination at specific sites in the genome, directed modification of target genes using Zinc finger nucleases, CRISPR (Clustered Regularly Interspaced Short Palindromic Repeats)/Cas9 system and technology variants or other gene editing technologies, which can edit the SLCO gene and its transcripts (directed transformation) to add and delete DNA fragments, delete and replace specific DNA bases, etc., to change the sequence, expression level or function of the SLCO gene or regulatory elements. In particular, editing (directed transformation) of the SLCO1B3 and/or SLCO1B1 genes and their transcripts to change the sequence, expression level or function of the SLCO1B3 and/or SLCO1B1 genes or regulatory elements. In particular, editing (directed transformation) of the human SLCO1B3 and/or human SLCO1B1 genes and their transcripts to change the sequence, expression level or function of the human SLCO1B3 and/or human SLCO1B1 genes or regulatory elements. The RNA interference (RNA interference, RNAi) described herein includes but is not limited to the use of double-stranded RNA (dsRNA), small

interfering RNA (siRNA), silencing RNA or RNA interference vehicle, the methods and reagents for degrading OATP mRNA, particularly include but are not limited to methods and reagents for degrading OATP1B1 and/or OATP1B3 mRNA, particularly include but are not limited to methods and reagents for degrading human OATP1B1 and/or human OATP1B3 mRNA. The methods and agents for PROTAC targeting OATP protein include, but are not limited to, methods and medicaments for degrading the target protein OATP using the ubiquitin-proteasome system (UPS), and the methods and agents may contain structures of a ubiquitin ligase ligand-linker-target protein OATP ligand, or other structures and methods that can ubiquitinate OATP or direct it to the proteasome. The methods and agents of PROTAC targeting OATP protein particularly include but are not limited to the methods and agents of PROTAC targeting OATP1B1 and/or OATP1B3 protein, particularly including but not limited to methods and agents for PROTAC targeting human OATP1B1 and/or human OATP1B3 protein.

The methods, agents and substances described herein for treating and/or preventing HBV and/or HDV infection and related diseases by preventing or reducing the interaction between HBV and/or HDV and OATP or by preventing or reducing the expression and/or function of OATP, can be used alone or in combination with agents that prevent or reduce the interaction between HBV and/or HDV and NTCP and/or agents and methods that can prevent or reduce the expression and/or function of NTCP in animals.

The NTCP described herein include, but are not limited to, mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old-world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some embodiments of the present invention, the NTCP includes, but is not limited to, NTCP derived from humans (SEQ ID NO: 7, NCBI Reference Sequence: NM_003049), NTCP derived from chimpanzees (NCBI Reference Sequence: XM_510035), derived from NTCP from tree shrews (NCBI Reference Sequence: XM_006171503), NTCP from bonobos (NCBI Reference Sequence: XM_003824101), NTCP from capuchin monkeys (NCBI Reference Sequence:XM_032245781), NTCP from squirrel monkeys (NCBI Reference Sequence:XM_003924480), NTCP from Macaca fascicularis (NCBI Reference Sequence:NM_001283323), NTCP from arctic ground squirrels (NCBI Reference Sequence:XM_026385176), NTCP from ground squirrels (NCBI Reference Sequence:XM_005322843), NTCP derived from rats (NCBI Reference Sequence: XM_032908052), NTCP derived from mice (NCBI Reference Sequence: NM_001177561), NTCP derived from pigs (NCBI Reference Sequence: XM_001927695), NTCP derived from dogs (NCBI Reference Sequence: XM_537494), as well as NTCP and its variants derived from other animals mentioned above.

In some preferred embodiments, the methods, agents and substances described herein for treating and/or preventing HBV and/or HDV infection and related diseases by preventing or reducing the interaction between HBV and/or HDV and OATP or by preventing or reducing the expression and/or function of OATP, when used in combination with agents that prevent or reduce the interaction between HBV and/or HDV and NTCP and/or agents and methods that can prevent or reduce NTCP expression and/or function in animals, the NTCP is that of humans, primates, rodents or avian animals, and is more preferably human NTCP. The agents and substances that prevent or reduce the interaction between HBV and/or HDV and NTCP and/or the agents, substances and methods that can prevent or reduce the expression and/or function of NTCP in animals include but are not limited to, peptides, proteins, antibodies, small molecule drugs that act with NTCP or with HBV, and thereby prevent or reduce the interaction between HBV and/or HDV and NTCP, polyribonucleotides, and other agents, as well as agents, substances, and methods currently known to act on NTCP. The currently known agents, substances and methods that act on NTCP include but are not limited to bile acids and their derivatives (including but not limited to cholic acid CA, deoxycholic acid DCA, chenodeoxycholic acid CDCA, ursodeoxycholic acid UDCA, lithocholic acid LCA and the products combining these bile acids with glycine, taurine, sulfuric acid, glucuronic acid, dimeric bile acid derivatives), organic anions (including but not limited to glycocholic acide, bromosulfophthalein ), peptides (including but not limited to EMD51921, angiopeptin, U71038, $\alpha$-amanitln, phe-phe-phe, ala-ala-ala, tyr-tyr), drugs (including but not limited to propranolol, progesterone, cyclosporine and their derivatives, ketoconazole, bumetanide, furosemide, chlorpropamide, theophylline, ephedrine, sodium valproate, nicotine, pseudoephedrine, salicylate, procainamide, diphenylhydantoin, nifedipine, caffeine, bupivacaine, lidocaine, quinidine and other drugs that interact with NTCP) and other agents that interact with NTCP and methods.

The agents, substances and methods described herein that prevent or reduce NTCP expression and/or function at least retain their effect on the NTCP encoding gene SLC10A1, or act on NTCP mRNA, or act on NTCP protein

expression, and/or modification, and/or transport, or degrade, to prevent or reduce the expression of NTCP protein, or reduce the biological function of the expressed NTCP protein, or enhance the degradation of NTCP protein. The methods described herein that act on the SLC10A1 gene include, but are not limited to, gene knockout, gene insertion, homologous recombination, gene editing, and other methods that inhibit gene transcription activity. The methods described herein that act on NTCP mRNA include, but are not limited to, antisense nucleic acids, RNA interference, and other methods that cleave and degrade the mRNA, destabilize the mRNA, or prevent the mRNA translation. The methods described herein to reduce the biological function of the expressed NTCP protein include but are not limited to inhibiting post-translational modifications such as protein glycosylation to reduce the biological function of the NTCP protein. The methods that act on NTCP protein transport include, but are not limited to, methods such as inhibiting protein sorting and Golgi apparatus to reduce the NTCP protein present on the cell membrane surface, or methods such as enhancing the endocytosis of the NTCP protein on the cell membrane surface to reduce the NTCP protein on the cell membrane surface. The methods of enhancing NTCP protein degradation include, but are not limited to, increasing protein ubiquitination, protein degradation targeting chimera (PROTAC) and other methods that destroy protein stability and/or enhance protein degradation.

The methods and agents and substances described herein that prevent or reduce the interaction between HBV and/or HDV and OATP, or by preventing or reducing the expression and/or function of OATP, can be used for the prevention and treatment of HBV and/or HDV infection and related diseases. In some embodiments, the disease is a liver disease associated with HBV and/or HDV viral infection. The various methods, agents and substances described herein can be used to prevent the transport of HBV and/or HDV viruses into cells via OATP, thereby avoiding or reducing viral infection of liver cells and preventing the occurrence and/or progression of the disease. In the event of accidental exposure to the virus, the methods, agents and substances described herein can be administered to prevent the entry of HBV and/or HDV viruses into liver cells, thereby preventing viral infection and achieving disease prevention. Therefore, the method of treating or preventing liver disease described herein is a method of treating or preventing liver disease caused by viral infection, which includes administering to a subject in need a therapeutically effective amount or a prophylactically effective amount of the method described herein to prevent or reduce HBV and/or HDV interacts with OATP, or by various agents or substances described herein that prevent or reduce OATP expression and/or function. In other embodiments, provided herein are methods of preventing HBV and/or HDV virus infection of liver tissue or liver cells and/or preventing the spread of the virus between liver cells, the method comprising administering to a subject in need thereof an effective amount of this article Various agents or substances described herein that prevent or reduce the interaction of HBV and/or HDV with OATP, or by preventing or reducing OATP expression and/or function. In some embodiments, also provided herein is the use of various substances or agents described herein in the manufacture of a medicament for the treatment or prevention of liver disease, especially liver disease associated with hepatitis B virus or hepatitis D virus infection, and in the manufacture of a medicament that prevent virus (such as hepatitis virus, such as hepatitis B virus, hepatitis D virus) infection of liver tissue or liver cells and/or prevent the spread of the virus between liver cells. In some embodiments, also provided herein are methods, medicaments, and substances for treating or preventing liver disease, particularly liver disease associated with hepatitis B virus or hepatitis D virus infection, and for preventing viruses (e.g., hepatitis viruses, such as Methods, medicaments and substances for infecting liver tissue or liver cells with hepatitis B virus or hepatitis D virus and/or preventing the spread of the virus between liver cells. In some embodiments, the disease is Acute Hepatitis B (AHB), Chronic Hepatitis B (CHB), Acute Hepatitis D (AHD), chronic hepatitis D (CHD).

In some embodiments, the methods or agents or substances for the treatment or prevention of HBV and/or HDV infection and related diseases described in the present invention can be used in combination with other HBV and/or HDV treatment or prevention drugs, including but not limited to nuclear Glycoside (acid) analogues (including but not limited to lamivudine, telbivudine, entecavir, tenofovir disoproxil, adefovir dipivoxil, clavudine, tenofovir disoproxil disoproxil and other nuclear Glycoside (acid) analogues), interferons (including but not limited to ordinary interferons (including but not limited to interferon alpha-2a, alpha-2b and other types of interferons), long-acting interferons, PEG interferons (including but not limited to Not limited to PEG interferon alpha-2a, alpha-2b and other types of PEG interferon) and other interferons), nucleocapsid inhibitors, RNA interference, immunomodulators and other agents for preventing or treating HBV or HDV infection, and Combinations of these agents are recommended.

In some embodiments of the present invention, the agent or substance that prevents or reduces the interaction between hepadnavirus and/or HDV and OATP is selected from hepadnavirus surface antigen-derived peptides that bind to OATP. Preferably, the OATP-binding hepadnavirus surface antigen-derived peptide is as described below.

Hepadnavirus surface antigen-derived peptide that binds to OATP

This invention also provides a polypeptide derived from a hepatophilic DNA virus and variants thereof, said polypeptide and variants thereof retaining at least the biological activity of binding to OATP, which will be referred to herein as simply OATP-binding peptide. The "derived from hepadnavirus" as used herein means that the polypeptide is derived from hepadnavirus, or is highly homologous to the corresponding polypeptide of hepadnavirus or has the same function but with certain differences in sequence. The "variant" as described herein refers to a polypeptide that has certain amino

acid differences compared with a wild-type polypeptide, but still has the biological activity of binding OATP as described herein. The OATP-binding peptides described herein can be used for the prevention and/or treatment of hepadnavirus and/or HDV infection and related diseases.

The hepadnaviruses include, but are not limited to, Orthohepadnavirus, Avihepadnavirus, Metahepadnavirus, Herpetohepadnavirus and other hepadnaviruses. The orthohepadnavirus DNA viruses includes but is not limited to infecting humans (HBV), woolly monkeys (Woolly monkey HBV, WMHBV), capuchin monkeys (Capuchin monkey HBV, CMHBV), squirrel monkey, woodchuck (Woodchuck hepatitis virus, WHV), arctic rat, ground squirrel (ground squirrel hepatitis virus, GSHV), tree shrew, chimpanzee, bat (Bat hepatitis virus, BHV) and other animals, example genome sequences can be found in GenBank accession numbers including but not limited to AF046996 (WMHBV), NC_043528 (CMHBV), NC_004107 (WHV), NC_001484 (GSHV), JX941468 (BHV). The Avihepadnavirus includes, but is not limited to, infecting ducks (duck HBV, DHBV), herons (heron HBV, HHBV) and other birds, and example genome sequences can be found in, but are not limited to, GenBank accession numbers NC_001344 (DHBV), NC_001486 (HHBV).

Orthohepadnavirus that infect humans (HBV) described herein include, but are not limited to, HBV genotype A, B, C, D, E, F, G, H, and I, and example genome sequences can be found in, but are not limited to, GenBank accession numbers KC875260 (genotype A), AY220704 (genotype B), AF461363 (genotype C), AY 796030 (genotype D), AB205129 (genotype E), DQ823095 (genotype F), HE981176 (genotype G) and AB179747 (genotype H). In some embodiments herein, the amino acid sequence of the OATP-binding hepadnavirus-derived peptide is derived from a viral envelope protein, also known as a surface antigen or surface protein, that is, a hepadnavirus surface antigen-derived peptide that binds to OATP or an OATP-binding peptide derived from a hepadnavirus surface antigen.

In further preferred embodiments herein, the amino acid sequence of the hepadnavirus surface antigen-derived peptide that binds to OATP is derived from the amino acid sequence of the Pre-S region of the viral surface antigen, including but not limited to the exemplary surface antigen Pre-S amino acid sequence SEQ ID NO: 8-22 and its variants. Polypeptides derived from the amino acid sequence of surface antigen of Pre-S1 region of human HBV (i.e., HBVPre-S1-derived peptides that bind to OATP or OATP-binding peptides derived from HBVPre-S1) are specifically included herein, including but not limited to, amino acid sequences of the Pre-S1 region of HBV genotypes A, B, C, D, E, F, G, H and I, including but not limited to the exemplary amino acid sequence of surface antigen of HBV Pre-S region which shown in SEQ ID NO: 15-22 and variants thereof. Specifically included herein are OATP-binding peptides derived from the amino acid sequence of the surface antigen of Pre-S1 region of human HBV, including but not limited to genotype C genotype HBVP Pre-S region amino acid sequence, including but not limited to the exemplary HBV surface antigen Pre-S1 region The amino acid sequence of SEQ ID NO: 17 and its variants. The hepadnavirus-derived peptides and their variants that bind to OATP, the hepadnavirus surface antigen-derived peptides and their variants that bind to OATP, and the HBVPre-S1-derived peptides and their variants that bind to OATP are collectively referred to as OATP-binding peptides in this invention.

The OATP-binding hepadnavirus-derived peptides and variants thereof retain at least the biological activity of binding to OATP. The OATP includes but is not limited to OATP superfamily derived from mammals, birds, fishes, amphibians and other animal sources, including but not limited to OATP1, OATP2, OATP3, OATP4, OATP5, OATP6, OATP7, OATP8, OATP9, OATP10, and OATP11 families, especially including but not limited to the OATP family expressed in hepatocytes; further including but not limited to the OATP1B subfamily, especially including but not limited to the OATP1B family expressed in hepatocytes; further including but not limited to OATP1B3, OATP1B1, OATP1B2 and other OATP1B subfamily members. The OATP1B3 includes, but is not limited to, OATP1B3 derived from humans (NCBI Reference Sequence: NM_019844), OATP1B3 derived from chimpanzees (NCBI Reference Sequence: XM_016923821), OATP1B3 derived from tree shrews (NCBI Reference Sequence: XM_006155085), derived from OATP1B3 from macaca fascicularis (NCBI Reference Sequence: NM_001283191), OATP1B3 from arctic ground squirrels (NCBI Reference Sequence: XM_026391932), OATP1B3 from rats (NCBI Reference Sequence: XM_032905480), OATP1B3 from pigs (NCBI Reference Sequence: NM_001315607), OATP1B3 from dogs (NCBI Reference Sequence: NM_001166047), and OATP1B3 from other animals. The OATP1B1 includes, but is not limited to, OATP1B1 derived from humans (NCBI Reference Sequence: NM_006446), OATP1B1 derived from bonobos (NCBI Reference Sequence: XM_003828901), OATP1B1 derived from macaca fascicularis (NCBI Reference Sequence: NM_001284540), and OATP1B1 derived from other animals. The OATP1B2 includes, but is not limited to, OATP1B2 derived from rats (NCBI Reference Sequence: NM_031650), OATP1B2 derived from mice (NCBI Reference Sequence: NM_020495), and OATP1B2 derived from other animals. In some preferred embodiments herein, the OATP-binding HBV Pre-S1-derived peptide and its variants retain at least the biological activity of binding to human OATP1B3 and/or human OATP1B1.

The term "OATP-binding hepadnavirus-derived peptide" or "OATP-binding peptide derived from hepadnavirus" means that the origin or source of the polypeptide is a hepadnavirus, including but not limited to natural, recombinant, Synthetic or purified polypeptides include, but are not limited to, full-length natural HBV polypeptides or fragments thereof, and variants of full-length natural polypeptides or fragments thereof. In some embodiments, the OATP-binding hepadnavirus-derived peptide can be derived from any hepadnavirus, including but not limited to Orthohepadnavirus (OHV) that

infects mammals, Avihepadnavirus that infects birds virus (AHV), fish-infecting Metahepadnavirus viruses, and amphibian-infecting Herpetohepadnavirus viruses; in some embodiments, the OATP-binding hepadnavirus surface antigen-derived peptide may be derived from any hepadnavirus surface antigen; in In some embodiments, the OATP-binding hepadnavirus surface antigen-derived peptide can be derived from any proto-hepadnavirus surface antigen; in some embodiments, the OATP-binding hepadnavirus surface antigen-derived peptide can be derived from any human hepatitis B virus (HBV) surface antigen, including, but not limited to, derived from HBV surface antigen of genotype A, B, C, D, E, F, G, H, and I; in some embodiments, the OATP-binding hepadnavirus surface antigen-derived peptide may be derived from any human hepatitis B virus (HBV) surface antigen Pre-S1 region, an OATP-binding HBVPre-S1-derived peptide or an OATP-binding peptide derived from HBVPre-S1.

The terms "OATP-binding HBVPre-S1-derived peptide" or "OATP-binding peptide derived from HBVPre-S1" as used herein refers to the polypeptide whose origin or source is the HBV surface antigen Pre-S1 region, including but not limited to natural, recombinant, synthetic or purified polypeptides, including but not limited to full-length natural HBV surface antigen Pre-S1 region or fragments thereof and variants thereof, full-length non-natural HBV surface antigen Pre-S1 region or fragments thereof and variants thereof, non- Full-length natural HBV surface antigen Pre-S1 region or fragments thereof and variants thereof, non-full-length non-natural HBV surface antigen Pre-S1 region or fragments thereof and variants thereof, and other forms of variants. In some embodiments, the OATP-binding HBVPre-S1 derived peptide may contain the entire surface antigen pre-S1 from the second amino acid G (glycine) in the HBV surface antigen pre-S1 region of any genotype district. In certain embodiments, the OATP-binding HBVPre-S1 derived peptides described herein may be derived from the pre-S1 region of the surface antigen of any one of HBV genotypes A, B, C, D, E, F, G, H and I. Example genome sequences of these HBV genotypes can be found in GenBank accession numbers KC875260, AY220704, AF461363, AY796030, AB205129, DQ823095, HE981176, and AB179747, and exemplary amino acid sequences of their surface antigen Pre-S1 regions can be found in, but are not limited to, SEQ ID NO: 8-22 and variants thereof. In certain embodiments, the OATP-binding HBVP Pre-S1 derived peptides described herein can be derived from the surface antigen of pre-S1 region of HBV genotype C. The OATP-binding HBVPre-S1 derived peptide at least retains the biological activity of binding to OATP, especially retains the biological activity of binding to OATP1B1, and/or OATP1B2 and/or OATP1B3, especially retains the biological activity of binding to human OATP1B1 and/or human OATP1B3.

In some embodiments, the OATP-binding peptides described herein may be 10-118 amino acids in length. For example, the polypeptide may be, but not limited to, 15-100, 15-80, 20-100, 20-80, 20-60, 25-60, 30-60, 35-60, or 40-60 (including all integers in these ranges) amino acids in length. In some embodiments, the OATP-binding peptides described herein may be at least 20 amino acids in length, e.g., at least 25, 30, 35 or 40 amino acids in length. In some embodiments, the OATP-binding peptides described herein may be but are not limited to 20, 25, 30, 35, 40, 47, 55 or 60 amino acids in length. In some embodiments, the OATP-binding peptides described herein may be 47 amino acids in length. The variants of varying lengths of the OATP-binding peptides described herein retain one or more biological activities associated with the corresponding polypeptide, including at least the biological activity of binding to OATP, particularly to OATP1B1, and/or OATP1B2, and/or OATP1B3, especially the biological activity of binding to human OATP1B1 and/or human OATP1B3.

In certain embodiments, the OATP-binding peptides described herein may be derived from the pre-S1 region of the surface antigen of HBV genotype C, including but not limited to amino acids 2-119, 2-69, 2-59, 13-119, 13-88, 13-72, 13-67, 13-59, 13-52, 13-47, 13-42, 13-37, 13-32 of the pre-S1 region and variants thereof, exemplary sequences include but not limited to SEQ ID NO: 23-35 and variants thereof.

In some embodiments, the OATP-binding peptides comprises the amino acid sequence of at least amino acid residues 13-44 at the N-terminal of the pre-S1 region of surface antigen of HBV genotype C described herein and variants thereof. In preferred embodiments, the OATP-binding peptide may be 32-47 amino acid residues in length. In a particularly preferred embodiment, the exemplary amino acid sequence of the OATP-binding peptide is shown in SEQ ID NO: 30; preferably, the OATP-binding peptide also includes variants of the amino acid sequence shown in SEQ ID NO: 30; In some embodiments, the OATP-binding peptide at least contains amino acid residues 1-32 in the sequence SEQ ID NO: 30 and variants thereof, and preferably, the OATP-binding peptide at least contains the sequence SEQ ID NO: 30 Amino acid residues from position 1 to position 33, 34, 35, 36, 37, 38, 39, 40, 41, 42, 43, 44, 45 or 46 in the sequence and variants thereof. In some embodiments, the OATP-binding peptide refers to at least the positions 13-44 of the N-terminus of the surface antigen of pre-S1 region of HBV genotypes A, B, F, H, and I described herein, a fragment of at least positions 2-33 of the N-terminus of the HBV surface pre-S1 region of genotype D or its variant, or a fragment of at least positions 12-43 of the N-terminus of the HBV surface pre-S1 region of genotype E and G or its variant. In some embodiments, the OATP-binding peptide refers to at least the positions 13-69 of the N-terminus of the surface antigen of pre-S1 region of HBV genotypes A, B, F, H, and I described herein, a fragment of at least positions 2-48 of the N-terminus of the HBV surface pre-S1 region of genotype D or its variant, or a fragment of at least positions 12-68 of the N-terminus of the HBV surface pre-S1 region of genotype E and G or its variant, exemplary sequences thereof include but are not limited to the exemplary sequences SEQ ID NO: 36-42 and variants thereof.

As used herein, a "variant" relative to an OATP-binding peptide refers to a polypeptide that differs in amino acid sequence from a given polypeptide but retains the biological activity of binding to OATP, particularly OATP1B3, and/or

OATP1B2, and/or OATP1B1, especially biological activity binding to human OATP1B3 and/or OATP1B1. For example, in some embodiments, one or more of the following amino acid substitutions may be introduced into the derivative peptide derived from the amino acid sequence of positions 13-59 of the Pre-S1 region of HBV genotype C: N15D, F25L, G35K, N39E, F45L, N46K, N48H or N48Y or N48K, D50A, H51Q or H51N, E54K or E54D, A55S, N56K or N56D, and QS7K, exemplary sequences include but are not limited to those shown by SEQ ID NO: 43-60 sequence. As used herein, the term "variant" also includes homologous polypeptide sequences found in different viral species, strains, or subtypes of the genus Hepatovirus. Based on the antigenic epitopes on its envelope protein, HBV is classified into four main serotypes (adr, adw, ayr, and ayw), and according to the variability of the nucleotide sequence in the genome, HBV is divided into nine genotypes (A to I). Thus, the term "variant" includes any of these homologous polypeptides found in HBV subtypes. "Variants" may also include polypeptides with natural flanking amino acid sequences from any of these HBV subtypes added at the N- and/or C-terminus, or variants thereof. For example, in some embodiments, the polypeptide derived from the 13-59 amino acid sequence of the genotype C surface antigen Pre-S1 region can be combined with the polypeptide including but not limited to derived from the surface antigen of pre-S1 region of HBV genotypes A, B, F, H, and I, a fragment of at least amino acid residues 2-12 at the N-terminus of the S1 region, or a fragment of at least amino acid residues 2-11 at the N-terminus of the surface antigen pre-S1 region of HBV genotype E or G, forms a fusion polypeptide, and its example sequence including but not limited to the sequences shown in SEQ ID NO: 61-66. For example, in some embodiments, the OATP-binding peptide derived from the HBV Pre-S1 region described herein may contain an amino acid sequence selected from, but not limited to, any of SEQ ID NO: 23-66, and natural amino acid sequence flanking the N- and/or C-terminus derived from the surface antigen of any of HBV genotypes A-I. In some embodiments, the natural flanking amino acid sequences can be derived from the amino acid sequences of HBV surface antigens, including but not limited to GenBank accession numbers KC875260 (genotype A), AY220704 (genotype B), AF461363 (genotype C), AY796030 (amino acid sequence of HBV surface antigen of genotype D), AB205129 (genotype E), DQ823095 (genotype F), HE981176 (genotype G) or AB179747 (genotype H). For example, the polypeptide described herein may contain the amino acid sequences shown by SEQ ID NO: 30 and contain at its N and/or C terminus a natural flanking amino acid sequence from the pre-S1 region of the HBV genotype C. Alternatively, the OATP-binding peptide described herein may contain the amino acid sequences shown by SEQ ID NO: 30 and contain at its N and/or C terminus a natural flanking amino acid sequence from the pre-S1 region of any of the HBV genotype A, B, C, D, E, F, G and H. In some embodiments, the N-terminus and/or C-terminus of the peptides described herein may independently contain a natural flanking amino acid sequence of 1-10, e.g., 1-8, 1-5, 1-3, (including all integers within these ranges) amino acid length. For example, the OATP-binding peptide described herein may contain the amino acid sequence shown in SEQ ID NO: 30 and at its N-terminus a 10 amino acid long native flanking amino acid sequence from the HBV genotype C pre-S1 region. In other words, the OATP-binding peptide may contain amino acids 2-59 of the HBV genotype C pre-S1 region (SEQ ID NO: 25). As another example, the OATP-binding peptide described herein may contain the amino acid sequence shown in SEQ ID NO: 30 and at its N-terminus a 9 amino acid long native flanking amino acid sequence from the HBV genotype E pre-S1 region. In other words, the OATP-binding peptide may contain amino acids 13-59 of the HBV genotype C pre-S1 region and amino acids 2-11 of the HBV genotype E pre-S1 region (SEQ ID NO: 63). It will be appreciated that any of the OATP-binding peptides described can have any length of native flanking amino acid sequence extending from its N and/or C terminus, and that the resulting polypeptide retains the biology of the original polypeptide binding to OATP, especially biological activity that binds to OATP1B3, and/or OATP1B2, and/or OATP1B1, especially biological activity that binds to human OATP1B3 and/or OATP1B1. As used herein, the "variant" relative to an OATP-binding peptide also includes one or more amino acid deletions, substitutions or insertions, while retaining the biological activity of binding to OATP, especially the biological activity of binding to OATP1B3, and/or OATP1B2, and/or OATP1B1, especially the biological activity of binding to human OATP1B3 and/or human OATP1B1. The OATP-binding peptide preferably retains the amino acid corresponding to the glycine at position 13 of the HBV genotype C pre-S1 region (i.e., the N-terminal glycine of SEQ ID NO: 30). In some embodiments, a polypeptide described herein may have one or more naturally occurring mutations in the HBV pre-S1 region. For example, in some embodiments, relative to the sequence from the HBV pre-S1 region, the OATP-binding peptide described herein may have 1-30, such as 1-20, 1-10, 1-8, 1-5 or 1-3 (including all integers within these ranges) amino acids deletions, substitutions or insertions. In some embodiments, the OATP-binding peptide described herein may have 1-30, such as 1-20, 1-10, 1-8, 1-5 or 1-3 (including all integers within these ranges) amino acid deletions, substitutions or insertions relative to any amino acid sequence selected from SEQ ID NO: 23-66. In some embodiments, the OATP-binding peptide described herein may have 1-30, such as 1-20, 1-10, 1-8, 1-5 or 1-3 (including all integers within these ranges) amino acid deletions, substitutions or insertions relative to any amino acid sequence selected from SEQ ID NO: 23-66. In some embodiments, the OATP-binding peptide described herein may have 1-3 amino acid deletions, substitutions, or insertions relative to the exemplary amino acid sequence of SEQ ID NO: 30. In some embodiments, the OATP-binding peptide described herein may have 1-30, such as 1-20, 1-10, 1-8, 1-5 or 1-3 (including all integers within these ranges) amino acid deletions, substitutions or insertions at the C-terminus relative to any amino acid sequence selected from SEQ ID NO: 23-66. For example, the OATP-binding peptide may include, but is not limited to, an amino acid sequence selected from any one of SEQ ID NO: 31-35. In various embodiments, the polypeptides herein include, but are not limited

to, and at least about 30%, 40%, 50%, 60%, 70%, 80%, 85%, 90%, 95%, 96%, 97%, 98% or 99% identical polypeptides. For example, the OATP-binding peptide may contain an amino acid sequence that is at least about 30%, 40%, 50%, 60%, 70%, 80%, 85%, 90%, 95%, 96%, 97%, 98% or 99% identity to any of the exemplary sequences in SEQ ID NO: 23-66; preferably, the amino acid sequence having said identity is from any one of the hepadnaviruses, especially Is derived from a orthohepadnavirus, more preferably from a orthohepadnavirus that infects humans (HBV), including but not limited to HBV genotypes A, B, C, D, E, F, G, H and I genotypes, more preferably from HBV genotype C. For example, the OATP-binding peptide may contain an amino acid sequence that is at least about 30%, 40%, 50%, 60%, 70%, 80%, 85%, 90%, 95%, 96%, 97%, 98% or 99% identity to any of the exemplary sequences in SEQ ID NO: 30; preferably, the amino acid sequence having said identity is from any one of the hepadnaviruses, especially Is derived from a orthohepadnavirus, more preferably from a orthohepadnavirus that infects humans (HBV), including but not limited to HBV genotypes A, B, C, D, E, F, G, H and I genotypes, more preferably from HBV genotype C. The variants having a certain sequence identity with the OATP-binding peptides described herein retain one or more biological activities associated with the corresponding polypeptide, including at least the biological activity of binding to OATP, particularly to OATP1B1, and/or OATP1B2, and/or OATP1B3, especially the biological activity of binding to human OATP1B1 and/or human OATP1B3.

The hepadnavirus-derived peptide and its variants that bind to OATP have a hydrophobic group modification at the N-terminus, such as but not limited to myristic acid (myr), palmitic acid (plam), stearic acid (stearoyl), cholesterol (chol), oleic acid, linoleic acid, polyethylene glycol (PEG), arachidonic acid or other hydrophobic groups. In some embodiments, the hydrophobic group may be selected from myristic acid, palmitic acid, stearic acid, and cholesterol. In some embodiments, the hydrophobic group can be myristic acid. In some embodiments, the OATP-binding peptide described herein may contain an amino acid sequence selected from any one of SEQ ID NO: 23-66 and variants thereof, wherein the N-terminus of the OATP-binding peptide may be selected from hydrophobic group modification of myristic acid, palmitic acid, stearic acid and cholesterol. In some embodiments, the polypeptide may comprise an amino acid sequence selected from any one of SEQ ID NO: 23-66 or a variant thereof, wherein the N-terminus of the HBV entry inhibitory OATP-binding peptide can be myristoylated. In some embodiments, the OATP-binding peptide described herein may contain the amino acid sequence shown in SEQ ID NO: 30 or SEQ ID NO: 60, wherein the N-terminus of the OATP-binding peptide may be myristoylated. In some embodiments, the C-terminus of the polypeptide can be modified. C-terminal modifications may be selected from, but are not limited to, amidation (amination), prenylation, and other C-terminal modifications, or may be deleted. In some embodiments, the C-terminal modification may be amidation ($NH_2$). For example, the OATP-binding peptide may contain the amino acid sequence shown in SEQ ID NO: 30 or SEQ ID NO: 60, and its C-terminus may be amidated. In some embodiments, the N-terminus of the OATP-binding peptide can be modified with a hydrophobic group, and/or the C-terminus can be otherwise modified. In some embodiments, the N-terminus of the OATP-binding peptide can be modified with a hydrophobic group, and/or the C-terminus can be amidated. In certain embodiments, the OATP-binding peptide may contain an amino acid sequence selected from any one of SEQ ID NO: 23-66 and variants thereof, the N-terminus of which may be modified by hydrophobic groups, and/or the C-terminus may be amidated. For example, the OATP-binding peptide may contain the amino acid sequence shown in SEQ ID NO: 30 or SEQ ID NO: 60, and its N-terminus being modified by hydrophobic group of myristic acid (myr), palmitic acid (plam), stearic acid (stearoyl), cholesterol (chol), the C-terminus of which is amidated, such as the polypeptide shown in SEQ ID NO: 67-71. In some embodiments, the N-terminus of the OATP-binding peptide can be modified by a myristic acid (myr) hydrophobic group, and/or the C-terminus can be amidated. In certain embodiments, the polypeptide may comprise an amino acid sequence selected from any one of SEQ ID NO: 23-66 and variants thereof, the N-terminus of which may be modified by myristoylation, and/or the C-terminus may be amidated. For example, the OATP-binding peptide may comprise the polypeptide shown in SEQ ID NO: 30, 36-42 or 60, the N-terminus of which is modified by a hydrophobic group of myristic acid (myr) hydrophobic group, and the C-terminus of which is amidated, as shown in any of SEQ ID NO: 67, 68 and 72-78.

The OATP-binding peptides derived from hepadnavirus and their variants provided herein at least retain the biological activity of binding to OATP and can be used for the prevention and/or treatment of hepadnavirus and/or HDV infection and related diseases. In some embodiments, the OATP-binding peptide is derived from viruses including, but not limited to, Orthohepadnavirus, Avihepadnavirus, Metahepadnavirus, Herpetohepadnavirus, and other hepadnaviruses. In some embodiments, the OATP-binding peptide is derived from a species including, but not limited to, human (HBV), Woolly monkey HBV (WMHBV), capuchin monkey HBV (CMHBV), squirrel monkey, woodchuck (woodchuck hepatitis virus, WHV), arctic ground squirrel, ground squirrel (ground squirrel hepatitis virus, GSHV), tree shrews, chimpanzees, bats (Bat hepatitis virus, BHV) and other animals, as well as infecting ducks Avihepadnavirus of HBV(duck HBV, DHBV), heron (heron HBV, HHBV) and other birds. In some embodiments, the OATP-binding peptide is derived from the surface antigen of Pre-S region of HBV including, but not limited to, genotypes A, B, C, D, E, F, G, H, and I. In some embodiments, the OATP-binding peptide is derived from including, but not limited to, amino acids 13-59 of HBV genotype C Pre-S. The N-terminus of the OATP-binding peptide is modified with a hydrophobic group, and the C-terminus may be modified with a stabilizing modification or deleted. In some embodiments, the N-terminal of the OATP-binding peptide is modified with a hydrophobic group of myristic acid (myr), palmitic acid (plam), stearic acid (stearoyl), and cholesterol (chol), and the C-terminal can be amidated. In some embodiments, the N-terminus of the OATP-binding peptide can be modified by a

myristic acid (myr) hydrophobic group, the C-terminus can be amidated. The OATP-binding peptide at least retains the biological activity of binding to OATP, especially retains the biological activity of binding to OATP1B1, and/or OATP1B2 and/or OATP1B3, especially retains the biological activity of binding to human OATP1B1 and/or human OATP1B3. In some embodiments, the structure of OATP-binding peptide derived from hepadnavirus is a polypeptide represented by any one of the exemplary structures shown in SEQ ID NO: 67-78 and variants thereof; particularly including, but not limited to, polypeptides containing the exemplary structure shown in SEQ ID NO: 67 and 68 and variants thereof.

In some embodiments, the OATP-binding peptide can be used for the prevention and/or treatment of hepadnavirus and/or HDV infection and related diseases, especially for the prevention and treatment of HBV and/or HDV infection and related diseases in humans. The diseases include, but are not limited to, acute or chronic infection with HBV and/or HDV, acute viral hepatitis B and/or D, chronic viral hepatitis B and/or D, explosive viral hepatitis B and/or D, subsevere viral hepatitis B and/or D, severe viral hepatitis B and/or D, HBV and/or HDV vehicle infection, liver disease associated with HBV and/or HDV infection, liver fibrosis associated with HBV and/or HDV infection, cirrhosis associated with HBV and/or HDV infection, liver cancer associated with HBV and/or HDV infection, liver function abnormalities associated with HBV and/or HDV infection, hepatic decompensation associated with HBV and/or HDV infection, liver failure associated with HBV and/or HDV infection and other related diseases. The method of treating or preventing liver disease described herein is a method of treating or preventing HBV and/or HDV infection and related diseases, including but not limited to administering a therapeutically effective amount or a prophylactically effective amount of the hepadnavirus-derived peptide to a subject in need, prevent or reduce the interaction between hepadnavirus and/or HDV and OATP, prevent or inhibit hepadnavirus and/or HDV infection. For example, but not limited to, in the case of accidental exposure to HBV and/or HDV viruses, the virus can be prevented from entering liver cells through the binding of the OATP-binding peptide to OATP by administering the OATP-binding peptide described herein, thereby preventing Viral infection and disease prevention. In some embodiments, provided herein are methods for preventing HBV and/or HDV virus infection of liver tissue or liver cells and/or preventing HBV and/or HDV transmission between liver cells, the method comprising administering an effective amount to a subject in need thereof Pharmaceutical compositions of OATP-binding peptides derived from HBVPre-S1. In some embodiments, the disease is a liver disease associated with HBV and/or HDV viral infection. The various OATP-binding peptides derived from HBV Pre-S1 described herein can be used to bind to OATP, thereby preventing the transport of viruses into cells via OATP, avoiding or reducing viral infection of liver cells, and preventing the occurrence and/or development of disease. In some embodiments, the prevent invention also provided herein is the use of OATP-binding peptides in the manufacture of drugs for treating or preventing liver disease, particularly liver disease associated with hepatitis B virus or hepatitis D virus infection, and in the manufacture of drugs for preventing HBV and/or HDV virus from infecting liver tissues or liver cells and/or preventing the transmission of said viruses between liver cells. In some embodiments, the OATP-binding peptides described herein can be used in combination with HBV and/or HDV treatment or prevention drugs, including but not limited to, nuclear glycoside (acid) analogues (including but not limited to lamivudine, telbivudine, entecavir, tenofovir disoproxil, adefovir dipivoxil, clavudine, tenofovir disoproxil disoproxil and other nuclear Glycoside (acid) analogues), interferons (including but not limited to ordinary interferons (including but not limited to interferon alpha-2a, alpha-2b and other types of interferons), long-acting interferons, PEG interferons (including but not limited to Not limited to PEG interferon alpha-2a, alpha-2b and other types of PEG interferon) and other interferons), nucleocapsid inhibitors, RNA interference, immunomodulators and other agents for preventing or treating HBV or HDV infection, and Combinations of these agents are recommended.

HBV and/or HDV infection model and its establishment method

The prevent invention also provides a method for establishing a cell or animal model susceptible to HBV and/or HDV, wherein, transfects the exogenous SLCO gene into the cell or animal, and/or expresses the exogenous OATP protein in the cell or animal, and the animals, cells or animals that are originally not susceptible to HBV and/or HDV or are not easily infected, acquire susceptibility to HBV and/or HDV after being transfected with the SLCO gene and/or expressing exogenous OATP protein. The present invention also includes drugs and lead compounds obtained by screening using the above-mentioned HBV and/or HDV infected cells and/or animal models and the drugs can be used to treat and/or prevent HBV and/or HDV infection. The method for establishing a cell or animal model susceptible to HBV and/or HDV infection includes constructing cells or animals that are transfected with exogenous SLCO genes and/or express exogenous OATP proteins, and obtain HBV and/or HDV susceptible cell and/or animal models. The cells or animals are not susceptible to HBV and/or HDV or are not easily infected before they are transfected with the exogenous SLCO gene and/or express the exogenous OATP protein.

The exogenous SLCO gene and exogenous OATP protein are derived from animals, including but are not limited to, mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans,

chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some embodiments herein, the exogenous SLCO gene and OATP protein are derived from mammals or birds. In some preferred embodiments herein, the exogenous SLCO gene and OATP protein are derived from humans, chimpanzees, bonobos, tree shrews, marmots, arctic ground squirrels, ground squirrels, bats, woolly monkeys, capuchin monkeys, and squirrel monkeys. In some embodiments herein, the exogenous SLCO gene and OATP protein are derived from humans, chimpanzees, and tree shrews. In some further preferred embodiments herein, the transfected exogenous SLCO gene and OATP protein derived from humans.

The exogenous SLCO genes include, but are not limited to, SLCO1, SLCO2, SLCO3, SLCO4, SLCO5, SLCO6, SLCO7, SLCO8, SLCO9, SLCO10, SLCO11 and other SLCO gene families. In some embodiments, the SLCO genes include, but are not limited to, the SLCO gene family expressed in hepatocytes. In some embodiments, the SLCO genes include, but are not limited to, the SLCO1B subfamily. In some preferred embodiments, the SLCO genes include, but are not limited to, the SLCO1B subfamily expressed in hepatocytes. In some preferred embodiments, the SLCO genes include, but are not limited to, SLCO1B3, SLCO1B1, SLCO1B2, and other SLCO1B gene subfamily members. In some embodiments herein, the transfected exogenous SLCO gene is SLCO1B3 and/or SLCO1B1 and/or SLCO1B2, or a pairwise combination thereof, or SLCO1B3 and SLCO1B1 and SLCO1B2. In some embodiments herein, the transfected exogenous SLCO1B3 gene originates from, but is not limited to, humans (for example sequences, see SEQ ID NO: 3, NCBI Reference Sequence: NM_019844), chimpanzees (for example sequences, see genebank Gene ID: 749254 ), tree shrews (for example sequences, see genebank GeneID: 102476084), Macaca fascicularis (for example sequences, see genebank GeneID: 102136593), arctic ground squirrels (genebank GeneID: 113185010), pigs (for example sequences, see genebank GeneID: 100620829), dogs (For example sequences, see genebank GeneID: 403519) and its variants. And the SLCO1B3 gene sequences and variants derived from the other animals mentioned above, including but not limited to gene bank NC_050564, NC_013676, NW_017869963, NW_006501111, NW_022196903, NW_003613944, NW_004801640, NW_004949097, NW_015351262, NW_024404945, NW_020539983, NW_023144717, NW_006711288, NW_019154091, NW_004569192, NC_044309, NW_020339853, NC_018729, NW_017619867, NW_023365217, NC_051831, NW_020656135, NW_022631003, NC_045619, NW_004451484, NC_043709, NW_020356453, NC_048233, NW_020312893, NW_020998839, NW_006383153, NW_023270504, NC_032654, NC_010447, NW_006781351, NW_023601653, NW_011889225, NC_009149, NW_007675154, NW_023458025, NW_006159939, NW_023666069, NW_012009027, NC_022282, NC_041764, NW_012010756, NC_044984, NC_037679, NW_012109719, NW_022681456, NW_016809129, NC_044558, NC_045443, NC_044614, NC_048251, NC_036891, NC_000012, NC_036915, NC_044403, NW_022611650, NW_024100944, NC_033666, NW_006408616 and other SLCO1B3 gene sequences. In some embodiments herein, the transfected exogenous SLCO1B1 gene includes, but is not limited to, the SLCO1B1 gene sequence derived from humans (SEQ ID NO: 6, genebank Gene ID: 10599), bonobos (genebank Gene ID: 100976318), Macaca fascicularis (genebank GeneID: 102137347) and other animals mentioned above, including but not limited to gene bank NC_000012, NC_041764, NC_036891, NC_022282, NC_048391, NW_012009027, NW_012014011, NC_037679, NW_012109719, NC_044614, NC_048251, NC_036915, NW_006399825, NW_023666069, NW_016809129, NC_044558, NC_044403, NW_022611650, NW_012112030, NC_045443, NW_006399825 and other SLCO1B1 gene sequences and variants. In some preferred embodiments, the transfected exogenous SLCO gene is derived from human SLCO1B3 (for example sequences, see SEQ ID NO: 3, NCBI Reference Sequence: NM_019844), chimpanzee SLCO1B3 (for example sequences, see genebank Gene ID: 749254), tree shrew SLCO1B3 (for example sequences, see genebank GeneID: 102476084), and/or human SLCO1B1 (for example sequences, see SEQ ID NO: 6, genebank Gene ID: 10599), chimpanzee or tree shrew. In some further preferred embodiments, the transfected exogenous SLCO gene is derived from human SLCO1B3 (exemplary sequence, see SEQ ID NO: 3, NCBI Reference Sequence: NM_019844) and/or human SLCO1B1 (exemplary sequence, see SEQ ID NO: 6, genebank Gene ID: 10599). The SLCO gene includes but is not limited to SLCO complete genome sequence and its variants, coding sequence (CDS) and its variants, open reading frame sequence (ORF) and its variants and other forms of genetic coding sequences. In some embodiments herein, the transfected exogenous SLCO gene also includes variants, fragments and homologous sequences of the above exogenous SLCO gene sequence. This invention also includes vehicles carrying the above-mentioned SLCO gene and its variants, fragments, and homologous sequences, including but not limited to DNA, RNA, plasmids, viruses, and other vehicles. The methods of transfecting exogenous SLCO genes include but are not limited to liposomes, cationic polypeptides, electrotransfection, viral vectors, transgenes, gene insertion, gene editing and other methods that can transfect cells or animals with the exogenous SLCO genes, and the method is technically characterized by transferring

DNA or RNA encoding the SLCO gene into cells or animals.

The exogenous OATP proteins include, but are not limited to, OATP1, OATP2, OATP3, OATP4, OATP5, OATP6, OATP7, OATP8, OATP9, OATP10, OATP11 and other OATP families. In some embodiments, the OATP family includes, but is not limited to, the OATP family expressed in hepatocytes. In some embodiments, the OATP includes, but is not limited to, the OATP1B subfamily. In some preferred embodiments, the OATP includes, but is not limited to, the OATP1B subfamily expressed in hepatocytes. In some preferred embodiments, the OATPs include, but are not limited to, OATP1B3, OATP1B1, OATP1B2, and other OATP1B subfamily members. In some embodiments herein, the transfected exogenous OATP is OATP1B3 and/or OATP1B1 and/or OATP1B2, or a pairwise combination thereof, or OATP1B3 and OATP1B1 and OATP1B2. In some embodiments herein, the expressed exogenous OATP1B3 protein originates from, including but not limited to, human (an exemplary amino acid sequence is shown in SEQ ID NO: 1, an exemplary coding sequence is shown in SEQ ID NO: 2, NCBI Reference Sequence: NM_019844 ), chimpanzees (exemplary amino acid sequences can be found in NCBI Reference Sequence: XM_016923821), tree shrews (exemplary amino acid sequences can be found in NCBI Reference Sequence:XM_006155085), macaca fascicularis (exemplary amino acid sequences can be found in NCBI reference Sequence: NM_001283191), arctic ground squirrel (exemplary amino acid sequence can be found in NCBI Reference Sequence: XM_026391932), pigs (exemplary amino acid sequences can be found in NCBI Reference Sequence: NM_001315607), dogs (exemplary amino acid sequence can be found in NCBI Reference Sequence: NM_001166047) and other animals mentioned above, and their variants. In some embodiments herein, the expressed exogenous OATP1B1 protein and its variant originates from, including but not limited to, human (an exemplary amino acid sequence shown in SEQ ID NO: 4, an exemplary coding sequence shown in SEQ ID NO: 5, NCBI Reference Sequence: NM_006446), bonobos (exemplary amino acid sequences can be found in NCBI Reference Sequence: XM_003828901), macaca fascicularis (exemplary amino acid sequences can be found in NCBI Reference Sequence: NM_ 001284540) and other animals mentioned above. In some preferred embodiments, the expressed exogenous OATP protein is derived from human OATP1B3 (an exemplary amino acid sequence can be found in SEQ ID NO: 1, an exemplary nucleic acid coding sequence can be found in SEQ ID NO: 2, NCBI Reference Sequence: NM_019844), chimpanzee OATP1B3 (exemplary amino acid sequence can be found in NCBI Reference Sequence: XM_016923821), tree shrew OATP1B3 (exemplary amino acid sequence can be found in NCBI Reference Sequence: XM_006155085), and/or human OATP1B1 (exemplary amino acid sequence can be found in NCBI Reference Sequence: NM_006446), chimpanzee OATP1B1 or tree shrew OATP1B1. In some further preferred embodiments, the expressed exogenous OATP protein is derived from human OATP1B3 (exemplary amino acid sequence is shown in SEQ ID NO:1, exemplary nucleic acid coding sequence is shown in SEQ ID NO:2, NCBI Reference Sequence: NM_019844) and/or human OATP1B1 (exemplary amino acid sequence can be seen in SEQ ID NO:4, exemplary nucleic acid coding sequence can be seen in SEQ ID NO:5, NCBI Reference Sequence: NM_006446). In some embodiments herein, the expressed exogenous OATP protein also includes variants, fragments and homologous sequences of the amino acid sequence of the above-mentioned exogenous OATP protein, variants, fragments and homologous sequences of the nucleic acid coding sequence. The method of expressing exogenous OATP protein includes, but is not limited to, incubating the OATP protein or recombination with cells, or performing cell fusion with cells expressing the OATP protein, or coupling the OATP protein to the cell surface, or Introducing DNA or RNA fragments or vectors containing the nucleic acid encoding the OATP protein into cells or animals, and other methods that allow cells or animals to express the exogenous OATP protein.

The cells or animal models susceptible to HBV and/or HDV and their establishment methods can be transfected with the exogenous SLCO gene alone, or can be transfected with the exogenous SLC10A1 gene at the same time; or the exogenous OATP protein can be expressed alone, or can be expressed with exogenous NTCP protein at the same time. The exogenous SLCOA1 gene and exogenous NTCP protein are derived from, including but not limited to, mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old-world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some embodiments herein, the exogenous SLC10A1 gene and NTCP protein are derived from mammals or birds. In some preferred embodiments of this invention, the exogenous SLC10A1 gene and NTCP protein are derived from humans, chimpanzees, bonobos, tree shrews, woodchuck, arctic ground squirrels, ground squirrels, bats, woolly monkeys, capuchin monkeys, Squirrel monkeys, rats and mice. In some embodiments herein, the exogenous SLC10A1 gene and NTCP protein are derived from humans, chimpanzees, and tree shrews. In some further preferred embodiments

herein, the transfected exogenous SLC10A1 gene and NTCP protein derived from humans. In some embodiments herein, the exogenous SLC10A1 gene that is transfected concomitantly with the exogenous SLCO gene is derived from includes, but is not limited to, human (for example sequence, SEQ ID NO:79, genebank GeneID: 6554),chimpanzee (for example, genebank GeneID: 453001), tree shrew (for example, genebank GeneID: 102494), bonobo (for example, genebank GeneID: 100985481), capuchin (for example, genebank GeneID: 116528519), squirrel monkey (for example, genebank GeneID: 101045215), macaca fascicularis (for example, genebank GeneID: 102138916), arctic ground squirrel (for example, genebank GeneID: 113180315), ground squirrel (for example, genebank GeneID: 113180315), rats (for example, genebank GeneID. 116905231), mice (for example, genebank GeneID: 20493), pig (for example, genebank GeneID: 100153302), dog (for example, genebank GeneID: 480372), as well as the SLC10A1 gene and variants thereof from the other animals mentioned above, including but not limited to the following example sequences: gene_bank_NC_000014.9, NC_051341.1, NC_000078.7, NC_041760.1, NC_037337.1, NC_007131.7, NC_030684.2, NC_036893.1, NC_051812.1, NC_048392.1, NC_008801.1, NW_004569144.1, NC_044068.1, NW_024429186.1, NC_048231.1, NC_010449.5, NW_005 395103.1, NW_017364105.1, NC_009167. 3. NW_019174280.1, NC_050533.1, NC_035899.1, NC_019880.2, NW_006890081.1, NW_005819088.1, NC_023185.1, NC_045166.1, NC_030440.1, NC_049537.1, NC_047591.1, NC_036849.1, NC_046375.1, NC_024351.1, NC_036457.1, NC_045686.1, NW_005179644.1, NC_039316.1, NC_041348.1, NC_044208.1, NW_017859650.1, NC_051245.1, NW_007 281450.1, NW_017713697.1, NW_017728947. 1. NC_048605.1, NW_020451983.1, NC_051776.1, NW_023336592.1, NC_048897.1, NW_009952265.1, NW_004973262.1, NC_034414.1, NC_052536.1, NW_022 044854.1, NC_031774.1, NW_014650483.1, NW_009152437.1, NW_008795283.1, NW_008824949.1, NC_041728.1, NC_045427.1, NC_050580.1, NW_004491405.1, NW_006804717.1, NW_022103915.1, N W_006438074.1, NW_023416306.1, NW_023537909.1, NW_020093309. 1. NW_022436999.1, NW_012007369.1, NC_022278.1, NW_012015021.1, NC_044982.1, NC_037675.1, NW_012106931.1, NC_044616.1, NC_048253.1, NC_0369 17.1, NW_020356506.1, NC_018728.3, NW_017619866.1, NW_020339215.1, NC_045600.1, NW_004450335.1, NW_023399451.1, NW_006383727.1, NW_023270825.1, NW_022589710.1, NW_02213 4863.1, NW_004438415.1, NC_047035.1, NW_022098036.1, NC_041224. 1. NC_045786.1, NW_003573429.1, NW_014638379.1, NW_007676261.1, NW_011515408.1, NC_044516.1, NW_018335685.1, NC_032659.1, NC_030817.1, NC_ 040258.1, NW_023435897.1, NC_050555.1, NW_003159375.1, NW_015351211.1, NW_020539924.1, NW_012267218.1, NW_003613686.1, NC_051075.1, NW_018657848.1, NC_034581.1, NC_034596.1, NC _046160.1, NT_176399.1, NW_004524583.1, NW_004624734. 1. NW_005857041.1, NW_023425344.1, NW_024100921.1, NW_003338865.1, NW_006399787.1, NW_015150892.1, NW_024425008.1, NW_007370659.1, N W_018734275.1, NC_044498.1, NW_009900513.1, NW_020455250.1, NC_040085.1, NW_021964165.1, NC_033662.1, NW_003852510.1, NC_042286.1, NC_048301.1, NC_040242.1, NW_020834750.1, NW_004955466.1, NW_020312 827.1, NW_020998607.1, NW_022196912.1, NC_045618. 1. NC_051448.1, NC_043708.1, NW_018503592.1, NW_018344057.1, NW_005842135.1, NC_050456.1, NW_021703778.1, NC_045764.1, NW_004545910.1, NC_ 051214.1, NW_024408640.1, NW_011494811.1, NW_018127943.1, NW_005081537.1, NC_052956.1, NW_006799940.1, NW_015112671.1, NW_015095634.1, NW_005054301.1, NW_009646445.1, NW_02360 1104.1, NW_023416313.1, NW_004504313.1, NW_017870438.1, NW_010972853. 1. NW_022681468.1, NC_045750.1, NW_009632203.1, NW_022665528.1, NC_041378.1, NW_018114435.1, NW_008609214.1, NW_005871133.1, NW_023425 404.1, NC_046289.1, NC_045004.1, NC_021677.1, NW_023666053.1, NW_023044993.1, NC_044308.2, NW_016820119.1, NC_044553.1, NC_044381.1, NC_046632.1, NC_041312.1, NW_006712282.1, NW_015505 194.1, NW_022631221.1, NC_022009.1, NC_046312. 1. NW_019029691.1, NW_019372207.1, NW_022611658.1, NC_050482.1, NW_008239553.1, NC_044575.1, NC_037555.1, NC_040903.2, NW_020837954.1, NC_ 046226.1, NW_023144640.1, NW_010582752.1, NW_017729025.1, NW_010537981.1, NW_010771612.1, NW_018150791.1, NW_022641422.1, NW_017805708.1, NW_005369967.1, NC_041820.1, NW_02065 6171.1, NW_006781042.1, NW_010035470.1, NW_009002855.1, NW_011888796. 1. NW_023455365.1, NW_004567114.1, NW_007577740.1, NW_015164181.1, NW_019776417.1, NC_040901.1, NC_042532.1, NW_010423023.1, NW_009928 727.1, NW_005087567.1,NC_043759.1, NW_006408597.1, NW_009874831.1, NW_009258894.1, NW_023528387.1, NW_020799101.1, NW_015090839.1, NW_014006077.1, NC_046656 .1, NC_041043.1, NC_040021.1, NC_045563.1, NC_044005. 1. NW_006288624.1, NW_006501064.1, NW_010112985.1, NC_024307.1, NW_006209531.1, NW_018030485.1, NC_047090.1, NC_050191.1, NW_023365123. 1. NW_015505036.1, NC_042571.1, NC_041720.1, NW_020448008.1, NW_006727797.1, NW_012116265.1, NC_043979.1, NW_012150840.1, NW_013185668.1, NC_045964.1, NC_045701.1, NC_041066.1, NW_00 9270392.1, NW_007729665.1, NW_021604585.1, NC_044003. 1. NC_045439.1, NC_046335.1, NW_008348666.1, NC_045481.1, NW_006921675.1, NW_020848012.1, NC_044039.1, NW_015553274.1, NW_019715876.1, NW _020172672.1, NC_044325.1, NW_019525097.1, NW_007014397.1, NC_046358.1, NW_020664447.1, NW_016107905.1, NC_047530.1, NW_004454173.1, NW_004443946.1, NW_019154104.1 and other SLC10A1 gene sequences and their variants body. In some preferred embodiments, the transfected exogenous SLC10A1 gene is derived

from human (for example sequences, see SEQ ID NO: 79, genebank GeneID: 6554), chimpanzee (for example sequences, see genebank Gene ID: 453001), tree shrew (for example sequences, see genebank GeneID: 102494). In some preferred embodiments, the transfected exogenous SLC10A1 gene is derived from human (for example sequences, see SEQ ID NO: 79, genebank GeneID: 6554). The SLC10A1 gene includes but is not limited to SLC10A1 complete genome sequence and its variants, coding sequence (CDS) and its variants, open reading frame sequence (ORF) and its variants and other forms of genetic coding sequences. In some embodiments herein, the transfected exogenous SLC10A1 gene also includes variants, fragments and homologous sequences of the above exogenous SLC10A1 gene sequence. In some embodiments herein, the transfected exogenous SLC10A1 gene also includes variants, fragments and homologous sequences of the above exogenous SLC10A1 gene sequence. This invention also includes vehicles carrying the above-mentioned SLC10A1 gene and its variants, fragments, and homologous sequences, including but not limited to DNA, RNA, plasmids, viruses, and other vehicles. The methods of transfecting exogenous SLC10A1 genes include but are not limited to liposomes, cationic polypeptides, electrotransfection, viral vectors, transgenes, gene insertion, gene editing and other methods that can transfect cells or animals with the exogenous SLC10A1 genes, and the method is technically characterized by transferring DNA or RNA encoding the SLC10A1 gene into cells or animals.

In some embodiments herein, the exogenous NTCP protein expressed simultaneously with the exogenous OATP protein is derived from, including but not limited to, human (exemplary amino acid sequence is shown in SEQ ID NO:7, exemplary nucleic acid coding sequence is shown in SEQ ID NO:80, NCBI Reference Sequence: NM_003049), chimpanzee (exemplary amino acid sequence can be found in NCBI Reference Sequence: XM_510035), tree shrew (exemplary amino acid sequence can be found in NCBI Reference Sequence: XM_006171503), bonobo (exemplary amino acid sequence can be found in NCBI Reference Sequence: XM_003824101), capuchin monkey (NCBI Reference Sequence: XM_032245781), squirrel monkey (NCBI Reference Sequence: XM_003924480), macaca fascicularis (exemplary amino acid sequence can be found in NCBI Reference Sequence: NM_001283323), arctic ground squirrel (exemplary amino acid sequence can be found in NCBI Reference Sequence: XM_026385176), ground squirrel (exemplary amino acid sequence can be found in NCBI Reference Sequence: XM_005322843), rats (exemplary amino acid sequence can be found in NCBI Reference Sequence: XM_032908052), mice (exemplary amino acid sequence can be found in NCBI Reference Sequence: NM_001177561), pig (exemplary amino acid sequence can be found in NCBI Reference Sequence: XM_001927695), dog (exemplary amino acid sequence can be found in NCBI Reference Sequence: XM_537494), as well as NTCP and its variants derived from the other animals mentioned above.

In some preferred embodiments, the expressed exogenous NTCP protein is derived from human (exemplary amino acid sequence is shown in SEQ ID NO:7, exemplary nucleic acid coding sequence is shown in SEQ ID NO:80, NCBI Reference Sequence: NM_003049), chimpanzee (exemplary amino acid sequence can be found in NCBI Reference Sequence: XM_510035), tree shrew (exemplary amino acid sequence can be found in NCBI Reference Sequence: XM_006171503). In some preferred embodiments, the expressed exogenous NTCP protein is derived from human (exemplary amino acid sequence is shown in SEQ ID NO:7, exemplary nucleic acid coding sequence is shown in SEQ ID NO:80, NCBI Reference Sequence: NM_003049). In some embodiments herein, the expressed exogenous NTCP protein further comprises variants, fragments, and homologous sequences of the amino acid sequence of the exogenous NTCP protein. The method of expressing exogenous NTCP protein includes, but is not limited to, incubating the NTCP protein with cells, or coupling the NTCP protein to the cell surface, or using DNA or RNA fragments containing coding sequences encoding the NTCP protein, or introducing the vehicle into cells or animals, and other methods can allow cells or animals to express the exogenous NTCP protein.

In one or more embodiments, said cells transfected with exogenous SLCO gene and/or expressing OATP protein include, but are not limited to, human liver cell lines, mice liver cell lines, rats liver cell lines, and primary liver cell lines or liver cell lines of other animal origin. Human liver cell lines include, but are not limited to, HepG2, Huh7, Hep RG, SMMC-7721, Bel-7402, MHCC97, Hep3B, PLC/PRF/5, HepG2.2.15, Hep AD38, HL-7702, QSG-7701, L-02, HL-7702BaPT, HCC- DJ711, THLE-3, HC-04 and other liver-derived cell lines. mice-derived liver lines include, but are not limited to, GHA1, AML12, BNL CL.2, NCTC 1469, NCTC 1469, FL83B, H2.35, Hepa1-6, Hep56.1D mice-derived hepatocytes. rats-derived liver lines include, but are not limited to, IAR20, H-4-II-E, H4-II-E-C3, BRL, BRL 3A, TC5123 rats-derived hepatocytes. Woodchuck-derived liver lines include, but are not limited to, WCH-17 and other woodchuck-derived hepatocytes. Tree shrew-derived liver lines include, but are not limited to, ITH48, ITH6.1 and other tree shrew-derived hepatocytes. Rabbit-derived liver lines include, but are not limited to, RNH/HL-034 and other rabbit-derived hepatocytes. Pig-derived liver lines include, but are not limited to, PNH/HL-059 and other pig-derived hepatocytes. This invention also includes liver cells from other animals. In some preferred embodiments, the cells are selected from, but are not limited to, the human liver cell lines HepG2, Huh7, Hep RG or the mice liver cell lines Hepa1-6, Hep56.1D or the rat liver cell line TC5123. In some further preferred embodiments, cells transfected with exogenous SLCO genes and/or expressing exogenous OATP proteins include, but are not limited to, human liver cell lines HepG2 or Huh7. In one or more embodiments, the animals transfected with exogenous SLCO gene and/or expressing OATP protein include, but are not limited to, non-human mammals, birds and other animals. Mammals include, but are not limited to, mice, rats, dog,

monkey, tree shrew, pig, rabbit, bonobo, tree shrew and other mammals. Birds include, but are not limited to, duck, heron, and other birds. Other animals include bat and other animals. mice include, but are not limited to, C57BL (including, but not limited to, C57BL/6, C57BL/10 and other strains), BALB/c (including, but not limited to, BALB/cd, BALB/cJ, BALB/cAnN and other strains), C3H (including, but not limited to, C3H/Bi, C3H/He, C3H/HeJ, C3H/St, C3HeB/FeJ, C3H/DiSn, C3H/Sf, C3H/Hel, and other strains), DBA/2 (including, but not limited to, DBA/2, DBA/2J, and other strains), CBA (including, but not limited to, CBA/Br, CBA/Ca, CBA/J, CBA/st, CBA/H, and other strains), A (including, but not limited to, A/ He, A/J, A/WySN, and other strains), AKR (including, but not limited to, AKR/A, AKR/J, AKR/N, and other strains), Kunming mice, golden gopher, NIH albino mice, ICR mice, Webster, LACA, and other mice. rats include, but are not limited to, Wistar, SD, ACI, F344, LEW, LOU/CN, LOU/MN, SHR, WKY, GH, Long-Evans, Brown Norway, and other ratss. Dogs include, but are not limited to, Beagles, Chinese Garden Dogs and other dogs. Monkeys include, but are not limited to, macaca fascicularis, rhesus monkeys, red-faced monkeys, bear monkeys, and other monkeys. Rabbits include, but are not limited to, Chinese native rabbits, golden rabbits, big-eared white rabbits, New Zealand white rabbits, Silver Fox, Himalayan albio rabbits, and Angora rabbits., Belgian Hare, Lop, California, Chekered Giant, Danish White Rabbit, West German Longhair Rabbit and other rabbits. In some embodiments, animals transfected with exogenous SLCO genes and/or expressing exogenous OATP proteins include, but are not limited to, mice and rats. In a further preferred embodiment, animals transfected with exogenous SLCO genes and/or expressing exogenous OATP proteins include but are not limited to C57BL mice, BALB/c mice, SD rats, and Wistar rats.

The present invention also includes lead compounds or drugs for the treatment and/or prevention of HBV and/or HDV infection and related diseases screened by use of the above-mentioned HBV and/or HDV susceptible cells and/or animal models, including but not limited to small molecule drugs, proteins, peptides, antibodies, polyRNA, polyDNA and other drugs. The lead compound or drug can be used for including but not limited to preventing or inhibiting HBV and/or HDV infection, preventing or inhibiting HBV and/or HDV replication, clearing HBV and/or HDV infection, preventing or inhibiting HBV and/or HDV releases, activates and/or modulates HBV and/or HDV-specific immune responses, and other methods for treating and/or preventing HBV and/or HDV infection and related diseases. In some embodiments herein, a library of agents is screened using HBV and/or HDV susceptible cells and/or animal models described herein to obtain lead compounds or drugs that can be used for the treatment and/or prevention of HBV and/or HDV infections and related diseases, the libraries of agents include, but are not limited to, libraries of small molecule compounds, libraries of proteins, libraries of antibodies, libraries of peptides, libraries of natural products, and other libraries of agents or lead structure libraries. In some embodiments herein, HBV and/or HDV susceptible cells and/or animal models described herein are used to obtain drug action targets of the life cycle of HBV and/or HDV including infection, replication, and progeny virus release. Design small molecule drugs, proteins, peptides, antibodies, polyRNA, polyDNA and other drugs based on the target, or screen small molecule compound libraries, protein libraries, antibody libraries, peptide libraries, and natural products based on the target library and other pharmaceutical or lead structure libraries to obtain lead compounds or drugs that can be used to treat and/or prevent HBV and/or HDV infection and related diseases.

A drug delivery method and vehicle targeting OATP

The prevent invention provides a method and vehicle for delivery of a drug or group, said method performing drug or group delivery by targeting or binding an OATP, said vehicle retaining at least the biological activity of targeting or binding the OATP and delivering the carried drug or group into a cell or organ.

The OATP targeted or bound by the delivery methods and vehicles include, but are not limited to, OATP of mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some embodiments herein, the OATP targeted by the vehicle is derived from humans or birds. In some preferred embodiments herein, the OATP targeted by the vehicle is derived from humans, chimpanzees, bonobos, tree shrews, marmots, arctic ground squirrels, ground squirrels, bats, woolly monkeys, capuchin monkeys, and squirrel monkeys. In some preferred embodiments herein, the vehicles targeting OATP is derived from humans, chimpanzees or tree shrews. In some preferred embodiments herein, vehicles targeting OATP is human OATP.

The delivery methods and vehicles targeting OATP including, but are not limited to, OATP 1, OATP2, OATP3,

OATP4, OATP5, OATP6, OATP7, OATP8, OATP9, OATP10, OATP11 and other OATP families. In some embodiments, the delivery methods and vehicles targeting OATP includes, but are not limited to, the OATP family expressed in hepatocytes. In some embodiments, the delivery methods and vehicles targeting OATP includes, but is not limited to, the OATP family expressed in hepatocytes. In some embodiments, the delivery methods and vehicles targeting OATP includes, but are not limited to, the OATP1B subfamily expressed in hepatocytes. In some preferred embodiments, the delivery methods and vehicles targeting OATP include, but are not limited to, OATP1B3, OATP1B1, OATP1B2, and other OATP1B subfamily members. In some embodiments herein, the delivery methods and vehicles targeting OATP are OATP1B3 and/or OATP1B1 and/or OATP1B2, or a pairwise combination thereof, or OATP1B3 and OATP1B1 and OATP1B2. In some embodiments herein, the delivery methods and vehicles targeting OATP, including but are not limited to, human OATP1B3 (an exemplary amino acid sequence is shown in SEQ ID NO: 1, an exemplary coding sequence is shown in SEQ ID NO: 2, NCBI Reference Sequence: NM_019844 ), chimpanzees OATP1B3 (exemplary amino acid sequences can be found in NCBI Reference Sequence: XM_016923821), tree shrews OATP1B3 (exemplary amino acid sequences can be found in NCBI Reference Sequence:XM_006155085), macaca fascicularis OATP1B3 (exemplary amino acid sequences can be found in NCBI Reference Sequence: NM_001283191), arctic ground squirrel OATP1B3 (exemplary amino acid sequence can be found in NCBI Reference Sequence: XM_026391932), pigs OATP1B3 (exemplary amino acid sequences can be found in NCBI Reference Sequence: NM_001315607), dogs OATP1B3 (exemplary amino acid sequence can be found in NCBI Reference Sequence: NM_001166047) and other OATP1B3 of animals mentioned above. In some embodiments herein, the delivery methods and vehicles targeting OATP, including but are not limited to, human OATP1B1 (exemplary amino acid sequence is shown in SEQ ID NO: 5, NCBI Reference Sequence: NM_006446 ), bonobo OATP1B1 (exemplary amino acid sequences can be found in NCBI Reference Sequence: XM _003828901), macaca fascicularis OATP1B1 (exemplary amino acid sequences can be found in NCBI Reference Sequence: NM_ 001284540), and other OATP1B1 of animals mentioned above. In some preferred embodiments, the delivery methods and vehicles targeting OATP is human OATP1B3 (an exemplary amino acid sequence is shown in SEQ ID NO: 1, NCBI Reference Sequence: NM_019844), chimpanzee OATP1B3 (exemplary amino acid sequence can be found in NCBI Reference Sequence: XM_016923821), tree shrew OATP1B3 (exemplary amino acid sequence can be found in NCBI Reference Sequence: XM_006155085), and/or human OATP1B1 (exemplary amino acid sequence can be found in NCBI Reference Sequence: NM_006446), chimpanzee OATP1B1 or tree shrew OATP1B1. In some further preferred embodiments, the delivery methods and vehicles targeting OATP is human OATP1B3 (an exemplary amino acid sequence is shown in SEQ ID NO: 1, NCBI Reference Sequence: NM_019844) and/or human OATP1B1 (exemplary amino acid sequence can be found in NCBI Reference Sequence: NM_006446). In some embodiments herein, the delivery methods and vehicles targeting OATP also includes variants, fragments, and homologous sequences of amino acid sequences, and variants, fragments, and homologous sequences of nucleic acid coding sequences of the above-mentioned OATP protein.

The delivery methods and vehicles can target or bind OATP alone, or can target or bind both OATP and NTCP. The delivery methods and vehicles targeting NTCP include, but are not limited to, NTCP of mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora.

In some embodiments herein, the delivery methods and vehicles simultaneously incorporating NTCP is NCTP of mammals or birds. In some preferred embodiments of this invention, the delivery methods and vehicles simultaneously incorporating NTCP are derived from humans, chimpanzees, bonobos, tree shrews, woodchuck, arctic ground squirrels, ground squirrels, bats, woolly monkeys, capuchin monkeys, Squirrel monkeys, rats and mice. In some preferred embodiments herein, the delivery methods and vehicles simultaneously incorporating NTCP is NCTP of human, chimpanzee or tree shrew. In some preferred embodiments herein, the delivery methods and vehicles simultaneously incorporating NTCP is human NCTP. In some embodiments herein, said OATP-targeted drug delivery method and OATP-targeted delivery vehicle while incorporating an NTCP is a NTCP that includes, but is not limited to human NTCP (exemplary amino acid sequence is shown in SEQ ID NO:7, NCBI Reference Sequence: NM_003049), chimpanzee NTCP (exemplary amino acid sequence can be found in NCBI Reference Sequence: XM_510035), tree shrew NTCP (exemplary amino acid sequence can be found in NCBI Reference Sequence: XM_006171503), bonobo NTCP (exemplary amino acid sequence can be found in NCBI Reference Sequence: XM_003824101), capuchin monkey NTCP (NCBI Reference Sequence: XM_032245781), squirrel monkey NTCP (NCBI Reference Sequence:

XM_003924480), macaca fascicularis NTCP (exemplary amino acid sequence can be found in NCBI Reference Sequence: NM_001283323), arctic ground squirrel NTCP (exemplary amino acid sequence can be found in NCBI Reference Sequence: XM_026385176), ground squirrel NTCP (exemplary amino acid sequence can be found in NCBI Reference Sequence: XM_005322843), rats NTCP (exemplary amino acid sequence can be found in NCBI Reference Sequence: XM_032908052), mice NTCP (exemplary amino acid sequence can be found in NCBI Reference Sequence: NM_001177561), pig NTCP (exemplary amino acid sequence can be found in NCBI Reference Sequence: XM_001927695), dog NTCP (exemplary amino acid sequence can be found in NCBI Reference Sequence: XM_537494), as well as NTCP and its variants derived from the other animals mentioned above, which includes, but is not limited to, the following example sequences: NC_000014.9, NC_051341.1, NC_000078.7, NC_041760.1, NC_037337.1, NC_007131.7, NC_030684.2, NC_036893.1, NC_051812.1, NC_048392.1, NC_008801.1, NW_004569144.1, NC_044068.1, NW_024429186.1, NC_048231.1, NC_010449.5, NW_005395103.1, NW_017364105.1, NC_009167.3, NW_019174280.1, NC_050533.1, NC_035899.1, NC_019880.2, NW_006890081.1, NW_005819088.1, NC_023185.1, NC_045166.1, NC_030440.1, NC_049537.1, NC_047591.1, NC_036849.1, NC_046375.1, NC_024351.1, NC_036457.1, NC_045686.1, NW_005179644.1, NC_039316.1, NC_041348.1, NC_044208.1, NW_017859650.1, NC_051245.1, NW_007281450.1, NW_017713697.1, NW_017728947.1, NC_048605.1, NW_020451983.1, NC_051776.1, NW_023336592.1, NC_048897.1, NW_009952265.1, NW_004973262.1, NC_034414.1, NC_052536.1, NW_022044854.1, NC_031774.1, NW_014650483.1, NW_009152437.1, NW_008795283.1, NW_008824949.1, NC_041728.1, NC_045427.1, NC_050580.1, NW_004491405.1, NW_006804717.1, NW_022103915.1, NW_006438074.1, NW_023416306.1, NW_023537909.1, NW_020093309.1, NW_022436999.1, NW_012007369.1, NC_022278.1, NW_012015021.1, NC_044982.1, NC_037675.1, NW_012106931.1, NC_044616.1, NC_048253.1, NC_036917.1, NW_020356506.1, NC_018728.3, NW_017619866.1, NW_020339215.1, NC_045600.1, NW_004450335.1, NW_023399451.1, NW_006383727.1, NW_023270825.1, NW_022589710.1, NW_022134863.1, NW_004438415.1, NC_047035.1, NW_022098036.1, NC_041224.1, NC_045786.1, NW_003573429.1, NW_014638379.1, NW_007676261.1, NW_011515408.1, NC_044516.1, NW_018335685.1, NC_032659.1, NC_030817.1, NC_040258.1, NW_023435897.1, NC_050555.1, NW_003159375.1, NW_015351211.1, NW_020539924.1, NW_012267218.1, NW_003613686.1, NC_051075.1, NW_018657848.1, NC_034581.1, NC_034596.1, NC_046160.1, NT_176399.1, NW_004524583.1, NW_004624734.1, NW_005857041.1, NW_023425344.1, NW_024100921.1, NW_003338865.1, NW_006399787.1, NW_015150892.1, NW_024425008.1, NW_007370659.1, NW_018734275.1, NC_044498.1, NW_009900513.1, NW_020455250.1, NC_040085.1, NW_021964165.1, NC_033662.1, NW_003852510.1, NC_042286.1, NC_048301.1, NC_040242.1, NW_020834750.1, NW_004955466.1, NW_020312827.1, NW_020998607.1, NW_022196912.1, NC_045618.1, NC_051448.1, NC_043708.1, NW_018503592.1, NW_018344057.1, NW_005842135.1, NC_050456.1, NW_021703778.1, NC_045764.1, NW_004545910.1, NC_051214.1, NW_024408640.1, NW_011494811.1, NW_018127943.1, NW_005081537.1, NC_052956.1, NW_006799940.1, NW_015112671.1, NW_015095634.1, NW_005054301.1, NW_009646445.1, NW_023601104.1, NW_023416313.1, NW_004504313.1, NW_017870438.1, NW_010972853.1, NW_022681468.1, NC_045750.1, NW_009632203.1, NW_022665528.1, NC_041378.1, NW_018114435.1, NW_008609214.1, NW_005871133.1, NW_023425404.1, NC_046289.1, NC_045004.1, NC_021677.1, NW_023666053.1, NW_023044993.1, NC_044308.2, NW_016820119.1, NC_044553.1, NC_044381.1, NC_046632.1, NC_041312.1, NW_006712282.1, NW_015505194.1, NW_022631221.1, NC_022009.1, NC_046312.1, NW_019029691.1, NW_019372207.1, NW_022611658.1, NC_050482.1, NW_008239553.1, NC_044575.1, NC_037555.1, NC_040903.2, NW_020837954.1, NC_046226.1, NW_023144640.1, NW_010582752.1, NW_017729025.1, NW_010537981.1, NW_010771612.1, NW_018150791.1, NW_022641422.1, NW_017805708.1, NW_005369967.1, NC_041820.1, NW_020656171.1, NW_006781042.1, NW_010035470.1, NW_009002855.1, NW_011888796.1, NW_023455365.1, NW_004567114.1, NW_007577740.1, NW_015164181.1, NW_019776417.1, NC_040901.1, NC_042532.1, NW_010423023.1, NW_009928727.1, NW_005087567.1, NC_043759.1, NW_006408597.1, NW_009874831.1, NW_009258894.1, NW_023528387.1, NW_020799101.1, NW_015090839.1, NW_014006077.1, NC_046656.1, NC_041043.1, NC_040021.1, NC_045563.1, NC_044005.1, NW_006288624.1, NW_006501064.1, NW_010112985.1, NC_024307.1, NW_006209531.1, NW_018030485.1, NC_047090.1, NC_050191.1, NW_023365123.1, NW_015505036.1, NC_042571.1, NC_041720.1, NW_020448008.1, NW_006727797.1, NW_012116265.1, NC_043979.1, NW_012150840.1, NW_013185668.1, NC_045964.1, NC_045701.1, NC_041066.1, NW_009270392.1, NW_007729665.1, NW_021604585.1, NC_044003.1, NC_045439.1, NC_046335.1, NW_008348666.1, NC_045481.1, NW_006921675.1, NW_020848012.1, NC_044039.1, NW_015553274.1, NW_019715876.1, NW_020172672.1, NC_044325.1, NW_019525097.1, NW_007014397.1, NC_046358.1, NW_020664447.1, NW_016107905.1, NC_047530.1, NW_004454173.1, NW_004443946.1, NW_019154104.1 and other NTCP and its variants. In some embodiments herein, the NTCP to which said delivery method and vehicle are simultaneously incorporated is NCTP of mammals or birds. In some preferred embodiments, the delivery methods and vehicles simultaneously incorporating NTCP is human NTCP (an exemplary amino acid sequence is

shown in SEQ ID NO: 7, NCBI Reference Sequence: NM_003049), chimpanzee NTCP (exemplary amino acid sequence can be found in NCBI Reference Sequence: XM_510035), tree shrew NTCP (exemplary amino acid sequence can be found in NCBI Reference Sequence: XM_006171503), and NTCP of bonobo, tree shrew, woodchuck, arctic ground squirrel, ground squirrel, bat, woolly monkey, capuchin monkey, squirrel monkey and duck. In some preferred embodiments, the delivery methods and vehicles simultaneously incorporating NTCP is human NTCP (an exemplary amino acid sequence is shown in SEQ ID NO: 7, NCBI Reference Sequence: NM_003049), chimpanzee NTCP (exemplary amino acid sequence can be found in NCBI Reference Sequence: XM_510035), tree shrew NTCP (exemplary amino acid sequence can be found in NCBI Reference Sequence: XM_006171503). In some preferred embodiments, the delivery methods and vehicles simultaneously incorporating NTCP is human NTCP (an exemplary NCBI Reference Sequence: NM_003049).

The vehicle forms of the vehicles targeting OATP delivery includes but is not limited to polypeptides, small molecule drugs, proteins, antibodies, nucleotides and other vehicle forms, which are characterized by being able to bind to OATP and transfer the carried drugs or groups for delivery into cells and/or organs, preferably, the delivery vehicle may also target or bind NTCP. The polypeptides, small molecule drugs, proteins, antibodies, nucleotides and other forms of OATP targeted delivery vehicles and variants thereof at least retain the biological activity of binding to OATP and optionally can also target or bind to NTCP for delivery of the carried drug or group into cells, and/or organs, and/or systems. In some preferred embodiments herein, the polypeptides, small molecule drugs, proteins, antibodies, nucleotides and other forms of OATP targeted delivery vehicles and variants thereof at least retain the biological activity of binding to OATP1B3, and/or OATP1B1, and/or OATP1B2 for delivery of the carried drug or group into hepatocytes, and/or liver, and/or digestive system. In some preferred embodiments herein, the polypeptides, small molecule drugs, proteins, antibodies, nucleotides and other forms of OATP targeted delivery vehicles and variants thereof at least retain the biological activity of binding to human OATP1B3, and/or human OATP1B1 for delivery of the carried drug or group into human hepatocytes, and/or human liver, and/or human digestive system. In some preferred embodiments herein, the OATP targeted delivery vehicles includes, but is not limited to, polypeptides, antibodies, proteins, small molecule drugs, nucleotides and other types of compounds or molecules, agents currently known to bind or act on OATP, and polypeptides derived from hepadnavirus surface antigens obtained through design and/or screening that can bind or interact with OATP.

In some embodiments herein, the polypeptides, antibodies, proteins, small molecule drugs, nucleotides, and other types of compounds or molecules that can bind or interact with OATP and optionally with NTCP can be obtained through design and/or screening, to prepare the OATP targeted delivery vehicles described herein. In some embodiments herein, the polypeptides, antibodies, proteins, small molecule drugs, nucleotides and other types of compounds or molecules that can bind or interact with OATP1B1 and/or OATP1B3 can be obtained through design and/or screening, to prepare the OATP targeted delivery vehicles described herein. In some embodiments herein, the polypeptide or peptide modifiers obtained by design and/or screening that binds or interacts with OATP1B1 and/or OATP1B3 includes the polypeptide or peptide modifiers obtained by Random peptide libraries, combinatorial peptide libraries, synthetic peptide libraries and other peptide libraries, phage presentation, yeast presentation, virus presentation and other peptide screening technologies, using OATP1B1 and/or OATP1B3 as a target. In some embodiments herein, an antibody obtained by design and/or screening that binds to or interacts with OATP1B1 and/or OATP1B3, including, but not limited to, anti-OATP1B1 and/or anti-OATP1B3 antibody sera, polyclonal antibodies, monoclonal antibodies or antibody fragments or antibody modifiers obtained by immunizing animals with OATP1B1 and/or OATP1B3 proteins or protein fragments or fusion proteins, Polyclonal antibodies, monoclonal antibodies, humanized antibodies obtained by hybridoma technology, phage presentation technology, yeast presentation technology, immunolibrary technology, ribosome presentation technology, mRNA presentation screening technology, nanobody (Nanobody, Nb) screening method, transgenic mice fully humanized antibody screening technology, single-cell photoconduction technology, flow cytometry, and other antibody screening techniques, fully humanized antibodies and other antibodies, or antibody fragments, or antibody modifiers, which targeting OATP1B1 and/or OATP1B3. In some embodiments herein, the proteins, amino acid fragments or protein modifiers that can bind or interact with OATP1B1 and/or OATP1B3 obtained through design and/or screening include, but are not limited to, through phage display technology, yeast display technology, proteins, amino acid fragments or protein modifications obtained by cell display technology, random libraries and other protein-protein interaction screening technologies, which targeting OATP1B1 and/or OATP1B3. In some embodiments herein, nucleotides obtained by design and/or screening that binds or interacts with OATP1B1 and/or OATP1B3, include, but are not limited to, polyDNA, oligoDNA, polyRNA, oligo-RNA, deoxyribonucleic acid protein complexes (DNP), or nucleic acid modifiers obtained by screening for targeting OATP1B1 and/or OATP1B3. In some embodiments herein, small molecules obtained by design and/or screening that can bind or interact with OATP1B1 and/or OATP1B3, include but are not limited to small molecules that bind or interact with OATP1B1 and/or OATP1B3 obtained by design or compound screening. In some preferred embodiments, including, but not limited to, peptides, antibodies, proteins, small molecule drugs, nucleotides, and other types of compounds or molecules that obtained by the above design and/or screening techniques that bind or interact with human OATP1B1 and/or human OATP1B3 can serve as delivery vehicles for targeting OATP. In some embodiments, OATP-targeted delivery vehicles described herein can be prepared by utilizing or modifying agents

currently known to bind or act with OATP. In some embodiments, OATP-targeted delivery vehicles described herein can be prepared by utilizing or modifying agents currently known to bind or act with OATP1B1 and/or OATP1B3. The agents currently known to interact with OATP1B1 and/or OATP1B3, including but not limited to, statins (including, but not limited to, atorvastatin, pitavastatin, srosuvastatin, simvatinast, patinravastatin, and other statins), sartans (including, but not limited to, valsartan, telmisartan, telmisartan, and other sartans), bosentan, docetaxel, fexofenadine, glecaprevir, glembuvir (glibenclamide), grazoprevir, nateglinide, paclitaxel, paritaprevir, repaglinide, simeprevir, steroid hormone metabolites, thyroid hormone metabolites, inflammatory mediators, bile acids, bilirubin, Bromosulfophthalein, Estradiol-17β-glucoside 17β-glucuronic acid, cholecystokinin octapeptide, testosterone 3-sulfate, liver function markers, mushroom toxins, antibiotics, antivirals, anticancer drugs, Myrcludex B (bulevirtide) and other drugs known to interact with OATP1B1 and/or OATP1B3. OATP1B1 and/or OATP1B3. The above agents can be tested for their ability to bind to OATP1B1 and/or OATP1B3 and deliver the drug; or they can be modified to give them the ability to bind to OATP1B1 and/or OATP1B3 and deliver the drug. In some embodiments, OATP-targeted delivery vehicles described herein can be prepared by utilizing or modifying agents currently known to bind or act with human OATP1B1 and/or human OATP1B3.

The delivery vehicle for targeting OATP contains a drug or group including, but not limited to, a small molecule drug, a nucleotide, a protein, an antibody, a peptide, or other drug or group, and is characterized in that said drug or group may be linked to said vehicle by covalent and/or non-covalent bonds. The drug or group is conjugated to the vehicle, and the drug or group conjugated to the vehicle is delivered into cells, organs, or tissues by the vehicle's binding or interaction with OATP. The drug or group may be linked to the OATP-targeted delivery vehicle via, but not limited to, covalent and/or non-covalent bonds or otherwise. The covalent bonds include, but are not limited to, peptide bonds, ester bonds, amide bonds, disulfide bonds, polar bonds, nonpolar bonds, coordination bonds, single bonds, double bonds, triple bonds, σ bonds, π bonds and other covalent bonds. The non-covalent bonds include, but are not limited to, hydrogen bonds, ion-dipole interactions, dipole-dipole interactions, charge interactions, hydrophobic interactions, hydrophilic interactions, van der Waals' gravitational forces, ionic bonds, salt bonds, intermolecular forces, protein interactions, protein-nucleic acid interactions, antigen-antibody interactions, receptor-ligand interactions, and other non-covalent bonding interactions. In said OATP-targeted delivery vectors, the drug or group may be linked either directly or through a linker. The small molecule drugs or groups include, but are not limited to, fluorescent groups, toxins, chemotherapeutic agents, signal modulating molecules, cell signaling agonists, cell signaling inhibitors, and other small molecule drugs or groups. The nucleotides include, but are not limited to, DNA, RNA and other nucleotide molecules or variants, including, but not limited to, oligo-DNA, oligo-RNA, poly-DNA, poly-RNA and other forms of DNA and/or RNA molecules. The nucleotide molecules have the ability to alter and regulate gene sequences, gene editing, gene transcription, mRNA translation, and their effects include, but are not limited to, transduction of exogenous genes, protein coding, and up-regulation or down-regulation of target gene expression. The nucleic acid molecules include plasmids, DNA gene fragments, RNA gene fragments, antisense nucleic acids, nucleases, RNA interference, gene editing vectors, and other forms of nucleic acid molecules. In some more preferred embodiments herein, the vehicle contains nucleotides that are protein-coding DNA or RNA, antisense nucleic acids, nucleic acids for RNA interference, nucleotides for gene editing. In some embodiments herein, the nucleic acids contained in the vehicles further include, but are not limited to, viruses. The OATP-targeting vehicles can link to a virus, or can fusion express with viral protein, or can insert its coding sequence into a virus, or can link to a virus by other ways, to enable the virus to acquire the biological activity to infect OATP-expressing cells and thereby deliver the nucleic acid code or fragment carried by the virus into a cell or organ or tissue. The viruses include, but are not limited to, adenoviruses, lentiviruses, retroviruses, adeno-associated viruses, poxviruses, stomatitis viruses, herpesviruses, rabies virus vectors and other viruses thereof. The proteins include, but are not limited to, proteins that modulate cellular physiological functions, proteins that have pharmacological effects, proteins that kill target cells, and proteins that have other activities. The antibody includes, but is not limited to, an antibody that can regulate the physiological function of a cell after binding to a target antigen, an antibody that has pharmacological effects, an antibody that can kill a target cell, an antibody that can regulate immunity, and an antibody that has other activities, and said antibody includes, but is not limited to, a polyclonal antibody, a monoclonal antibody, a bispecific antibody, a tricomponent antibody, a tetraspecific antibody, a humanized antibody, an all-humanized antibody, an antibody fragment and other antibodies, and said antibody includes, but is not limited to, an antibody that can regulate immunity after binding to a target antigen. other antibodies. The proteins or antibodies and fragments thereof may be fusion expressed by (but not limited to) with the vehicles, or expressed and then coupled to the vehicles, or otherwise coupled to the vehicles.

The described OATP-targeted delivery methods and vehicle-delivered drugs can be used in include, but are not limited to, mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not

limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some preferred embodiments herein, the vehicle is used for drug delivery in humans, chimpanzees, bonobos, tree shrews, ducks, marmots, arctic ground squirrels, ground squirrels, bats, woolly monkeys, capuchin monkeys, and squirrel monkeys. In some preferred embodiments herein, the vehicle is used for drug delivery in humans.

In the delivery methods and vehicles for targeting OATP, the drug delivered enters systems including, but not limited to, the digestive, respiratory, circulatory, nervous, locomotor, urinary, reproductive, endocrine, and other systems; drugs delivered into organs and tissues including, but not limited to, the liver, kidneys, lungs, heart, muscle, fat, brain, nerves, intestines, pancreas, and other organs or tissues. The delivered drug preferably enters the digestive system, the delivered drug preferably enters the liver. In some embodiments herein, the systems to which the vehicle drug delivery is applied include, but are not limited to, the digestive system, the respiratory system, the circulatory system, the nervous system, the locomotor system, the urinary system, the reproductive system, the endocrine system, and other systems. In some embodiments herein, the organs and tissues to which said vehicle drug is applied for delivery include, but are not limited to, the liver, kidneys, lungs, heart, muscle, fat, brain, nerves, intestines, pancreas, and other organs or tissues. In some embodiments herein, cells to which vehicle drug delivery is applied include, but are not limited to, hepatocytes, lung cells, kidney cells, nerve cells, muscle cells, adipocytes, blood cells, lymphocytes, and other cells. In some preferred embodiments herein, the vehicle is applied to deliver the drug into the digestive system, and/or the liver, and/or hepatocytes.

In a preferred embodiment, the drug scaffold vehicle for OATP described herein is preferably an OATP scaffold vehicle derived from a hepadnavirus surface scaffold as described below.

OATP targeted delivery polypeptide vehicle derived from hepadnavirus surface antigen

In some embodiments herein, the OATP targeted delivery vehicle described herein includes, but is not limited to, a polypeptide derived from a hepadnavirus surface antigen that retains at least the biological activity that bound to OATP and will be delivered into cells with carried drugs or groups, that is, an OATP targeted delivery polypeptide vehicle derived from the surface antigen of hepadnavirus, referred to as the OATP targeted delivery polypeptide vehicle.

The hepadnaviruses include, but are not limited to, Orthohepadnavirus, Avihepadnavirus, Metahepadnavirus, Herpetohepadnavirus and other hepadnaviruses. The orthohepadnavirus DNA viruses includes but is not limited to infecting humans (HBV), woolly monkeys (Woolly monkey HBV, WMHBV), capuchin monkeys (Capuchin monkey HBV, CMHBV), squirrel monkey, woodchuck (Woodchuck hepatitis virus, WHV), arctic ground squirrel, ground squirrel (ground squirrel hepatitis virus, GSHV), tree shrew, chimpanzee, bat (Bat hepatitis virus, BHV) and other animals, example genome sequences can be found in GenBank accession numbers including but not limited to AF046996 (WMHBV), NC_043528 (CMHBV), NC_004107 (WHV), NC_001484 (GSHV), JX941468 (BHV). The Avihepadnavirus includes, but is not limited to, infecting ducks (duck HBV, DHBV), herons (heron HBV, HHBV) and other birds, and example genome sequences can be found in, but are not limited to, GenBank accession numbers NC_001344 (DHBV), NC_001486 (HHBV). The orthohepadnavirus that infect humans (HBV) described herein include, but are not limited to, HBV genotype A, B, C, D, E, F, G, H, and I, and example genome sequences can be found in, but are not limited to, GenBank accession numbers KC875260 (genotype A), AY220704 (genotype B), AF461363 (genotype C), AY 796030 (genotype D), AB205129 (genotype E), DQ823095 (genotype F), HE981176 (genotype G) and AB179747 (genotype H).

The amino acid sequence of the OATP targeted delivery vehicle derived from the surface antigen of hepatophilic DNA virus is derived from a viral envelope protein, also known as surface antigen or surface protein. The hepadnavirus including, but not limited to, human (HBV), Woolly monkey HBV (WMHBV), capuchin monkey HBV (CMHBV), squirrel monkey, woodchuck (woodchuck hepatitis virus, WHV), arctic ground squirrel, ground squirrel (ground squirrel hepatitis virus, GSHV), tree shrews, chimpanzees, bats (Bat hepatitis virus, BHV) and other animals, as well as infecting ducks Avihepadnavirus of HBV(duck HBV, DHBV), heron (heron HBV, HHBV) and other birds. In some preferred embodiments herein, the amino acid sequence of the OATP targeted delivery vehicle is derived from the amino acid sequence of the Pre-S region of the viral surface antigen, including but not limited to the exemplary surface antigen Pre-S amino acid sequence SEQ ID NO: 8-22 and its variants. In some preferred embodiments herein, the amino acid sequence of the OATP targeted delivery vehicle is derived the surface antigen of Pre-S1 region of human HBV, including but not limited to genotypes A, B, C, D, E, F, G, H and I HBVP Pre-S1 region amino acid sequences and variants thereof, including but not limited to the exemplary amino acid sequence of surface antigen of HBV Pre-S1 shown in SEQ ID NO: 15-22 and variants thereof. In some embodiments herein, the OATP targeted delivery polypeptide vehicle may be derived from the pre-S1 region of the HBV genotype C surface antigen. In some embodiments, the length of the OATP targeted delivery polypeptide vehicle may consist of 10-118 amino acids. For example, the polypeptide may be 15-100, 15-80, 20-100, 20-80, 20-60, 25-60, 30-60,

35-60, or 40-60 (including all integers in these ranges) amino acids in length. In some embodiments, OATP targeted delivery vehicle described herein may be at least 20 amino acids in length, e.g., at least 25, 30, 35 or 40 amino acids in length. In some embodiments, OATP targeted delivery vehicle described herein may be 20, 25, 30, 35, 40, 47, 55 or 60 amino acids in length. In some embodiments, the OATP targeted delivery vehicle may be 47 amino acids in length. The variants with different lengths of the OATP targeted delivery polypeptide vehicle retain one or more biological activities related to the corresponding polypeptide, including at least: the biological activity that binds to OATP (especially OATP1B1 and OATP1B3, especially human OATP1B1 and human OATP1B3) and transports drugs into cells.

The terms "OATP targeted delivery polypeptide vehicle derived from hepadnavirus surface antigen", "polypeptide vehicle derived from hepadnavirus surface antigen" and "polypeptide vehicle derived from hepadnavirus" refer to that the origin or source of the polypeptide vehicle is hepadnavirus, including but not limited to natural, recombinant, synthetic or purified polypeptide vehicles, including but not limited to full-length natural HBV polypeptides or fragments thereof, and full-length natural polypeptides or fragments variants thereof. In some embodiments, the polypeptide vehicle derived from a hepadnavirus can be derived from any hepadnavirus, including but not limited to, orthohepadnavirus (OHV) that infects mammals, Avihepadnavirus that infects birds (AHV), metahepadnavirus viruses that infects fish, and herpetohepadnavirus that infects amphibians; in some embodiments, the polypeptide vehicle derived from a hepadnavirus may be derived from any hepadnavirus surface antigen; in some embodiments, the polypeptide vehicle derived from a hepadnavirus can be derived from any orthohepadnavirus surface antigen; in some embodiments, the polypeptide vehicle derived from a hepadnavirus can be derived from any human hepatitis B virus (HBV) surface antigen, including, but not limited to, derived from surface antigen of HBV genotype A, B, C, D, E, F, G, H, and I; in some embodiments, the polypeptide vehicle derived from a hepadnavirus may be derived from any human hepatitis B virus (HBV) surface antigen Pre-S1 region, an OATP-binding HBVPre-S1-derived peptide or an OATP-binding peptide derived from HBVPre-S1.

The terms "OATP targeted delivery polypeptide vehicle derived from HBVP Pre-S1" as used herein refers to the polypeptide whose origin or source is the surface antigen of HBV Pre-S1 region, including but not limited to natural, recombinant, synthetic or purified polypeptides, including but not limited to full-length natural surface antigen of HBV Pre-S1 region or fragments thereof and variants thereof, full-length non-natural surface antigen of HBV Pre-S1 region or fragments thereof and variants thereof, non- Full-length natural surface antigen of HBV Pre-S1 region or fragments thereof and variants thereof, non-full-length non-natural surface antigen of HBV Pre-S1 region or fragments thereof and variants thereof, and other forms of variants. In some embodiments, the OATP targeted delivery polypeptide vehicle derived from HBVP Pre-S1 may contain the entire surface antigen pre-S1 region from the second amino acid G (glycine) of the surface antigen pre-S1 region of HBV of any genotype. In certain embodiments, the OATP targeted delivery polypeptide vehicle derived from HBVP Pre-S1 described herein may be derived from the pre-S1 region of the surface antigen of any one of HBV genotypes A, B, C, D, E, F, G, H and I. The example genome sequences of these HBV genotypes can be found in GenBank accession numbers KC875260, AY220704, AF461363, AY796030, AB205129, DQ823095, HE981176, and AB179747, and exemplary amino acid sequences of their surface antigen Pre-S1 regions can be found in, but are not limited to, SEQ ID NO: 8-22 and variants thereof. In some embodiments, the OATP targeted delivery polypeptide vehicle derived from HBVP Pre-S1 may be derived from the pre-S1 region of the HBV genotype C surface antigen. The OATP targeted delivery polypeptide vehicle derived from HBVP Pre-S1 at least retains the biological activity of binding to OATP, especially retains the biological activity of binding to OATP1B1, and/or OATP1B2 and/or OATP1B3, especially retains the biological activity of binding to human OATP1B1 and/or human OATP1B3.

In some embodiments of the invention, the OATP targeted delivery polypeptide vector derived from hepadnavirus surface antigen is a polypeptide having the structure of the following formula (I):

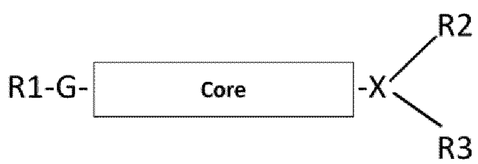


(I)

among them, G is glycine, the core sequence is the amino acid sequence derived from the surface antigen of hepadnavirus, X is an amino acid residue with two amino groups or two carboxyl groups or other groups available for linkage reaction, used as a vehicle, and R1 is a N-terminal modification of the vehicle, R2 does not exist, or is a flanking sequence or other group, R3 is a carried pharmacophore group or a linking group that can interact with a drug or group.

The core amino acid sequence is derived from hepadnavirus envelope protein, also known as surface antigen or surface protein. The hepadnavirus including, but not limited to, human (HBV), Woolly monkey HBV (WMHBV), capuchin monkey HBV (CMHBV), squirrel monkey, woodchuck (woodchuck hepatitis virus, WHV), arctic ground squirrel, ground squirrel (ground squirrel hepatitis virus, GSHV), tree shrews, chimpanzees, bats (Bat hepatitis virus, BHV) and other

animals, as well as infecting ducks Avihepadnavirus of HBV (duck HBV, DHBV), heron (heron HBV, HHBV) and other birds. In some preferred embodiments herein, the polypeptide vector derived from Hepadnaviridae has its core amino acid sequence derived from the amino acid sequence of the Pre-S region of the viral surface antigen, including but not limited to the exemplary surface antigen Pre-S amino acid sequence SEQ ID NO: 8-22 and its variants. In some preferred embodiments herein, the polypeptide vehicle core sequence is derived from a polypeptide of the amino acid sequence of the surface antigen of Pre-S region of human HBV, including but not limited to genotypes A, B, C, D, E, F, G, H and Genotype I HBVP Pre-S1 region amino acid sequences, including but not limited to exemplary amino acid sequences of surface antigen of HBV Pre-S region shown in SEQ ID NO: 15-22 and variants thereof. In some preferred embodiments herein, the polypeptide vehicle core sequence is derived from a polypeptide of the amino acid sequence of the Pre-S region of human HBV genotype C surface antigen, including but not limited to exemplary amino acid sequences SEQ ID NO: 17 and variants thereof. In some embodiments herein, the polypeptide vehicle core sequence is derived from a human C genotype HBV polypeptide comprising amino acids 3-119, 4-119, 3-69, 4-69, 3-59, 4-59, 3-48, 4-48, 14-119, 14-88, 14-72, 14-67, 14-59, 14-56, 14-55, 14-53, 14-48,14-47, 14-45, 14-42, 14-37, 14-32 of the surface antigen pre-S1 region, the example sequences include but are not limited to SEQ ID NO: 81-102 and variants thereof. In some embodiments, the vehicle core sequence comprises the amino acid sequence of at least amino acid residues 14-32 at the N-terminal of the pre-S1 region of HBV genotype C surface antigen described herein. In preferred embodiments, the core sequence may be 19-106 amino acid residues in length. In a particularly preferred embodiment, the core sequence is, for example, but not limited to, the exemplary sequence of SEQ ID NO: 97, which contains at least, but is not limited to, the amino acid residues 1-19 of the exemplary sequence of SEQ ID NO: 97. Preferably, the core sequence contains at least but it is not limited to the amino acid residues from position 1 to position 24, 25, 26, 27, 28, 29, 30, 31, 32, 33, 34 or 35 of the exemplary sequence of SEQ ID NO: 30. In some embodiments, the core sequence has a fragment of at least the positions 14-32 of the N-terminus of the surface antigen of pre-S1 region of HBV genotypes A, B, F, H, and I described herein, a fragment of at least positions 3-21 of the N-terminus of the HBV surface pre-S1 region of genotype D, or a fragment of at least positions 13-31 of the N-terminus of the HBV surface pre-S1 region of genotype E and G. In some embodiments, the core sequence has a fragment of at least the positions 14-48 of the N-terminus of the surface antigen of pre-S1 region of HBV genotypes A, B, F, H, and I described herein, a fragment of at least positions 3-37 of the N-terminus of the HBV surface pre-S1 region of genotype D, or a fragment of at least positions 13-47 of the N-terminus of the HBV surface pre-S1 region of genotype E and G. Example sequences thereof include but are not limited to SEQ ID NO: 103-110.

As used herein, a "variant" relative to a core sequence refers to one that differs in amino acid sequence from a given polypeptide but retains the biological activity of binding to OATP and transporting drugs or groups into cells, particularly OATP1B3, and/or OATP1B2 and/or OATP1B1 to bind and transport drugs or groups into hepatocytes, especially the biological activity of binding to human OATP1B3 and/or OATP1B1 and transporting drugs or groups into human hepatocytes. For example, in some embodiments, N15D, F25L, G35K, N39E, N46K, F45L, N48H, N48Y, N48K, D50A, H51Q, H51N, E54K, E54D, A55S, N56K, N56D, Q57K amino acid substitutions or mutation combinations can be introduced into the core sequence derived from the 14-59 amino acid sequence of the genotype C HBVPre-S1 region, example sequences thereof include but are not limited to SEQ ID NO: 111-128. As used herein, the term "variant" also includes homologous core sequences found in different viral species, strains, or subtypes of the genus hepatovirus. Based on the antigenic epitopes on its envelope protein, HBV is classified into four main serotypes (adr, adw, ayr, and ayw), and according to the variability of the nucleotide sequence in the genome, HBV is divided into nine genotypes (A to I). Thus, the term "variant" includes any of these homologous core polypeptides found in HBV subtypes. "Variants" may also include core sequences with natural flanking amino acid sequences from any of these HBV subtypes added at the N- and/or C-terminus, or variants thereof. For example, in some embodiments, the polypeptide derived from the 13-48 amino acid sequence of the genotype C surface antigen Pre-S1 region can be combined with the polypeptide including but not limited to derived from the surface antigen of pre-S1 region of HBV genotypes A, B, F, H, and I, a fragment of at least amino acid residues 3-12 at the N-terminus of the S1 region, or a fragment of at least amino acid residues 3-11 at the N-terminus of the surface antigen pre-S1 region of HBV genotype E or G, forms a fusion core sequence, and its example sequence including but not limited to the sequences shown in SEQ ID NO: 129-134. For example, in some embodiments, the core sequences described herein may contain an amino acid sequence selected from, but not limited to, any of SEQ ID NO: 81-128, and natural amino acid sequence flanking the N- and/or C-terminus derived from the surface antigen of any of HBV genotypes A-I. In some embodiments, the natural flanking amino acid sequences can be derived from the amino acid sequences of HBV surface antigens, including but not limited to GenBank accession numbers KC875260 (genotype A), AY220704 (genotype B), AF461363 (genotype C), AY796030 (amino acid sequence of HBV surface antigen of genotype D), AB205129 (genotype E), DQ823095 (genotype F), HE981176 (genotype G) or AB179747 (genotype H). For example, the core sequence described herein may contain the amino acid sequences shown by SEQ ID NO: 97 and contain at its N and/or C terminus a natural flanking amino acid sequence from the pre-S1 region of the HBV genotype C. Alternatively, the core sequence described herein may contain the amino acid sequences shown by SEQ ID NO: 97 and contain at its N and/or C terminus a natural flanking amino acid sequence from the pre-S1 region of any of the HBV genotype A, B, C, D, E, F, G and H. In some embodiments, the N-terminus and/or C-terminus of the core sequence described herein may

independently contain a natural flanking amino acid sequence of 1-10, e.g., 1-8, 1-5, 1-3, (including all integers within these ranges) amino acid length. For example, the core sequence described herein may contain the amino acid sequence shown in SEQ ID NO: 97 and at its N-terminus a 10 amino acid long native flanking amino acid sequence from the pre-S1 region of HBV genotype C. In other words, the core sequence may contain amino acids 3-59 of the HBV genotype C pre-S1 region (SEQ ID NO: 87). For another example, the core sequence described herein may contain the amino acid sequence shown in SEQ ID NO: 97 and at its N-terminus a 9 amino acid long native flanking amino acid sequence from the pre-S1 region of HBV genotype E or G. In other words, the polypeptide may contain amino acids 14-48 of the HBV genotype C pre-S1 region and amino acids 3-12 of the HBV genotype E or G pre-S1 region (SEQ ID NO: 131 and 133). It will be understood that any of the vector core sequences described herein can have any length of native flanking amino acid sequence extending from its N and/or C terminus, and that the resulting core sequence retains the biology of the original polypeptide vector, and the activity at least includes: binding to OATP (especially OATP1B1 and OATP1B3) and transporting drugs or groups into cells. "Variant" related to the core sequence also includes one or more amino acid deletions, substitutions or insertions, while the resulting core sequence retains a variety of biological activities of the original polypeptide vector, including at least: binding to OATP (especially It is OATP1B1 and OATP1B3, especially human OATP1B1 and human OATP1B3) and transport drugs or groups into cells. For example, in some embodiments, relative to the core sequence from the HBV pre-S1 region, the polypeptide described herein may have 1-30, such as 1-20, 1-10, 1-8, 1-5 or 1-3 (including all integers within these ranges) amino acids deletions, substitutions or insertions. For example, in some embodiments, the polypeptide described herein may have 1-30, such as 1-20, 1-10, 1-8, 1-5 or 1-3 (including all integers within these ranges) amino acid deletions, substitutions or insertions relative to any amino acid sequence selected from SEQ ID NO: 81-128. For example, in some embodiments, the vehicle core sequence described herein may have 1-30, such as 1-20, 1-10, 1-8, 1-5 or 1-3 (including all integers within these ranges) amino acid deletions, substitutions or insertions relative to any amino acid sequence selected from SEQ ID NO: 166. For example, in some embodiments, the vehicle core sequence described herein may have 1-3 amino acid deletions, substitutions, or insertions relative to the exemplary amino acid sequence of SEQ ID NO: 97. For example, in some embodiments, the OATP-binding peptide described herein may have 1-30, such as 1-20, 1-10, 1-8, 1-5 or 1-3 (including all integers within these ranges) amino acid deletions, substitutions or insertions at the N-terminus or C-terminus relative to any amino acid sequence selected from SEQ ID NO: 81-128. For example, the vehicle core sequences described herein may contain an amino acid sequence selected from any of those shown in SEQ ID NO: 81-102. In various embodiments, a vehicle core sequence described herein may have at least about 30%, 40%, 50%, 60%, 70%, 80%, 85%, 90%, 95%, 96%, 97%, 98% or 99% identical to any of the vehicle core sequences described herein. For example, the polypeptide may contain an amino acid sequence that is at least about 30%, 40%, 50%, 60%, 70%, 80%, 85% 90%, 95%, 96%, 97%, 98% or 99% identical to any of the exemplary sequences of SEQ ID NOs: 81-134. In some embodiments, the vector core sequence may contain an amino acid sequence that is at least about 30%, 40%, 50%, 60%, 70%, 80%, 85%, 90%, 95%, 96%, 97%, 98% or 99% identical to any of the exemplary sequences of SEQ ID NOs: 81-134. In some embodiments, the vector core sequence may contain an amino acid sequence that is at least about 30%, 40%, 50%, 60%, 70%, 80%, 85%, 90%, 95%, 96%, 97%, 98% or 99% identical to any of the exemplary sequences of SEQ ID NOs: 97. Variants having some sequence identity to the core sequences described herein such that the resulting polypeptide vehicles retain at least the biological activity of binding to OATPs (in particular OATP1B1 and OATP1B3, and in particular human OATP1B1 and human OATP1B3) and transporter of the drug or group into the cell.

R1 is a necessary N-terminal modification group for the OATP targeted delivery polypeptide vehicle derived from the surface antigen of hepadnavirus. In some embodiments, R1 can be modified with a hydrophobic group, such as but not limited to myristic acid (myr), palmitic acid (plam), stearic acid (stearoyl), cholesterol (chol), oleic acid, linoleic acid, polyethylene glycol (PEG), arachidonic acid or other hydrophobic groups. In some embodiments, the hydrophobic group of R1 may be selected from myristic acid, palmitic acid, stearic acid, and cholesterol. In some embodiments, the hydrophobic group of R1 can be myristic acid. In some embodiments, the vehicle core sequence described herein may contain an amino acid sequence selected from any one of SEQ ID NO: 81-134, wherein the G of N-terminus of the polypeptide may be selected from hydrophobic group modification of myristic acid, palmitic acid, stearic acid and cholesterol. In some embodiments, the vehicle core sequence may comprise an amino acid sequence selected from any one of SEQ ID NO: 81-134, wherein the G of N-terminus of the HBV entry inhibitory OATP-binding peptide can be myristoylated. In some embodiments, the vehicle core sequence may comprise an amino acid sequence selected from any one of SEQ ID NO: 9794, wherein the G of N-terminus of the peptide vehicle can be myristoylated.

In formula (I), G is connected to the core sequence through an amide bond; X is connected to the core sequence through an amide bond.

R2 is a flanking sequence or other group, and R2 can be deleted. In some embodiments herein, R2 is derived from an amino acid sequence derived from a surface antigen of a hepadnavirus including, but not limited to, human (HBV), Woolly monkey (WMHBV), capuchin monkey HBV (CMHBV), squirrel monkey, woodchuck (woodchuck hepatitis virus, WHV), arctic ground squirrel, ground squirrel (ground squirrel hepatitis virus, GSHV), tree shrews, chimpanzees, bats (Bat hepatitis virus, BHV) and other animals, as well as infecting ducks Avihepadnavirus of HBV (duck HBV, DHBV), heron (heron HBV, HHBV) and other birds. In some preferred embodiments herein, the polypeptide vehicle derived from

Hepadnaviridae has its R2 flanking amino acid sequence derived from the amino acid sequence of the Pre-S region of the viral surface antigen, including but not limited to the exemplary surface antigen Pre-S amino acid sequence SEQ ID NO: 8-22 and its variants. In some preferred embodiments herein, the R2 flanking sequence of the polypeptide vehicle is derived from a polypeptide of the amino acid sequence of the surface antigen of Pre-S region of human HBV, including but not limited to genotypes A, B, C, D, E, F, G, H and I HBVP Pre-S1 region amino acid sequences, including but not limited to exemplary amino acid sequences of surface antigen of HBV Pre-S region shown in SEQ ID NO: 15-22 and variants thereof. In some preferred embodiments herein, the R2 flanking sequence of the polypeptide vehicle is derived from the amino acid sequence of the surface antigen of human HBV Pre-S region, including but not limited to exemplary amino acid sequences shown in SEQ ID NO: 17 and variants thereof. In some embodiments herein, the peptide vector R2 flanking sequence is derived from a human HBV genotype C polypeptide comprising amino acids 50-119, 50-88, 50-72, 50-69, 50-67, 58-69, 50-59 or 50-52 of the pre-S1 region of the surface antigen, and exemplary sequences thereof include, but are not limited to, SEQ ID NO:135-142 and variants thereof. In some embodiments herein, said R2 flanking sequence does not contain an amino acid, or contains amino acid residues 50-52 at the N-terminal end of the surface antigen of pre-S1 region of HBV genotype C described herein. In some embodiments herein, the R2 flanking sequence may be 0-70 amino acid residues in length. n some preferred embodiments, the R2 flanking sequence contains at least amino acid residues 1-3 of SEQ ID NO:141, preferably said flanking sequence contains at least amino acid residues 1 to 2, 3, 4, 5, 6, 7, 8, 9 or 10 of SEQ ID NO:141; in some embodiments, the R2 flanking sequence is shown in SEQ ID NO:141. In some embodiments, the fragment has a fragment of at least the positions 50-52 of the N-terminus of the surface antigen of pre-S1 region of the HBV genotypes A, B, F, H, and I described herein, a fragment of at least positions 39-41 of the N-terminus of the HBV surface pre-S1 region of genotype D, or a fragment of at least positions 49-51 of the N-terminus of the HBV surface pre-S1 region of genotype E and G. In some embodiments, the fragment has a fragment of at least the positions 50-59 of the N-terminus of the surface antigen of pre-S1 region of HBV genotypes A, B, F, H, and I described herein, a fragment of at least positions 39-48 of the N-terminus of the HBV surface pre-S1 region of genotype D, or a fragment of at least positions 49-58 of the N-terminus of the HBV surface pre-S1 region of genotype E and G. Example sequences thereof include but are not limited to SEQ ID NO: 143-149.

As used herein, a "variant" related to an R2 flanking sequence described herein, a flanking sequence derived from HBV, or an HBV-derived flanking sequence refers to a flanking sequence that differs from a given flanking sequence (i.e., a flanking sequence described herein, a flanking sequence derived from HBV, or an HBV-derived flanking sequence) in its amino acid sequence but that retains the biological activity to bind to the OATP binding and the biological activity of transporting the drug or group into the cell, particularly the biological activity to bind to the OATP1B3, and/or OATP1B2 and/or OATP1B1 and transport drugs or groups into hepatocytes, especially the biological activity of binding to human OATP1B3 and/or OATP1B1 and transporting drugs or groups into human hepatocytes. For example, in some embodiments, D50A, H51Q,or H51N, E54K, or E54D, A55S, N56K, or N56D, Q57K amino acid substitutions or mutation combinations can be introduced into the core sequence derived from the 50-59 amino acid sequence of the genotype C HBVPre-S1 region, example sequences thereof include but are not limited to SEQ ID NO: 150-158. As used herein, the term "variant" also includes homologous core sequences found in different viral species, strains, or subtypes of the genus hepatovirus. Based on the antigenic epitopes on its envelope protein, HBV is classified into four main serotypes (adr, adw, ayr, and ayw), and according to the variability of the nucleotide sequence in the genome, HBV is divided into nine genotypes (A to I). Thus, the term "variant" includes any of these homologous flanking polypeptides found in HBV subtypes. "Variants" may also include flanking sequences with natural flanking amino acid sequences from any of these HBV subtypes added at the N- and/or C-terminus, or variants thereof. In some embodiments, the natural flanking amino acid sequences can be derived from the amino acid sequences of HBV surface antigens, including but not limited to GenBank accession numbers KC875260 (genotype A), AY220704 (genotype B), AF461363 (genotype C), AY796030 (amino acid sequence of HBV surface antigen of genotype D), AB205129 (genotype E), DQ823095 (genotype F), HE981176 (genotype G) or AB179747 (genotype H).

In some embodiments, the N-terminus and/or C-terminus of the flanking sequence described herein may independently contain a natural flanking amino acid sequence of 1-10, e.g., 1-8, 1-5, 1-3, (including all integers within these ranges) amino acid length. "Variant" related to the flanking sequence also includes one or more amino acid deletions, substitutions or insertions, while the resulting flanking sequence retains a variety of biological activities of the original polypeptide vector, including at least: binding to OATP (especially It is OATP1B1 and OATP1B3, especially human OATP1B1 and human OATP1B3) and transport drugs or groups into cells. For example, in some embodiments, relative to the sequence from the HBV pre-S1 region, the flanking sequence described herein may have 1-30, such as 1-20, 1-10, 1-8, 1-5 or 1-3 (including all integers within these ranges) amino acids deletions, substitutions or insertions. In some embodiments, the flanking sequence described herein may have 1-30, such as 1-20, 1-10, 1-8, 1-5 or 1-3 (including all integers within these ranges) amino acid deletions, substitutions or insertions relative to any amino acid sequence selected from SEQ ID NO: 135-158. In some embodiments, the vehicle flanking core sequence described herein may have 1-10, such as 1-10, 1-8, 1-5, 1-3 or 1 (including all integers within these ranges) amino acid deletions, substitutions or insertions relative to any amino acid sequence selected from SEQ ID NO: 141. In some embodiments, the vehicle flanking

core sequence described herein may have 1-3 amino acid deletions, substitutions, or insertions relative to the exemplary amino acid sequence of SEQ ID NO: 141. For example, in some embodiments, the vehicle flanking core sequence described herein may have 1-30, such as 1-20, 1-10, 1-8, 1-5 or 1-3 (including all integers within these ranges) amino acid deletions, substitutions or insertions at the N-terminus or C-terminus relative to any amino acid sequence selected from SEQ ID NO: 135-158. For example, the vehicle core sequences described herein may contain an amino acid sequence selected from any of those shown in SEQ ID NO: 135-142.

In various embodiments, the vehicle flanking core sequence described herein may have at least about 30%, 40%, 50%, 60%, 70%, 80%, 85%, 90% similarity to any of the vehicle flanking core sequence described herein. For example, the flanking sequence may contain an amino acid sequence that is at least about 30%, 40%, 50%, 60%, 70%, 80%, 85% 90%, 95%, 96%, 97%, 98% or 99% identical to any of the exemplary sequences of SEQ ID NOs: 135-158. In some embodiments, the flanking core sequence may contain an amino acid sequence that is at least about 30%, 40%, 50%, 60%, 70%, 80%, 85%, 90%, 95%, 96%, 97%, 98% or 99% identical to any of the exemplary sequences of SEQ ID NO: 135-158. In some embodiments, the vector core sequence may contain an amino acid sequence that is at least about 30%, 40%, 50%, 60%, 70%, 80%, 85%, 90%, 95%, 96%, 97%, 98% or 99% identical to any of the exemplary sequences of SEQ ID NO: 141. Variants having some sequence identity to the flanking sequence described herein such that the resulting polypeptide vehicles retain at least the biological activity of binding to OATPs (in particular OATP1B1 and OATP1B3, and in particular human OATP1B1 and human OATP1B3) and transporter of the drug or group into the cell. In some embodiments, the C-terminus of the flanking sequence can be modified. C-terminal modifications of flanking sequence may be selected from, but are not limited to, amidation (amination), prenylation, and other C-terminal modifications, or may be deleted. For example, in some embodiments, the flanking sequence C-terminal modification is selected from amidated (NH2). In some embodiments herein, the flanking sequence is selected from SEQ ID NO: 141, 158, 140, and its C-terminus is amidated. In some embodiments herein, R2 may not contain a flanking sequence but only a modifying group, or may be deleted. In some embodiments, R2 may be amidated, isopentanediolated, or other groups.

In formula (I), the amino acid with two amino groups in X can be, for example, lysine, arginine, histidine or asparagine; the amino acid with two carboxyl groups can be, for example, aspartate Acid or glutamic acid, the amino acid having other groups available for linkage reaction may be, for example, methionine. Therefore, in formula (I) herein, R2 and R3 can react with the two groups of X respectively to form an amide bond or other chemical bond for covalent linkage. These reactions include, but are not limited to, acylation reactions, nucleophilic reactions, sulfonylation reactions, and salt-forming reactions. Preferably, when R2 is a flanking sequence, R2 and X are connected by an amide bond.

R3 is a pharmacophore group, or a linking group that can interact with a drug or group (i.e. linker). The pharmacophore group or linking group is directly connected to the backbone of the vehicle or indirectly connected to the linking group, and binds to or acts on the OATP through the vehicle, to deliver the pharmacophore, drug or group linked to the vehicle portion into a cell, organ or tissue. The pharmacophore, drug or group can be linked to the OATP targeted delivery polypeptide vehicle derived from hepadnavirus surface antigen through, but not limited to, covalent bonds and/or non-covalent bonds or other means. For example, in some embodiments, R3 is a carried drug group, and the pharmacophore group and the vehicle are connected through a covalent bond, and there is or is not a linker between the pharmacophore and the vehicle. The covalent bonds include, but are not limited to, peptide bonds, ester bonds, amide bonds, disulfide bonds, polar bonds, nonpolar bonds, coordination bonds, single bonds, double bonds, triple bonds, $\sigma$ bonds, $\pi$ bonds and other covalent bonds. In some embodiments, the pharmacophore group and the vehicle are covalently linked through a linker, which can be selected from, but is not limited to, amino acid sequences, nucleotide sequences, polyethylene glycol, and other linkers. In some embodiments, the linker may be an amino acid sequence and may be selected from, but is not limited to, glycine G (Gly, G) and/or serine (Ser, S)-rich flexible linkers or other amino acid sequence linkers. In some embodiments, R3 is a linking group that can interact with a drug or group, and the linking group is connected to the drug or group through a non-covalent bond. The non-covalent bonds include, but are not limited to, hydrogen bonds, ion-dipole interactions, dipole-dipole interactions, charge interactions, hydrophobic interactions, hydrophilic interactions, van der Waals' gravitational forces, ionic bonds, salt bonds, intermolecular forces, protein interactions, protein-nucleic acid interactions, antigen-antibody interactions, receptor-ligand interactions, and other non-covalent bonding interactions. The non-covalent bonds include, but are not limited to, hydrogen bonds, ion-dipole interactions, dipole-dipole interactions, charge interactions, hydrophobic interactions, hydrophilic interactions, van der Waals' gravitational forces, ionic bonds, salt bonds, intermolecular forces, protein interactions, protein-nucleic acid interactions, antigen-antibody interactions, receptor-ligand interactions, and other non-covalent bonding interactions.

The OATP-targeted delivery peptide vehicle derived from hepatophilic DNA viral surface antigen contains a pharmacophore, drug or group including, but not limited to, small molecule drugs, nucleotides, proteins, antibodies, peptides or other drugs or groups. The small molecule drugs or groups include, but are not limited to, fluorescent groups, toxins, chemotherapeutic agents, signal modulating molecules, cell signaling agonists, cell signaling inhibitors, and other small molecule drugs or groups. The nucleotides include, but are not limited to, DNA, RNA and other nucleotide molecules or variants, including, but not limited to, oligo-DNA, oligo-RNA, poly-DNA, poly-RNA and other forms of DNA and/or RNA molecules. The nucleotide molecules have the ability to alter and regulate gene sequences, gene editing, gene

transcription, mRNA translation, and their effects include, but are not limited to, transduction of exogenous genes, protein coding, and up-regulation or down-regulation of target gene expression. The nucleic acid molecules include plasmids, DNA gene fragments, RNA gene fragments, antisense nucleic acids, nucleases, RNA interference, gene editing vectors, and other forms of nucleic acid molecules. In some more preferred embodiments herein, the vehicle contains nucleotides that are protein-coding DNA or RNA, antisense nucleic acids, nucleic acids for RNA interference, nucleotides for gene editing. In some embodiments of the invention, the OATP targeted delivery polypeptide vector derived from hepadnavirus surface antigen further include, but are not limited to, viruses. The OATP targeted delivery polypeptide vector derived from hepadnavirus surface antigen can link to a virus, or can fusion express with viral protein, or can link to a virus by other ways, to enable the virus to acquire the biological activity to infect OATP-expressing cells and thereby deliver the nucleic acid code or fragment carried by the virus into a cell or organ or tissue. The viruses include, but are not limited to, adenoviruses, lentiviruses, retroviruses, adeno-associated viruses, poxviruses, stomatitis viruses, herpesviruses, rabies virus vectors and other viruses thereof. The proteins include, but are not limited to, proteins that modulate cellular physiological functions, proteins that have pharmacological effects, proteins that kill target cells, and proteins that have other activities. The antibody includes, but is not limited to, an antibody that can regulate the physiological function of a cell after binding to a target antigen, an antibody that has pharmacological effects, an antibody that can kill a target cell, an antibody that can regulate immunity, and an antibody that has other activities, and said antibody includes, but is not limited to, a polyclonal antibody, a monoclonal antibody, a bispecific antibody, a tricomponent antibody, a tetraspecific antibody, a humanized antibody, an all-humanized antibody, an antibody fragment and other antibodies, and said antibody includes, but is not limited to, an antibody that can regulate immunity after binding to a target antigen. other antibodies. The proteins or antibodies and fragments thereof may be fusion expressed by (but not limited to) with the vehicles, or expressed and then coupled to the vehicles, or otherwise coupled to the vehicles.

In some embodiments, R3 is a fluorescent group, including but not limited to, FITC, CY3, CY5. In some embodiments, R3 is a FITC fluorescent group. Fluorescent groups such as FITC can react nucleophilically with the ε-amino group of K to form a thiourea linkage.

In some embodiments, R3 is a nucleotide, including but not limited to, DNA, RNA and other nucleotide molecules, including but not limited to, oligo-DNA, oligo-RNA, poly-DNA, poly-RNA and other forms of DNA and/or RNA molecules. The nucleotide molecules have the ability to alter and regulate gene sequences, gene editing, gene transcription, mRNA translation, and their effects on transduction of exogenous genes, and up-regulation or down-regulation of target gene expression. The nucleic acid molecules include plasmids, DNA gene fragments, RNA gene fragments, antisense nucleic acids, nucleases, RNA interference, gene editing vectors, and other forms of nucleic acid molecules.

In some embodiments herein, R3 may be a peptide, protein, or antibody for modulating the physiological function of a target cell or for killing a target cell. The peptide, protein or antibody group may be expressed by (but is not limited to) fusion with a peptide vector, or coupled after expression, e.g., by chemistry.

In some embodiments herein, the R3 group may be a virus, expressed by fusing the peptide vector to a viral protein, or linking or coupling the peptide vector to a virus, e.g., by chemistry.

In some embodiments herein, R3 is a linking group that interacts with the contained drug, which may interact with the contained drug by means of covalent or non-covalent bonding to achieve linkage between the drug and the OATP-targeting vehicle.

In some embodiments herein, the OATP targeted delivery polypeptide vehicle derived from hepatophilic DNA viral surface antigen has a core sequence comprising an amino acid sequence selected from any of those shown in SEQ ID NO: 81-134 and variants thereof; R1 may be selected from myristic acid, palmitic acid, stearic acid, polyethylene glycol and cholesterol any hydrophobic group; the R2 flanking sequence contains an amino acid sequence or variant selected from any of those shown in SEQ ID NO: 135-158, the C-terminal end of which may be amidated, or may be a modifying group only, or may be missing; R3 may be selected from any pharmacophore group of a small molecule drug, nucleotide, peptide, protein or antibody, or a linking group that may interact with any drug of a small molecule drug, nucleotide, peptide, protein or antibody.

In some embodiments herein, the OATP targeted delivery polypeptide vehicle derived from hepatophilic DNA viral surface antigen has a core sequence comprising an amino acid sequence selected from any of those shown in SEQ ID NO: 97, 94, 103-128 and variants thereof; R1 may be selected from myristic acid hydrophobic group; the R2 flanking sequence contains an amino acid sequence or variant selected from any of those shown in SEQ ID NO: 141, 140, 143-158 and variants thereof, the C-terminal end of which may be amidated, or may be a modifying group only, or may be missing; R3 may be selected from any pharmacophore group of a small molecule drug, nucleotide, peptide, protein or antibody, or a linking group that may interact with any drug of a small molecule drug, nucleotide, peptide, protein or antibody.

In some embodiments herein, the OATP targeted delivery polypeptide vehicle derived from hepatophilic DNA viral surface antigen has a core sequence comprising an amino acid sequence selected from any of those shown in SEQ ID NO: 97, 94 and variants thereof; R1 may be selected from myristic acid hydrophobic group; the R2 flanking sequence contains an amino acid sequence or variant selected from any of those shown in SEQ ID NO: 141, 140 and variants thereof, the C-terminal end of which may be amidated, or may be a modifying group only, or may be missing; R3 may be selected

from any pharmacophore group of a small molecule drug, nucleotide, peptide, protein or antibody, or a linking group that may interact with any drug of a small molecule drug, nucleotide, peptide, protein or antibody.

In some embodiments herein, the OATP targeted delivery polypeptide vehicle derived from hepatophilic DNA viral surface antigen has a core sequence comprising an amino acid sequence selected from any of those shown in SEQ ID NO: 97 and variants thereof; R1 is selected from myristic acid, palmitic acid, stearic acid, polyethylene glycol and cholesterol any hydrophobic group, respectively; the R2 flanking sequence contains an amino acid sequence or variant selected from any of those shown in SEQ ID NO: 141, the C-terminus is amidated; R3 may be selected from any pharmacophore group of a small molecule drug, nucleotide, peptide, protein or antibody, or a linking group that may interact with any drug of a small molecule drug, nucleotide, peptide, protein or antibody. The vehicles have structures shown in example structures of SEQ ID NO: 159-163 and variants thereof.

In some embodiments herein, the OATP targeted delivery polypeptide vector derived from hepadnavirus surface antigen has a core sequence comprising an amino acid sequence selected from any of those shown in SEQ ID NO: 97 and 94 and variants thereof respectively; R1 is a myristic acid group; The R2 flanking sequences are selected from the amino acid sequences shown in SEQ ID NO: 158 and 140 and their variants, respectively, the C-terminus is amidated; R3 may be selected from any pharmacophore group of a small molecule drug, nucleotide, peptide, protein or antibody, or a linking group that may interact with any drug of a small molecule drug, nucleotide, peptide, protein or antibody. The vehicles have structures shown in example structures of SEQ ID NO: 164-165 and variants thereof.

In some preferred embodiments herein, the OATP targeted delivery polypeptide vector derived from hepadnavirus surface antigen comprises an amino acid sequence selected from the exemplary sequences shown in any one of SEQ ID NO: 95-99, 103-110, and variants thereof; R1 is a myristic acid hydrophobic group; R2 is missing; R3 may be selected from any pharmacophore group of a small molecule drug, nucleotide, peptide, protein or antibody, or a linking group that may interact with any drug of a small molecule drug, nucleotide, peptide, protein or antibody. The vehicles have structures shown in SEQ ID NO: 166-178 and variants thereof.

In some preferred embodiments herein, the OATP targeted delivery polypeptide vehicle derived from hepadnavirus surface antigen comprises an amino acid sequence selected from the exemplary sequences shown in any one of SEQ ID NO: 97, and variants thereof; R1 is a myristic acid hydrophobic group; the R2 flanking sequence contains an amino acid sequence selected from the exemplary sequence shown in SEQ ID NO: 141 and variants thereof, the C-terminus is amidated; R3 is a FITC group.

The OATP targeted delivery polypeptide vector derived from hepadnavirus surface antigen can deliver a pharmacophore or a drug or group linked on, which can be used on animals including, but not limited to, mammals, birds, fish, amphibians, and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some preferred embodiments herein, the vehicle is used for drug delivery in humans, chimpanzees, bonobos, tree shrews, ducks, marmots, arctic ground squirrels, ground squirrels, bats, woolly monkeys, capuchin monkeys, and squirrel monkeys. In some preferred embodiments herein, the vehicle is used for drug delivery in humans.

The OATP targeted delivery polypeptide vehicle derived from hepadnavirus surface antigen can deliver the pharmacophore group or the drug or group connected to it into the systems, including but not limited to, digestive system, respiratory system, and circulatory system, nervous system, motor system, urinary system, reproductive system, endocrine system and other systems; can deliver the pharmacophore group or the drug or group connected to it into organs and tissues, including but not limited to, liver, kidney, lungs, heart, muscle, fat, brain, nerves, intestines, pancreas and other organs or tissues. The OATP targeted delivery polypeptide vehicle derived from hepadnavirus surface antigen can deliver the pharmacophore group or the drug or group connected to it preferably into digestive system; can deliver the pharmacophore group or the drug or group connected to it into preferably into the liver. In some embodiments herein, the OATP targeted delivery polypeptide vehicle derived from hepadnavirus surface antigen can deliver the pharmacophore group or the drug or group connected to it is applied to systems include, but are not limited to, the digestive system, the respiratory system, the circulatory system, the nervous system, the locomotor system, the urinary system, the reproductive system, the endocrine system, and other systems. In some embodiments herein, the OATP targeted delivery polypeptide vehicle derived from hepadnavirus surface antigen can deliver the pharmacophore group or the drug or group connected to it is applied to the organs and tissues include, but are not limited to, the liver, kidneys, lungs, heart, muscle, fat,

brain, nerves, intestines, pancreas, and other organs or tissues. In some embodiments herein, the OATP targeted delivery polypeptide vehicle derived from hepadnavirus surface antigen can deliver the pharmacophore group or the drug or group connected to it is applied to cells include, but are not limited to, hepatocytes, lung cells, kidney cells, nerve cells, muscle cells, adipocytes, blood cells, lymphocytes, and other cells. In some embodiments herein, the OATP targeted delivery polypeptide vehicle derived from hepadnavirus surface antigen can deliver the pharmacophore group or the drug or group connected to it is applied to deliver the drug into the digestive system, and/or the liver, and/or hepatocytes.

A hepadnavirus surface antigen-derived peptide that binds to OATP and inhibits its transport function and its application

This invention also provides a polypeptide that binds OATP and inhibits its transport function. The polypeptide is derived from the hepadnavirus surface antigen and at least retains the effect of binding OATP and inhibiting the transport function of OATP, that is, the polypeptide is derived from the hepadnavirus surface antigen binding OATP and inhibiting its transport function or "OATP-binding inhibitory peptide derived from hepadnavirus surface antigen", and which is referred to as OATP-binding inhibitory polypeptide. In this article, the OATP-binding inhibitory peptide can be used to inhibit the OATP transport function, regulate the pharmacokinetics of drugs using OATP as a transporter, and improve the efficacy and safety of these drugs. Therefore, in some embodiments, provided herein is the use of an OATP-binding inhibitory peptide described herein in the manufacture of drugs that modulate the pharmacokinetics of drugs that use OATP as a transporter and improve the efficacy and safety of these drugs.

The OATPs bound and inhibited by the OATP-binding inhibitory peptide described herein, include, but are not limited to, OATP superfamily derived from mammals, birds, fishes, amphibians, and other animals. In some embodiments, the OATP superfamily includes, but is not limited to, OATP1, OATP2, OATP3, OATP4, OATP5, OATP6, OATP7, OATP8, OATP9, OATP10, OATP11 and other OATP families. In some embodiments, the OATP includes, but is not limited to, the OATP1B subfamily. In some preferred embodiments, the OATPs include, but are not limited to, OATP1B3, OATP1B1, OATP1B2, and other OATP1B subfamily members.

The OATPs bound and inhibited by the OATP-binding inhibitory peptide described herein, include, but are not limited to, OATP derived from mammals, birds, fishes, amphibians, and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some embodiments herein, In some embodiments herein, the OATP1B3 bound and inhibited by the OATP-binding inhibitory peptide described herein includes, but is not limited to, the OATP1B3 derived from humans (SEQ ID NO: 1, NCBI Reference Sequence: NM_019844), the OATP1B3 derived from chimpanzees (NCBI Reference Sequence: XM_016923821), the OATP1B3 derived from tree shrews (NCBI Reference Sequence: XM_006155085), the OATP1B3 derived from Macaca fascicularis (NCBI Reference Sequence: NM_001283191), the OATP1B3 derived from arctic ground squirrel (NCBI Reference Sequence: XM_026391932), the OATP1B3 derived from rats (NCBI Reference Sequence: XM_032905480), the OATP1B3 derived from pigs (NCBI Reference Sequence: NM_001315607), the OATP1B3 derived from dogs (NCBI Reference Sequence: NM_001166047), and the OATP1B3 derived from the other animals mentioned above. In some preferred embodiments herein, the OATP1B3 bound to and modulating its transport function is that of a human primate, rodent, or bird. In some further preferred embodiments herein, In some embodiments herein, the OATP1B3 bound and inhibited by the OATP-binding inhibitory peptide described herein is human OATP1B3. In some embodiments herein, the polypeptide binds to and inhibits the transport function of OATP1B1, including but not limited to OATP1B1 derived from humans (an example sequence can be found in SEQ ID NO: 4, NCBI Reference Sequence: NM_006446), OATP1B1 derived from bonobos (Example sequences can be found in NCBI Reference Sequence: XM _003828901), OATP1B1 derived from Macaca fascicularis (Example sequences can be found in NCBI Reference Sequence: NM_ 001284540), and the OATP1B1 derived from the other animals mentioned above. In some preferred embodiments herein, the OATP1B1 bound to and modulating its transport function is that of a human primate, rodent, or bird. In some further preferred embodiments herein, the OATP binding inhibiting polypeptide binds human OATP1B1 and inhibits its transport function. In some embodiments herein, the OATP1B2 bound and inhibited by the OATP-binding inhibitory peptide described herein includes, but is not limited to, the OATP1B2 derived from rats (NCBI Reference Sequence: NM_031650), the OATP1B2 derived from mice (NCBI Reference Sequence: NM_020495),

and the OATP1B2 derived from the other animals mentioned above. In some preferred embodiments herein, the OATP that bound and inhibited its transport function by the OATP-binding inhibitory peptide is human OATP1B3 and/or human OATP1B1.

In some embodiments herein, the OATP-binding inhibitory peptide can bind and inhibit the OATP transport function alone, or can simultaneously bind and modulate the OATP and NTCP transport functions. In some embodiments herein, when the polypeptide simultaneously binds and modulates OATP and NTCP, the bound and modulated NTCP includes, but is not limited to, mammals, birds, fishes, amphibians and other animals. The mammals include, but are not limited to, primates, tree shrews (tupaia, tree threw), rodents, bats, Artiodactyla, Carnivora and other mammals. The birds include, but are not limited to, ducks, herons, and other birds. The primates include, but are not limited to, hominoid species, New World monkeys, old World monkeys, and other primates. The hominoid species includes, but is not limited to, humans, chimpanzees, gorillas, gibbons, orangutans, and other humanoid species. The New World monkeys include, but are not limited to, Woolly monkeys, capuchin monkeys, Squirrel monkeys and other New World monkeys. The old world monkeys include, but are not limited to, Macaca fascicularis and other old world monkeys. The rodents include, but are not limited to, animals of the family Sciuridae, myomorpha, and other rodents. The Sciuridae animals include, but are not limited to, arctic ground squirrels, ground squirrels, woodchucks, and other Sciuridae animals. The myomorpha animals include, but are not limited to, rats, mice and other myomorpha animals. The artiodactyla animals include, but are not limited to, pigs and other artiodactyla animals. The animals of the order Carnivora include, but are not limited to, dogs and other animals of the order Carnivora. In some embodiments of the present invention, the NTCP simultaneously bind to the OATP-binding inhibitory peptide includes, but is not limited to, NTCP derived from humans (SEQ ID NO: 7, NCBI Reference Sequence: NM_003049), NTCP derived from chimpanzees (NCBI Reference Sequence: XM_510035), derived from NTCP from tree shrews (NCBI Reference Sequence: XM_006171503), NTCP from bonobos (NCBI Reference Sequence: XM_003824101), NTCP from capuchin monkeys (NCBI Reference Sequence:XM_032245781), NTCP from squirrel monkeys (NCBI Reference Sequence:XM_003924480), NTCP from macaca fascicularis (NCBI Reference Sequence:NM_001283323), NTCP from arctic ground squirrels (NCBI Reference Sequence:XM_026385176), NTCP from ground squirrels (NCBI Reference Sequence:XM_005322843), NTCP derived from rats (NCBI Reference Sequence: XM_032908052), NTCP derived from mice (NCBI Reference Sequence: NM_001177561), NTCP derived from pigs (NCBI Reference Sequence: XM_001927695), NTCP derived from dogs (NCBI Reference Sequence: XM_537494), as well as NTCP and its variants derived from other animals mentioned above. In some preferred embodiments herein, the NTCP simultaneously bind to the OATP-binding inhibitory peptide is that of a human primate, rodent, or bird. In some further preferred embodiments herein, the NTCP simultaneously bind to the OATP-binding inhibitory peptide is human NTCP.

In some embodiments herein, said inhibited OATP transport function includes, but is not limited to, OATP transport of the following substrates. Exemplary substrates of OATP1B1 include, but are not limited to, sartans, statins, bile acids and their derivatives, tinibs, platinum drugs, paclitaxel drugs, chemotherapy drugs, chloride drugs, non-Solifenadine, methotrexate, bosentan, atrasentan, rifampin, SN-38, irinotecan, cephalosporins, repaglinide, sirolimus, saquinavir, Lopinavir, torsemide and other drugs. Exemplary substrates of OATP1B3 include sartans, statins, bile acids and their derivatives, tinibs, platinums, paclitaxels, chemotherapy drugs, bosentan, digoxin, hama, fexofenadine, rifampicin, atrasentan, methotrexate, CCK-8, nefcillin and other drugs. The statins include, but are not limited to, simvastatin, lovastatin, pravastatin, fluvastatin, atorvastatin, rosuvastatin, cerivastatin, rosuvastatin, pitavastatin and other statin drugs. The sartan drugs include, but are not limited to, telmisartan, valsartan, irbesartan, olmesartan, valsartan, and other sartan drugs. The platinum drugs include but are not limited to cisplatin, carboplatin, oxaliplatin and other platinum drugs. The paclitaxel drugs include but are not limited to paclitaxel, docetaxel, albumin paclitaxel, liposomal paclitaxel and other paclitaxel drugs and derivatives. Tinib drugs include, but are not limited to, imatinib, gefitinib, erlotinib, sunitinib, dasatinib, lapatinib, nilotinib, ruxolitinib, and crazole icotinib, icotinib, tofacitinib, bosutinib, cabozantinib, ponatinib, axitinib, ibrutinib, trametinib, ceritinib, apatinib, alectinib, lenvatinib, osimertinib, neratinib, acalabrutinib, brigatinib, baricitinib, and other tinib drugs and their derivatives. Cephalosporins include, but are not limited to, cefoperazone, cefazolin, cefditoren, cefradine, cefmetazole, cephalexin and other cephalosporins. The prici drugs include but are not limited to captopril, enalapril, benazepril, lisinopril, ramipril, perindopril, fosinopril, imidapril, Cilazapril and other cilazapril drugs and their derivatives.

the OATP-binding inhibitory peptide is derived from the surface antigen of hepadnaviridae, and at least retains the biological activity of binding OATP and inhibiting the OATP substrate transport function. The hepadnaviruses include, but are not limited to, Orthohepadnavirus, Avihepadnavirus, Metahepadnavirus, Herpetohepadnavirus and other hepadnaviruses. The orthohepadnavirus DNA viruses includes but is not limited to infecting humans (HBV), woolly monkeys (Woolly monkey HBV, WMHBV), capuchin monkeys (Capuchin monkey HBV, CMHBV), squirrel monkey, woodchuck (Woodchuck hepatitis virus, WHV), arctic ground squirrel, ground squirrel (ground squirrel hepatitis virus, GSHV), tree shrew, chimpanzee, bat (Bat hepatitis virus, BHV) and other animals, example genome sequences can be found in GenBank accession numbers including but not limited to AF046996 (WMHBV), NC_043528 (CMHBV), NC_004107 (WHV), NC_001484 (GSHV), JX941468 (BHV). The Avihepadnavirus includes, but is not limited to, infecting ducks (duck

HBV, DHBV), herons (heron HBV, HHBV) and other birds, and example genome sequences can be found in, but are not limited to, GenBank accession numbers NC_001344 (DHBV), NC_001486 (HHBV). Orthohepadnavirus that infect humans (HBV) described herein include, but are not limited to, HBV genotype A, B, C, D, E, F, G, H, and I, and example genome sequences can be found in, but are not limited to, GenBank accession numbers KC875260 (genotype A), AY220704 (genotype B), AF461363 (genotype C), AY 796030 (genotype D), AB205129 (genotype E), DQ823095 (genotype F), HE981176 (genotype G) and AB179747 (genotype H). In some preferred embodiments herein, the amino acid sequence of the OATP-binding inhibitory peptide is derived from the amino acid sequence of the Pre-S region of the viral surface antigen, including but not limited to the exemplary surface antigen Pre-S amino acid sequence SEQ ID NO: 8-22 and its variants. In some preferred embodiments herein, the amino acid sequence of the OATP-binding inhibitory peptide is derived the surface antigen of Pre-S region of human HBV, including but not limited to genotypes A, B, C, D, E, F, G, H and I HBVP Pre-S region amino acid sequences and variants thereof, including but not limited to the exemplary amino acid sequences of surface antigen of HBV Pre-S region shown in SEQ ID NO: 15-22 and variants thereof. In some preferred embodiments herein, the amino acid sequence of the OATP-binding inhibitory peptide is derived the surface antigen of Pre-S region of human HBV, including but not limited to genotypes C HBVP Pre-S region amino acid sequences and variants thereof, including but not limited to the exemplary amino acid sequences of surface antigen of HBV Pre-S region shown in SEQ ID NO: 17 and variants thereof.

The term "OATP-binding inhibitory peptide derived from hepadnavirus surface antigen " or "hepadnavirus surface antigen-derived peptide that binds OATP and inhibits its transport function", which may also be referred to as "OATP-binding inhibitory peptide", is refers to polypeptides whose origin or source is hepadnavirus, including but not limited to natural, recombinant, Synthetic or purified polypeptides include, but are not limited to, full-length natural HBV polypeptides or fragments thereof, and variants of full-length natural polypeptides or fragments thereof. In some embodiments, the OATP-binding inhibitory peptide can be derived from any hepadnavirus, including but not limited to, orthohepadnavirus (OHV) that infects mammals, avihepadnavirus that infects birds (AHV), metahepadnavirus viruses that infects fish, and herpetohepadnavirus that infects amphibians; in some embodiments, the OATP-binding inhibitory peptide may be derived from any hepadnavirus surface antigen; in some embodiments, the OATP-binding inhibitory peptide can be derived from any orthohepadnavirus surface antigen; in some embodiments, the OATP-binding inhibitory peptide can be derived from any human hepatitis B virus (HBV) surface antigen, including, but not limited to, derived from surface antigen of HBV genotype A, B, C, D, E, F, G, H, and I; in some embodiments, the OATP-binding inhibitory peptide may be derived from any human hepatitis B virus (HBV) surface antigen Pre-S1 region, an OATP-binding HBVPre-S1-derived inhibitory peptide or an OATP-binding inhibitory peptide derived from HBVPre-S1.

The terms "OATP-binding inhibitory peptide derived from HBVPre-S1" or "OATP-binding HBVPre-S1-derived inhibitory peptide" as used herein refers to the polypeptide whose origin or source is the surface antigen of Pre-S1 region of HBV, including but not limited to natural, recombinant, synthetic or purified polypeptides, including but not limited to full-length natural surface antigen of HBV Pre-S1 region or fragments thereof and variants thereof, full-length non-natural surface antigen of HBV Pre-S1 region or fragments thereof and variants thereof, non- Full-length natural surface antigen of HBV Pre-S1 region or fragments thereof and variants thereof, non-full-length non-natural surface antigen of HBV Pre-S1 region or fragments thereof and variants thereof, and other forms of variants. In some embodiments, OATP-binding inhibitory peptide derived from HBVPre-S1 may contain the entire surface antigen pre-S1 from the second amino acid G (glycine) in the surface antigen of Pre-S1 region of any HBV genotype. In certain embodiments, the OATP-binding inhibitory peptide derived from HBVPre-S1 described herein may be derived from the pre-S1 region of the surface antigen of any one of HBV genotypes A, B, C, D, E, F, G, H and I. Example genome sequences of these HBV genotypes can be found in GenBank accession numbers KC875260, AY220704, AF461363, AY796030, AB205129, DQ823095, HE981176, and AB179747, and exemplary amino acid sequences of their surface antigen of Pre-S regions can be found in, but are not limited to, SEQ ID NO: 8-22 and variants thereof. In some embodiments, the OATP-binding inhibitory peptide derived from HBVPre-S1 described herein can be derived from the surface antigen of pre-S1 region of the HBV genotype C. The OATP-binding inhibitory peptide derived from HBVPre-S1 at least retains the biological activity of binding to OATP, especially retains the biological activity of binding to OATP1B1, and/or OATP1B2 and/or OATP1B3, especially retains the biological activity of binding to human OATP1B1 and/or human OATP1B3.

In some embodiments, the OATP-binding inhibitory peptide described herein may be 10-118 amino acids in length. For example, the polypeptide may be 15-100, 15-80, 20-100, 20-80, 20-60, 25-60, 30-60, 35-60, or 40-60 (including all integers in these ranges) amino acids in length. In some embodiments, the OATP-binding inhibitory peptide described herein may be at least 20 amino acids in length, e.g., at least 25, 30, 35 or 40 amino acids in length. In some embodiments, the OATP-binding inhibitory peptide described herein may be but are not limited to 20, 25, 30, 35, 40, 47, 55 or 60 amino acids in length. In some embodiments, the OATP-binding inhibitory peptide described herein may be 47 amino acids in length. Variants of OATP-binding inhibitory peptides of different lengths described herein retain one or more biological activities associated with the corresponding polypeptides, including at least: binding to OATP (especially OATP1B1 and/or OATP1B3) and inhibiting OATP (especially OATP1B1 and/or OATP1B3) transporter function.

In some embodiments, the OATP-binding inhibitory peptides described herein can be derived from the HBV

genotype C surface antigen pre-S1 region, and the OATP binding inhibitory peptides include but are not limited to amino acids 2-119, 2-69, 2-59, 13-119, 13-88, 13-72, 13-67, 13-59, 13-52, 13-47, 13-42, 13-37, 13-32, and the exemplary sequences thereof include but are not limited to SEQ ID NO: 23-35.

In some embodiments, the OATP-binding inhibitory peptides comprises the amino acid sequence of at least amino acid residues 13-44 at the N-terminal of the pre-S1 region of surface antigen of HBV genotype C described herein and variants thereof. In preferred embodiments, the OATP-binding inhibitory peptide may be 32-47 amino acid residues in length. In a particularly preferred embodiment, the OATP binding inhibitory peptide is an amino acid sequence of the amino acid sequence shown in SEQ ID NO: 30 and its variants, which contains at least amino acid residue at position 1-32 of the sequence of SEQ ID NO: 30. Preferably, the OATP-binding inhibitory peptide contains at least amino acid residues and variants thereof at positions 1 to 33, 34, 35, 36, 37, 38, 39, 40, and 41 of the exemplary sequence SEQ ID NO: 30. In some embodiments, the OATP-binding inhibitory peptide refers to at least the positions 13-44 of the N-terminus of the surface antigen of Pre-S1 region of HBV genotypes A, B, F, H, and I described herein, a fragment of at least positions 2-33 of the N-terminus of the HBV surface pre-S1 region of genotype D or its variant, or a fragment of at least positions 12-43 of the N-terminus of the HBV surface pre-S1 region of genotype E and G or its variant. In some embodiments, the OATP-binding inhibitory peptide refers to at least the positions 13-69 of the N-terminus of the surface antigen of Pre-S1 region of HBV genotypes A, B, F, H, and I described herein, a fragment of at least positions 2-48 of the N-terminus of the HBV surface pre-S1 region of genotype D or its variant, or a fragment of at least positions 12-68 of the N-terminus of the HBV surface pre-S1 region of genotype E and G or its variant, example sequences thereof include but are not limited to the example sequences SEQ ID NO: 36-42 and variants thereof.

The "variant" related to the OATP-binding inhibitory peptide derived from HBVPre-S1 refers to a peptide that differs in amino acid sequence from a given polypeptide at least retains the biological activity of binding to OATP, especially retains the biological activity of binding to OATP1B1, and/or OATP1B2 and/or OATP1B3, especially retains the biological activity of binding to human OATP1B1 and/or human OATP1B3. For example, in some embodiments, N15D, F25L, G35K, N39E, N46K, F45L, N48H, N48Y, N48K, D50A, H51Q, H51N, E54K, E54D, A55S, N56K, N56D, Q57K amino acid substitutions or mutation combinations can be introduced into the derived peptides derived from the 13-59 amino acid sequence of the genotype C HBVPre-S1 region, example sequences thereof include but are not limited to SEQ ID NO: 43-60. As used herein, the term "variant" also includes homologous polypeptide sequences found in different viral species, strains, or subtypes of the genus Hepatovirus. Based on the antigenic epitopes on its envelope protein, HBV is classified into four main serotypes (adr, adw, ayr, and ayw), and according to the variability of the nucleotide sequence in the genome, HBV is divided into nine genotypes (A to I). Thus, the term "variant" includes any of these homologous polypeptides found in HBV subtypes. "Variants" may also include polypeptides with natural flanking amino acid sequences from any of these HBV subtypes added at the N- and/or C-terminus, or variants thereof. For example, in some embodiments, the polypeptide derived from the 13-59 amino acid sequence of the genotype C surface antigen Pre-S1 region can be combined with the polypeptide including but not limited to derived from the surface antigen of Pre-S1 region of HBV genotypes A, B, F, H, and I, a fragment of at least amino acid residues 2-12 at the N-terminus of the S1 region, or a fragment of at least amino acid residues 2-11 at the N-terminus of the surface antigen pre-S1 region of HBV genotype E or G, forms a fusion polypeptide, and its example sequence including but not limited to the sequences shown in SEQ ID NO: 61-66. For example, in some embodiments, the polypeptide derived from the HBV Pre-S1 region described herein may contain an amino acid sequence selected from, but not limited to, any of SEQ ID NO: 23-66, and natural amino acid sequence flanking the N- and/or C-terminus derived from the surface antigen of any of HBV genotypes A-I. In some embodiments, the natural flanking amino acid sequences can be derived from the amino acid sequences of HBV surface antigens, including but not limited to GenBank accession numbers KC875260 (genotype A), AY220704 (genotype B), AF461363 (genotype C), AY796030 (amino acid sequence of HBV surface antigen of genotype D), AB205129 (genotype E), DQ823095 (genotype F), HE981176 (genotype G) or AB179747 (genotype H). For example, the polypeptide described herein may contain the amino acid sequences shown by SEQ ID NO: 30 and contain at its N and/or C terminus a natural flanking amino acid sequence from the pre-S1 region of the HBV genotype C. Alternatively, the polypeptide described herein may contain the amino acid sequences shown by SEQ ID NO: 30 and contain at its N and/or C terminus a natural flanking amino acid sequence from the pre-S1 region of any of the HBV genotype A, B, C, D, E, F, G and H. In some embodiments, the N-terminus and/or C-terminus of the OATP-binding inhibitory peptide described herein may independently contain a natural flanking amino acid sequence of 1-10, e.g., 1-8, 1-5, 1-3, (including all integers within these ranges) amino acid length. For example, the OATP-binding inhibitory peptide described herein may contain the amino acid sequence shown in SEQ ID NO: 30 and at its N-terminus a 10 amino acid long native flanking amino acid sequence from the pre-S1 region of HBV genotype C. In other words, the peptide may contain amino acids 2-59 of the HBV genotype C pre-S1 region (SEQ ID NO: 25). As another example, the OATP-binding inhibitory peptide described herein may contain the amino acid sequence shown in SEQ ID NO: 30 and at its N-terminus a 9 amino acid long native flanking amino acid sequence from the HBV genotype E pre-S1 region. In other words, the peptide may contain amino acids 13-59 of the HBV genotype C pre-S1 region and amino acids 2-11 of the HBV genotype E pre-S1 region (SEQ ID NO: 63). It will be appreciated that any of the OATP-binding inhibitory peptide described can have any length of native flanking amino acid sequence extending from its

N and/or C terminus, and that the resulting polypeptide retains the biology of the original polypeptide binding to OATP and inhibiting its transport function, especially biological activity that binds to OATP1B3, and/or OATP1B2, and/or OATP1B1 and inhibiting its transport function, especially biological activity that binds to human OATP1B3 and/or OATP1B1 and inhibiting its transport function. The "variant" related to the OATP-binding inhibitory peptide derived from HBVPre-S1 refers to a peptide that comprising having one or more amino acid deletions, substitutions or insertions retains the biological activity of binding to OATP, especially retains the biological activity of binding to OATP1B1, and/or OATP1B2 and/or OATP1B3, especially retains the biological activity of binding to human OATP1B1 and/or human OATP1B3. The OATP-binding inhibitory peptide preferably retains the amino acid corresponding to the glycine at position 13 of the HBV genotype C pre-S1 region (i.e., the N-terminal glycine of SEQ ID NO: 30). In some embodiments, the OATP-binding inhibitory peptide described herein may have one or more naturally occurring mutations in the HBV pre-S1 region. For example, in some embodiments, relative to the sequence from the HBV pre-S1 region, the OATP-binding inhibitory peptide described herein may have 1-30, such as 1-20, 1-10, 1-8, 1-5 or 1-3 (including all integers within these ranges) amino acids deletions, substitutions or insertions. In some embodiments, the OATP-binding inhibitory peptide described herein may have 1-30, such as 1-20, 1-10, 1-8, 1-5 or 1-3 (including all integers within these ranges) amino acid deletions, substitutions or insertions relative to any amino acid sequence selected from SEQ ID NO: 23-66. In some embodiments, the OATP-binding inhibitory peptide described herein may have 1-30, such as 1-20, 1-10, 1-8, 1-5 or 1-3 (including all integers within these ranges) amino acid deletions, substitutions or insertions relative to any amino acid sequence selected from SEQ ID NO: 30. In some embodiments, the OATP-binding inhibitory peptide described herein may have 1-3 amino acid deletions, substitutions, or insertions relative to the exemplary amino acid sequence of SEQ ID NO: 30. In some embodiments, the OATP-binding inhibitory peptide described herein may have 1-30, such as 1-20, 1-10, 1-8, 1-5 or 1-3 (including all integers within these ranges) amino acid deletions, substitutions or insertions at the C-terminus relative to any amino acid sequence selected from SEQ ID NO: 23-66. For example, the OATP-binding inhibitory peptide may include, but is not limited to, an amino acid sequence selected from any one of SEQ ID NO: 31-35. In various embodiments, the present description includes, but are not limited to, the OATP-binding inhibitory peptide and other polypeptides that has at least about 30%, 40%, 50%, 60%, 70%, 80%, 85%, 90%, 95%, 96%, 97%, 98% or 99% identity with any of the polypeptides. For example, the OATP-binding inhibitory peptide may contain an amino acid sequence that is at least about 30%, 40%, 50%, 60%, 70%, 80%, 85% 90%, 95%, 96%, 97%, 98% or 99% identical to any of the exemplary sequences of SEQ ID NOs: 23-66. In some embodiments, the OATP-binding inhibitory peptide may contain an amino acid sequence that is at least about 30%, 40%, 50%, 60%, 70%, 80%, 85% 90%, 95%, 96%, 97%, 98% or 99% identical to any of the exemplary sequences of SEQ ID NOs: 23-66. In some embodiments, the OATP-binding inhibitory peptide may contain an amino acid sequence that is at least about 30%, 40%, 50%, 60%, 70%, 80%, 85% 90%, 95%, 96%, 97%, 98% or 99% identical to the exemplary sequences of SEQ ID NO: 30. The variants having a certain sequence identity with the OATP-binding inhibitory peptides described herein retain one or more biological activities associated with the corresponding polypeptide, including at least the biological activity of binding to OATP and inhibiting its transport function, particularly to OATP1B1, and/or OATP1B2, and/or OATP1B3 and inhibiting its transport function, especially the biological activity of binding to human OATP1B1 and/or human OATP1B3 and inhibiting its transport function.

The OATP-binding inhibitory peptide derived from hepadnavirus surface antigen its variants have a hydrophobic group modification at the N-terminus, such as but not limited to myristic acid (myr), palmitic acid (plam), stearic acid (stearoyl), cholesterol (chol), oleic acid, linoleic acid, polyethylene glycol (PEG), arachidonic acid or other hydrophobic groups. In some embodiments, the hydrophobic group may be selected from myristic acid, palmitic acid, stearic acid, and cholesterol. In some embodiments, the hydrophobic group can be myristic acid. In some embodiments, the OATP-binding inhibitory peptide described herein may contain an amino acid sequence selected from any one of SEQ ID NO: 23-66 and variants thereof, wherein the N-terminus of the OATP-binding inhibitory peptide may be selected from hydrophobic group modification of myristic acid, palmitic acid, stearic acid and cholesterol. In some embodiments, the OATP-binding inhibitory peptide may comprise an amino acid sequence selected from any one of SEQ ID NO: 23-66 or a variant thereof, wherein the N-terminus of the OATP-binding inhibitory peptide can be myristoylated. In some embodiments, the OATP-binding inhibitory peptide described herein may contain the amino acid sequence shown in SEQ ID NO: 30 and SEQ ID NO: 92, wherein the N-terminus of the OATP-binding inhibitory peptide may be myristoylated. In some embodiments, the C-terminus of the OATP-binding inhibitory peptide may be modified. C-terminal modifications may be selected from, but are not limited to, amidation (amination), prenylation, and other C-terminal modifications, or may be deleted. In some embodiments, the C-terminal modification may be amidation ($NH_2$). For example, but not limited to, the OATP-binding inhibitory peptide may contain the amino acid sequence shown in SEQ ID NO: 30 and SEQ ID NO: 60, and its C-terminus may be amidated. In some embodiments, the N-terminus of the OATP-binding inhibitory peptide can be modified with a hydrophobic group, and/or the C-terminus can be otherwise modified. In some embodiments, the N-terminus of the OATP-binding inhibitory peptide can be modified with a hydrophobic group, and/or the C-terminus can be amidated. In certain embodiments, the OATP-binding inhibitory peptide may contain an amino acid sequence selected from any one of SEQ ID NO: 23-66 and variants thereof, the N-terminus of which may be modified by hydrophobic groups, and/or the C-terminus may be amidated. For example, the OATP-binding inhibitory peptide may contain the amino acid

sequence shown in SEQ ID NO: 30 and SEQ ID NO: 60, and its N-terminus being modified by hydrophobic group of myristic acid (myr), palmitic acid (plam), stearic acid (stearoyl), cholesterol (chol), the C-terminus of which is amidated, such as the polypeptide shown in SEQ ID NO: 67-71. In some embodiments, the N-terminus of the OATP-binding inhibitory peptide can be modified by a myristic acid (myr) hydrophobic group, and/or the C-terminus can be amidated. In certain embodiments, the OATP-binding inhibitory peptide may comprise an amino acid sequence selected from any one of SEQ ID NO: 23-66 and variants thereof, the N-terminus of which may be modified by myristoylation, and/or the C-terminus may be amidated. For example, but not limited to, the OATP-binding inhibitory peptide may comprise the polypeptide shown in SEQ ID NO: 30, 36-42, 60, the N-terminus of which is modified by a hydrophobic group of myristic acid (myr) hydrophobic group, and the C-terminus of which is amidated, as shown in any of SEQ ID NO: 67, 68, 72-78.

The polypeptide and variants thereof of the OATP-binding inhibitory peptide derived from hepadnavirus surface antigen retain at least the biological activity of binding to OATP and inhibiting its translocation function. In some embodiments, the OATP-binding inhibitory peptide is derived from viruses including, but not limited to, Orthohepadnavirus, Avihepadnavirus, Metahepadnavirus, Herpetohepadnavirus, and other hepadnaviruses. In some embodiments, the OATP-binding inhibitory peptide is derived from a species including, but not limited to, human (HBV), Woolly monkey HBV (WMHBV), capuchin monkey HBV (CMHBV), squirrel monkey, woodchuck (woodchuck hepatitis virus, WHV), arctic ground squirrel, ground squirrel (ground squirrel hepatitis virus, GSHV), tree shrews, chimpanzees, bats (Bat hepatitis virus, BHV) and other animals, as well as infecting ducks Avihepadnavirus of HBV(duck HBV, DHBV), heron (heron HBV, HHBV) and other birds. In some embodiments, the OATP-binding inhibitory peptide is derived from the surface antigen of Pre-S region of HBV, including but not limited to, genotypes A, B, C, D, E, F, G, H, and I. In some embodiments, the OATP-binding inhibitory peptide is derived from including, but not limited to, amino acids 13-59 of HBV genotype C Pre-S. The N-terminus of the OATP-binding inhibitory peptide is modified with a hydrophobic group, and the C-terminus may be modified with a stabilizing modification or deleted. In some embodiments, the N-terminal of the OATP-binding inhibitory peptide is modified with a hydrophobic group of myristic acid (myr), palmitic acid (plam), stearic acid (stearoyl), and cholesterol (chol), and the C-terminal can be amidated. In some embodiments, the N-terminus of the OATP-binding inhibitory peptide can be modified by a myristic acid (myr) hydrophobic group, the C-terminus can be amidated. The OATP-binding inhibitory peptide derived from HBVPre-S1 at least retains the biological activity of binding to OATP and inhibiting its translocation function, especially retains the biological activity of binding to OATP1B1, and/or OATP1B2 and/or OATP1B3 and inhibiting its translocation function, especially retains the biological activity of binding to human OATP1B1 and/or human OATP1B3 and inhibiting its translocation function. In some embodiments, the OATP-binding inhibitory peptide specifically including but not limited to the polypeptides shown in SEQ ID NO: 67-78 and variants thereof; particularly including but not limited to the polypeptides shown in SEQ ID NO: 67 (myrC13-59 NH2) and SEQ ID NO: 68 (MyrC13-59NH2 (Q57K) and variants thereof.

In some embodiments, the OATP-binding inhibitory peptide can treat or prevent disease by binding to and inhibiting OATP substrate transport. Specifically, treatment and prevention of disease are accomplished by administering an optional dose of the OATP-binding inhibitory peptide. In some embodiments, a method of treating or preventing a disease described herein is administering the OATP-binding inhibitory peptide and a therapeutic agent, wherein therapeutic agent is an OATP substrate, transported via OATP; in some embodiments, provided herein are uses of the OATP-binding inhibitory peptides described herein and therapeutic agents in the manufacture of medicaments for the treatment or prevention of disease. Preferably, therapeutic agent is as described in any embodiment of this article, especially the sartan drugs, statin drugs, bile acid derivatives, anti-tumor drugs, etc. described below. Through the OATP binding inhibitory peptide described herein, it binds to OATP and inhibits the transport of therapeutic agent by OATP, thereby improving the pharmacokinetic properties of therapeutic agent, Including but not limited to increasing the blood concentration of therapeutic agent, prolonging the survival time of therapeutic agent in the blood, extending the half-life of therapeutic agent, increasing the concentration of therapeutic agent in the target tissue or organ, thereby improving therapeutic effect of therapeutic agent. In some preferred embodiments herein, therapeutic agent is a sartan drug, and the OATP-binding inhibitory peptide described in this article is used in combination with sartan drugs, which can inhibit the transport of sartan drugs by OATP and improve the efficacy of sartan drugs.

In other embodiments, a method of treating or preventing a disease described herein is by administering an OATP-binding inhibitory peptide described herein and a therapeutic agent, wherein therapeutic agent is an OATP substrate and is cytotoxic. The OATP binding inhibitory polypeptide described herein binds to OATP, inhibits the transport of therapeutic agents by OATP, and reduces intracellular drug concentrations (especially in hepatocytes), reduces the toxic effects of therapeutic agents on cells, improves the safety of therapeutic agents. These therapeutic agents include, but are not limited to, statin drugs, cholic acid derivatives (including, but not limited to, obeticholic acid, ursodeoxycholic acid, and other cholic acid derivatives), and other drugs. Statin drugs include but are not limited to simvastatin, atorvastatin, rosuvastatin, pravastatin, pitavastatin, lovastatin, Atorvastatin, fluvastatin, cerivastatin, mevastatin and other statin drugs. In some preferred embodiments herein, therapeutic agent is a statin drug, and the OATP-binding inhibitory peptide described in this article is used in combination with statin drugs, which can inhibit the transport of statin drugs by OATP and improve the efficacy of statin drugs.

In other embodiments, a method of treating or preventing a disease described herein is by administering an OATP-binding inhibitory peptide described herein and a therapeutic agent. While reducing the concentration of intracellular therapeutic agents and reducing the cytotoxicity of therapeutic agents, it also improves the pharmacokinetic properties of therapeutic agents and improves the efficacy of therapeutic agents, thus achieving both improved efficacy and reduced toxic and side effects. In some preferred embodiments herein, therapeutic agent is an anti-tumor drug, and the OATP-binding inhibitory peptide described in this article is used in combination with anti-tumor drugs, which can inhibit the transport of anti-tumor drugs by OATP and improve the efficacy of anti-tumor drugs. At the same time, it improves the pharmacokinetic properties of anti-tumor drugs and improves the efficacy of anti-tumor drugs. The anti-tumor drugs include, but are not limited to, platinum, paclitaxel, tinib, methotrexate and other anti-tumor drugs.

In other embodiments, a method of treating or preventing a disease described herein is the administration of an OATP-binding inhibitory peptide described herein. The OATP-binding inhibitory polypeptide described herein binds to OATP, inhibits the transport of physiological substrates by OATP in the body, regulates the distribution of physiological substrates, and thereby prevents or treats corresponding diseases. The OATP physiological substrates include but are not limited to bile acids, hormones, organic anions and other OATP physiological substrates. The bile acids include, but are not limited to, cholic acid (CA), deoxycholic acid (DCA), chenodeoxycholic acid (CDCA), ursodeoxycholic acid (UDCA), lithocholic acid (LCA), and the combination of these bile acids with Glycine, taurine, sulfuric acid, glucuronic acid conjugation products, and other bile acids. In some embodiments herein, diseases that can be used to prevent or treat OATP-binding inhibitory peptides as described herein include, but are not limited to, liver diseases, metabolic diseases, neurological diseases, digestive system diseases, fundus diseases, circulatory system diseases, and immune system diseases and other systemic diseases. The liver diseases include, but are not limited to, fatty liver, alcoholic fatty liver disease, alcoholic steatohepatitis, non-alcoholic fatty liver disease (NAFLD), non-alcoholic steatohepatitis (NASH), cholestasis, primary biliary hepatitis Cholangitis, biliary cholangitis, primary biliary cirrhosis (PBC) and other liver diseases related to bile acid, cholesterol, fat, sugar metabolism and other liver diseases. Metabolic diseases include but are not limited to type 1 diabetes, type 2 diabetes, hyperlipidemia, hypercholesterolemia, hypertriglyceridemia, hyperglycemia, high LDL-C, obesity, metabolic syndrome and other cholesterol-related diseases. Metabolic diseases related to fat and glucose metabolism, and other metabolic diseases. Circulatory system diseases include cardiovascular disease, atherosclerosis, coronary heart disease, hypertension, hyperlipidemia, hypercholesterolemia and other circulatory system diseases.

In some embodiments, the diseases described herein are diseases outside the liver, such as tumors, and the tumors including but not limited to melanoma, Hodgkin's disease, non-Hodgkin's lymphoma, acute lymphoblastic leukemia, chronic lymphocytic leukemia, multiple myeloma, neuroblastoma, breast cancer, ovarian cancer, lung cancer, Wilms' tumor, cervical cancer, testicular cancer, soft tissue sarcoma, chronic lymphocytic leukemia, primary macroglobulinemia, bladder cancer, chronic myelogenous leukemia, primary brain cancer, malignant melanoma, small cell lung cancer, gastric cancer, colon cancer, malignant pancreatic isletoma, malignant carcinoid cancer, malignant melanoma, choriocarcinoma, mycosis fungoides Granuloma, head or neck cancer, osteogenic sarcoma, pancreatic cancer, acute myeloid leukemia, hairy cell leukemia, rhabdomyosarcoma, Kaposi's sarcoma, genitourinary tumors, thyroid cancer, esophageal cancer, malignant hypercalcemia, cervical hyperplasia, renal cell carcinoma, endometrial cancer, polycythemia vera, essential thrombocythemia, adrenocortical cancer, skin cancer and prostate cancer, etc. Therefore, therapeutic agents used are therapeutic agents for these tumors and are transported via OATP.

The "therapeutically effective amount", "prophylactically effective amount" or "effective amount" of the composition of OATP-binding inhibitory peptides described herein refers to the amount of the OATP-binding inhibitory peptide or composition or therapeutic agent that can effectively carry out treatment or prevention in the prevention or treatment of disease. The "therapeutically effective amount", "prophylactically effective amount" or "effective amount" may depend on the peptide, therapeutic agent, mode of administration, disease and severity, health, age, weight, family history, genetic makeup, stage of pathological development, type of pre-medication and concurrent treatment, etc., as well as the vary depending on other individual characteristics of the treatment subject. "Treatment" as used herein includes treating a subject (e.g., a mammal, such as a human) or cell to alter the current course of the subject or cell. Treatment includes, for example, administration of an OATP binding inhibitory peptide described herein or a pharmaceutical composition containing the polypeptide, and treatment can be carried out in a prophylactic manner or can be initiated after the occurrence of a pathological event or exposure to causative substances. Treatment also includes "preventive" treatment, which is directed at reducing the rate of progression of the disease or condition to be treated, delaying the onset of the disease or condition, or reducing the severity of its onset. "Treatment" or "prevention" does not necessarily mean complete eradication, cure, or prevention of the occurrence of a disease or condition or related symptoms. As used herein, "administering" or "administering" includes administering the OATP binding inhibitory peptide and therapeutic agent described herein in the form of local or systemic administration. The modes of administration include, but are not limited to, subcutaneous injection, intravenous injection, intramuscular injection, oral administration, pulmonary inhalation, transdermal administration, intracranial administration, intrathecal administration and other administration modes. In some embodiments, the OATP-binding inhibitory peptide can be administered by, but not limited to, subcutaneous injection,

intravenous injection, intravenous drip, oral administration, transdermal administration, etc. When using an OATP-binding inhibiting polypeptide and a therapeutic agent described herein, the two can be in the same formulation, or in separate formulations. The two may be administered separately, in which case the polypeptides described herein may be administered before, simultaneously with, or after therapeutic agent.

In some optimized embodiments of this article, the OATP binding inhibitory peptide can be any polypeptide described herein, and the N and/or C termini of the polypeptide can be modified or unmodified, preferably both ends are processed modified as described herein. In a particularly preferred embodiment, the OATP-binding inhibitory peptide is the sequence described in SEQ ID NO: 30, 60 and variants thereof, and the N-terminus and/or C-terminus of the sequence variant optionally have Any modification described herein, preferably N-terminal myristoylation modification and C-terminal amidation modification, can form the polypeptide structure shown in SEQ ID NO: 67 and 68. In other embodiments, the OATP binding inhibitory peptide binds and inhibits OATP1B1 and/or OATP1B3. In some embodiments, the OATP binding inhibitory polypeptide binds to and inhibits the transport of OATP1B1 and/or OATP1B3 on statins, sartans, platinums, paclitaxels, and tinibs. One or more OATP binding inhibitory polypeptides or pharmaceutical compositions thereof can be used to implement the disease treatment or prevention methods described herein as needed.

Pill box

In some embodiments, provided herein is a kit containing an OATP-binding inhibitory peptide described herein and a therapeutic agent. The OATP binding inhibitory peptide and therapeutic agent can be packaged independently or provided in the form of a mixture. Generally, therapeutic agents are provided in kits herein in their conventional administration dosages. The OATP binding inhibitory peptide is provided in the pharmaceutical kit herein in an amount that can effectively inhibit the OATP transport function. The amounts of the OATP binding inhibitor and therapeutic agent in the kit can be administered to the patient one or more times.

The patient described herein generally refers to a mammal, preferably a human.

Sequences

The amino acid sequences in some of the polypeptides or modified polypeptides described herein are as follows.
SEQ ID NO: 1

MDQHQHLNKTAESASSEKKKTRRCNGFKMFLAALSFSYIAKALGGIIMKISITQIERR
FDISSSLAGLIDGSFEIGNLLVIVFVSYFGSKLHRPKLIGIGCLLMGTGSILTSLPHFFMGY
YRYSKETHINPSENSTSSLSTCLINQTLSFNGTSPEIVEKDCVKESGSHMWIYVFMGNM
LRGIGETPIVPLGISYIDDFAKEGHSSLYLGSLNAIGMIGPVIGFALGSLFAKMYVDIGYVD
LSTIRITPKDSRWVGAWWLGFLVSGLFSIISSIPFFFLPKNPNKPQKERKISLSLHVLKTN
DDRNQTANLTNQGKNVTKNVTGFFQSLKSILTNPLYVIFLLLTLLQVSSFIGSFTYVFKYM
EQQYGQSASHANFLLGIITIPTVATGMFLGGFIIKKFKLSLVGIAKFSFLTSMISFLFQLLYF
PLICESKSVAGLTLTYDGNNSVASHVDVPLSYCNSECNCDESQWEPVCGNNGITYLSP
CLAGCKSSSGIKKHTVFYNCSCVEVTGLQNRNYSAHLGECPRDNTCTRKFFIYVAIQVIN
SLFSATGGTTFILLTVKIVQPELKALAMGFQSMVIRTLGGILAPIYFGALIDKTCMKWSTNS
CGAQGACRIYNSVFFGRVYLGLSIALRFPALVLYIVFIFAMKKKFQGKDTKASDNERKVM
DEANLEFLNNGEHFVPSAGTDSKTCNLDMQDNAAAN.

SEQ ID NO: 2

atggaccaacatcaacatttgaataaaacagcagagtcagcatcttcagagaaaaagaaaacaagacgctgcaa
tggattcaagatgttcttggcagccctgtcattcagctatattgctaaagcactaggtggaatcattatgaaaatttccatcactc
aaatagaaaggagatttgacatatcctcttctcttgctggtttaattgatggaagctttgaaattggaaatttgcttgtgattgtattt
gtaagttactttggatctaaactacacagaccgaagttaattggaattggttgtctccttatgggaactggaagtattttgacatct

ttaccacatttcttcatgggatattataggtattctaaagaaacccatattaatccatcagaaaattcaacatcaagtttatcaac
ctgtttaattaatcaaaccttatcattcaatggaacatcacctgagatagtagaaaaagattgtgtaaaggaatctgggtcaca
catgtggatctatgtcttcatggggaatatgcttcgtggcataggggaaacccccatagtaccattggggatttcatacattgat
gattttgcaaaagaaggacattcttccttgtatttaggtagtttgaatgcaataggaatgattggtccagtcattggctttgcactg
ggatctctgtttgctaaaatgtacgtggatattggatatgtagatctgagcactatcagaataactcctaaggactctcgttgggt
tggagcttggtggcttggtttccttgtgtctggactattttccattatttcttccataccattttttttcttgccgaaaaatccaaataaac
cacaaaaagaaagaaaaatttcactatcattgcatgtgctgaaaacaaatgatgatagaaatcaaacagctaatttgacca
accaaggaaaaaatgttaccaaaaatgtgactggtttttccagtctttgaaaagcatccttaccaatcccctgtatgttatatttc
tgcttttgacattgttacaagtaagcagctttattggttcttttacttacgtctttaaatatatggagcaacagtacggtcagtctgca
tctcatgctaactttttgttgggaatcataaccattcctacggttgcaactggaatgtttttaggaggatttatcattaaaaaattca
aattgtctttagttggaattgccaaattttcatttcttacttcgatgatatccttcttgtttcaacttctatatttccctctaatctgcgaaa
gcaaatcagttgccggcctaaccttgacctatgatggaaataattcagtggcatctcatgtagatgtaccactttcttattgcaa
ctcagagtgcaattgtgatgaaagtcagtgggaaccagtctgtgggaacaatggaataacttacctgtcaccttgtctagcag
gatgcaaatcctcaagtggtattaaaaagcatacagtgttttataactgtagttgtgtggaagtaactggtctccagaacagaa
attactcagcacacttgggtgaatgcccaagagataatacttgtacaaggaaatttttcatctatgttgcaattcaagtcataaa
ctctttgttctctgcaacaggaggtaccacatttatcttgttgactgtgaagattgttcaacctgaattgaaagcacttgcaatggg
tttccagtcaatggttataagaacactaggaggaattctagctccaatatattttggggctctgattgataaaacatgtatgaagt
ggtccaccaacagctgtggagcacaaggggcttgtaggatatataattccgtatttttttggaagggtctacttgggcttatctat
agctttaagattcccagcacttgttttatatattgttttcatttttgctatgaagaaaaaatttcaaggaaaagataccaaggcatc
ggacaatgaaagaaaagtaatggatgaagcaaacttagaattcttaaataatggtgaacattttgtaccttctgctggaaca
gatagtaaaacatgtaatttggacatgcaagacaatgctgctgccaactaa.

SEQ ID NO: 3

agactttaacatcagaaaaaggatggacttgttgcagttgctgtagcattcaaagtcaagttgtgctttgcgatgctgagt
ttactgttttaaatggacttcaagcatagccctgaagtgttgtcccgttgttcccacctcctagagggcctgacagaagaggtac
atatgctatgtgatcatttcaaaccaagcatcagcaacaattaaaaatattcacttggtatctgtagtttaataatggaccaaca
tcaacatttgaataaaacagcagagtcagcatcttcagagaaaaagaaaacaagacgctgcaatggattcaagatgttctt
ggcagccctgtcattcagctatattgctaaagcactaggtggaatcattatgaaaatttccatcactcaaatagaaaggagatt
tgacatatcctcttctcttgctggtttaattgatggaagctttgaaattggaaatttgcttgtgattgtatttgtaagttactttggatcta
aactacacagaccgaagttaattggaattggttgtctccttatgggaactggaagtattttgacatctttaccacatttcttcatgg
gatattataggtattctaaagaaacccatattaatccatcagaaaattcaacatcaagtttatcaacctgtttaattaatcaaacc
ttatcattcaatggaacatcacctgagatagtagaaaaagattgtgtaaaggaatctgggtcacacatgtggatctatgtcttc
atggggaatatgcttcgtggcataggggaaacccccatagtaccattggggatttcatacattgatgattttgcaaaagaagg
acattcttccttgtatttaggtagtttgaatgcaataggaatgattggtccagtcattggctttgcactgggatctctgtttgctaaaa
tgtacgtggatattggatatgtagatctgagcactatcagaataactcctaaggactctcgttgggttggagcttggtggcttggt
ttccttgtgtctggactattttccattatttcttccataccattttttttcttgccgaaaaatccaaataaaccacaaaaagaaagaa
aaatttcactatcattgcatgtgctgaaaacaaatgatgatagaaatcaaacagctaatttgaccaaccaaggaaaaaatgt
taccaaaaatgtgactggtttttccagtctttgaaaagcatccttaccaatcccctgtatgttatatttctgcttttgacattgttaca
agtaagcagctttattggttcttttacttacgtctttaaatatatggagcaacagtacggtcagtctgcatctcatgctaactttttgtt
gggaatcataaccattcctacggttgcaactggaatgtttttaggaggatttatcattaaaaaattcaaattgtctttagttggaatt
gccaaattttcatttcttacttcgatgatatccttcttgtttcaacttctatatttccctctaatctgcgaaagcaaatcagttgccggc
ctaaccttgacctatgatggaaataattcagtggcatctcatgtagatgtaccactttcttattgcaactcagagtgcaattgtgat

gaaagtcagtgggaaccagtctgtgggaacaatggaataacttacctgtcaccttgtctagcaggatgcaaatcctcaagtg
gtattaaaaagcatacagtgttttataactgtagttgtgtggaagtaactggtctccagaacagaaattactcagcacacttgg
gtgaatgcccaagagataatacttgtacaaggaaatttttcatctatgttgcaattcaagtcataaactctttgttctctgcaacag
gaggtaccacatttatcttgttgactgtgaagattgttcaacctgaattgaaagcacttgcaatgggtttccagtcaatggttata
agaacactaggaggaattctagctccaatatattttggggctctgattgataaaacatgtatgaagtggtccaccaacagctg
tggagcacaaggggcttgtaggatatataattccgtatttttggaagggtctacttgggcttatctatagctttaagattcccagc
acttgttttatatattgttttcatttttgctatgaagaaaaaatttcaaggaaaagataccaaggcatcggacaatgaaagaaaa
gtaatggatgaagcaaacttagaattcttaaataatggtgaacattttgtaccttctgctggaacagatagtaaaacatgtaatt
tggacatgcaagacaatgctgctgccaactaacattgcattgattcattaagatgttatttttgaggtgttcctggtctttcactgac
aattccaacattctttacttacagtggaccaatggataagtctatgcatctataataaactataaaaaatgggagtacccatgg
ttaggatatagctatgcctttatggttaagattagaatatatgatccataaaaatttaaagtgagaggcatggttagtgtgtgata
caataaaaagtaattgtttggtagttgtaactgctaataaaaccagtgactagaatataagggaggtaaaaaggacaagat
agattaatagcctaaataaagagaaaagcctgatgcctttaaaaaaaatgaaacactttggatgtattacttaggccaaaat
ctggcctggatttatgctataatatatattttcatgttaagttgtatatttttcagaaattataaatattattaatttaaaatttgaatttgtg
tttgactaacaacctcgatggatcttcttccaacctcccattaagatcctgcagaagaaatagaaatattcaaatattgcaagg
tgtaattgtgagacaacttattataatacgtgttaagtttctactggacccatggaagtggattaagaaaaaccgactttagcttg
agaaagcaaaa.

SEQ ID NO: 4

MDQNQHLNKTAEAQPSENKKTRYCNGLKMFLAALSLSFIAKTLGAIIMKSSIIHIERRFEISSSLVGFIDGSFEIGNLLVIVFVSYFGSKLHRPKLIGIGCFIMGIGGVLTALPHFFMGYYRYSKETNINSSENSTSTLSTCLINQILSLNRASPEIVGKGCLKESGSYMWIYVFMGNMLRGIGETPIVPLGLSYIDDFAKEGHSSLYLGILNAIAMIGPIIGFTLGSLFSKMYVDIGYVDLSTIRITPTDSRWVGAWWLNFLVSGLFSIISSIPFFFLPQTPNKPQKERKASLSLHVLETNDEKDQTANLTNQGKNITKNVTGFFQSFKSILTNPLYVMFVLLTLLQVSSYIGAFTYVFKYVEQQYGQPSSKANILLGVITIPIFASGMFLGGYIIKKFKLNTVGIAKFSCFTAVMSLSFYLLYFFILCENKSVAGLTMTYDGNNPVTSHRDVPLSYCNSDCNCDESQWEPVCGNNGITYISPCLAGCKSSSGNKKPIVFYNCSCLEVTGLQNRNYSAHLGECPRDDACTRKFYFFVAIQVLNLFFSALGGTSHVMLIVKIVQPELKSLALGFHSMVIRALGGILAPIYFGALIDTTCIKWSTNNCGTRGSCRTYNSTSFSRVYLGLSSMLRVSSLVLYIILIYAMKKKYQEKDINASENGSVMDEANLESLNKNKHFVPSAGADSETHC.

SEQ ID NO: 5

atggaccaaaatcaacatttgaataaaacagcagaggcacaaccttcagagaataagaaaacaagatactgcaatggattgaagatgttcttggcagctctgtcactcagctttattgctaagacactaggtgcaattattatgaaaagttccatcattcatatagaacggagatttgagatatcctcttctcttgttggttttattgacggaagctttgaaattggaaatttgcttgtgattgtatttgtgagttactttggatccaaactacatagaccaaagttaattggaatcggttgtttcattatgggaattggaggtgttttgactgctttgccacatttcttcatgggatattacaggtattctaaagaaactaatatcaattcatcagaaaattcaacatcgaccttatccacttgtttaattaatcaaattttatcactcaatagagcatcacctgagatagtgggaaaaggttgtttaaaggaatctgggtcatacatgtggatatatgtgttcatgggtaatatgcttcgtggaataggggagactcccatagtaccattggggctttcttacattgatgatttcgctaaagaaggacattcttctttgtatttaggtatattgaatgcaatagcaatgattggtccaatcattggctttaccctgggatctctgttttctaaaatgtacgtggatattggatatgtagatctaagcactatcaggataactcctactgattctcgatgggttggagcttggtggcttaatttccttgtgtctggactattctccattatttcttccataccattctttttcttgccccaaactccaaataaaccacaaaaa

gaaagaaaagcttcactgtctttgcatgtgctggaaacaaatgatgaaaaggatcaaacagctaatttgaccaatcaagga
aaaaatattaccaaaaatgtgactggtttttccagtcttttaaaagcatccttactaatcccctgtatgttatgtttgtgcttttgacgt
tgttacaagtaagcagctatattggtgcttttacttatgtcttcaaatacgtagagcaacagtatggtcagccttcatctaaggcta
acatcttattgggagtcataaccatacctatttttgcaagtggaatgtttttaggaggatatatcattaaaaaattcaaactgaac
accgttggaattgccaaattctcatgttttactgctgtgatgtcattgtccttttacctattatatttttttcatactctgtgaaaacaaatc
agttgccggactaaccatgacctatgatggaaataatccagtgacatctcatagagatgtaccactttcttattgcaactcaga
ctgcaattgtgatgaaagtcaatgggaaccagtctgtggaaacaatggaataacttacatctcaccctgtctagcaggttgca
aatcttcaagtggcaataaaaagcctatagtgttttacaactgcagttgtttggaagtaactggtctccagaacagaaattact
cagcccatttgggtgaatgcccaagagatgatgcttgtacaaggaaattttactttttttgttgcaatacaagtcttgaatttatttttc
tctgcacttggaggcacctcacatgtcatgctgattgttaaaattgttcaacctgaattgaaatcacttgcactgggtttccactca
atggttatacgagcactaggaggaattctagctccaatatattttggggctctgattgatacaacgtgtataaagtggtccacc
aacaactgtggcacacgtgggtcatgtaggacatataattccacatcattttcaagggtctacttgggcttgtcttcaatgttaag
agtctcatcacttgttttatatattatattaatttatgccatgaagaaaaaatatcaagagaaagatatcaatgcatcagaaaat
ggaagtgtcatggatgaagcaaacttagaatccttaaataaaaataaacattttgtcccttctgctggggcagatagtgaaac
acattgttaa.

SEQ ID NO: 6

aaagggtggacttgttgcagttgctgtaggattctaaatccaggtgattgtttcaaactgagcatcaacaacaaaaaca
tttgtatgatatctatatttcaatcatggaccaaaatcaacatttgaataaaacagcagaggcacaaccttcagagaataaga
aaacaagatactgcaatggattgaagatgttcttggcagctctgtcactcagctttattgctaagacactaggtgcaattattat
gaaaagttccatcattcatatagaacggagatttgagatatcctcttctcttgttggttttattgacggaagctttgaaattggaaat
ttgcttgtgattgtatttgtgagttactttggatccaaactacatagaccaaagttaattggaatcggttgtttcattatgggaattgg
aggtgttttgactgctttgccacatttcttcatgggatattacaggtattctaaagaaactaatatcaattcatcagaaaattcaac
atcgaccttatccacttgtttaattaatcaaattttatcactcaatagagcatcacctgagatagtgggaaaaggttgtttaaagg
aatctgggtcatacatgtggatatatgtgttcatgggtaatatgcttcgtggaataggggagactcccatagtaccattggggct
ttcttacattgatgatttcgctaaagaaggacattcttctttgtatttaggtatattgaatgcaatagcaatgattggtccaatcattg
gctttaccctgggatctctgttttctaaaatgtacgtggatattggatatgtagatctaagcactatcaggataactcctactgattc
tcgatgggttggagcttggtggcttaatttccttgtgtctggactattctccattatttcttccataccattctttttcttgccccaaactc
caaataaaccacaaaaagaaagaaaagcttcactgtctttgcatgtgctggaaacaaatgatgaaaaggatcaaacagc
taatttgaccaatcaaggaaaaaatattaccaaaaatgtgactggtttttccagtcttttaaaagcatccttactaatcccctgta
tgttatgtttgtgcttttgacgttgttacaagtaagcagctatattggtgcttttacttatgtcttcaaatacgtagagcaacagtatgg
tcagccttcatctaaggctaacatcttattgggagtcataaccatacctatttttgcaagtggaatgtttttaggaggatatatcatt
aaaaaattcaaactgaacaccgttggaattgccaaattctcatgttttactgctgtgatgtcattgtccttttacctattatattttttca
tactctgtgaaaacaaatcagttgccggactaaccatgacctatgatggaaataatccagtgacatctcatagagatgtacc
actttcttattgcaactcagactgcaattgtgatgaaagtcaatgggaaccagtctgtggaaacaatggaataacttacatctc
accctgtctagcaggttgcaaatcttcaagtggcaataaaaagcctatagtgttttacaactgcagttgtttggaagtaactggt
ctccagaacagaaattactcagcccatttgggtgaatgcccaagagatgatgcttgtacaaggaaattttactttttgttgcaat
acaagtcttgaatttatttttctctgcacttggaggcacctcacatgtcatgctgattgttaaaattgttcaacctgaattgaaatca
cttgcactgggtttccactcaatggttatacgagcactaggaggaattctagctccaatatattttggggctctgattgatacaac
gtgtataaagtggtccaccaacaactgtggcacacgtgggtcatgtaggacatataattccacatcattttcaagggtctactt
gggcttgtcttcaatgttaagagtctcatcacttgttttatatattatattaatttatgccatgaagaaaaaatatcaagagaaaga
tatcaatgcatcagaaaatggaagtgtcatggatgaagcaaacttagaatccttaaataaaaataaacattttgtcccttctgc

tggggcagatagtgaaacacattgttaaggggagaaaaaaagccacttctgcttctgtgtttccaaacagcattgcattgatt
cagtaagatgttatttttgaggagttcctggtcctttcactaagaatttccacatcttttatggtggaagtataaataagcctatgaa
cttataataaaacaaactgtaggtagaaaaaatgagagtactcattgttacattatagctacatatttgtggttaaggttagact
atatgatccatacaaattaaagtgagagacatggttactgtgtaataaaagaaaaaatacttgttcaggtaattctaattcttaa
taaaacaaatgagtatcatacaggtagaggttaaaaaggaggagctagattcatatcctaagtaaagagaaatgcctagt
gtctattttattaaacaaacaaacacagagtttgaactataatactaaggcctgaagtctagcttggatatatgctacaataata
tctgttactcacataaaattatatatttcacagactttatcaatgtataattaacaattatcttgtttaagtaaatttagaatacatttaa
gtattgtggaagaaataaagacattccaatatttgcaa.

SEQ ID NO: 7

MEAHNASAPFNFTLPPNFGKRPTDLALSVILVFMLFFIMLSLGCTMEFSKIKAHLWKPKGLAIALVAQYGIMPLTAFVLGKVFRLKNIEALAILVCGCSPGGNLSNVFSLAMKGDMNLSIVMTTCSTFCALGMMPLLLYIYSRGIYDGDLKDKVPYKGIVISLVLVLIPCTIGIVLKSKRPQYMRYVIKGGMIIILLCSVAVTVLSAINVGKSIMFAMTPLLIATSSLMPFIGFLLGYVLSALFCLNGRCRRTVSMETGCQNVQLCSTILNVAFPPEVIGPLFFFPLLYMIFQLGEGLLLIAIFWCYEKFKTPKDKTKMIYTAATTEETIPGALGNGTYKGEDCSPCTA.

The amino acid sequence of hepadnavirus Pre-S region is shown in the table below.

| SEQ ID NO: | Source | Gene bank ID | Amino acid sequence |
|---|---|---|---|
| 8 | DHBV | NC_00134 4 | MGQQPAKSMDVRRIEGGELLLNQLAGRMIPKGTVTWSGKFPTIDHLLDHVQTMEEVNTLQQQGAWPAGAGRRLGLTNPTPHETPQPQWTPEEDQKAREAFRRYQEERPPETTTIAPTSPTPWKLQPGDDPLLENKSLLETHPLYQNPEPAVPVIKTPPLKKKK |
| 9 | HHBV | NC_00148 6 | MGHTQAKSTTDRRVEGGELLLQHLAGRMIPPEFSGPITTAGKFPTIQHVMDHIDSVEELRTLQAGGHWPEGTARRLGLDQPRPTPPPITWTEEEDKKAKEFFKQYQENRPKPAETAPPPITELHAAEPPQWKISPEDPLLKAKALIPVKEPEVPILKVPKLTNKKK |
| 10 | WMHBV | AF046996 | MGLNQSTFNPLGFFPSHQLDPLFKANAGSADWDKNPNKDPWPQAHDTAVGAFGPGLVPPHGGLLGWSSQAQGLSVTVPDTPPPPSTNRDKGRKPTPATPPLRDTHPQA |
| 11 | CMHBV | NC_04352 8 | MGLNQSTINPLGFFPSHQLDPLFKANSPAADWDQNPHKDPWPSAHEVPVGAFGPGLVPPHGGLLGWNPQSQGQVTLLPKDPPAQLPNRNSARVPTPITPPLRDTHPQA |
| 12 | WHV | NC_00410 7 | MGNNIKVTFNPDKIAAWWPAVGTYYTTTYPQNQSVFQPGIYQTTSLINPKNQQELDSVLINRYKQIDWNTWQGFPVDQKLPLVSRDPPPKPYINQSAQTFEIKPGPIIVPGIRDIPRGLVPPQTPTNRDQGRKPTPPTPPLRDTHPHLT |
| 13 | GSHV | NC_00148 4 | MKNQTGHLQGFAEGLRALTTSDHHNSAYGDPFTTLSPVVPTVSTTLSPPLTIGDPVLSTEMSPSGLLGLLAGLQVVYFLWTKILTIAQSLDWWWTSLSFPGGIPECTGQNLQFQTCKHLPTSCPPTCNGFRW |
| 14 | BHV | JX941468 | MGQNWSIPNPLGFLPEHQLPNPYAPVYQDWDLNQDKDQWPQAREVSPGAYGLGFVPPHGGLTGNLPFAQEGNLTSNNPETIVYQIPKAKDESQTQVIQLPAPPRTTTNRKKGRQPTPPTPPVRVTHPHL |
| | | | N |
| 15 | HBV (Genotype A) | KC875260 | MGGWSSKPRKGMGTNLSVPNPLGFFPDHQLDPAFGANSNNPDWDFNPIKDHWPAGNQVGVGAFGPRLTPPHGGILGWSPQAQGILATVSTIPPPASTNRQSGRQPTPISPPLRDSHPEA |

(continued)

| SEQ ID NO: | Source | Gene bank ID | Amino acid sequence |
|---|---|---|---|
| 16 | HBV (Genotype B) | AY220704 | MGGWSSKPRKGMGTNLSVPNPLGFFPDHQLDPAFKANSENPDWDLNPHKDNWPDANKVGVGAFGPGFTPPHGGLLGWSPQAQGLLTTVPAAPPPASTNRQSGRQPTPLSPPLRDTHPQA |
| 17 | HBV (Genotype C) | AF461363 | MGGWSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA |
| 18 | HBV (Genotype D) | AY796030 | MGQNLSTSNPLGFFPDHQLDPAFRANTANPDWDFNPNKDTWPDANKVGAGAFGLGFTPPHGGLLGWSPQAQGILQTLPANPPPASTHRQSGRQPTPLSPPLRNTHPQA |
| 19 | HBV (Genotype E) | AB205129 | MGLSWTVPLEWGKNHSTTNPLGFFPDHQLDPAFRANTRNPDWDHNPDKDHWTEANKVGVGAFGPGFTPPHGGLLGWSPQAQGMLKTLPADPPPASTNRQSGRQPTPITPPLRDTHPQA |
| 20 | HBV (Genotype F) | DQ823095 | MGAPLSTTRRGMGQNLSVPNPLGFFPDHQLDPLFRANSSSPDWDFNKNKDNWPMANKVGVGGYGPGFTPPHGGLLGWSPQAQGVLTTLPADPPPASTNRRSGRKPTPVSPPLRDTHPQA |
| 21 | HBV (Genotype G) | HE981176 | MGLSWTVPLEWGKNLSTSNPLGFLPDHQLDPAFRANTNNPDWDFNPKKDPWPEANKVGVGAYGPGFTPPHGGLLGWSLQSQGTLTTLPADPPPASTNRQSGRQPTPISPPLRDSHPQA |
| 22 | HBV (Genotype H) | AB179747 | MGAPLSTARRGMGQNLSVPNPLGFFPDHQLDPLFRANSSSPDWDFNTNKDNWPMANKVGVGGFGPGFTPPHGGLLGWSPQAQGILTTSPPDPPPASTNRRSGRKPTPVSPPLRDTHPQA |

| Number | Name | Amino acid sequence | Source |
|---|---|---|---|
| 23 | C2-119 | GGWSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (2-119) |
| 24 | C2-69 | GGWSSKPRQGMGTNLSVPNPLGFFPDHQLDPAFGANSNNPDWDFNPNKDHWPEANQVGAGAFGPGFTP | Genotype C Pre-S1 (2-69) |
| 25 | C2-59 | GGWSSKPRQGMGTNLSVPNPLGFFPDHQLDPAFGANSNNPDWDFNPNKDHWPEANQVG | Genotype C Pre-S1 (2-59) |
| 26 | C13-119 | GTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (13-119) |

(continued)

| Number | Name | Amino acid sequence | Source |
|---|---|---|---|
| 27 | C13-88 | GTNLSVPNPLGFFPDHQLDPAFGANSNNPDWDFNPNKDHWPEANQVGAGAFGPGFTPPHGGLLGWSPQAQGILTTV | Genotype C Pre-S1 (13-88) |
| 28 | C13-72 | GTNLSVPNPLGFFPDHQLDPAFGANSNNPDWDFNPNKDHWPEANQVGAGAFGPGFTPPHG | Genotype C Pre-S1 (13-72) |
| 29 | C13-67 | GTNLSVPNPLGFFPDHQLDPAFGANSNNPDWDFNPNKDHWPEANQVGAGAFGPGF | Genotype C Pre-S1 (13-67) |
| 30 | C13-59 | GTNLSVPNPLGFFPDHQLDPAFGANSNNPDWDFNPNKDHWPEANQVG | Genotype C Pre-S1 (13-59) |
| 23 | C2-119 | GGWSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (2-119) |
| 24 | C2-69 | GGWSSKPRQGMGTNLSVPNPLGFFPDHQLDPAFGANSNNPDWDFNPNKDHWPEANQVGAGAFGPGFTP | Genotype C Pre-S1 (2-69) |
| 25 | C2-59 | GGWSSKPRQGMGTNLSVPNPLGFFPDHQLDPAFGANSNNPDWDFNPNKDHWPEANQVG | Genotype C Pre-S1 (2-59) |
| 26 | C13-119 | GTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (13-119) |
| 27 | C13-88 | GTNLSVPNPLGFFPDHQLDPAFGANSNNPDWDFNPNKDHWPEANQVGAGAFGPGFTPPHGGLLGWSPQAQGILTTV | Genotype C Pre-S1 (13-88) |
| 31 | C13-52 | GTNLSVPNPLGFFPDHQLDPAFGANSNNPDWDFNPNKDHW | Genotype C Pre-S1 (13-52) |
| 32 | C13-47 | GTNLSVPNPLGFFPDHQLDPAFGANSNNPDWDFNP | Genotype C Pre-S1 (13-47) |
| 33 | C13-42 | GTNLSVPNPLGFFPDHQLDPAFGANSNNPD | Genotype C Pre-S1 (13-42) |
| 34 | C13-37 | GTNLSVPNPLGFFPDHQLDPAFGAN | Genotype C Pre-S1 (13-37) |
| 35 | C13-32 | GTNLSVPNPLGFFPDHQLDP | Genotype C Pre-S1 (13-32) |
| 36 | A13-59 | GTNLSVPNPLGFFPDHQLDPAFGANSNNPDWDFNPVKDDWPAANQVG | Genotype A Pre-S1 (13-59) |
| 37 | B13-59 | GTNLSVPNPLGFFPDHQLDPAFKANSENPDWDLNPNKDNWPDANKVG | Genotype B Pre-S1 (13-59) |
| 38 | D2-48 | GQNLSTSNPLGFFPDHQLDPAFRANTANPDWDFNPNKDTWPDANKVG | Genotype D Pre-S1 (2-48) |

(continued)

| Number | Name | Amino acid sequence | Source |
|---|---|---|---|
| 39 | E12-58 | GKNISTTNPLGFFPDHQLDPAFRANTRNPDWDHNPNKDHWTEANKVG | Genotype E Pre-S1 (12-58) |
| 40 | F13-59 | GQNLSVPNPLGFFPDHQLDPLFRANSSSPDWDFNTNKDSWPMANKVG | Genotype F Pre-S1 (13-59) |
| 41 | G12-58 | GKNLSASNPLGFLPDHQLDPAFRANTNNPDWDFNPKKDPWPEANKVG | Genotype G Pre-S1 (12-58) |
| 42 | H13-59 | GQNLSVPNPLGFFPDHQLDPLFRANSSSPDWDFNTNKDNWPMANKVG | Genotype H Pre-S1 (13-59) |
| 43 | C13-59 (N15D) | GTDLSVPNPLGFFPDHQLDPAFGANSNNPDWDFNPNKDHWPEANQVG | Genotype C Pre-S1 (13-59) |
| 44 | C13-59 (F25L) | GTNLSVPNPLGFLPDHQLDPAFGANSNNPDDFNPNKDHWPEANQVG | Genotype C Pre-S1 (13-59) |
| 45 | C13-59 (G35K) | GTNLSVPNPLGFFPDHQLDPAFKANSNNPDWDFNPNKDHWPEANQVG | Genotype C Pre-S1 (13-59) |
| 46 | C13-59 | GTNLSVPNPLGFFPDHQLDPAFGANSENPDWDFNPNKD | Genotype C |
| 23 | C2-119 | GGWSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (2-119) |
| 24 | C2-69 | GGWSSKPRQGMGTNLSVPNPLGFFPDHQLDPAFGANSNNPDWDFNPNKDHWPEANQVGAGAFGPGFTP | Genotype C Pre-S1 (2-69) |
| 25 | C2-59 | GGWSSKPRQGMGTNLSVPNPLGFFPDHQLDPAFGANSNNPDWDFNPNKDHWPEANQVG | Genotype C Pre-S1 (2-59) |
| 26 | C13-119 | GTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (13-119) |
| 27 | C13-88 | GTNLSVPNPLGFFPDHQLDPAFGANSNNPDWDFNPNKDHWPEANQVGAGAFGPGFTPPHGGLLGWSPQAQGILTTV | Genotype C Pre-S1 (13-88) |
| | (N39E) | HWPEANQVG | Pre-S1 (13-59) |
| 47 | C13-59 (N46K) | GTNLSVPNPLGFFPDHQLDPAFGANSNNPDWDFKPNKDHWPEANQVG | Genotype C Pre-S1 (13-59) |
| 48 | C13-59 (F45L) | GTNLSVPNPLGFFPDHQLDPAFGANSNNPDWDLNPNKDHWPEANQVG | Genotype C Pre-S1 (13-59) |
| 49 | C13-59 (N48H) | GTNLSVPNPLGFFPDHQLDPAFGANSNNPDWDFNPHKDHWPEANQVG | Genotype C Pre-S1 (13-59) |
| 50 | C13-59 (N48Y) | GTNLSVPNPLGFFPDHQLDPAFGANSNNPDWDFNPYKDHWPEANQVG | Genotype C Pre-S1 (13-59) |

(continued)

| Number | Name | Amino acid sequence | Source |
|---|---|---|---|
| 51 | C13-59 (N48K) | GTNLSVPNPLGFFPDHQLDPAFGANSNNPDWDFNPKKD HWPEANQVG | Genotype C Pre-S1 (13-59) |
| 52 | C13-59 (D50A) | GTNLSVPNPLGFFPDHQLDPAFGANSNNPDWDFNPNKA HWPEANQVG | Genotype C Pre-S1 (13-59) |
| 53 | C13-59 (H51Q) | GTNLSVPNPLGFFPDHQLDPAFGANSNNPDWDFNPNKD QWPEANQVG | Genotype C Pre-S1 (13-59) |
| 54 | C13-59 (H51N) | GTNLSVPNPLGFFPDHQLDPAFGANSNNPDWDFNPNKD NWPEANQVG | Genotype C Pre-S1 (13-59) |
| 55 | C13-59 (E54K) | GTNLSVPNPLGFFPDHQLDPAFGANSNNPDWDFNPNKD HWPKANQVG | Genotype C Pre-S1 (13-59) |
| 56 | C13-59 (E54D) | GTNLSVPNPLGFFPDHQLDPAFGANSNNPDWDFNPNKD HWPDANQVG | Genotype C Pre-S1 (13-59) |
| 57 | C13-59 (A55S) | GTNLSVPNPLGFFPDHQLDPAFGANSNNPDWDFNPNKD HWPESNQVG | Genotype C Pre-S1 (13-59) |
| 58 | C13-59 (N56K) | GTNLSVPNPLGFFPDHQLDPAFGANSNNPDWDFNPNKD HWPEAKQVG | Genotype C Pre-S1 (13-59) |
| 59 | C13-59 (N56D) | GTNLSVPNPLGFFPDHQLDPAFGANSNNPDWDFNPNKD HWPEADQVG | Genotype C Pre-S1 (13-59) |
| 60 | C13-59 (Q57K) | GTNLSVPNPLGFFPDHQLDPAFGANSNNPDWDFNPNKD HWPEANKVG | Genotype C Pre-S1 (13-59) |
| 61 | A2-12+ C13-59 | GGWSSKPRKGMGTNLSVPNPLGFFPDHQLDPAFGANSN NPDWDFNPNKDHWPEANQVG | Genotype A Pre-S1(2-12)+C |
| 23 | C2-119 | GGWSSKPRQGMGTNLSVPNPLGFFPDHQLDPAFGANSN NPDWDFNPNKDHWPEANQVGAGAFGPGFTPPHGGLLG WSPQAQGILTTVPVAPPPASTNRQSGRQPTPISPPLRDS HPQA | Genotype C Pre-S1 (2-119) |
| 24 | C2-69 | GGWSSKPRQGMGTNLSVPNPLGFFPDHQLDPAFGANSN NPDWDFNPNKDHWPEANQVGAGAFGPGFTP | Genotype C Pre-S1 (2-69) |
| 25 | C2-59 | GGWSSKPRQGMGTNLSVPNPLGFFPDHQLDPAFGANSN NPDWDFNPNKDHWPEANQVG | Genotype C Pre-S1 (2-59) |
| 26 | C13-119 | GTNLSVPNPLGFFPDHQLDPAFGANSNNPDWDFNPNKD HWPEANQVGAGAFGPGFTPPHGGLLGWSPQAQGILTTV PVAPPPASTNRQSGRQPTPISPPLRDSHPQA | Genotype C Pre-S1 (13-119) |
| 27 | C13-88 | GTNLSVPNPLGFFPDHQLDPAFGANSNNPDWDFNPNKD HWPEANQVGAGAFGPGFTPPHGGLLGWSPQAQGILTTV | Genotype C Pre-S1 (13-88) |
| | | | Pre-S1 (13-59) |

(continued)

| Number | Name | Amino acid sequence | Source |
|---|---|---|---|
| 62 | B2-12+ C13-59 | GGWSSKPRKGMGTNLSVPNPLGFFPDHQLDPAFGANSN NPDWDFNPNKDHWPEANQVG | Genotype B Pre-S1(2-12)+C Pre-S1 (13-59) |
| 63 | E2-11+ C13-59 | GLSWTVPLEWGTNLSVPNPLGFFPDHQLDPAFGANSNN PDWDFNPNKDHWPEANQVG | Genotype E Pre-S1(2-11)+C Pre-S1 (13-59) |
| 64 | F2-12+ C13-59 | GAPLSTTRRGMGTNLSVPNPLGFFPDHQLDPAFGANSNN PDWDFNPNKDHWPEANQVG | Genotype F Pre-S1(2-12)+C Pre-S1 (13-59) |
| 65 | G2-11+ C13-59 | GLSWTVPLEWGTNLSVPNPLGFFPDHQLDPAFGANSNN PDWDFNPNKDHWPEANQVG | Genotype G Pre-S1(2-11)+C Pre-S1 (13-59) |
| 66 | H2-12+ C13-59 | GAPLSTARRGMGTNLSVPNPLGFFPDHQLDPAFGANSNN PDWDFNPNKDHWPEANQVG | Genotype H Pre-S1(2-12)+C Pre-S1 (13-59) |

| Number | Name | N-terminal modification | Amino acid sequence | C-terminal modification | Sourse |
|---|---|---|---|---|---|
| 67 | myrC13-59 NH2 | Myr | GTNLSVPNPLGFFPDHQLDPA FGANSNNPDWDFNPNKDHW PEANQVG | NH2 | Genotype C Pre-S1 (13-59) |
| 68 | MyrC13-59 NH2 (Q57K) | Myr | GTNLSVPNPLGFFPDHQLDPA FGANSNNPDWDFNPNKDHW PEANKVG | NH2 | Genotype C Pre-S1 (13-59) |
| 69 | plam C13-59 NH2 | Plam | GTNLSVPNPLGFFPDHQLDPA FGANSNNPDWDFNPNKDHW PEANQVG | NH2 | Genotype C Pre-S1 (13-59) |
| 70 | stea C13-59 NH2 | Stearoyl | GTNLSVPNPLGFFPDHQLDPA FGANSNNPDWDFNPNKDHW PEANQVG | NH2 | Genotype C Pre-S1 (13-59) |
| 71 | chol C13-59 NH2 | Chol | GTNLSVPNPLGFFPDHQLDPA FGANSNNPDWDFNPNKDHW PEANQVG | NH2 | Genotype C Pre-S1 (13-59) |
| 72 | MyrA13-59 NH2 | Myr | GTNLSVPNPLGFFPDHQLDPA FGANSNNPDWDFNPVKDDWP AANQVG | NH2 | Genotype A Pre-S1 (13-59) |
| 73 | MyrB13-59 NH2 | Myr | GTNLSVPNPLGFFPDHQLDPA FKANSENPDWDLNPNKDNWP DANKVG | NH2 | Genotype B Pre-S1 (13-59) |

(continued)

| Number | Name | N-terminal modification | Amino acid sequence | C-terminal modification | Sourse |
|---|---|---|---|---|---|
| 74 | MyrD2-48 NH2 | Myr | GQNLSTSNPLGFFPDHQLDPA FRANTANPDWDFNPNKDTWP DANKVG | NH2 | Genotype D Pre-S1 (2-48) |
| 75 | MyrE12-58 NH2 | Myr | GKNISTTNPLGFFPDHQLDPA FRANTRNPDWDHNPNKDHWT EANKVG | NH2 | Genotype E Pre-S1 (12-58) |
| 76 | MyrF13-59 NH2 | Myr | GQNLSVPNPLGFFPDHQLDPL FRANSSSPDWDFNTNKDSWP MANKVG | NH2 | Genotype F Pre-S1 (13-59) |
| 77 | MyrG 12-58 NH2 | Myr | GKNLSASNPLGFLPDHQLDPA FRANTNNPDWDFNPKKDPWP EANKVG | NH2 | Genotype G Pre-S1 (12-58) |
| 78 | MyrH13-59 NH2 | Myr | GQNLSVPNPLGFFPDHQLDPL FRANSSSPDWDFNTNKDNWP MANKVG | NH2 | Genotype H Pre-S1 (13-59) |
| Note: The sequence numbers indicate only the amino acid sequence itself, excluding modifications. | | | | | |

SEQ ID NO: 79

gattaaagaaggcatccagcaagaactgcacaagaaacggagtcagccggagaacaaggagtggtcttccact gcctcacaggaggatggaggcccacaacgcgtctgccccattcaacttcaccctgccacccaactttggcaagcgcccca cagacctggcactgagcgtcatcctggtgttcatgttgttcttcatcatgctctcgctgggctgcaccatggagttcagcaagat caaggctcacttatggaagcctaaagggctggccatcgccctggtggcacagtatggcatcatgcccctcacggcctttgtg ctgggcaaggtcttccggctgaagaacattgaggcactggccatcttggtctgtggctgctcacctggagggaacctgtcca atgtcttcagtctggccatgaagggggacatgaacctcagcattgtgatgaccacctgctccaccttctgtgcccttggcatga tgcctctcctcctgtacatctactccaggggggatctatgatggggacctgaaggacaaggtgccctataaaggcatcgtgata tcactggtcctggttctcattccttgcaccatagggatcgtcctcaaatccaaacggccacaatacatgcgctatgtcatcaag ggagggatgatcatcattctcttgtgcagtgtggccgtcacagttctctctgccatcaatgtggggaagagcatcatgtttgcca tgacaccactcttgattgccacctcctccctgatgccttttattggctttctgctgggttatgttctctctgctctcttctgcctcaatgg acggtgcagacgcactgtcagcatggagactggatgccaaaatgtccaactctgttccaccatcctcaatgtggcctttccac ctgaagtcattggaccacttttcttctttcccctcctctacatgattttccagcttggagaagggcttctcctcattgccatattttggt gctatgagaaattcaagactcccaaggataaaacaaaaatgatctacacagctgccacaactgaagaaacaattccagg agctctgggaaatggcacctacaaaggggaggactgctccccttgcacagcctagcccttcccctggtggcctggattctgg

tcccaaagcaattctgaaagccagtgtggtaaactagagagagcagcaaaaacaccagtcttgcctgagtctttctccagc atttccagtacatctatcagaatcatcaagtcttggccgggaacacagacagggtgtctacccaagaagcctcacctatccc caacttagaatttgctacttattttaaagacttgttcagtgactgtaaactctatgaaaccagaaaccgaatctgcctcttgctgg gatctctaaaagtgtctgataagcatcttaaagtcactcaattcctgaactaatcaatatatatgtttaacccattactcaaatac ccaaatcccattccaagttttgtgacccaaaagagaaataaatgctcacaagtgctgtagaattaaacttcagaagttctaac ctaaaaagttcagatcctattccttccccttttgacattattgggatgatgctcccgaaaagtcaaatttgacatcaagtatgcaaa agtgaacacagtaagatgcaatcaggcaaaacaaactcaaaaaatagctaatgaaatgaaaaaactgggcgaatgcat catgttagtagaggaggaaaacttttgacaaggaaaaaccaggaaacaaacacatacattaacacaatgttacctcacta ataatctctttttaagttcagtaggtatttggttttaacacaaggcgcccccaaaactgggtacagcaaactactgccaaaatg agtcgtttcccaattcaaagaaaaatgttcaaatacctagaattcacaaatttcaaattgtctctaataaaatttaaacattttgc atatca.

SEQ ID NO: 80

Atggaggcccacaacgcgtctgccccattcaacttcaccctgccacccaactttggcaagcgccccacagacctgg cactgagcgtcatcctggtgttcatgttgttcttcatcatgctctcgctgggctgcaccatggagttcagcaagatcaaggctca cttatggaagcctaaagggctggccatcgccctggtggcacagtatggcatcatgcccctcacggcctttgtgctgggcaag gtcttccggctgaagaacattgaggcactggccatcttggtctgtggctgctcacctggagggaacctgtccaatgtcttcagt ctggccatgaaggggggacatgaacctcagcattgtgatgaccacctgctccaccttctgtgcccttggcatgatgcctctcctc ctgtacatctactccaggggggatctatgatggggacctgaaggacaaggtgccctataaaggcatcgtgatatcactggtcc tggttctcattccttgcaccatagggatcgtcctcaaatccaaacggccacaatacatgcgctatgtcatcaagggagggatg atcatcattctcttgtgcagtgtggccgtcacagttctctctgccatcaatgtggggaagagcatcatgtttgccatgacaccact cttgattgccacctcctccctgatgccttttattggctttctgctgggttatgttctctctgctctcttctgcctcaatggacggtgcaga cgcactgtcagcatggagactggatgccaaaatgtccaactctgttccaccatcctcaatgtggcctttccacctgaagtcatt ggaccacttttcttctttcccctcctctacatgattttccagcttggagaagggcttctcctcattgccatattttggtgctatgagaa attcaagactcccaaggataaaacaaaaatgatctacacagctgccacaactgaagaaacaattccaggagctctggga aatggcacctacaaaggggaggactgctccccttgcacagcctag.

The core amino acid sequence of OATP targeting polypeptide vector HBV Pre-S1 is shown in the table below.

| **Number** | Name | Amino acid sequence | Source |
|---|---|---|---|
| 81 | C3-119 | GWSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(3-119) |
| 82 | C4-119 | WSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(4-119) |
| 83 | C3-69 | GWSSKPRQGMGTNLSVPNPLGFFPDHQLDPAFGANSNNPD WDFNPNKDHWPEANQVGAGAFGPGFTP | Genotype C Pre-S1 (3-69) |

(continued)

| Number | Name | Amino acid sequence | Source |
|---|---|---|---|
| 84 | C4-69 | WSSKPRQGMGTNLSVPNPLGFFPDHQLDPAFGANSNNPD WDFNPNKDHWPEANQVGAGAFGPGFTP | Genotype C Pre-S1 (4-69) |
| 85 | C3-59 | GWSSKPRQGMGTNLSVPNPLGFFPDHQLDPAFGANSNNPD WDFNPNKDHWPEANQVG | Genotype C Pre-S1 (3-59) |
| 86 | C4-59 | WSSKPRQGMGTNLSVPNPLGFFPDHQLDPAFGANSNNPD WDFNPNKDHWPEANQVG | Genotype C Pre-S1 (4-59) |
| 87 | C3-48 | GWSSKPRQGMGTNLSVPNPLGFFPDHQLDPAFGANSNNPD WDFNPN | Genotype C Pre-S1 (3-48) |
| 88 | C4-48 | WSSKPRQGMGTNLSVPNPLGFFPDHQLDPAFGANSNNPD WDFNPN | Genotype C Pre-S1 (4-48) |
| 89 | C14-119 | TNLSVPNPLGFFPDHQLDPAFGANSNNPDWDFNPNKDHWP EANQVGAGAFGPGFTPPHGGLLGWSPQAQGILTTVPVAPP PASTNRQSGRQPTPISPPLRDSHPQA | Genotype C Pre-S1 (14-119) |
| 90 | C14-88 | TNLSVPNPLGFFPDHQLDPAFGANSNNPDWDFNPNKDHWP EANQVGAGAFGPGFTPPHGGLLGWSPQAQGILTTV | Genotype C Pre-S1 (14-88) |
| 91 | C14-72 | TNLSVPNPLGFFPDHQLDPAFGANSNNPDWDFNPNKDHWP EANQVGAGAFGPGFTPPHG | Genotype C Pre-S1 (14-72) |
| 92 | C14-67 | TNLSVPNPLGFFPDHQLDPAFGANSNNPDWDFNPNKDHWP EANQVGAGAFGPGF | Genotype C Pre-S1 (14-67) |
| 93 | C14-59 | TNLSVPNPLGFFPDHQLDPAFGANSNNPDWDFNPNKDHWP EANQVG | Genotype C Pre-S1 (14-59) |
| 97 | C14-56 | TNLSVPNPLGFFPDHQLDPAFGANSNNPDWDFNPNKDHWP EAN | Genotype C Pre-S1 (14-56) |
| 95 | C14-55 | TNLSVPNPLGFFPDHQLDPAFGANSNNPDWDFNPNKDHWP EA | HBV (Genotype C) Pre-S1 (14-55) |
| 96 | C14-53 | TNLSVPNPLGFFPDHQLDPAFGANSNNPDWDFNPNKDHWP | Genotype C Pre-S1 (14-53) |
| 97 | C14-48 | TNLSVPNPLGFFPDHQLDPAFGANSNNPDWDFNPN | Genotype C Pre-S1 (14-48) |
| 98 | C14-47 | TNLSVPNPLGFFPDHQLDPAFGANSNNPDWDFNP | HBV (Genotype C) Pre-S1 (14-47) |
| 99 | C14-45 | TNLSVPNPLGFFPDHQLDPAFGANSNNPDWDF | HBV (Genotype C) Pre-S1 (14-45) |

(continued)

| Num ber | Name | Amino acid sequence | Source |
|---|---|---|---|
| 100 | C14-42 | TNLSVPNPLGFFPDHQLDPAFGANSNNPD | Genotype C Pre-S1 (14-42) |
| 101 | C14-37 | TNLSVPNPLGFFPDHQLDPAFGAN | Genotype C Pre-S1 (14-37) |
| 102 | C14-32 | TNLSVPNPLGFFPDHQLDP | Genotype C Pre-S1 (14-32) |
| 103 | A14-48 | TNLSVPNPLGFFPDHQLDPAFGANSNNPDWDFNPV | Genotype A |
| 81 | C3-119 | GWSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(3-119) |
| 82 | C4-119 | WSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(4-119) |
| 83 | C3-69 | GWSSKPRQGMGTNLSVPNPLGFFPDHQLDPAFGANSNNPD WDFNPNKDHWPEANQVGAGAFGPGFTP | Genotype C Pre-S1 (3-69) |
| 84 | C4-69 | WSSKPRQGMGTNLSVPNPLGFFPDHQLDPAFGANSNNPD WDFNPNKDHWPEANQVGAGAFGPGFTP | Genotype C Pre-S1 (4-69) |
| 85 | C3-59 | GWSSKPRQGMGTNLSVPNPLGFFPDHQLDPAFGANSNNPD WDFNPNKDHWPEANQVG | Genotype C Pre-S1 (3-59) |
| 86 | C4-59 | WSSKPRQGMGTNLSVPNPLGFFPDHQLDPAFGANSNNPD WDFNPNKDHWPEANQVG | Genotype C Pre-S1 (4-59) |
| 87 | C3-48 | GWSSKPRQGMGTNLSVPNPLGFFPDHQLDPAFGANSNNPD WDFNPN | Genotype C Pre-S1 (3-48) |
| 88 | C4-48 | WSSKPRQGMGTNLSVPNPLGFFPDHQLDPAFGANSNNPD WDFNPN | Genotype C Pre-S1 (4-48) |
| 89 | C14-119 | TNLSVPNPLGFFPDHQLDPAFGANSNNPDWDFNPNKDHWP EANQVGAGAFGPGFTPPHGGLLGWSPQAQGILTTVPVAPP PASTNRQSGRQPTPISPPLRDSHPQA | Genotype C Pre-S1 (14-119) |
| 90 | C14-88 | TNLSVPNPLGFFPDHQLDPAFGANSNNPDWDFNPNKDHWP EANQVGAGAFGPGFTPPHGGLLGWSPQAQGILTTV | Genotype C Pre-S1 (14-88) |
| | | | Pre-S1 (14-48) |
| 104 | B14-48 | TNLSVPNPLGFFPDHQLDPAFKANSENPDWDLNPN | Genotype B Pre-S1 (14-59) |
| 105 | D3-37 | GQNLSTSNPLGFFPDHQLDPAFRANTANPDWDFNPN | Genotype D Pre-S1 (3-37) |
| 106 | E13-47 | KNISTTNPLGFFPDHQLDPAFRANTRNPDWDHNPN | Genotype E Pre-S1(13-47) |

(continued)

| Number | Name | Amino acid sequence | Source |
|---|---|---|---|
| 107 | F14-48 | QNLSVPNPLGFFPDHQLDPLFRANSSSPDWDFNTN | Genotype F Pre-S1 (14-48) |
| 108 | G13-47 | KNLSASNPLGFLPDHQLDPAFRANTNNPDWDFNPK | Genotype G Pre-S1(13-47) |
| 109 | G 13-46 | KNLSASNPLGFLPDHQLDPAFRANTNNPDWDFNP | Genotype G Pre-S1 (13-46) |
| 110 | H14-48 | QNLSVPNPLGFFPDHQLDPLFRANSSSPDWDFNTN | Genotype H Pre-S1 (14-48) |
| 81 | C3-119 | GWSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1(3-119) |
| 82 | C4-119 | WSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1(4-119) |
| 83 | C3-69 | GWSSKPRQGMGTNLSVPNPLGFFPDHQLDPAFGANSNNPDWDFNPNKDHWPEANQVGAGAFGPGFTP | Genotype C Pre-S1 (3-69) |
| 84 | C4-69 | WSSKPRQGMGTNLSVPNPLGFFPDHQLDPAFGANSNNPDWDFNPNKDHWPEANQVGAGAFGPGFTP | Genotype C Pre-S1 (4-69) |
| 85 | C3-59 | GWSSKPRQGMGTNLSVPNPLGFFPDHQLDPAFGANSNNPDWDFNPNKDHWPEANQVG | Genotype C Pre-S1 (3-59) |
| 86 | C4-59 | WSSKPRQGMGTNLSVPNPLGFFPDHQLDPAFGANSNNPDWDFNPNKDHWPEANQVG | Genotype C Pre-S1 (4-59) |
| 87 | C3-48 | GWSSKPRQGMGTNLSVPNPLGFFPDHQLDPAFGANSNNPDWDFNPN | Genotype C Pre-S1 (3-48) |
| 88 | C4-48 | WSSKPRQGMGTNLSVPNPLGFFPDHQLDPAFGANSNNPDWDFNPN | Genotype C Pre-S1 (4-48) |
| 89 | C14-119 | TNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (14-119) |
| 90 | C14-88 | TNLSVPNPLGFFPDHQLDPAFGANSNNPDWDFNPNKDHWPEANQVGAGAFGPGFTPPHGGLLGWSPQAQGILTTV | Genotype C Pre-S1 (14-88) |
| 111 | C14-48 (N15D) | TDLSVPNPLGFFPDHQLDPAFGANSNNPDWDFNPN | Genotype C Pre-S1 (14-48) |
| 112 | C14-48 (F25L) | TNLSVPNPLGFLPDHQLDPAFGANSNNPDWDFNPN | Genotype C Pre-S1 (14-48) |
| 113 | C14-48 (G35K) | TNLSVPNPLGFFPDHQLDPAFKANSNNPDWDFNPN | Genotype C Pre-S1 (14-48) |

(continued)

| Num ber | Name | Amino acid sequence | Source |
|---|---|---|---|
| 114 | C14-48 (N39E) | TNLSVPNPLGFFPDHQLDPAFGANSENPDWDFNPN | Genotype C Pre-S1 (14-48) |
| 115 | C14-48 (N46K) | TNLSVPNPLGFFPDHQLDPAFGANSNNPDWDFKPN | Genotype C Pre-S1 (14-48) |
| 116 | C14-48 (F45L) | TNLSVPNPLGFFPDHQLDPAFGANSNNPDWDLNPN | Genotype C Pre-S1 (14-48) |
| 117 | C14-48 (N48H) | TNLSVPNPLGFFPDHQLDPAFGANSNNPDWDFNPH | Genotype C Pre-S1 (14-48) |
| 118 | C14-48 | TNLSVPNPLGFFPDHQLDPAFGANSNNPDWDFNPY | Genotype C |
| 81 | C3-119 | GWSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(3-119) |
| 82 | C4-119 | WSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(4-119) |
| 83 | C3-69 | GWSSKPRQGMGTNLSVPNPLGFFPDHQLDPAFGANSNNPD WDFNPNKDHWPEANQVGAGAFGPGFTP | Genotype C Pre-S1 (3-69) |
| 84 | C4-69 | WSSKPRQGMGTNLSVPNPLGFFPDHQLDPAFGANSNNPD WDFNPNKDHWPEANQVGAGAFGPGFTP | Genotype C Pre-S1 (4-69) |
| 85 | C3-59 | GWSSKPRQGMGTNLSVPNPLGFFPDHQLDPAFGANSNNPD WDFNPNKDHWPEANQVG | Genotype C Pre-S1 (3-59) |
| 86 | C4-59 | WSSKPRQGMGTNLSVPNPLGFFPDHQLDPAFGANSNNPD WDFNPNKDHWPEANQVG | Genotype C Pre-S1 (4-59) |
| 87 | C3-48 | GWSSKPRQGMGTNLSVPNPLGFFPDHQLDPAFGANSNNPD WDFNPN | Genotype C Pre-S1 (3-48) |
| 88 | C4-48 | WSSKPRQGMGTNLSVPNPLGFFPDHQLDPAFGANSNNPD WDFNPN | Genotype C Pre-S1 (4-48) |
| 89 | C14-119 | TNLSVPNPLGFFPDHQLDPAFGANSNNPDWDFNPNKDHWP EANQVGAGAFGPGFTPPHGGLLGWSPQAQGILTTVPVAPP PASTNRQSGRQPTPISPPLRDSHPQA | Genotype C Pre-S1 (14-119) |
| 90 | C14-88 | TNLSVPNPLGFFPDHQLDPAFGANSNNPDWDFNPNKDHWP EANQVGAGAFGPGFTPPHGGLLGWSPQAQGILTTV | Genotype C Pre-S1 (14-88) |
| | (N48Y) | | Pre-S1 (14-48) |
| 119 | C14-48 (N48K) | TNLSVPNPLGFFPDHQLDPAFGANSNNPDWDFNPK | Genotype C Pre-S1 (14-48) |

(continued)

| Number | Name | Amino acid sequence | Source |
|---|---|---|---|
| 120 | C14-59 (D50A) | TNLSVPNPLGFFPDHQLDPAFGANSNNPDWDFNPNKAHWPEANQVG | Genotype C Pre-S1(14-59) |
| 121 | C14-59 (H51Q) | TNLSVPNPLGFFPDHQLDPAFGANSNNPDWDFNPNKDQWPEANQVG | Genotype C Pre-S1(14-59) |
| 122 | C14-59 (H51N) | TNLSVPNPLGFFPDHQLDPAFGANSNNPDWDFNPNKDNWPEANQVG | Genotype C Pre-S1(14-59) |
| 123 | C14-59 (E54K) | TNLSVPNPLGFFPDHQLDPAFGANSNNPDWDFNPNKDHWPKANQVG | Genotype C Pre-S1(14-59) |
| 124 | C14-59 (E54D) | TNLSVPNPLGFFPDHQLDPAFGANSNNPDWDFNPNKDHWPDANQVG | Genotype C Pre-S1(14-59) |
| 125 | C14-59 (A55S) | TNLSVPNPLGFFPDHQLDPAFGANSNNPDWDFNPNKDHWPESNQVG | Genotype C Pre-S1(14-59) |
| 81 | C3-119 | GWSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1(3-119) |
| 82 | C4-119 | WSSKPRQGMGTNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1(4-119) |
| 83 | C3-69 | GWSSKPRQGMGTNLSVPNPLGFFPDHQLDPAFGANSNNPDWDFNPNKDHWPEANQVGAGAFGPGFTP | Genotype C Pre-S1 (3-69) |
| 84 | C4-69 | WSSKPRQGMGTNLSVPNPLGFFPDHQLDPAFGANSNNPDWDFNPNKDHWPEANQVGAGAFGPGFTP | Genotype C Pre-S1 (4-69) |
| 85 | C3-59 | GWSSKPRQGMGTNLSVPNPLGFFPDHQLDPAFGANSNNPDWDFNPNKDHWPEANQVG | Genotype C Pre-S1 (3-59) |
| 86 | C4-59 | WSSKPRQGMGTNLSVPNPLGFFPDHQLDPAFGANSNNPDWDFNPNKDHWPEANQVG | Genotype C Pre-S1 (4-59) |
| 87 | C3-48 | GWSSKPRQGMGTNLSVPNPLGFFPDHQLDPAFGANSNNPDWDFNPN | Genotype C Pre-S1 (3-48) |
| 88 | C4-48 | WSSKPRQGMGTNLSVPNPLGFFPDHQLDPAFGANSNNPDWDFNPN | Genotype C Pre-S1 (4-48) |
| 89 | C14-119 | TNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (14-119) |

(continued)

| Num ber | Name | Amino acid sequence | Source |
|---|---|---|---|
| 90 | C14-88 | TNLSVPNPLGFFPDHQLDPAFGANSNNPDWDFNPNKDHWP EANQVGAGAFGPGFTPPHGGLLGWSPQAQGILTTV | Genotype C Pre-S1 (14-88) |
| 126 | C14-59 (N56K) | TNLSVPNPLGFFPDHQLDPAFGANSNNPDWDFNPNKDHWP EAKQVG | Genotype C Pre-S1(14-59) |
| 127 | C14-59 197(N56D ) | TNLSVPNPLGFFPDHQLDPAFGANSNNPDWDFNPNKDHWP EADQVG | Genotype C Pre-S1(14-59) |
| 128 | C14-59 (Q57K) | TNLSVPNPLGFFPDHQLDPAFGANSNNPDWDFNPNKDHWP EANKVG | Genotype C Pre-S1(14-59) |
| 129 | A3-13+C1 4-48 | GWSSKPRKGMGTNLSVPNPLGFFPDHQLDPAFGANSNNPD WDFNPN | Genotype A Pre-S1(3-12)+C Pre-S1 (14-48) |
| 130 | B3-13+C1 4-48 | GWSSKPRKGMGTNLSVPNPLGFFPDHQLDPAFGANSNNPD WDFNPN | Genotype B Pre-S1(3-12)+C Pre-S1 (14-48) |
| 131 | E3-12+C1 4-48 | LSWTVPLEWGTNLSVPNPLGFFPDHQLDPAFGANSNNPDW DFNPN | Genotype E Pre-S1(3-12)+C |
| 81 | C3-119 | GWSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(3-119) |
| 82 | C4-119 | WSSKPRQGMGTNLSVPNPLGFFPDHQLDPAFGANSNNPD WDFNPNKDHWPEANQVGAGAFGPGFTPPHGGLLGWSPQA QGILTTVPVAPPPASTNRQSGRQPTPISPPLRDSHPQA | Genotype C Pre-S1(4-119) |
| 83 | C3-69 | GWSSKPRQGMGTNLSVPNPLGFFPDHQLDPAFGANSNNPD WDFNPNKDHWPEANQVGAGAFGPGFTP | Genotype C Pre-S1 (3-69) |
| 84 | C4-69 | WSSKPRQGMGTNLSVPNPLGFFPDHQLDPAFGANSNNPD WDFNPNKDHWPEANQVGAGAFGPGFTP | Genotype C Pre-S1 (4-69) |
| 85 | C3-59 | GWSSKPRQGMGTNLSVPNPLGFFPDHQLDPAFGANSNNPD WDFNPNKDHWPEANQVG | Genotype C Pre-S1 (3-59) |
| 86 | C4-59 | WSSKPRQGMGTNLSVPNPLGFFPDHQLDPAFGANSNNPD WDFNPNKDHWPEANQVG | Genotype C Pre-S1 (4-59) |
| 87 | C3-48 | GWSSKPRQGMGTNLSVPNPLGFFPDHQLDPAFGANSNNPD WDFNPN | Genotype C Pre-S1 (3-48) |
| 88 | C4-48 | WSSKPRQGMGTNLSVPNPLGFFPDHQLDPAFGANSNNPD WDFNPN | Genotype C Pre-S1 (4-48) |

(continued)

| Number | Name | Amino acid sequence | Source |
|---|---|---|---|
| 89 | C14-119 | TNLSVPNPLGFFPDHQLDPAFGANSNNPDWDFNPNKDHWPEANQVGAGAFGPGFTPPHGGLLGWSPQAQGILTTVPVAPPPASTNRQSGRQPTPISPPLRDSHPQA | Genotype C Pre-S1 (14-119) |
| 90 | C14-88 | TNLSVPNPLGFFPDHQLDPAFGANSNNPDWDFNPNKDHWPEANQVGAGAFGPGFTPPHGGLLGWSPQAQGILTTV | Genotype C Pre-S1 (14-88) |
| | | | Pre-S1 (14-48) |
| 132 | F3-13+C1 4-48 | APLSTTRRGMGTNLSVPNPLGFFPDHQLDPAFGANSNNPDWDFNPN | Genotype F Pre-S1(3-12)+C Pre-S1 (14-48) |
| 133 | G3-12+C1 4-48 | LSWTVPLEWGTNLSVPNPLGFFPDHQLDPAFGANSNNPDWDFNPN | Genotype G Pre-S1(3-12)+C Pre-S1 (14-48) |
| 134 | H3-13+C1 4-48 | APLSTARRGMGTNLSVPNPLGFFPDHQLDPAFGANSNNPDWDFNPN | Genotype H Pre-S1(3-12)+C Pre-S1 (14-48) |

When R2 is the HBV Pre-S flanking sequence, the amino acid sequence is shown in the table below.

| | Name | Amino acid sequence | Source |
|---|---|---|---|
| 135 | C50-119 | DHWPEANQVGAGAFGPGFTPPHGGLLGWSPQAQGILTTVPVAPPPASTNRQSGRQPTPISPPLRDSHPQA | Genotype C Pre-S1(50-119) |
| 136 | C50-88 | DHWPEANQVGAGAFGPGFTPPHGGLLGWSPQAQGILTTV | Genotype C Pre-S1 (50-88) |
| 137 | C50-72 | DHWPEANQVGAGAFGPGFTPPHG | Genotype C Pre-S1(50-72) |
| 138 | C50-69 | DHWPEANQVGAGAFGPGFTP | Genotype C Pre-S1 (50-69) |
| 139 | C50-67 | DHWPEANQVGAGAFGPGF | Genotype C Pre-S1 (50-67) |
| 140 | C58-59 | VG | Genotype C Pre-S1 (58-59) |
| 141 | C50-59 | DHWPEANQVG | Genotype C Pre-S1 (50-59) |
| 142 | C50-52 | DHW | Genotype C Pre-S1(50-52) |
| 143 | A50-59 | DDWPAANQVG | Genotype A Pre-S1 (50-59) |
| 144 | B50-59 | DNWPDANKVG | Genotype B Pre-S1 (50-59) |
| 145 | D39-48 | DTWPDANKVG | Genotype D Pre-S1 (39-48) |

(continued)

| | Name | Amino acid sequence | Source |
|---|---|---|---|
| 146 | D49-58 | DHWTEANKVG | Genotype E Pre-S1 (49-58) |
| 147 | F50-59 | DSWPMANKVG | Genotype F Pre-S1 (50-59) |
| 148 | G49-58 | DPWPEANKVG | Genotype G Pre-S1 (49-58) |
| 149 | G50-59 | DNWPMANKVG | Genotype H Pre-S1 (50-59) |
| 150 | C50-59 (D50A) | AHWPEANQVG | Genotype C Pre-S1 (50-59) |
| 151 | C50-59 (H51Q) | DQWPEANQVG | Genotype C Pre-S1 (50-59) |
| 152 | C50-59 (H51N) | DNWPEANQVG | Genotype C Pre-S1 (50-59) |
| 153 | C50-59 (E54K) | DHWPKANQVG | Genotype C Pre-S1 (50-59) |
| 154 | C50-59 (E54D) | DHWPDANQVG | Genotype C Pre-S1 (50-59) |
| 155 | C50-59 (A55S) | DHWPESNQVG | Genotype C Pre-S1 (50-59) |
| 135 | C50-119 | DHWPEANQVGAGAFGPGFTPPHGGLLGWSPQAQGILT TVPVAPPPASTNRQSGRQPTPISPPLRDSHPQA | Genotype C Pre-S1(50-119) |
| 136 | C50-88 | DHWPEANQVGAGAFGPGFTPPHGGLLGWSPQAQGILT TV | Genotype C Pre-S1 (50-88) |
| 137 | C50-72 | DHWPEANQVGAGAFGPGFTPPHG | Genotype C Pre-S1(50-72) |
| 138 | C50-69 | DHWPEANQVGAGAFGPGFTP | Genotype C Pre-S1 (50-69) |
| 139 | C50-67 | DHWPEANQVGAGAFGPGF | Genotype C Pre-S1 (50-67) |
| 140 | C58-59 | VG | Genotype C Pre-S1 (58-59) |
| 141 | C50-59 | DHWPEANQVG | Genotype C Pre-S1 (50-59) |
| 156 | C50-59 (N56K) | DHWPEAKQVG | Genotype C Pre-S1 (50-59) |
| 157 | C50-59 (N56D) | DHWPEADQVG | Genotype C Pre-S1 (50-59) |
| 158 | C50-59 (Q57K) | DHWPEANKVG | Genotype C Pre-S1 (50-59) |

An example structure of a targeting vector is as follows (the sequence number only refers to the amino acid sequence of G+Core+K+R2, and the C-terminus of R2 has NH2 modification).

| Sequences Serial number | R1 | G | Vehicle Core area | | K | R2 | |
|---|---|---|---|---|---|---|---|
| | | | Amino acid sequence | Source | | Amino acid sequence | Source |
| 159 | Myr | G | TNLSVPNPLGFFPDHQLD PAFGANSNNPDWDFNPN | Genotype C Pre-S1(14-4 8) | K | DHWPEANQV G | Genotype C Pre-S1 (50-59) |
| 160 | Plam | G | TNLSVPNPLGFFPDHQLD PAFGANSNNPDWDFNPN | Genotype C Pre-S1(14-4 8) | K | DHWPEANQV G | Genotype C Pre-S1 (50-59) |
| 161 | Stearoyl | G | TNLSVPNPLGFFPDHQLD PAFGANSNNPDWDFNPN | Genotype C Pre-S1(14-4 8) | K | DHWPEANQV G | Genotype C Pre-S1 (50-59) |
| 162 | Chol | G | TNLSVPNPLGFFPDHQLD PAFGANSNNPDWDFNPN | Genotype C Pre-S1(14-4 8) | K | DHWPEANQV G | Genotype C Pre-S1 (50-59) |
| 163 | Peg | G | TNLSVPNPLGFFPDHQLD | Genotype C | K | DHWPEANQV | Genotype C |
| | | | PAFGANSNNPDWDFNPN | Pre-S1(14-4 8) | | G | Pre-S1 (50-59) |
| 164 | Myr | G | TNLSVPNPLGFFPDHQLD PAFGANSNNPDWDFNPN | Genotype C Pre-S1(14-4 8) | K | DHWPEANKV G | Genotype C Pre-S1 (50-59, Q57K) |
| 165 | Myr | G | TNLSVPNPLGFFPDHQLD PAFGANSNNPDWDFNPN KDHWPEAN | Genotype C Pre-S1(14-5 6) | K | VG | Genotype C Pre-S1 (58-59) |
| 166 | Myr | G | TNLSVPNPLGFFPDHQLD PAFGANSNNPDWDFNPN KDHWPEA | Genotype C Pre-S1(14-5 5) | K | / | |
| 167 | Myr | G | TNLSVPNPLGFFPDHQLD PAFGANSNNPDWDFNPN KDHWP | Genotype C Pre-S1(14-5 3) | K | / | |
| 168 | Myr | G | TNLSVPNPLGFFPDHQLD PAFGANSNNPDWDFNPN | Genotype C Pre-S1(14-4 8) | K | / | |
| 169 | Myr | G | TNLSVPNPLGFFPDHQLD PAFGANSNNPDWDFNP | Genotype C Pre-S1(14-4 7) | K | / | |
| 170 | Myr | G | TNLSVPNPLGFFPDHQLD PAFGANSNNPDWDF | Genotype C Pre-S1(14-4 5) | K | / | |
| 171 | Myr | G | TNLSVPNPLGFFPDHQLD PAFGANSNNPDWDFNPV | Genotype A Pre-S1(14-4 8) | K | / | |
| 172 | Myr | G | TNLSVPNPLGFFPDHQLD PAFKANSENPDWDLNPN | Genotype B Pre-S1(14-5 9) | K | / | |

(continued)

| Sequences Serial number | R1 | G | Vehicle Core area | | K | R2 | |
|---|---|---|---|---|---|---|---|
| | | | Amino acid sequence | Source | | Amino acid sequence | Source |
| 173 | Myr | G | GQNLSTSNPLGFFPDHQLDPAFRANTANPDWDFNPN | Genotype D Pre-S1(3-37 ) | K | / | |
| 174 | Myr | G | KNISTTNPLGFFPDHQLDPAFRANTRNPDWDHNPN | Genotype E Pre-S1(13-4 7) | K | / | |
| 175 | Myr | G | QNLSVPNPLGFFPDHQLDPLFRANSSSPDWDFNTN | Genotype F Pre-S1(14-4 8) | K | / | |
| 176 | Myr | G | KNLSASNPLGFLPDHQLDPAFRANTNNPDWDFNPK | Genotype G Pre-S1(13-4 | K | / | |
| | | | | 7) | | | |
| 177 | Myr | G | KNLSASNPLGFLPDHQLDPAFRANTNNPDWDFNP | Genotype G Pre-S1(13-4 6) | K | / | |
| 178 | Myr | G | QNLSVPNPLGFFPDHQLDPLFRANSSSPDWDFNTN | Genotype H Pre-S1(14-4 8) | K | / | |
| Note: "/" means does not exist. | | | | | | | |

The following examples are provided for illustrative purposes and should not be construed as limiting the scope of the invention.

EXAMPLE

Example 1

This example uses flow cytometry to detect the binding of polypeptides derived from the surface antigen of hepadnavirus to OATP and/or NTCP.

In this example, the combination of the human C genotype HBVPre-S1 region (shown in SEQ ID NO: 17) fragment and human OATP1B3 (hOATP1B3, GenBank NM_019844) and/or human NTCP (hNTCP, GenBank NM_003049) were selected and tested.

In this example, the polypeptide derived from the surface antigen of hepadnavirus (shown in SEQ ID NO:67, polypeptide code L47) is selected from the polypeptide derived from the 13-59 amino acid sequence (shown in SEQ ID NO: 30) of the human genotype C HBVPre-S1 region (shown in SEQ ID NO: 17), and its N-terminus is modified with myristic acid, and its C-terminus is amidated. In this example, the OATP is human OATP1B3 (hOATP1B3), and the exemplary amino acid and nucleic acid coding sequences are shown in SEQ ID NO: 1 and 2 (GenBank NM_019844) respectively. In this example, the NTCP is human NTCP (hNTCP), and the exemplary amino acid and nucleic acid coding sequences are shown in SEQ ID NO: 7 and 80 (GenBank NM_003049) respectively.

In this example, the core sequence of the OATP targeting polypeptide vehicle is selected from the polypeptide of the 14-48 amino acid sequence (shown in SEQ ID NO: 94) of the genotype C HBVPre-S1 region (shown in SEQ ID NO: 17); R1 is selected from the myristic acid hydrophobic group; R2 flanking sequence is selected from the polypeptide of the 50-59 amino acid sequence (shown in SEQ ID NO: 141) of the C genotype HBVPre-S1 region (shown in SEQ ID NO: 17),

and its N-terminal is amidated. Exemplary sequences are shown in SEQ ID NO: 159. FITC (fluorescein isothiocyanate) is a kind of fluorescein. Its main principle is to use the N=C=S group on FITC to react chemically with the free -NH2 on the protein, and then generate a chemical reaction at a wavelength of 490-495nm.Under the excitation of a light source, the maximum emission wavelength is 520~530nm, showing bright yellow-green fluorescence. In this example, the R3 example motif is chosen to be FITC, which is coupled to K (lysine), and the product obtained is technically equivalent to FITC labeling of amino acid K at position 49 of L47, example vehicle code FITC-L47 in this example.

Incubate Hela cells with FITC-L47 with stabilized expression of hOATPB3 and/or hNTCP, and detect the fluorescence intensity of the cells by flow cytometry to display the binding of FITC-L47 to hOATPB3 and/or hNTCP.

The experimental materials used in this example are as follows:

1. Cells

Hela-hOATP1B3 cells: Hela cells stably transfected with human OATP1B3. Transfection was lentivirus-mediated, hOATP1B3 nucleic acid coding sequence CDS was adopted from GenBank NM_019844.4 coding sequence (shown in SEQ ID NO:2), and the recombinant lentivirus profile is shown in Figure 1 and contains thaumatin B resistance. After vehicle construction was completed (recombinant lentivirus number WL1142), it was sequenced to be identical to the target hOATP1B3 CDS. Hygromycin B (Hygromycin B) is an aminoglycoside antibiotic produced by the metabolism of Streptomyces hygroscopicus that kills prokaryotic, eukaryotic (e.g., yeast), and mammalian cells by inhibiting protein synthesis. The thaumatin resistance gene (hyg or hph) of Escherichia coli (Escherichia coli.) origin encodes thaumatin B phosphotransferase, which converts thaumatin B into a biologically inactive phosphorylated product that acts as a detoxifier. The recombinant lentivirus WL1142 carries hygromycin resistance, and the infected cells can acquire hygromycin resistance. Drug susceptibility test results showed that 250 μg/mL hygromycin could kill all cultured HeLa cells, so only HeLa cells infected with lentivirus WL1142 could survive under these conditions. The cultured HeLa cells were added with recombinant lentivirus WL1142. After 48 hours of infection, the medium containing 250 μg/mL hygromycin B was replaced and the culture was continued for 9 days. All mock-infected (mock) Hela cells died; WL1142-infected Hela cells survived and were subcultured to form Hela cells stably transfected with WL1142 Hela-hOATP1B3.

Hela-hNTCP cells: Hela cells stably transfected with human NTCP. Transfection was lentivirus-mediated, hNTCP nucleic acid coding sequence CDS was adopted from GenBank NM_003049.4 coding sequence (shown in SEQ ID NO:80), and the recombinant lentivirus profile is shown in Figure 2 and contains puromycin resistance. After the vector was constructed (recombinant lentivirus number WL1143), it was completely consistent with the target hNTCP CDS sequence after sequencing. The pac gene found in the puromycin-producing bacterium Streptomyces alboniger encodes puromycin N-acetyltransferase (PAC), which confers resistance to puromycin in cells. This feature is now commonly used to screen mammalian stably transfected cell lines carrying specific pac gene plasmids. The recombinant lentivirus WL1143 carries puromycin resistance, and the infected cells can acquire puromycin resistance. Drug susceptibility test results showed that 1μg/mL puromycin could kill all cultured HeLa cells, so only HeLa cells infected with lentivirus WL1143 could survive under these conditions. The cultured HeLa cells were added with recombinant lentivirus WL1143. After 48 hours of infection, the medium containing 1 μg/ml puromycin was replaced and cultured for 9 days. All mock-infected (mock) Hela cells died; WL1143-infected Hela cells survived and were subcultured to form Hela cells stably transfected with WL1143 Hela-hNTCP.

Hela-hNTCP/hOATP1B3 cells: the cultured Hela-hNTCP cells were added with recombinant lentivirus WL1142. After 48 hours of infection, the medium containing 250 μg/mL hygromycin B and 1 μg/ml puromycin was replaced and cultured for 9 days. All mock-infected (mock) Hela-hNTCP cells died; WL1142-infected Hela-hNTCP cells survived. Therefore, only Hela-hNTCP cells infected with lentivirus WL1142 can survive under these conditions. After subculture, Hela-hNTCP cells stably transfected with WL1142, Hela-hNTCP/hOATP1B3, were formed.

2. Culture medium

| Components | Concentration |
|---|---|
| DMEM (GIBCO) | - |
| Fetal bovine serum | 10% |
| Penicillin | 100u/mL |
| Streptomycin | 100μg/mL |

3. Reagents and equipments

(1) Hygromycin B (Sigma-Aldrich, product number: V900372-1G, molecular weight: 527.52).
(2) Puromycin (Shanghai Yuanpei Biotechnology Co., Ltd., product number: S250JO, molecular weight: 544.43).
(3) L47-FITC: The amino acid sequence of L47 is shown in SEQ ID NO:30, and the N-terminus of L47 is modified by myristoylation (Myr), and the C-terminus is amidated (NH2). The 49th amino acid K of L47 was modified by FITC to obtain L47-FITC. The structure is shown in Figure 3 (A). L47-FITC was synthesized by China Peptide Biochemical Co., Ltd. (product number 871031), with HPLC purity of 95% (Figure 3, B). The mass spectrometry identification of the synthesized peptide was consistent with the molecular weight of the target peptide (Figure 3, C).
(4) PI: Shanghai Univ Biotechnology Co., Ltd.; abs9358-1ml. PI (propidium iodide) is a nuclear staining reagent that can be used for DNA staining. It cannot penetrate intact cell membranes, but PI can stain the nucleus red through damaged cell membranes. PI emits bright fluorescence at 600nm when excited by green light (540nm wavelength), and releases 20-30 times more red fluorescence after being embedded in double-stranded DNA.
(5) Phosphate buffer PBS 1X: GBICO 8120015.
(6) 3.7% formaldehyde: add 3.7 g formaldehyde to PBS and adjust the volume to 100ml.
(7) Flow cytometer: BD FACSDiva 8.0.1.1. Use 488nm blue laser for excitation, select 530/30 505LP channel for FITC signal detection, and select 575/25 550LP channel for PI signal detection.

The method of this example includes the following steps:
Culture Hela, Hela-hNTCP, Hela-hOATP1B3, and Hela-hNTCP/ hOATP1B3 cells. Cells in the logarithmic growth phase were plated in a 6-well plate at a cell density of 2×105/ml. Cultivate overnight, and the cell number should be about 70% cell coverage on the second day. Select two wells of HeLa cells and wash them gently twice with PBS. Discard the PBS, add 1 ml of 3.7% paraformaldehyde, and fix at 37°C for 10 minutes. Discard the supernatant from all cells and gently wash the cells twice with PBS. Prepare L47-FITC (10μg/ml) and PI (10μg/ml) working solutions according to the following table.

| EP tube number | PBS 1X | L47-FITC(1mg/ml) | PI (1mg/ml) |
|---|---|---|---|
| Blank control | 5 ml | 0 | 0 |
| L47-FITC | 5 ml | 50μL | 0 |
| PI | 5 ml | 0 | 50μL |
| L47-FITC/PI | 5 ml | 50μL | 50μL |

Add the above working solutions to the corresponding 6-well plates and incubate at room temperature for 15 minutes in the dark. Discard the incubation solution, add 500 μL of digestion solution to each well, and digest at 37°C for 2 minutes. Remove the digestion solution, add 1 ml PBS to each well, and collect cells in a 1.5 ml centrifuge tube. Use a cell strainer to filter each group of cells into flow tubes for flow cytometry detection. The sample loading list is shown in the table below:

| Cell | | staining | | | |
|---|---|---|---|---|---|
| | | 1 | 2 | 3 | 4 |
| Hela (fixed) | B | L47-FITC | PI | - | - |
| Hela | C | PBS | L47-FITC | PI | L47-FITC+PI |
| Hela-hNTCP | D | PBS | L47-FITC | PI | L47-FITC+PI |
| Hela-hOATP1B3 | E | PBS | L47-FITC | PI | L47-FITC+PI |
| Hela-hNTCP/ hOATP1B3 | F | PBS | L47-FITC | PI | L47-FITC+PI |

Hela cells incubated with PBS were used as negative controls for FITC and PI staining, and Hela cells fixed with paraformaldehyde and incubated with L47-FITC and PI were used as positive controls for FITC and PI staining. Adjust the parameters of the flow cytometer to detect FITC and PI signals so that the signal fluorescence intensity of FITC and PI negative control cells is less than 103, and the signal fluorescence intensity of FITC and PI positive control cells is greater than 103 (as shown in Figure 5).

Under the same parameters, flow cytometry was performed on cells in each group. The blank group (BLANK) is Hela, Hela-hNTCP, Hela-hOATP1B3, and Hela-hNTCP/hOATP1B3 cells incubated with PBS. The FITC and PI signals are negative, indicating that the above cells have no FITC and PI autofluorescence signals (Figure 6).

The single-staining group is Hela, Hela-hNTCP, Hela-hOATP1B3, and Hela-hNTCP/hOATP1B3 cells incubated with L47-FITC or PI. The FITC and PI signals are shown in Figure 7. The PI staining signals of the cells in each group were very low and almost negative, indicating that the cell membranes of the cells in each group were intact, excluding FITC staining due to cell membrane leakage. L47-FITC single staining showed that there were obvious differences in the FITC signals of cells in each group.

The double-staining group is Hela, Hela-hNTCP, Hela-hOATP1B3, and Hela-hNTCP/ hOATP1B3 cells incubated with L47-FITC+PI. The FITC and PI signals are shown in Figure 8. After L47-FITC+PI double staining, the PI staining signals of the cells in each group were very low and almost negative, indicating that the cell membranes of the cells in each group were intact, excluding FITC staining due to cell membrane leakage. Compared with L47-FITC + PI double staining and L47-FITC single staining, the FITC signals of cells in each group were consistent, excluding the influence of PI on L47-FITC staining in L47-FITC + PI double staining.

Compare the FITC signals of blank PBS-stained Hela cells and L47-FITC/PI double-stained Hela, Hela-hNTCP, Hela-hOATP1B3, and Hela-hNTCP/ hOATP1B3 cells (Figure 9). After Hela cells were stained with PBS blank control and L47-FITC/PI, the FITC signal was compared, which showed that there was a certain degree of non-specific binding between L47-FITC and Hela cells. After Hela-hOATP1B3 cells and Hela were stained with L47-FITC/PI, the FITC signal was compared. The FITC signal after staining of Hela-hOATP1B3 cells was significantly stronger, significantly surpassing the FITC signal of non-specific binding of Hela cells and L47-FITC, showing that L47 -FITC specifically binds to Hela-hOATP1B3 cells, that is, L47-FITC specifically binds to hOATP1B3. After Hela-hNTCP cells and Hela were stained with L47-FITC/PI, the FITC signal was compared. The FITC signal after staining of Hela-hNTCP cells was significantly stronger, significantly surpassing the FITC signal of non-specific binding of Hela cells and L47-FITC, showing that L47 -FITC specifically binds to Hela-hNTCP cells, that is, L47-FITC specifically binds to hNTCP. After Hela-hNTCP/ hOATP1B3 cells and Hela were stained with L47-FITC/PI, the FITC signal was compared. The FITC signal after staining of Hela-hNTCP/ hOATP1B3 cells was significantly stronger, significantly surpassing the non-specific binding of FITC between Hela cells and L47-FITC. Signal showing the specific binding of L47-FITC to Hela-hNTCP/hOATP1B3 cells. After L47-FITC/PI staining of Hela-hNTCP/ hOATP1B3 cells and Hela-hNTCP cells, the FITC signal intensity was compared. The FITC signal intensity of Hela-hNTCP/ hOATP1B3 cells after L47-FITC/PI staining was stronger. After comparing the FITC signals of Hela-hNTCP/ hOATP1B3 cells and Hela- hOATP1B3 cells after L47-FITC/PI staining, the FITC signal intensity of Hela-hNTCP/ hOATP1B3 cells after L47-FITC/PI staining was stronger.

The average fluorescence intensity of the FITC signal of blank PBS-stained Hela cells, L47-FITC/PI double-stained Hela, Hela-hNTCP, Hela-hOATP1B3, and Hela-hNTCP/hOATP1B3 cells is shown in the table below, and the fluorescence intensity comparison is shown in Figure 10.

| Cell | staining | FITC average fluorescence intensity |
|---|---|---|
| Hela | PBS | 151 |
| Hela | L47-FITC/PI | 1087 |
| Hela-hNTCP | L47-FITC/PI | 3624 |
| Hela-hOATP1B3 | L47-FITC/PI | 3120 |
| Hela-hNTCP/ hOATP1 B3 | L47-FITC/PI | 4258 |

After Hela cells were stained with PBS blank control and L47-FITC/PI, the FITC signal was compared. After Hela cells were stained with L47-FITC/PI, the average fluorescence intensity of FITC was higher than that of Hela cells stained with PBS blank (1087 vs. 151), showing a certain degree of non-specific binding between L47-FITC and HeLa cells. Comparing the average fluorescence intensity of FITC between Hela-hOATP1B3 cells and Hela after L47-FITC/PI staining, the average fluorescence intensity of FITC after Hela-hOATP1B3 cells was significantly increased (3120 vs. 1087), exceeding the average fluorescence intensity of FITC that binds non-specifically to Hela cells and L47-FITC shows that L47-FITC specifically binds to Hela-hOATP1B3 cells, that is, L47-FITC specifically binds to hOATP1B3. After Hela-hNTCP cells and Hela were stained with L47-FITC/PI, the FITC signal was compared. After Hela-hNTCP cells were stained, the average fluorescence intensity of FITC increased significantly (3624 vs. 1087), surpassing the non-specific binding of Hela cells to L47-FITC. The average fluorescence intensity of FITC shows that L47-FITC specifically binds to Hela-hNTCP cells, that is, L47-FITC specifically binds to hNTCP. After Hela-hNTCP/ hOATP1B3 cells and Hela were stained with L47-FITC/PI, compared with the average fluorescence of FITC, the average fluorescence intensity of FITC after staining of Hela-hNTCP/ hOATP1B3 cells was significantly increased (4258 vs. 1087), surpassing that of Hela cells

and L47- The average fluorescence intensity of FITC non-specifically bound to FITC, showing L47-FITC and Hela-hNTCP/ Specific binding exists in hOATP1B3 cells. Compared with Hela-hOATP1B3 cells and Hela-hNTCP cells after L47-FITC/PI staining, the average fluorescence intensity of FITC in Hela-hNTCP/hOATP1B3 cells after L47-FITC/PI staining was significantly increased (4258 vs. 3120, 4258 vs. 3624), showing There is a mutually enhanced synergistic effect between hNTCP and hOATP1B3 on the binding of L47-TITC.

These results prove that L47-FITC specifically binds to hOATP1B3; L47-FITC specifically binds to hNTCP, and hOATP1B3 and hNTCP synergistically enhance the binding effect on L47-FITC.

NTCP is considered to be a functional receptor for HBV and HDV infection [Yan H et al. Elife. 2012;1:e00049.; Ni Y et al. Gastroenterology. 2014;146(4):1070-83.], NTCP from multiple species including human, rat, mice and canine NTCP can bind to HBVPre-S1 derived peptides [Lempp FA, te al. Hepatology. 2017;66(3):703-716.]. This example also shows that the HBVPre-S1 derived example polypeptide can bind to human NTCP, which is consistent with literature reports. This example reveals that polypeptides derived from the surface antigen of hepadnavirus can bind to OATP alone, and that in cells transfected with OATP and NTCP simultaneously, the binding of polypeptides derived from the surface antigen of hepadnavirus shows a synergistic effect.

Through this example, it is shown that a polypeptide derived from the surface antigen of hepadnavirus can bind to OATP. In particular, it is shown that the polypeptide derived from the 13-59 amino acid sequence of the human C genotype HBVPre-S1 region (shown in SEQ ID NO: 17) (an example amino acid sequence is shown in SEQ ID NO: 30), whose N It has myristic acid modification at the end and amidation modification at the C-terminus (shown in SEQ ID NO: 67, polypeptide code L47), which can be compared with human OATP1B3 (shown in SEQ ID NO:1 and 2, GenBank NM_019844) combined.

Example 2

This example uses fluorescence microscopy to examine the binding of polypeptides derived from the surface antigen of hepadnavirus to OATP and/or NTCP, and its role in mediating cell entry.

In this example, the combination of the human C genotype HBVPre-S1 region (shown in SEQ ID NO: 17) fragment and human OATP1B3 (hOATP1B3, GenBank NM_019844) and/or human NTCP (hNTCP, GenBank NM_003049) were selected and tested.

In this example, the polypeptide derived from the surface antigen of hepadnavirus (shown in SEQ ID NO:67, polypeptide code L47) is selected from the polypeptide derived from the 13-59 amino acid sequence (shown in SEQ ID NO: 30) of the human C genotype HBVPre-S1 region (shown in SEQ ID NO: 17), and its N-terminus is modified with myristic acid, and its C-terminus is amidated. In this example, the OATP is human OATP1B3 (hOATP1B3), and the exemplary amino acid and nucleic acid coding sequences are shown in SEQ ID NO: 1 and 2 (GenBank NM_019844) respectively. In this example, the NTCP is human NTCP (hNTCP), and the exemplary amino acid and nucleic acid coding sequences are shown in SEQ ID NO: 7 and 80 (GenBank NM_003049) respectively.

In this example, the core sequence of the OATP targeting polypeptide vehicle is selected from the polypeptide of the 14-48 amino acid sequence (shown in SEQ ID NO: 94) of the genotype C HBVPre-S1 region (shown in SEQ ID NO: 17); R1 is selected from the myristic acid hydrophobic group; R2 flanking sequence is selected from the polypeptide of the 50-59 amino acid sequence (shown in SEQ ID NO: 141) of the C genotype HBVPre-S1 region (shown in SEQ ID NO: 17), and its N-terminal is amidated. Exemplary sequences are shown in SEQ ID NO: 159. FITC (fluorescein isothiocyanate) is a kind of fluorescein. Its main principle is to use the N=C=S group on FITC to react chemically with the free -NH2 on the protein, and then generate a chemical reaction at a wavelength of 490-495nm. In this example, the R3 example motif is chosen to be FITC, which is coupled to K (lysine), and the product obtained is technically equivalent to FITC labeling of amino acid K at position 49 of L47, example vehicle code FITC-L47 in this example.

Incubate FITC-L47 with Hela cells that stably express hOATPB3 and/or hNTCP, and observe the combination of fluorescence and the distribution of fluorescence in the cells under a fluorescence microscope. This will show the binding of FITC-L47 to hOATPB3 and/or hNTCP, and fluorescence mediated into cells.

The experimental materials used in this example are as follows:

1. Cells

Hela-hOATP1B3 cells, Hela-hNTCP cells and Hela-hNTCP/hOATP1B3 cells are as described in Example 1.

2. Culture medium

| Components | Concentration |
|---|---|
| DMEM (GIBCO) | - |
| Fetal bovine serum | 10% |
| Penicillin | 100u/mL |
| Streptomycin | 100μg/mL |

3. Reagents and equipment

(1) Hygromycin B (Sigma-Aldrich, product number: V900372-1G, molecular weight: 527.52).
(2) Puromycin (Shanghai Yuanpei Biotechnology Co., Ltd., product number: S250JO, molecular weight: 544.43).
(3) L47-FITC: The amino acid sequence of L47 is shown in SEQ ID NO:30. The N-terminus of L47 is modified by myristoylation (Myr), and the C-terminus is amidated (NH2). The 49th amino acid K of L47 was modified by FITC to obtain L47-FITC. The structure is shown in Figure 3 (A). L47-FITC was synthesized by China Peptide Biochemical Co., Ltd. (product number 871031), with HPLC purity of 95% (Figure 3, B). The mass spectrometry identification of the synthesized peptide was consistent with the molecular weight of the target peptide (Figure 3, C).
(4) Phosphate buffer PBS 1X: GBICO 8120015.
(5) Fluorescence excitation light source: Leica LED8, using cyan 475±14nm wavelength excitation light.
(6) Fluorescence microscope: Leica DMIRBE, including I3 fluorescence module (Cat. No. 513828).

The method of this example includes the following steps:
Hela, Hela-hNTCP, Hela-hOATP1B3, and Hela-hNTCP/hOATP1B3 in logarithmic growth phase were spread into 24-well plates. Culture in ordinary medium, medium containing 1 mg/ml puromycin, medium containing 250 ug/ml hygromycin B, medium containing 1 mg/ml puromycin + 250 ug/ml hygromycin B, respectively, with 500uL per well. Cultivate overnight, and the cell number should be about 50% coverage. Discard the supernatant and wash twice with PBS. Add 500ul of the solution to each well as shown in the table below, and incubate at 37°C in the dark for 10 minutes. Then discard the supernatant, wash twice with PBS, add 500 ul PBS to each well, and observe and photograph under a fluorescence microscope.

| Cell | Blank control (PBS) | FITC-L47 (10ug/ml PBS solution) |
|---|---|---|
| Hela | 500ul | 500ul |
| Hela-hNTCP | 500ul | 500ul |
| Hela-hOATP1B3 | 500ul | 500ul |
| Hela-hNTCP/ hOATP1B3 | 500ul | 500ul |

As shown in Figure 11, the Leica LED8 cyan Under the irradiation of 475±14nm wavelength excitation light, among the cells incubated with blank control (PBS), neither Hela nor Hela-hNTCP cells showed fluorescence (No. 1, 2); cells transfected with hOATP1B3 gene (Hela-hOATP1B3 and Hela -hNTCP/hOATP1B3 cells, No. 3, 4) show orange-yellow fluorescence, which should be the fluorescence emitted by the expressed hOATP1B3 protein under excitation light. However, cells in each group did not show green fluorescence, so cells in each group did not show green autofluorescence; after 10 minutes of incubation with L47-FITC, Hela cells did not show fluorescence (number 5), so Hela cells did not bind to L47-FITC. To exclude non-specific binding; after 10 minutes of incubation with L47-FITC, Hela-hNTCP cells showed fluorescence (number 6). The fluorescence was distributed on the cell membrane, the cell outline was clear, and no obvious green fluorescence signal was displayed in the cytoplasm. Considering that Hela cells do not bind to L47-FITC (No. 5) and Hela-hNTCP has no spontaneous green fluorescence (No. 2), it is shown that Hela-hNTCP displays green fluorescence after incubation with L47-FITC, indicating that L47-FITC binds to hNTCP, and the binding is located in the cell membrane part, and L47-FITC does not enter the cell cytoplasm. After 10 minutes of incubation with L47-FITC, Hela-hOATP1B3 cells showed green fluorescence, which was located in the cytoplasm and the cell outline was blurred; orange fluorescence was also visible in the cells (No. 7). Considering that Hela cells do not bind to L47-FITC (No. 5), and Hela-hOATP1B3 emits only orange fluorescence but no green fluorescence under excitation light (No. 3), it shows the green color that Hela-hOATP1B3 appears after incubation with L47-FITC. Fluorescence shows that L47-FITC binds to hOATP1B3, and the binding is located in the cytoplasm of the cell, indicating that hOATP1B3 can mediate L47-FITC

to enter the cell cytoplasm. After incubation with L47-FITC for 10 minutes, Hela-hNTCP/hOATP1B3 cells showed green fluorescence, which was located in the cytoplasm and the cell outline was blurred; orange fluorescence was also visible in the cells (No. 8). Compared with the incubation of Hela-hOATP1B3 and L47-FITC (No. 7), the green fluorescence intensity is enhanced, indicating that hNTCP has enhanced the effect of hOATP1B3 on mediating L47-FITC into cells.

Since both NTCP and OATP1B3 are considered to be specifically expressed in liver cells, Hela cells are cells of non-liver origin and should not express NTCP and OATP1B3. Moreover, in this example, Hela cells showed no fluorescence after incubation with FITC-L47, which also excludes the expression of NTCP and OATP1B3 in Hela cells. The above results prove that both hNTCP and hOATP1B3 can bind to L47-FITC, but the binding of hNTCP to L47-FITC is limited to the cell membrane and does not mediate the entry of L47-FITC into cells. hOATP1B3 can mediate the entry of L47-FITC into cells, and hNTCP enhances the effect of hOATP1B3 on mediating the entry of L47-FITC into cells.

NTCP is considered to be a functional receptor for HBV and HDV infection [Yan H et al. Elife. 2012;1:e00049.; Ni Y et al. Gastroenterology. 2014;146(4):1070-83.], NTCP from multiple species including human, rat, mice and canine NTCP can bind to HBVPre-S1 derived peptides [Lempp FA, te al. Hepatology. 2017;66(3):703-716.], this example also shows that the HBVPre-S1 derived example polypeptide can bind to human NTCP, which is consistent with literature reports. In this example, it is not shown that NTCP has the effect of mediating the entry of hepadnavirus surface antigen polypeptide into cells. This example reveals that polypeptides derived from hepadnavirus surface antigens can bind to OATP alone and mediate the entry of hepadnavirus surface antigen polypeptides into cells within 10 minutes. When NTCP is expressed simultaneously with OATP, the intensity of cell-bound fluorescence can be enhanced. In view of the literature reports that OATP and NTCP can form heterodimers, combined with this example showing that the surface antigen polypeptide derived from hepadnavirus specifically binds to OATP and can be mediated by OATP to enter cells, it can be reasonably inferred that OATP is a component of HBV and NTCP. The infection receptor of HDV, or together with NTCP, forms the infection co-receptor (co-receptor) of HBV and HDV.

Through this example and Example 1, it is shown that the polypeptide derived from the surface antigen of hepadnavirus can bind to OATP; the polypeptide derived from the surface antigen of hepadnavirus mediates entry into cells after binding to OATP. In particular, it is shown that the polypeptide derived from the 13-59 amino acid sequence of the human C genotype HBVPre-S1 region (shown in SEQ ID NO: 17) (an example amino acid sequence is shown in SEQ ID NO: 30), whose N It has myristic acid modification at the end and amidation modification at the C-terminus (shown in SEQ ID NO: 67, polypeptide code L47), which can be compared with human OATP1B3 (shown in SEQ ID NO:1 and 2, GenBank NM_019844) combined.

Through this example and Example 1, it is shown that OATP is a co-receptor for HBV and/or HDV infection, which can be achieved by preventing or reducing the interaction between HBV and/or HDV and OATP co-receptor, or by preventing or reducing OATP expression and/or or function, implement or prepare methods and medicaments for treating and/or preventing HBV and/or HDV infection and related diseases. In particular, methods and agents for treating and/or preventing infections and related diseases of HBV and/or HDV can be implemented or prepared by preventing or reducing the interaction of HBV and/or HDV with human OATP1B3 and/or OATP1B1, or by preventing or reducing human OATP1B3 and/or OATP1B1 expression and/or function. One embodiment is that the polypeptide derived from the surface antigen of hepadnavirus can be combined with OATP to competitively prevent or weaken the interaction between the surface antigen of hepadnavirus and OATP, so as to implement or prepare the treatment and/or prevention of HBV and/or HBV. Or methods and agents for HDV infection and related diseases. In particular, methods and agents for the treatment and/or prevention of HBV and/or HDV infections and related diseases are implemented or prepared by competitively preventing or attenuating the action of the human HBVPre-S1 region with human OATP1B3 and/or OATP1B1 by means of polypeptides derived from the human HBVPre-S1 region and human OATP1B3 and/or OATP1B1.

By this Example and Example 1, OATP is shown to be a co-receptor for HBV and or HDV infection, and cellular and/or animal models of susceptibility to HBV and/or HDV can be constructed by transferring an exogenous SLCO gene or expressing an exogenous OATP protein. In particular, cellular and/or animal models susceptible to HBV and/or HDV are constructed by transferring exogenous SLCO1B3 and/or human SLCO1B1 genes or expressing exogenous OATP1B3 and/or OATP1B3 proteins. The cellular and/or animal models can also be utilized for drug screening to obtain drugs for the treatment or prevention of diseases associated with HBV and/or HDV infection.

Through this example and Example 1, it is shown that the vehicle targeting OATP can achieve intracellular delivery of drugs. In particular vectors targeting OATP1B3 and/or OATP1B3, in particular OATP-targeted peptide vectors derived from hepatophilic DNA viral surface antigens, in particular peptide vectors with a core sequence selected from a polypeptide with an amino acid sequence of amino acids 14-48 in the region of the C-genotype HBVPre-S1 region; R1 selected from the cardamomate hydrophobic motif; and a R2-flanking sequence selected from a polypeptide with an N-terminal selection modified from a polypeptide of amino acid sequence of amino acids 50-59 in the region of the C-genotype HBVPre -S1 region amino acid sequences at amino acids 50-59 of the polypeptide with the N-terminal selected for amidation modification, and the exemplary vector sequence is shown in SEQ ID NO:159. In this example, FITC is selected as an exemplary pharmacophore group for R3, and the OATP targeting vehicle can achieve effective intracellular delivery.

Example 3

This example tests the inhibitory effect of polypeptides derived from the surface antigen of hepadnavirus on OATP transport function.

In this example, the polypeptide derived from the surface antigen of hepadnavirus (shown in SEQ ID NO:67, polypeptide code L47 or Hepalatide) is selected from the polypeptide derived from the 13-59 amino acid sequence (shown in SEQ ID NO: 30) of the human C genotype HBVPre-S1 region (shown in SEQ ID NO: 17), and its N-terminus is modified with myristic acid, and its C-terminus is amidated. In this example, the OATP is human OATP1B3 (hOATP1B3) and human OATP1B1 (hOATP1B1), and the exemplary amino acid and nucleic acid coding sequences are shown in SEQ ID NO: 2 (GenBank NM_019844) and SEQ ID NO: 5 (GenBank NM_006446) respectively. In this example, human OCT2, OAT1 and OAT3 were selected as other transporters. In this example, the standard substrate for human OATP1B3 (hOATP1B3) and human OATP1B1 (hOATP1B1) is 17β-glucoside-estradiol (E217βG), and rifampicin is used as the positive Inhibitors for human OATP1B3 (hOATP1B3) and human OATP1B1 (hOATP1B1). In this example, the substrates for human OCT2, OAT1 and OAT3 are respectively metformin (MET), para-aminohippuric acid (PAH) and estrone sulfate (ES), quinidine was selected as the OCT2 positive inhibitor, and probenecid was selected As a positive inhibitor of OAT1 and OAT3.

The experimental materials used in this example are as follows:

1. Cells

HEK293 cell line stably expressing human OATP1B1, OATP1B3, OAT1, OAT3, OCT2, MATE1 and MATE2K (HEK293-OATP1B1, HEK293-OATP1B3, HEK293-OAT1, HEK293-OAT3 and HEK293-OCT2), and transgenic HEK293 cell line transfected with blank plasmid (HEK293-MOCK), and the above cells were provided by GenoMembrane Company of Japan.

2. Main chemical reagents

17β-glucoside-estradiol (Cat. No. Sigma E1127), metformin hydrochloride (Cat. No. USP 1396309), p-aminohippuric acid (Cat. No. Sigma A1422), estrone sulfate (Cat. No. Sigma E0251), rifampin (Cat. No. Sigma R3501), quinidine (Cat. No. TRC Q685000), probenecid (Cat. No. Sigma P8761), DMEM medium (Cat. No. Gibco 11885-084), trypsin (Cat. No. Gibco 25300-30), penicillin chain mycin mixed solution (Cat. No. Gibco 15140-122), fetal bovine serum (Cat. No. Gibco 10099-141), G418 hydrochloride (Cat. No. Sigma A17200), BCA kit (Cat. No. Thermo 23227), 24-well lysine-coated culture plate (Cat. No. CORNING 354414).

Internal standard information: tolbutamide (Cat. No. Sigma T0891), buspirone (Cat. No. Sigma B7148).

Hepalatide (number: L47), molecular weight 5399.1Da, sequence is shown in SEQ ID NO: 67. L47 was synthesized by China Peptide Biochemical Co., Ltd. (Product No. 820734), with HPLC purity of 99.9% (Figure 4, A). The mass spectrometry identification of the synthesized peptide was consistent with the molecular weight of the target peptide (Figure 4, B).

3. LC-MS/MS system:

includes LEAP CTC HTS PAL automatic sampling system (Swiss CTC Analytical Instruments Co., Ltd.), Shimadzu LC20 (Japanese Shimadzu Corporation) liquid chromatography system and API5000 triple quadrupole detector (American Applied Biosystems Co., Ltd.). The operating software is Analyst 1.6.3.

The method of this example includes the following steps:
Cells for experiments were cultured in DMEM medium at 37°C and 5% CO2. Among them, DMEM medium contains 10% fetal calf serum, penicillin-streptomycin solution (100 U/mL and 0.1 mg/mL) and 0.5 mg/mL geneticin. Before the transfer experiment, cells were seeded in a 24-well plate at a density of 4×105 cells/well. The transfer experiment can be performed when the cell confluence reaches 80-90%.

The concentrations of substrates and positive inhibitors for each transporter are as follows. The final concentrations tested for L47 are 10 μM, 5 μM, 1 μM, 0.5 μM and 0.1 μM.

| SLC transporter | Substrate (final concentration) | Positive inhibitor (final concentration) |
|---|---|---|
| OATP1B1 | 17β-glucoside-estradiol (20 μM) | Rifampicin (100 μM) |
| OATP1B3 | 17β-glucoside-estradiol (30 μM) | |
| OCT2 | Metformin (100 μM) | Quinidine (500 μM) |

(continued)

| SLC transporter | Substrate (final concentration) | Positive inhibitor (final concentration) |
|---|---|---|
| OAT1 | Para-aminohippuric acid (20 μM) | Probenecid (50 μM) |
| OAT3 | Estrone sulfate (20 μM) | |

Manufacture of L47 test substance (hepalatide) working solution: Use DMSO to prepare a test substance stock solution with a concentration of 2 mM, and then dilute it with DMSO in a gradient to 2000 μM, 1000 μM, 200 μM, 100 μM and 20 μM concentration working solution, and dilute the above working solutions of different concentrations to 20 μM, 10 μM, 2 μM, 1 μM and 0.2 μM concentration dosing solutions respectively, so that the final concentrations of the test substance in the test system are 10 μM, 5 μM and 5 μM respectively. μM, 1 μM, 0.5 μM and 0.1 μM.

Pre-incubation of OATP1B1 and OATP1B3: Aspirate the culture medium from the culture plate, and wash the cells twice with 37°C pre-warmed TB buffer (pH7.4), 0.5 mL each time. During the second wash, leave the buffer in the culture plate and equilibrate at 37°C for 5 minutes; discard the buffer in the culture plate and add 1× pre-incubation dosing solution or blank buffer (NC group which includes MOCK cells and each transporter cell. The MOCK cell group is used to calculate the uptake ratio and passive transport volume. The same below) or selective inhibitor (PC group) containing different concentrations of test substances pre-warmed at 37°C and placed in incubator and incubate for 30 minutes; discard the solution in the culture plate, add different concentrations of test substance containing probe substrate or blank buffer or selective inhibitor mixed dosage solution pre-warmed at 37°C, and continue incubating for 10 minutes. Pre-incubation of the remaining transporters: Aspirate the culture medium from the culture plate, and wash the cells twice with 37°C pre-warmed buffer (pH7.4), 0.5 mL each time. During the second wash, leave the buffer in the culture plate and equilibrate at 37°C for 5 minutes; discard the buffer in the culture plate and add a mixed dosing solution containing probe substrate and test substance of different concentrations pre-warmed at 37°C or a mixed dosing solution of probe substrate and TB buffer (NC group) or probe Mixed dosing solution of substrate and selective inhibitor (PC group), continue incubation for 10 minutes.

After the incubation, immediately aspirate the solution in the culture plate and wash it three times with 4°C pre-cooled buffer, 0.5 mL each time, to remove the remaining dosing solution in the plate. After washing, add 0.3 mL of distilled water to each well, and repeatedly freeze and thaw with liquid nitrogen three times (-196°C to 37°C) to completely lyse the cells. Precipitate 100 μL of cell lysate and methanol containing internal standard at a ratio of 1:4, centrifuge at 12000 rpm, 4°C for 5 min, take the supernatant, and use the LC-MS/MS method to detect the amount of probe substrate in the supernatant. See point 6 for instruments and methods. Take 25 μL of cell lysate and use the Pierce® BCA Protein Assay Kit to detect the cell protein concentration of the sample.

The LC-MS/MS method is used to detect the substrate concentration in the cell lysate. The LC-MS/MS analysis method of 17β-glucoside-estradiol is as follows:
Chromatographic conditions: chromatographic system Shimadzu LC 20 series; column Phenomenex SynergiTM 4 μm Hydro-RP 80 A30*2 mm; column temperature 40 °C; mobile phase A: 0.1% formic acid in water (v/v), B: 0.1% formic acid in acetonitrile (v/v); flow rate 450 μL/min; running time 2.5min; injection volume 5 μL.

| | Time (min) | %A | %B |
|---|---|---|---|
| gradient | 0.01 | 90 | 10 |
| | 0.6 | 5 | 95 |
| | 1.5 | 5 | 95 |
| | 1.51 | 90 | 10 |
| | 2.5 | 90 | 10 |

Mass spectrometry conditions: mass spectrometry system API 5000; scan mode ESI-, MRM; collision gas 8 psi; curtain gas 20 psi; nebulization gas 50 psi; auxiliary gas 50 psi; ion voltage -4500 volts; temperature 500°C.

MS/MS parameters

| Compound | Q1 | Q3 | Dwell time | DP (v) | EP (v) | CE (v) | CXP (v) |
|---|---|---|---|---|---|---|---|
| 17β-glucoside-estradiol | 447.50 | 74.90 | 50 ms | -100 | -10 | -58 | -10 |
| Tolbutamide (internal standard) | 269.30 | 170.00 | 50 ms | -55 | -10 | -25 | -10 |

Data analysis

(1) The substrate uptake rate (U, pmol/mg/min) is calculated using the following formula:

$$U=C_lysate/(P\times T)$$

Among them, Clysate is the amount of transported substrate in the cell lysate (pmol); P is the amount of cellular protein (mg); T is the incubation time (min).

(2) The net uptake rate of the substrate (NU, pmol/mg/min) is calculated using the following formula:

$$NU = \text{average of } U\text{-}U_MOCK$$

(3) Relative uptake activity (% of NC) is calculated using the following formula:

$$\%\text{ of NC} = NU_{TA} / NU_{NC}\text{ average }*100$$

$NU_{TA}$ is the net uptake rate of the experimental group or positive control group, and $NU_{NC}$ is the net uptake rate of the negative control group.

(4) Uptake Ratio is calculated using the following formula;

UR = average substrate uptake rate of transporter cells in the NC group / the average --> substrate uptake rate of MOCK cells in the NC group.

When the uptake ratio is greater than or equal to 2, it indicates that the transport system is working properly.

(5) The half inhibitory concentration ($IC_{50}$) is calculated using the following formula:

$$Y=100/(1+10\^{}(X\text{-}LogIC50))$$

Among them, X is the concentration of the test substance (Log μM), Y is the relative activity (% of NC), and the $IC_{50}$ and 95% confidence interval are calculated by the software Prism.

(6) Data processing: Both original data and calculated values retain three significant figures.

Results and discussion

In the negative control group (NC) without test substance, the uptake ratios of probe substrates mediated by transporters OATP1B1, OATP1B3, OAT1, OAT3 and OCT2 were 15.0 (Table 1), 3.70 (Table 2), 315 (Table 3), 156 (Table 4) and 48.2 (Table 5), and after adding selective inhibitors of each transporter to the positive control group, the transport activity of the substrate was reduced to 0.826% (Table 1), 0.297% (Table 2), 6.54% (Table 3), and 19.0% of the negative control group, respectively. (Table 4) and 0.00% (Table 5). The results of the above control group indicate that the test system of each transporter is suitable for the study of this experiment.

Under the conditions of test concentrations of 0.1 μM, 0.5 μM, 1 μM, 5 μM and 10 μM, L47 has a concentration-dependent inhibitory effect on the transport activity of OATP1B3 and OATP1B3. The half inhibitory concentrations (IC50) can be calculated to be 0.813 μM (Table 1 and Figure 12) and 0.559 μM (Table 2 and Figure 13) respectively; L47 has no obvious inhibitory effect on the transport activities of OAT1, OAT3 and OCT2 (Table 3-5).

Table 1: Inhibitory effect of L47 on OATP1B1 uptake of E217βG activity

| Test system/test concentration (μM) | uptake rate (pmol/mg/min) | | Net uptake rate (pmol/mg/min) | | relative uptake activity (% of NC) | |
|---|---|---|---|---|---|---|
| | mean | standard deviation | mean | standard deviation | mean | standard deviation |
| MOCK | 3.33 | 1.31 | NA | NA | NA | NA |
| OATP1B1/NC | 50.0 | 2.03 | 46.6 | 2.03 | 100 | 4.36 |
| OATP1B1/PC | 3.41 | 1.00 | 0.385 | NA | 0.826 | NA |
| 10 | 4.60 | 1.47 | 1.28 | NA | 2.75 | NA |
| 5 | 5.49 | 0.326 | 2.16 | 0.326 | 4.63 | 0.700 |
| 1 | 18.1 | 2.88 | 14.8 | 2.88 | 31.7 | 6.19 |
| 0.5 | 39.6 | 4.53 | 36.2 | 4.53 | 77.7 | 9.71 |
| 0.1 | 49.5 | 4.20 | 46.2 | 4.20 | 99.1 | 9.00 |
| | | | 15.0 | | | |
| $IC_{50}$(μM) | | | 0.813 | | | |
| 95% confidence interval(μM) | | | 0.534-1.24 | | | |
| *: The calculated value is a negative number and is counted as "0.00"; NA: Not applicable. The mean is the mean of three samples. | | | | | | |

Table 2: Inhibitory effect of L47 on OATP1B3 uptake of E217βG activity

| Test system/test concentration (μM) | uptake rate (pmol/mg/min) | | Net uptake rate (pmol/mg/min) | | relative uptake activity (% of NC) | |
|---|---|---|---|---|---|---|
| | mean | standard deviation | mean | standard deviation | mean | standard deviation |
| MOCK | 3.83 | 0.747 | NA | NA | NA | NA |
| OATP1 B3/NC | 14.2 | 2.23 | 10.3 | 2.23 | 100 | 21.5 |
| OATP1 B3/PC | 3.27 | 0.726 | 0.0308 | NA | 0.297 | NA |
| 10 | 3.73 | 2.84 | 0.00 | NA | 0.00 | NA |
| 5 | 3.22 | 0.619 | 0.0314 | NA | 0.303 | NA |
| 1 | 5.53 | 0.498 | 1.70 | 0.498 | 16.4 | 4.82 |
| 0.5 | 10.9 | 2.46 | 7.10 | 2.46 | 68.7 | 23.8 |
| 0.1 | 13.9 | 1.61 | 10.1 | 1.61 | 97.5 | 15.6 |
| Uptake Ratio UR=NC/MOCK | | | 3.70 | | | |
| $IC_{50}$(μM) | | | 0.559 | | | |
| 95% confidence interval(μM) | | | 0.312-1.00 | | | |
| *: The calculated value is a negative number and is counted as "0.00"; NA: Not applicable. The mean is the mean of three samples. | | | | | | |

Table 3: Inhibitory effect of L47 on substrate uptake activities of OAT1, OAT2 and OCT2 relative uptake activity (% of NC)

| Test system/test concentration (μM) | OAT1 | | OAT3 | | OCT2 | |
|---|---|---|---|---|---|---|
| | mean | standard deviation | mean | standard deviation | mean | standard deviation |
| MOCK | NA | NA | NA | NA | NA | NA |

(continued)

| Test system/test concentration (μM) | OAT1 | | OAT3 | | OCT2 | |
|---|---|---|---|---|---|---|
| | mean | standard deviation | mean | standard deviation | mean | standard deviation |
| NC | 100 | 8.40 | 100 | 8.32 | 100 | 4.17 |
| PC | 6.54 | 1.37 | 19.0 | 1.21 | 0.00 | NA |
| 10 | 110 | 14.5 | 106 | 8.64 | 108 | 5.86 |
| 5 | 110 | 3.67 | 104 | 4.13 | 106 | 6.00 |
| 1 | 109 | 8.07 | 97.2 | 8.52 | 95.1 | 7.47 |
| 0.5 | 109 | 13.3 | 97.6 | 7.35 | 92.8 | 2.94 |
| 0.1 | 111 | 14.1 | 95.9 | 7.67 | 103 | 6.92 |
| Uptake Ratio UR=NC/MOCK | 315 | | 156 | | 48.2 | |
| $IC_{50}$(μM) | NA | | NA | | NA | |
| 95% confidence interval(μM) | NA | | NA | | NA | |
| NA: Not applicable. The mean is the mean of three samples. | | | | | | |

In this example, the HEK293 cell lines stably expressing the human transporter (HEK293-OATP1B1, HEK293-OATP1B3, HEK293-OAT1, HEK293-OAT3, HEK293-OCT2, HEK293-MATE1 and HEK293-MATE2K) were used to evaluate the Inhibition of transport effect of the test substance L47 on the above five SLC transporters at test concentrations of 0.1 μM, 0.5 μM, 1 μM, 5 μM and 10 μM. The results of this experiment show that in the blank negative control group (NC) without test substance or inhibitor, the uptake ratio of each probe substrate is greater than the benchmark value of 2.0 that is judged as a substrate, suggesting that each transporter testing system is suitable for research in this experiment. Test substance L47 has a concentration-dependent inhibitory effect on the transport activities of OATP1B1 and OATP1B3, and its half inhibitory concentrations (IC50) can be calculated to be 0.813 μM and 0.559 μM respectively; within the test concentration range of 0.1 μM-10 μM, L47 had no obvious inhibitory effect on the transport activities of OAT1, OAT3 and OCT2.

Through this experiment, the inhibitory effect of polypeptide derived from the surface antigen of hepadnavirus on the OATP transport function is shown. In particular, the polypeptide derived from the amino acid sequence 13-59 of the human C genotype HBVPre-S1 region, with myristic acid modification at the N terminus and amidation modification at the C terminus (an example structure is shown in SEQ ID NO: 67, peptide code L47 or hepalatide), inhibits the transport function of human OATP1B3 (hOATP1B3) and human OATP1B1 (hOATP1B1).

Example 4

This example tests the inhibitory effect of polypeptides derived from the surface antigen of hepadnavirus on the function of OATP transporting drugs.

In this example, the polypeptide derived from the surface antigen of hepadnavirus (shown in SEQ ID NO:67, polypeptide code L47 or Hepalatide) is selected from the polypeptide derived from the 13-59 amino acid sequence (shown in SEQ ID NO: 30) of the human C genotype HBVPre-S1 region (shown in SEQ ID NO: 17), and its N-terminus is modified with myristic acid, and its C-terminus is amidated. In this example, the OATP is human OATP1B3 (hOATP1B3) and human OATP1B1 (hOATP1B1), and the exemplary amino acid and nucleic acid coding sequences are shown in SEQ ID NO: 2 (GenBank NM_019844) and SEQ ID NO: 5 (GenBank NM_006446) respectively. The drugs transported by OATP are statins and sartans, and atorvastatin and valsartan are further selected as examples of OATP transported drugs. In this example, rifampicin was used as a positive inhibitor of human OATP1B3 (hOATP1B3) and human OATP1B1 (hOATP1B1).

The experimental materials used in this example are as follows:

1. Cells

HEK293 cell lines stably expressing human OATP1B1 and OATP1B3 transporters respectively (HEK293-OATP1B1, HEK293-OATP1B3) and Transgenic HEK293 cell line transfected with blank plasmid (HEK293-MOCK) were provided by GenoMembrane Company of Japan.

2. Main reagents and instruments

Atorvastatin (Cat. No. PHR1422), valsartan (Cat. No. PHR1315), G418 hydrochloride (Cat. No. A1720) from Sigma; DMEM culture medium (Cat. No. 11885-084), trypsin (Cat. No. 25300-062), penicillin-streptomycin mixture solution (Cat. No. 15140-122), fetal bovine serum (Cat. No. 10099-141) from Gibco; BCA kit (catalog number 23225) is from Thermo; 24-well lysine-coated culture plate (Cat. No. 354414) from CORNING. Internal standard information: Tolbutamide (Cat. No. T0891) is from Sigma Company; Verapamil (Cat. No. 107868) is from J&K Company. Hepalatide (number: L47), molecular weight 5399.1Da, sequence is shown in SEQ ID NO: 099. L47 was synthesized by China Peptide Biochemical Co., Ltd. (Product No. 820734), with HPLC purity of 99.9% (Figure 4, A). The mass spectrometry identification of the synthesized peptide was consistent with the molecular weight of the target peptide (Figure 4, B).

LC-MS/MS system includes LEAP CTC HTS PAL automatic sampling system (Swiss CTC Analytical Instruments Co., Ltd.), Shimadzu LC20 liquid chromatography system (Shimadzu Corporation, Japan) and API5000 triple quadrupole detector (American Applied Biosystems Co., Ltd.). The operating software is Analyst 1.6.3.

The method of this example includes the following steps:

Cells for experiments were cultured in DMEM medium at 37°C and 5% CO2. Among them, DMEM medium contains 10% fetal calf serum, penicillin-streptomycin solution (100 U/mL and 0.1 mg/mL) and 0.5 mg/mL geneticin. Before the transfer experiment, cells were seeded in a 24-well plate at a density of 4×105 cells/well. The transfer experiment can be performed when the cell confluence reaches 80-90%.

The concentrations of substrates and positive inhibitors for each transporter are as follows. The final concentrations tested for L47 are 10 μM, 5 μM, 1 μM, 0.5 μM and 0.1 μM.

| SLC transporter | Probe substrate (final concentration) | Inhibitor (final concentration) |
|---|---|---|
| **OATP1B1** | Valsartan (5 μM), Atorvastatin calcium (0.05 μM) | Rifampicin (100 μM) |
| **OATP1B3** | Valsartan (5 μM), Atorvastatin calcium (0.05 μM) | |

Preparation of working solution: Use PBS to prepare a test substance stock solution with a concentration of 6 mM, and then dilute it with PBS in a gradient to 2000 μM, 1000 μM, 200 μM, 100 μM and 20 μM concentration working solution, and dilute the above working solutions of different concentrations to 20 μM, 10 μM, 2 μM, 1 μM and 0.2 μM concentration dosing solutions (Dosing Solution) respectively, so that the final concentrations of the test substance in the test system are 10 μM, 5 μM and 5 μM respectively. μM, 1 μM, 0.5 μM and 0.1 μM.

Pre-incubation of OATP1B1 and OATP1B3: Aspirate the culture medium from the culture plate, and wash the cells twice with 37°C pre-warmed TB buffer (pH7.4), 0.5 mL each time. During the second wash, leave the buffer in the culture plate and equilibrate at 37°C for 5 minutes; discard the buffer in the culture plate and add 1× pre-incubation dosing solution or blank buffer (NC) or selective inhibitor (PC) containing different concentrations of test substances pre-warmed at 37°C and placed in incubator and incubate for 30 minutes; discard the solution in the culture plate, add different concentrations of test substance containing probe substrate or blank buffer or selective inhibitor mixed dosage solution pre-warmed at 37°C, and continue incubating for 5 minutes. Among them, the NC group includes MOCK cells and each transporter cell, and the MOCK cell group is used to calculate the uptake ratio and passive transport amount.

After the incubation, immediately aspirate the solution in the culture plate and wash it three times with 4°C pre-cooled buffer, 0.5 mL each time, to remove the remaining dosing solution in the plate. After washing, add 0.3 mL of distilled water to each well, and repeatedly freeze and thaw with liquid nitrogen three times (-196°C to -37°C) to completely lyse the cells. Precipitate 100 μL of cell lysate and methanol containing internal standard at a ratio of 1:4, centrifuge at 12000 rpm, 4°C for 5 min, take the supernatant, and use the LC-MS/MS method to detect the amount of probe substrate in the supernatant. Take 25 μL of cell lysate and use the Pierce® BCA Protein Assay Kit to detect the cell protein concentration of the sample.

Detection of atorvastatin calcium and valsartan concentrations in samples using LC-MS/MS method. The LC-MS/MS analysis method of atorvastatin calcium is as follows.

Chromatographic conditions: chromatographic system Shimadzu LC 20 series; column Phenomenex SynergiTM 4 μm Hydro-RP 80 A30*2 mm; column temperature 40 °C; mobile phase A: 0.1% formic acid in water (v/v), B: 0.1% formic acid in acetonitrile (v/v); flow rate 450 μL/min; running time 2.50 min; injection volume 5 μL.

| gradient | Time (min) | %A | %B |
|---|---|---|---|
| | 0.01 | 90 | 10 |
| | 0.60 | 5 | 95 |

(continued)

| gradient | Time (min) | %A | %B |
|---|---|---|---|
| | 1.50 | 5 | 95 |
| | 1.51 | 90 | 10 |
| | 2.50 | 90 | 10 |

Mass spectrometry conditions: mass spectrometry system API 5000; scan mode ESI+, MRM; collision gas 8 psi; curtain gas 10 psi; nebulization gas 50 psi; auxiliary gas 50 psi; ion voltage 5500 volts; temperature 550°C.

MS/MS parameters

| Compound | Q1 | Q3 | Dwell time | DP (v) | EP (v) | CE (v) | CXP (v) |
|---|---|---|---|---|---|---|---|
| Atorvastatin calcium | 559.30 | 440.30 | 50 ms | 91 | 10 | 33 | 10 |
| Tolbutamide (internal standard) | 271.10 | 155.00 | 50 ms | 57 | 10 | 26 | 15 |

The LC-MS/MS analysis method of valsartan is as follows. Chromatographic conditions: chromatographic system Shimadzu LC 20 series; column Phenomenex SynergiTM 4 μm Hydro-RP 80 A30*2 mm; column temperature 40°C; mobile phase A: 0.1% formic acid in water (v/v), B: 0.1% formic acid in acetonitrile (v/v); flow rate 450 μL/min; running time 2.50 min; injection volume 5 μL.

| gradient | Time (min) | %A | %B |
|---|---|---|---|
| | 0.01 | 90 | 10 |
| | 0.60 | 5 | 95 |
| | 1.50 | 5 | 95 |
| | 1.51 | 90 | 10 |
| | 2.50 | 90 | 10 |

Mass spectrometry conditions: mass spectrometry system API 5000; scan mode ESI+, MRM; collision gas 8 psi; curtain gas 20 psi; nebulization gas 50 psi; auxiliary gas 50 psi; ion voltage 5500 volts; temperature 550°C.

MS/MS parameters

| Compound | Q1 | Q3 | Dwell time | DP (v) | EP (v) | CE (v) | CXP (v) |
|---|---|---|---|---|---|---|---|
| valsartan | 436.50 | 291.10 | 50 ms | 91 | 10 | 33 | 10 |
| Tolbutamide (internal standard) | 271.10 | 155.00 | 50 ms | 57 | 10 | 26 | 15 |

The data analysis method is the same as that in Example 3. The results of this example show that in the negative control group (NC) without test substance, the uptake ratios of valsartan mediated by the transporters OATP1B1 and OATP1B3 were 3.25 and 6.90 respectively (Table 4), indicating that the drug base in this implementation system Established through OATP1B1 and OATP1B3 transport, and excluding the uptake and leakage of substrates by MOCK cells, the uptake ratios of drug substrates are all greater than the benchmark value of 2.0 that is judged as a substrate, indicating that each transporter testing system is suitable for the study of this experiment. After adding selective inhibitors of each transporter to the positive control group (PC), the transport activity of the substrate was reduced to 0.00% and 0.00% of that of the negative control group respectively (Table 4), indicating that OATP1B1 and OATP1B3 are sensitive to the inhibitors. The implementation system is suitable for detecting the inhibitory effect of test substances on transporters.

In the negative control group (NC) without test substance, the uptake ratios of atorvastatin mediated by the transporters OATP1B1 and OATP1B3 were 6.32 and 8.58, respectively (Table 4), indicating that the drug substrate in this implementation system passes through OATP1B1 and OATP1B3 transport function established, and excluding the uptake and leakage of substrates by MOCK cells, the uptake ratios of drug substrates are all greater than the benchmark value of 2.0 that is judged as a substrate, indicating that each transporter testing system is suitable for the study of this experiment. After adding selective inhibitors of each transporter to the positive control group (PC), the transport activity of the substrate was reduced to 1.52% and 30.9% of that of the negative control group respectively (Table 4), indicating that OATP1B1 and OATP1B3 are sensitive to the inhibitors. The implementation system is suitable for detecting the inhibitory effect of test substances on transporters.

The results showed that hepalatide had a significant inhibitory effect on the valsartan transport activity of OATP1B1 and OATP1B3 at concentrations of 0.1 μM, 0.5 μM, 1 μM, 5 μM and 10 μM, and the half inhibitory concentration was calculated (IC50) are 0.322 μM (Table 4 and Figure 14) and 0.609 μM (Table 4 and Figure 15) respectively. The transport activity of atorvastatin also significantly inhibits the transport of OATP1B1 and OATP1B3, with IC50s of 0.166 μM (Table 4 and Figure 16) and <0.1 μM (Table 4) respectively.

Through this experiment, the inhibitory effect of a polypeptide derived from the surface antigen of hepadnavirus on the OATP transport drug function is shown. In particular, the polypeptide derived from the amino acid sequence 13-59 of the human C genotype HBVPre-S1 region, with myristic acid modification at the N terminus and amidation modification at the C terminus (an example structure is shown in SEQ ID NO: 67, peptide code L47 or hepalatide), inhibits the transport function of human OATP1B3 (hOATP1B3) and human OATP1B1 (hOATP1B1) to statins and sartans.

Table 4: Inhibitory effect of hepalatide on the uptake drug activity of OATP1B1 and OATP1B3. relative uptake activity (% of NC)

| Test system/test concentration (μM) | **OATP1B1** | | | | **OATP1B3** | | | |
|---|---|---|---|---|---|---|---|---|
| | valsartan | | Atorvastatin | | valsartan | | **Atorvastatin** | |
| | mean | standard deviation | mean | standard deviation | mean | standard deviation | mean | standard deviation |
| MOCK | NA | NA | NA | NA | NA | NA | NA | NA |
| OATP/NC | 100 | 2.70 | 100 | 8.21 | 100 | 0.794 | 100 | 20.6 |
| OATP/PC | 0.00 | NA | 1.52 | NA | 0.00 | NA | 30.9 | 0.711 |
| 10 | 0.00 | NA | 10.9 | 1.68 | 0.00 | NA | 14.0 | 1.05 |
| 5 | 0.00 | NA | 11.7 | 0.556 | 0.00 | NA | 14.7 | 1.49 |
| 1 | 8.71 | NA | 31.6 | 3.32 | 21.8 | 3.30 | 43.9 | 2.07 |
| 0.5 | 45.3 | 5.87 | 21.4 | 4.95 | 73.7 | 5.08 | 27.9 | 3.53 |
| 0.1 | 86.4 | 15.0 | 54.4 | 4.60 | 90.0 | 10.2 | 38.4 | 0.547 |
| Uptake Ratio UR=NC/MOCK | 3.25 | | 6.32 | | 6.90 | | 8.58 | |
| $IC_{50}$(μM) | 0.322 | | 0.166 | | 0.609 | | < 0.1 | |
| 95% confidence interval(μM) | 0.214-0.484 | | 0.109-0.254 | | 0.390-0.951 | | NA | |
| *: The calculated value is a negative number and is counted as "0.00"; NA: Not applicable. The mean is the mean of three samples. | | | | | | | | |

Example 5

This example tests the inhibitory effect of polypeptides derived from the surface antigen of hepadnavirus on OATP transport function.

In this example, the polypeptide derived from the surface antigen of hepadnavirus (shown in SEQ ID NO:67, polypeptide code L47 or Hepalatide) is selected from the polypeptide derived from the 13-59 amino acid sequence (shown in SEQ ID NO: 30) of the human C genotype HBVPre-S1 region (shown in SEQ ID NO: 17),and its N-terminus is modified with myristic acid, and its C-terminus is amidated and its shortened peptide from the N-terminus. In this example, the OATP is human OATP1B3 (hOATP1B3), and the exemplary nucleic acid coding sequences are shown in SEQ ID NO: 2

(GenBank NM_019844). In this example, the standard substrate of human OATP1B3 (hOATP1B3) is 17β-glucoside-estradiol (E217βG), and rifampicin is used as a positive inhibitor.

The experimental materials used in this example are as follows:

1. Cells

HEK293 cell lines stably expressing OATP1B3 transporters respectively (HEK293-OATP1B3) and Transgenic HEK293 cell line transfected with blank plasmid (HEK293-MOCK) were provided by GenoMembrane Company of Japan.

2. Main chemical reagents

17β-glucoside-estradiol (Cat. No. E1127), rifampicin (Cat. No. R3501), G418 hydrochloride (Cat. No. A1720) from Sigma; DMEM culture medium (Cat. No. 11885-084), trypsin (Cat. No. 25300-062), penicillin-streptomycin mixture solution (Cat. No. 15140-122), fetal bovine serum (Cat. No. 10099-141) from Gibco; BCA kit (Cat. No. 23225) from Thermo.

Internal standard information: Tolbutamide (catalog number T0891) from Sigma.

Test peptide:

Hepalatide (number: L47), molecular weight 5399.1Da, sequence is shown in SEQ ID NO: 67. L47 was synthesized by China Peptide Biochemical Co., Ltd. (Product No. 820734), with HPLC purity of 99.9% (Figure 4, A). The mass spectrometry identification of the synthesized peptide was consistent with the molecular weight of the target peptide (Figure 4, B). Compared with L47, the N-terminus of P(2-48) polypeptide has no myristic acid modification (myr), P(12-48) N-terminal truncation of 10 amino acids without myristic acid modification (myr), P(21-48) N-terminal truncation of 19 amino acids without myristic acid modification (myr), P(29-48) has an N-terminal truncation of 27 amino acids and no myristic acid modification (myr), and P(34-48) has an N-terminal truncation of 32 amino acids and no myristic acid modification (myr), and the C-terminal amidation modification of each peptide and HPLC purity identification were all greater than 95%. The mass spectrometry identification of each peptide was consistent with the molecular weight of the target peptide. The amino acid sequences of P(12-48), P(21-48), P(29-48) and P(34-48) are shown in SEQ ID NO:179-182, respectively.

P(2-48)(SEQ ID NO: 67):
Myr-GTNLSVPNPLGFFPDHQLDPAFGANSNNPDWDFNPNKDHWPEANQVG-NH2
P(12-48)(SEQ ID NO: 179):
Myr-GFFPDHQLDPAFGANSNNPDWDFNPNKDHWPEANQVG-NH2
P(21-48)(SEQ ID NO: 180):
Myr-PAFGANSNNPDWDFNPNKDHWPEANQVG-NH2
P(29-48)(SEQ ID NO: 181):
Myr-NPDWDFNPNKDHWPEANQVG-NH2
P(34-48)(SEQ ID NO: 182):
Myr-FNPNKDHWPEANQVG-NH2

Instruments and equipment:

LC-MS/MS system includes LEAP CTC HTS PAL automatic sampling system (Swiss CTC Analytical Instruments Co., Ltd.), Shimadzu LC20 liquid chromatography system (Shimadzu Corporation, Japan), liquid Chromatography System and API5000 triple quadrupole detector (American Applied Biosystems Co., Ltd.). The operating software is Analyst 1.6.3.

The method of this example includes the following steps:
Cells for experiments were cultured in DMEM medium at 37°C and 5% CO2. Among them, DMEM medium contains 10% fetal calf serum, penicillin-streptomycin solution (100 U/mL and 0.1 mg/mL) and 0.5 mg/mL geneticin. Before the transfer experiment, cells were seeded in a 24-well plate at a density of 4×105 cells/well. The transfer experiment can be performed when the cell confluence reaches 80-90%.

Preparation of working solution: Use PBS to prepare a test substance stock solution with a concentration of 6 mM, and then dilute it with PBS in a gradient to 200 μM concentration working solution, and dilute the above working solutions of different concentrations to 2 μM concentration dosing solutions (Dosing Solution), so that the final concentrations of the test substance in the test system are 1 μM.

Pre-incubation OATP1B3: aspirate the culture medium from the culture plate, and wash the cells twice with 37°C

pre-warmed buffer (pH7.4), 0.5 mL each time. During the second wash, leave the buffer in the culture plate and equilibrate at 37°C for 5 minutes; discard the buffer in the culture plate and add 1× pre-incubation dosing solution or blank buffer (NC) or selective inhibitor (PC) containing different concentrations of test substances pre-warmed at 37°C and placed in incubator and incubate for 30 minutes; discard the solution in the culture plate, add different concentrations of test substance containing probe substrate or blank buffer or selective inhibitor mixed dosage solution pre-warmed at 37°C, and continue incubating for 10 minutes. The NC group includes MOCK cells and each transporter cell, and the MOCK cell group is used to calculate the uptake ratio and passive transport amount.

After the incubation, immediately aspirate the solution in the culture plate and wash it three times with 4°C pre-cooled buffer, 0.5 mL each time, to remove the remaining dosing solution in the plate. After washing, add 0.3 mL of distilled water to each well, and repeatedly freeze and thaw with liquid nitrogen three times (-196°C to -37°C) to completely lyse the cells. Precipitate 100 μL of cell lysate and methanol containing internal standard at a ratio of 1:4, centrifuge at 12000 rpm, 4°C for 5 min, take the supernatant, and use the LC-MS/MS method to detect the amount of probe substrate in the supernatant. Take 25 μL of cell lysate and use the Pierce® BCA Protein Assay Kit to detect the cell protein concentration of the sample.

The LC-MS/MS method is used to detect the substrate concentration in the cell lysate. The LC-MS/MS analysis method of 17β-glucoside-estradiol is as follows:

Chromatographic conditions: chromatographic system Shimadzu LC 20 series; column Phenomenex Synergi 4 μm Hydro-RP 80 A30*2 mm; column temperature 45°C; mobile phase A: 0.1% formic acid in water (v/v), B: 0.1% formic acid in acetonitrile (v/v); flow rate 450 μL/min; running time 1.50 min; injection volume 5 μL.

| | Time (min) | %A | %B |
|---|---|---|---|
| gradient | 0.00 | 90 | 10 |
| | 0.70 | 5 | 95 |
| | 1.20 | 5 | 95 |
| | 1.21 | 90 | 10 |
| | 1.50 | 90 | 10 |

Mass spectrometry conditions: mass spectrometry system API 4000; scan mode ESI-, MRM; collision gas 6 psi; curtain gas 20 psi; nebulization gas 50 psi; auxiliary gas 50 psi; ion voltage -4500 volts; temperature 500°C.

MS/MS parameters

| Compound | Q1 | Q3 | Dwell time | DP (v) | EP (v) | CE (v) | CXP (v) |
|---|---|---|---|---|---|---|---|
| 17β-glucoside-estradiol | 447.5 | 74.9 | 100 ms | -100 | -10 | -58 | -12 |
| Tolbutamide (internal standard) | 269.3 | 170.0 | 100 ms | -55 | -10 | -25 | -9 |

The data analysis method is the same as that in Example 3. The results showed that in the negative control group (NC) without test substance, the uptake ratio of the probe substrate mediated by OATP1B3 was 14.2, and in the positive control group after adding a selective inhibitor of the transporter, the relative uptake activity was 5.59 % (Table 5). The results of the above control group indicate that the test system of transporter is suitable for the study of this experiment. Under the test concentration of 1 μM, the test substance L47 (hepalatide) has an inhibitory effect on the transport activity of OATP1B3, and the relative uptake activity is 40.1%; the test substances P(2-48), P(12-48), P(21-48), P(29-48) and P(34-48) have no obvious inhibitory effect on the transport activity of OATP1B3, and the relative uptake activity 118%, 103%, 89.9%, 78.5% and 104% respectively (Table 5).

Through this example, the hydrophobic modification of the N-terminal is necessary for the inhibitory effect of polypeptide derived from the surface antigen of hepadnavirus on the OATP transport function is shown.

Table 5: Inhibitory effects of L47 and N-terminal shortening peptide on the E217βG uptake activity of OATP1B1

| Test system/test concentration | uptake rate (pmol/mg/min) | | Net uptake rate (pmol/mg/min) | | relative uptake activity (% ofNC) | |
|---|---|---|---|---|---|---|
| | mean | standard deviation | mean | standard deviation | mean | standard deviation |
| MOCK | 1.40 | NA | NA | NA | NA | NA |
| OATP1B3/NC | 19.9 | 2.46 | 18.5 | 2.46 | 100 | 13.3 |
| OATP1 B3/PC | 2.44 | NA | 1.03 | NA | 5.59 | NA |
| Hepalatide, 1μM | 8.83 | 0.614 | 7.43 | 0.614 | 40.1 | 3.32 |
| P(2-48), 1μM | 23.3 | 3.91 | 21.9 | 3.91 | 118 | 21.1 |
| P(12-48), 1μM | 20.5 | 3.28 | 19.1 | 3.28 | 103 | 17.7 |
| P(21-48), 1μM | 18.1 | 0.745 | 16.7 | 0.745 | 89.9 | 4.03 |
| P(29-48), 1μM | 15.9 | 1.10 | 14.5 | 1.10 | 78.5 | 5.92 |
| P(34-48), 1μM | 20.6 | 2.11 | 19.2 | 2.11 | 104 | 11.4 |
| Uptake Ratio UR=NC/MOCK | | | 14.2 | | | |
| NA: Not applicable. The mean is the mean of three samples. | | | | | | |

Example 6

This example examines OATP and/or NTCP mediating HBV infection. In this example, OATP is selected as human OATP1B3 (hOATP1B3) or human OATP1B1 (hOATP1B1), and NTCP is selected as human NTCP (hNTCP). In this example, the exemplary amino acid and nucleic acid coding sequences of human OATP1B3 (hOATP1B3), human OATP1B1 (hOATP1B1), and human NTCP (hNTCP) are shown in GenBank NM_019844, NM_006446, and GenBank NM_003049 respectively.

In this example, HBV was incubated with HepG2 cells stably expressing hOATPB3, or hOATPB1 and/or hNTCP, ELISA detected the culture supernatant HBsAg, HBeAg, and fluorescence quantitative PCR detected the culture supernatant HBV DNA, showing that OATP and/or NTCP mediates HBV infection.

The experimental materials used in this example are as follows:

1. Cells

HepG2-hOATP1B3 cells: HepG2 cells stably transfected with human OATP1B3. Transfection was lentivirus-mediated, hOATP1B3 nucleic acid coding sequence CDS was adopted from GenBank NM_019844.4 coding sequence (shown in SEQ ID NO:2), and the recombinant lentivirus profile is shown in Figure 1 and contains thaumatin B resistance. After vehicle construction was completed (recombinant lentivirus number WL1142), it was sequenced to be identical to the target hOATP1B3 CDS. Drug susceptibility test results showed that 250 μg/mL hygromycin could kill all cultured HeLa cells, so only HeLa cells infected with lentivirus WL1142 could survive under these conditions. The HepG2 cells cultured were added with recombinant lentivirus WL1142. After 48 hours of infection, the medium containing 250 μg/mL hygromycin B was replaced and the culture was continued for 9 days. All mock-infected (mock) HepG2 cells died; WL1142-infected HepG2 cells survived and were subcultured to form HepG2 cells stably transfected with WL1142 HepG2-hOATP1B3.

HepG2-hOATP1B1 cells: HepG2 cells stably transfected with human OATP1B1. Transfection was mediated by lentivirus, hOATP1B1 nucleic acid coding sequence CDS adopts NM_006446 coding sequence, hOATP1B1 protein C-terminal added 1X FLAG tag N-DYKDDDDK-C, and the recombinant lentivirus map is shown in Figure 17, including blasticidin BSD (Blasticidin) resistance. After vehicle construction was completed (recombinant lentivirus number WL1281), it was sequenced to be identical to the target hOATP1B3-FLAG CDS. BSD is a peptidyl nucleoside antibiotic produced from Streptomyces griseochromogenes (Streptomyces griseochromogenes). Blasticidin mainly inhibits the synthesis of prokaryotic and eukaryotic cell proteins by interfering with the formation of peptide bonds in ribosomes. Blasticidin is used to select transfected cells carrying BSD resistance genes. The recombinant lentivirus WL1281 carries BSD resistance, and the infected cells can acquire BSD resistance. Drug susceptibility test results showed that 2 μg/mL BSD could kill all cultured HepG2 cells, so only HepG2 cells infected with lentivirus WL1281 could survive under these conditions. Recombinant lentivirus WL1281 was added to the cultured HepG2 cells. After 48 hours of infection, the culture

medium containing 2 μg/mL BSD was replaced and cultured for 9 days. All mock-infected (mock) HepG2 cells died; WL1281 infected HepG2 cells survived and were subcultured to form HepG2 cells stably transfected with WL1281 HepG2-hOATP1B1.

HepG2-hNTCP cells: HepG2 cells stably transfected with human NTCP. Transfection was lentivirus-mediated, hNTCP nucleic acid coding sequence CDS was adopted from GenBank NM_003049.4 coding sequence (shown in SEQ ID NO:80), and the recombinant lentivirus profile is shown in Figure 2 and contains puromycin resistance. After the vector was constructed (recombinant lentivirus number WL1143), it was completely consistent with the target hNTCP CDS sequence after sequencing. Drug susceptibility test results showed that 1μg/mL puromycin could kill all cultured HepG2 cells, so only HepG2 cells infected with lentivirus WL1143 could survive under these conditions. The cultured HepG2 cells were added with recombinant lentivirus WL1143. After 48 hours of infection, the medium containing 1 μg/ml puromycin was replaced and cultured for 9 days. All mock-infected (mock) HepG2 cells died; WL1143-infected HepG2 cells survived and were subcultured to form HepG2 cells stably transfected with WL1143 HepG2-hNTCP.

HepG2-hNTCP/hOATP1B3 cells: the cultured HepG2-hNTCP cells were added with recombinant lentivirus WL1142. After 48 hours of infection, the medium containing 250 μg/mL hygromycin B and 1 μg/ml puromycin was replaced and cultured for 9 days. All mock-infected (mock) HepG2-hNTCP cells died; WL1142-infected HepG2-hNTCP cells survived. Therefore, only HepG2-hNTCP cells infected with lentivirus WL1142 can survive under these conditions. After subculture, HepG2-hNTCP cells stably transfected with WL1142, HepG2-hNTCP/hOATP1B3, were formed.

HepG2-hNTCP/hOATP1B1 cells: the cultured HepG2-hNTCP cells were added with recombinant lentivirus WL1281. After 48 hours of infection, the medium containing 2 μg/mL BSD and 1 μg/ml puromycin was replaced and cultured for 9 days. All mock-infected (mock) HepG2-hNTCP cells died; WL1281-infected HepG2-hNTCP cells survived. Therefore, only HepG2-hNTCP cells infected with lentivirus WL1281 can survive under these conditions. After subculture, HepG2-hNTCP cells stably transfected with WL1281, HepG2-hNTCP/hOATP1B1, were formed.

2. HBV virus

Using TNE buffer (Tris-HCl pH 7.4 20mM; NaCl 140mM; EDTA 1mM; pH 7.4) as the solvent, prepare 60%, 45%, 35%, 25%, and 15% *(w/v)* sucrose solutions respectively, and Discontinuous sucrose density gradients (6 ml of 15% sucrose, 6 ml of 25% sucrose, 4 ml of 35% sucrose, 4 ml of 45% sucrose, 4 ml of 60% sucrose) were prepared top-down in high-speed centrifuge tubes (Beckman-Coulter, USA, 40 ml volume; model 357002). HBV DNA from serum of HBeAg-negative chronic hepatitis B patients ≥107 IU/ml. After centrifugation at 1500g for 10 minutes, collect the supernatant and add it to the top of the sucrose density gradient high-speed centrifuge tube, 12ml per test tube. After high-speed centrifugation at 70,000 g for 5 hours at 4°C, use a syringe to extract 3 ml of solution from the bottom of the high-speed centrifuge tube and discard; extract 6 ml of the solution and add it to an ultrafiltration centrifuge tube (Sartorius, Germany, volume 20 ml; filter membrane pore size >100,000 MWD; model Vivaspin 20), centrifuge at 5000g for 30 minutes at 4°C to filter out the sucrose solution, and then refill the ultrafiltration centrifuge tube with TNE, centrifuge at 5000g for 30 minutes at 4°C, and collect the HBV virus purification concentrate in the ultrafiltration centrifuge tube. Use the HBV DNA fluorescence quantitative PCR kit to detect the HBV DNA titer according to the instructions, and freeze the HBV solution at -80 degrees for later use.

3. Reagents and equipment

(1) Culture medium

| Components | Concentration |
|---|---|
| DMEM (GIBCO) | - |
| Fetal bovine serum | 10% |
| Penicillin | 100u/mL |
| Streptomycin | 100μg/mL |

(2) Hygromycin B (Sigma-Aldrich, product number: V900372-1G, molecular weight: 527.52).
(3) Puromycin (Shanghai Yuanpei Biotechnology Co., Ltd., product number: S250JO, molecular weight: 544.43).
(4) BSD (Invivogen, product number: ant-b1-1, molecular weight: 458.9).
(5) Hepalatide (number: L47), molecular weight 5399.1Da, sequence is shown in SEQ ID NO: 67. L47 was synthesized by China Peptide Biochemical Co., Ltd. (Product No. 820734), with HPLC purity of 99.9% (Figure 4, A). The mass

spectrometry identification of the synthesized peptide was consistent with the molecular weight of the target peptide (Figure 4, B).
(6) Phosphate buffer PBS 1X: GBICO 8120015.
(7) HBV DNA fluorescence quantitative detection kit: Zhengzhou Antu Bioengineering Co., Ltd.
(8) HBsAg chemiluminescence detection kit: Zhengzhou Antu Bioengineering Co., Ltd. (detection limit 0.05 IU/ml, linear detection range 0.05 -250 IU/ml).
(9) HBeAg chemiluminescence detection kit: Zhengzhou Antu Bioengineering Co., Ltd. (detection limit 0.1 PEIU/ml, linear detection range 0.1 -200 PEIU /ml, and 1 PEIU/ml≈1.05IU/ml).
(10) Fluorescence quantitative PCR instrument: MyIQ2, Bio-Rad Company.
(11) ELICA detector: Zhengzhou Antu Bioengineering Co., Ltd., model: LUMO.

The method of this example includes the following steps:
Culture HepG2, HepG2-hNTCP, HepG2-hOATP1B3, HepG2-hOATP1B1, HepG2-hNTCP/ hOATP1B3, HepG2-hNTCP/ hOATP1B1 cells. Cells in the logarithmic growth phase are plated into a 6-well plate at a cell density of 5×104/ml and cultured overnight. The cell number should be approximately 70% with a cell coverage rate of about 70% on the second day. Take out the purified HBV solution from the refrigerator, with a titer of about 108 IU/mL, and place it in an ice box at 4°C to thaw overnight. Remove the medium from the cultured cells and replace fresh medium containing HBV and/or heparin (day0) according to the table below. The medium containing HBV was 200 ul of the above HBV solution mixed with 1.8 ml of medium, which at the time of infection did not contain polyethylene glycol (PEG) or dimethyl sulfoxide (DMSO). Collect the supernatant after continuing to culture for 24 hours (day 1), rinse with PBS 3 times, replace with fresh medium without HBV and hepalatide, replace with fresh medium after 48 hours of culture, and collect and replace the culture supernatant every 3 days thereafter. Fresh medium (day6, day9). The remaining inoculation medium and collected supernatant on Day 0 were frozen at -80°C.

Experiment 1

| Serial number | Cell | HBV solution (Final concentration 10% V/V) | Hepalatide (Final concentration 10 ug/L) |
|---|---|---|---|
| 1 | HepG2 | - | - |
| 2 | HepG2 | + | - |
| 3 | HepG2-hNTCP | + | - |
| 4 | HepG2-hOATP1B3 | + | - |
| 5 | HepG2-hNTCP/ hOATP1 B3 | + | - |
| 6 | HepG2-hOATP1B3 | + | + |
| 7 | HepG2-hNTCP/ hOATP1 B3 | + | + |

Experiment 2

| Serial number | Cell | HBV solution (Final concentration 10% V/V) | Hepalatide (Final concentration 1 ug/L) |
|---|---|---|---|
| 8 | HepG2 | - | - |
| 9 | HepG2 | + | - |
| 10 | HepG2-hNTCP | + | - |
| 11 | HepG2-hOATP1B1 | + | - |
| 12 | HepG2-hNTCP/ hOATP1B1 | + | - |
| 13 | HepG2-hOATP1B1 | + | + |
| 14 | HepG2-hNTCP/ hOATP1B1 | + | + |

Use the HBV DNA fluorescence quantitative detection kit to detect the HBV DNA concentration in the inoculation medium by fluorescence quantitative PCR; the HBsAg chemiluminescence detection kit was used to detect the HBsAg concentration in the inoculation medium and each collected supernatant using the chemiluminescence immunoassay (CLIA) method; the HBeAg chemiluminescence detection kit was used to detect the HBeAg concentration in the inoculation medium and each collected supernatant using the chemiluminescence immunoassay (CLIA) method. The test results of the inoculation medium are as follows:

| Serial number | HBV DNA IU/ml | HBsAg(IU/ml) | HBeAg(PEIU /ml) |
|---|---|---|---|
| Experiment 1 | $5.8\times10^6$ | 638 | 0.028 |
| Experiment 2 | $5.1\times10^6$ | 709 | 0.031 |

The test results of each collected culture supernatant are shown in the table below:

| Serial number | Groups | HBsAg(IU/ml) | | | HBeAg(PEIU /ml) | | |
|---|---|---|---|---|---|---|---|
| | | Day1 | Day6 | Day9 | Day1 | Day6 | Day9 |
| 1 | HepG2 (blank) | 0.055 | 0.042 | 0.055 | 0.025 | 0.026 | 0.021 |
| 2 | HepG2 (HBV) | 196.961 | 0.028 | 0.054 | 0.027 | 0.019 | 0.041 |
| 3 | HepG2-hNTCP(HBV) | 182.045 | 0.037 | 0.045 | 0.032 | 0.027 | 0.023 |
| 4 | HepG2-hOATP1B3(HBV) | 201.253 | 0.204 | 0.571 | 0.031 | 0.142 | 0.333 |
| 5 | HepG2-hNTCP/ hOATP1B3(HBV) | 194.616 | 0.279 | 0.865 | 0.026 | 0.373 | 0.755 |
| 6 | HepG2-hOATP1B3(HBV+L47) | 197.828 | 0.035 | 0.036 | 0.028 | 0.026 | 0.027 |
| 7 | HepG2-hNTCP/ hOATP1B3(HBV+L47) | 194.635 | 0.029 | 0.039 | 0.014 | 0.030 | 0.041 |
| 8 | HepG2 (blank) | 0.044 | 0.029 | 0.038 | 0.038 | 0.035 | 0.019 |
| 9 | HepG2 (HBV) | 217.893 | 0.039 | 0.043 | 0.024 | 0.027 | 0.048 |
| 10 | HepG2-hNTCP(HBV) | 208.638 | 0.029 | 0.049 | 0.028 | 0.031 | 0.042 |
| 11 | HepG2-hOATP1 B1 (HBV) | 212.386 | 0.269 | 0.492 | 0.026 | 0.159 | 0.327 |
| 12 | HepG2-hNTCP/ hOATP1B1(HBV) | 209.346 | 0.305 | 0.672 | 0.037 | 0.415 | 0.809 |
| 13 | HepG2-hOATP1B1(HBV+L47) | 209.752 | 0.053 | 0.048 | 0.034 | 0.048 | 0.029 |
| 14 | HepG2-hNTCP/ hOATP1B1(HBV+L47) | 217.964 | 0.075 | 0.041 | 0.029 | 0.043 | 0.042 |

The results of the test one showed that the injection contained high concentrations of HBV DNA and HBsAg, indicating that the injection contained HBV virus at a lower titer than the same; the HBeAg detection value was lower than the detection limit and the injection did not contain HBeAg. In the culture supernatants of day1, day6, and day9 of HepG2 blank control group (No. 1), the detection values of HBsAg and HBeAg were lower than the detection limit, indicating that the detection method is reliable. Similar levels of HBsAg were present in the supernatants collected 24 hours after infection (day 1) in each other group (numbered 2-7), indicating that the degree of HBV inoculation in the inoculation medium of each infection group was similar. After HepG2 was inoculated with HBV virus (No. 2), the detection values of HBsAg and HBeAg in the supernatant on day 6 and day 9 were equivalent to those in the blank control group (No. 1), indicating that HepG2 was not infected by HBV, which is consistent with the report of reference 1[Yan H, Zhong G, Xu G, He W, Jing Z, Gao Z, Huang Y, Qi Y, Peng B, Wang H, Fu L, Song M, Chen P, Gao W, Ren B, Sun Y, Cai T, Feng X, Sui J, Li W. Sodium taurocholate cotransporting polypeptide is a functional receptor for human hepatitis B and D virus. Elife. 2012 Nov 13;3. doi: 10.7554/eLife.00049.] and reference 2 [Ni Y, Lempp FA, Mehrle S, Nkongolo S, Kaufman C, Fälth M, Stindt J, Königer C, Nassal M, Kubitz R, Sültmann H, Urban S. Hepatitis B and D viruses exploit sodium taurocholate co-transporting polypeptide for species-specific entry into hepatocytes. Gastroenterology. 2014 Apr;146(4):1070-83.].

HepG2 cells stably transfected with hNTCP (HepG2-hNTCP) were inoculated with HBV virus (No. 3) without PEG or DMSO in the culture medium. The detection values of HBsAg and HBeAg in the supernatant on day 6 and day 9 were the same as those in the HepG2 infected group (No. 2). In reference 1 and reference 2, HepG2 cells stably transfected with hNTCP could be infected with HBV or HDV in the presence of PEG.

HepG2 cells stably transfected with hOATP1B3 (HepG2-hOATP1B3) without PEG or DMSO in the medium, as

shown in Figure 18, showed significantly higher HBsAg and HBeAg detection values in the supernatants of day6 and day9 after inoculation with HBV virus (No. 4) than those of the HepG2-infected group (No. 2) and the HepG2-hNTCP-infected group (No. 3), showing that HepG2-hOATP1B3 can be infected by HBV. In particular, the culture medium does not contain HBeAg, and HBeAg in the supernatants of day6 and day9 after HepG2-hOATP1B3 inoculation with HBV can only be re-expressed and secreted into the supernatants by HBV-infected cells. Thus, the appearance and elevation of HBeAg after HepG2-hOATP1B3 inoculation with HBV clearly confirms that HepG2-hOATP1B3 can be infected by HBV and that hOATP1B3 mediates HBV infection.

L47 has been shown to bind hOATP1B3 in Example 1 and was shown to prevent HBV infection in reference 3 [Li J, Shi TD, Han JF, Zeng XG, Fan CL, Han C, Liu HL, Wu YZ. A systematic study of Tupaia as a model for human acute hepatitis B infection. J Vet Med Sci. 2021 Jul 10;83(6):1004-1011. doi: 10.1292/jvms.21-0026. Epub 2021 Apr 29. ]. In the process of HepG2-hOATP1B3 inoculation with HBV, the addition of L47 (No. 6) to the inoculation medium resulted in the detection values of HBsAg and HBeAg in the supernatants on day6 and day9 after inoculation were comparable to those of the HepG2-infected group (No. 2) and significantly lower compared with those of the HepG2-hOATP1B3-infected group (No. 4) on day6 and day9 after inoculation (shown in Figure 18), indicating that the markedly elevated HBsAg and HBeAg values in the supernatants of the HepG2-hOATP1B3-inoculated HBV group on day6 and day9 were due to HBV infection, that HBV can be infected through the hOATP1B3-mediated infection, and that L47 can prevent HBV infection mediated by hOATP1B3.

HepG2 cells stably transfected with hOATP1B3 and hNTCP (HepG2-hNTCP/hOATP1B3) without PEG or DMSO in the medium, as shown in Figure 18, showed significantly higher HBsAg and HBeAg detection values in the supernatants of day6 and day9 after inoculation with HBV virus (No. 5) than those of the HepG2-infected group (No. 2) and the HepG2- hNTCP-infected group (No. 3), showing that HepG2-hNTCP/hOATP1B3 can be infected by HBV. In particular, the culture medium does not contain HBeAg, and HBeAg in the supernatants of day6 and day9 after HepG2-hOATP1B3 inoculation with HBV can only be re-expressed and secreted into the supernatants by HBV-infected cells. Thus, the appearance and elevation of HBeAg after HepG2-hNTCP/hOATP1B3 inoculation with HBV clearly confirms that HepG2-hNTCP/hOATP1B3 can be infected by HBV. Compared with the HBsAg and HBeAg detection values in the supernatants of day6 and day9 after HBV inoculation with HepG2-hOATP1B3 (No. 4), the HBsAg and HBeAg detection values in the supernatants of HBV on day6 and day9 after HepG2-hNTCP/hOATP1B3 inoculation with HBV were significantly higher, indicating that the combination of hNTCP and hOATP1B3 could mediate HBV infection more effectively.

In the process of HepG2-hNTCP/hOATP1B3 inoculation with HBV, the addition of L47 (No. 7) to the inoculation medium resulted in the detection values of HBsAg and HBeAg in the supernatants on day6 and day9 after inoculation were comparable to those of the HepG2-infected group (No. 2) and significantly lower compared with those of the HepG2-hNTCP/hOATP1B3-infected group (No. 5) on day6 and day9 after inoculation (shown in Figure 18), indicating that the markedly elevated HBsAg and HBeAg values in the supernatants of the HepG2-hOATP1B3-inoculated HBV group on day6 and day9 were due to HBV infection, that HBV can be infected through the hOATP1B3-mediated infection, and that L47 can prevent HBV infection mediated by hOATP1B3 combined with hNTCP.

The results of the test two showed that the injection contained high concentrations of HBV DNA and HBsAg, indicating that the injection contained HBV virus at a lower titer than the same; the HBeAg detection value was lower than the detection limit and the injection did not contain HBeAg. In the culture supernatants of day1, day6, and day9 of HepG2 blank control group (No. 8), the detection values of HBsAg and HBeAg were lower than the detection limit, indicating that the detection method is reliable. Similar levels of HBsAg were present in the supernatants collected 24 hours after infection (day 1) in each other group (numbered 9-14), indicating that the degree of HBV inoculation in the inoculation medium of each infection group was similar. The HBsAg and HBeAg detection values in the supernatants of HepG2 on day6 and day9 after inoculation with HBV virus (No. 9) were comparable to those of the blank control group (No. 8), indicating that HepG2 was not infected by HBV, which is in agreement with the reports of Reference 1 and Reference 2.

HepG2 cells stably transfected with hNTCP (HepG2-hNTCP) were inoculated with HBV virus (No. 10) without PEG or DMSO in the culture medium. The detection values of HBsAg and HBeAg in the supernatant on day 6 and day 9 were the same as those in the HepG2 infected group (No. 9). In reference 1 and reference 2, HepG2 cells stably transfected with hNTCP could be infected with HBV or HDV in the presence of PEG.

HepG2 cells stably transfected with hOATP1B1 (HepG2-hOATP1B1) without PEG or DMSO in the medium, as shown in Figure 19, showed significantly higher HBsAg and HBeAg detection values in the supernatants of day6 and day9 after inoculation with HBV virus (No. 11) than those of the HepG2-infected group (No. 9) and the HepG2-hNTCP-infected group (No. 10), showing that HepG2-hOATP1B1 can be infected by HBV. In particular, the culture medium does not contain HBeAg, and HBeAg in the supernatants of day6 and day9 after HepG2-hOATP1B1 inoculation with HBV can only be re-expressed and secreted into the supernatants by HBV-infected cells. Thus, the appearance and elevation of HBeAg after HepG2-hOATP1B1 inoculation with HBV clearly confirms that HepG2-hOATP1B1 can be infected by HBV and that hOATP1B1 mediates HBV infection.

L47 has been shown to bind hOATP1B1 in Example 1 and was shown to prevent HBV infection in reference 3. In the process of HepG2-hOATP1B1 inoculation with HBV, the addition of L47 (No. 13) to the inoculation medium resulted in

the detection values of HBsAg and HBeAg in the supernatants on day6 and day9 after inoculation were comparable to those of the HepG2-infected group (No. 9) and significantly lower compared with those of the HepG2-hOATP1B1-infected group (No. 11) on day6 and day9 after inoculation (shown in Figure 19), indicating that the markedly elevated HBsAg and HBeAg values in the supernatants of the HepG2-hOATP1B1-inoculated HBV group on day6 and day9 were due to HBV infection, that HBV can be infected through the hOATP1B1-mediated infection, and that L47 prevented the hOATP1B1-mediated HBV infection.

HepG2 cells stably transfected with hOATP1B1 and hNTCP (HepG2-hNTCP/hOATP1B1) without PEG or DMSO in the medium, as shown in Figure 19, showed significantly higher HBsAg and HBeAg detection values in the supernatants of day6 and day9 after inoculation with HBV virus (No. 12) than those of the HepG2-infected group (No. 9) and the HepG2- hNTCP-infected group (No. 10), showing that HepG2-hNTCP/hOATP1B1 can be infected by HBV. In particular, the culture medium does not contain HBeAg, and HBeAg in the supernatants of day6 and day9 after HepG2-hNTCP/hOATP1B1 inoculation with HBV can only be re-expressed and secreted into the supernatants by HBV-infected cells. Thus, the appearance and elevation of HBeAg after HepG2-hNTCP/hOATP1B1 inoculation with HBV clearly confirms that HepG2-hNTCP/hOATP1B1 can be infected by HBV. Compared with the HBsAg and HBeAg detection values in the supernatants of day6 and day9 after HBV inoculation with HepG2-hOATP1B1 (No. 11), the HBsAg and HBeAg detection values in the supernatants of HBV on day6 and day9 after HepG2-hNTCP/hOATP1B1 inoculation with HBV were significantly higher, indicating that the combination of hNTCP and hOATP1B1 could mediate HBV infection more effectively.

In the process of HepG2-hNTCP/hOATP1B1 inoculation with HBV, the addition of L47 (No. 14) to the inoculation medium resulted in the detection values of HBsAg and HBeAg in the supernatants on day6 and day9 after inoculation were comparable to those of the HepG2-infected group (No. 9) and significantly lower compared with those of the HepG2-hNTCP/hOATP1B1-infected group (No. 12) on day6 and day9 after inoculation (shown in Figure 19), indicating that the markedly elevated HBsAg and HBeAg values in the supernatants of the HepG2-hOATP1B1-inoculated HBV group on day6 and day9 were due to HBV infection, that HBV can be infected through the hOATP1B1-mediated infection, and that L47 can prevent HBV infection mediated by hOATP1B1 combined with hNTCP.

## Claims

1. Use of a substance in the manufacture of a medicament for preventing and/or treating hepatitis B virus (HBV) and/or hepatitis D virus (HDV) infections or diseases related to said infections in an animal, wherein the substance prevents or reduces the interaction between HBV and/or HDV and an organic anion transporting polypeptide (OATP) in the animal, and/or prevents or reduces the expression and/or function of OATP in the animal.

2. The use according to claim 1, wherein, the substance prevents or reduces the interaction between HBV and/or HDV and OATP1B3 and/or OATP1B1 in animals, and/or prevents or reduces the expression and/or function of OATP1B3 and/or OATP1B1 in animals.

3. The use according to claim 2, wherein, the animal is a human, a chimpanzee, a bonobo, a tree shrew, a marmot, an arctic ground squirrel, a ground squirrel, a bat, a woolly monkey, a capuchin monkey or a squirrel monkey.

4. The use according to claim 2 and claim 3, wherein, the substance prevents or reduces the interaction between HBV and/or HDV and OATP1B3 and/or OATP1B1 in human, and/or prevents or reduces the expression and/or function of OATP1B3 and/or OATP1B1 in human.

5. The use according to any one of claims 1 to 4, wherein, the substance is a polypeptide, antibody, protein, small molecule, and polynucleotide that at least retains the activity acting on OATP, or the activity acting on the interaction region between HBV or HDV and OATP, or the activity preventing or reducing the expression and/or function of OATP.

6. The use of any one of claims 1 to 5, wherein, the substance is used alone, or used in combination with an agent that can prevent or reduce the interaction between HBV and/or HDV and NTCP and/or an agent or method that can prevent or reduce the expression and/or function of NTCP, or in combination with other medicament that prevent or treat HBV and/or HDV infection and related diseases.

7. The use according to any one of claims 1 to 6, wherein, the substance is a polypeptide derived from the Hepadnaviridae surface antigen, and the polypeptide at least retains the biological activity of binding to OATP.

8. The use according to claim 7, wherein, the polypeptide is derived from the human HBV surface antigen Pre-S1 region

and has the biological activity of binding to human OATP1B3 and/or human OATP1B1, and its N-terminus is modified with a hydrophobic group, the C-terminus is modified or unmodified.

**9.** The use according to claim 8, wherein, the polypeptide is derived from the surface antigen Pre-S1 region of human HBV of genotype A, B, C, D, E, F, G, H or I.

**10.** The use according to claim 8, wherein, the N-terminus of the polypeptide is modified with fatty acid.

**11.** The use according to claim 10, wherein, the N-terminus of the polypeptide is modified with myristic acid.

**12.** The use according to any one of claims 7-11, wherein, the polypeptide is selected from: polypeptides or variants thereof derived from amino acids at positions 2-119, 2-69, 2-59, 13-119, 13-88, 13-72, 13-67, 13-59, 13-52, 13-47, 13-42, 13-37, or 13-32 of the surface antigen Pre-S1 region of the human HBV of genotype C.

**13.** The use according to any one of claims 7-11, wherein, the polypeptide is selected from: polypeptides or variants thereof derived from amino acids at positions 13-59, 13-59, 2-48, 12-58, 13-59, 12-58, 13-59, 13-59 of the surface antigen Pre-S1 region of the human HBV of genotype A, B, D, E, F, G, H, I.

**14.** The use according to any one of claims 12 and 13, wherein, the N-terminus of the polypeptide is modified with a fatty acid, and the C-terminus of the polypeptide is modified or not modified.

**15.** The use according to claim 14, wherein, the N-terminus of the polypeptide has a fatty acid modification and the C-terminus has an amidation modification, and the sequence is as shown in SEQ ID NO: 67-78.

**16.** The use of any one of claims 12-15, wherein, the polypeptide is derived from the surface antigen Pre-S1 region of human HBV of genotype C, and its amino acid sequence has one or more substitution mutations selected from the group consisting of: N15D, F25L, G35K, N39E, N46K, F45L, N48H, N48Y, N48K, D50A, H51Q, H51N, E54K, E54D, A55S, N56K, N56D and Q57K.

**17.** A method for establishing a cell or animal model susceptible to HBV and/or HDV, comprising: transfecting the exogenous SLCO gene into the cell or animal, and/or expressing the exogenous OATP protein in the cell or animal, and the cells or animals that are originally not susceptible to HBV and/or HDV or are not easily infected, acquire susceptibility to HBV and/or HDV after being transfected with the SLCO gene and/or expressing exogenous OATP protein.

**18.** The method according to claim 17, wherein, the cells or animals are transfected with exogenous SLCO1B3 and/or SLCO1B1 genes or variants thereof, and/or are made to express exogenous OATP1B3 and/or OATP1B1 proteins or variants thereof.

**19.** The method according to claim 18, wherein the SLCO1B3 and/or SLCO1B1 exogenous genes are derived from humans, chimpanzees or tree shrews, and the OATP1B3 and/or OATP1B1 exogenous proteins are derived from humans, chimpanzees or tree shrews.

**20.** The method according to any one of claims 17 to 19, wherein, the SLCO gene is transfected alone, or the SLCO gene and the exogenous SLC10A1 gene is transfected at the same time; or the exogenous OATP protein is expressed alone, or the exogenous OATP and the exogenous NTCP protein is expressed at the same time.

**21.** The method according to any one of claims 17 to 20, wherein, the cells are not human embryonic stem cells, but are preferably hepatocytes or liver-derived cell lines transfected with exogenous SLCO genes and/or expressing exogenous OATP proteins, the animal is a non-human mammal, preferably a Rat, Mouse, dog or monkey transfected with an exogenous SLCO gene or expressed an exogenous OATP protein.

**22.** The method according to claim 21, wherein the cells are hepatocytes or liver-derived cell lines which transfected with SLCO1B3 and/or SLCO1B1 exogenous genes or variants thereof derived from humans, chimpanzees or tree shrews, or expressed OATP1B3 and/or OATP1B1 exogenous proteins or variants thereof derived from humans, chimpanzees or tree shrews; the animals are rats, mice, dogs or monkeys which transfected with SLCO1B3 and/or SLCO1B1 exogenous genes or variants thereof derived from humans, chimpanzees or tree shrews, or expressed OATP1B3 and/or OATP1B1 exogenous proteins or variants thereof derived from humans, chimpanzees or tree shrews.

**23.** The method according to claim 22, wherein the cells are hepatocytes or liver-derived cell lines which transfected with SLCO1B3 and/or SLCO1B1 exogenous genes or variants thereof derived from humans, chimpanzees or tree shrews, or expressed OATP1B3 and/or OATP1B1 exogenous proteins or variants thereof derived from humans, chimpanzees or tree shrews, and simultaneously transfected with SLC10A1 exogenous genes or variants thereof derived from humans, chimpanzees or tree shrews, or expressed NTCP exogenous proteins or variants thereof derived from humans, chimpanzees or tree shrews; the animals are ratss, mice, dogs or monkeys which transfected with SLCO1B3 and/or SLCO1B1 exogenous genes or variants thereof derived from humans, chimpanzees or tree shrews, or expressed OATP1B3 and/or OATP1B1 exogenous proteins or variants thereof derived from humans, chimpanzees or tree shrews, and simultaneously transfected with SLC10A1 exogenous genes or variants thereof derived from humans, chimpanzees or tree shrews, or expressed NTCP exogenous proteins or variants thereof derived from humans, chimpanzees or tree shrews.

**24.** Cells transfected with exogenous SLCO genes and/or expressed exogenous OATP proteins, wherein the cells are not susceptible to HBV and/or HDV or are not susceptible to infection before transfection or expression, and after transfection with the SLCO genes and/or expression of exogenous OATP protein, the cells are susceptible to HBV and/or HDV; preferably, the transfection and expression are as described in any one of claims 18-23.

**25.** A drug or group delivery vehicle, wherein, the vehicle at least retains the biological activity of binding OATP and delivering the carried drug or group into cells.

**26.** The delivery vehicle according to claim 25, wherein the OATP is OATP1B3 and/or OATP1B1.

**27.** The delivery vehicle according to claim 26, wherein the OATP is human OATP1B3 and/or human OATP1B1.

**28.** The delivery vehicle according to claim 25, wherein the vehicle simultaneously binds NTCP.

**29.** The delivery vehicle according to claim 28, wherein the vehicle simultaneously binds human NTCP.

**30.** The delivery vehicle according to claim 25, wherein the vehicle is a polypeptide, a small molecule compound, a protein, an antibody or a nucleic acid, and the vehicle combines with **OATP** and delivers the carried drug or group into cells and/or organs.

**31.** The delivery vehicle according to claim 25, wherein the drug or group carried by the vehicle is a small molecule drug, nucleic acid, protein, antibody and/or polypeptide, and the drug or group connected to the backbone of the vehicles through covalent bonds and/or non-covalent bonds; wherein the backbone of the vehicle and the drug or group are connected directly or through a linker.

**32.** The delivery vehicle according to claim 25, wherein the drug or group carried by the vehicle enters hepatocytes or liver.

**33.** The delivery vehicle according to claim 32, wherein the drug or group carried by the vehicle enters human hepatocytes or human liver.

**34.** The delivery vehicle according to any one of claims 31-33, wherein the vehicle is a polypeptide derived from the surface antigen of hepadnavirus, which at least retains the biological activity of binding to OATP and delivers the carried drug or group into cells.

**35.** The delivery vehicle of claim 34, wherein the delivery vehicle has a structure represented by the following formula (I):

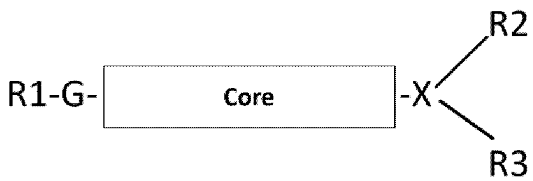

(I)

wherein, G is glycine, the core sequence is the amino acid sequence derived from the surface antigen of hepadnavirus, X is an amino acid residue with two amino groups or two carboxyl groups or other groups available for linkage reaction, and R1 is a N-terminal modification of the vehicle, R2 does not exist, or is a flanking sequence or other group, R3 is a carried pharmacophore group or a linking group that can interact with a drug or group.

**36.** The delivery vehicle according to claim 35, wherein the core sequence of the delivery vehicle is derived from the surface antigen Pre-S region of the hepadnavirus.

**37.** The delivery vehicle according to claim 36, wherein the core sequence of the delivery vehicle is derived from the surface antigen Pre-S region of the human HBV.

**38.** The delivery vehicle according to claim 37, wherein, the core sequence of the delivery vehicle is derived from amino acids 3-119, 4-119, 3-69, 4-69, 3-59, 4-59, 3-48, 4-48, 14-119, 14-88, 14-72, 14-67, 14-59, 14-56, 14-55, 14-53, 14-48, 14-47, 14-45, 14-42, 14-37 or 14-32 of the surface antigen Pre-S1 region of human HBV genotype C.

**39.** The delivery vehicle according to claim 37, wherein the core sequence of the delivery vehicle is derived from the amino acids 14-48, 14-48, 3-37, 13-47, 14-48, 13-47, 14-48 or 14-48 of the surface antigen Pre-S1 region of the human HBV genotype A, B, D, E, F, G, H or I.

**40.** The delivery vehicle according to claim 35, wherein R1 of the delivery vehicle is a fatty acid group.

**41.** The delivery vehicle according to claim 40, wherein R1 of the delivery vehicle is a myristic acid group.

**42.** The delivery vehicle of claim 35, wherein the R2 flanking sequences of the delivery vehicle are derived from amino acids at positions 50-119, 50-88, 50-72, 50-69, 50-67, 58-59, 50-59 or 50-52 of the surface antigen Pre-S1 region of human HBV genotype C, or variants thereof, wherein, the N-terminus of R2 can be amidated.

**43.** The delivery vehicle of claim 35, wherein the R2 flanking sequences of the delivery vehicle are derived from amino acids at positions 50-59, 50-59, 39-48, 49-58, 50-59, 49-58, 50-59 or 50-59 of the surface antigen Pre-S1 region of human HBV genotype A, B, D, E, F, G, H or I, or variants thereof, wherein, the N-terminus of R2 can be amidated.

**44.** The delivery vehicle according to claim 35, wherein R3 of the delivery vehicle is a pharmacophore group of a small molecule compound, nucleic acid, polypeptide, protein, antibody or virus, etc., or is a linking group that can interact with drugs or groups such as small molecule compound, nucleic acid, polypeptide, proteins, antibodies or viruses.

**45.** The delivery vehicle according to claim 44, wherein R3 of the delivery vehicle is a small molecule pharmacophore group with cell regulatory activity, or is a biologically active group derived from: a active protein, coding nucleic acids of a active protein or biologically active nucleic acid, wherein, the biologically active nucleic acid includes interference or antisense active protein mRNA, gene encoding vehicles, viral vehicles, etc; or, the R3 is a linking group which can interact with the small molecule pharmacophore group, active protein or biologically active nucleic acid.

**46.** The delivery vehicle according to any one of claims 35 to 45, wherein, the core sequence of the delivery vehicle is derived from amino acids at positions 14-48 of the surface antigen Pre-S1 region of the human HBV genotype C, or a variant thereof; R1 is myristic acid, palmitic acid, stearic acid, cholesterol, polyethylene glycol groups; the flanking sequence described by R2 is derived from amino acids 50-59 of the surface antigen Pre-S1 region of human HBV genotype C, with its C-terminus amidated; preferably, the amino acid sequence of the delivery vehicle is as shown in any one of SEQ ID NO: 228-233.

**47.** The delivery vehicle according to any one of claims 35 to 45, wherein, the core sequence of the delivery vehicle is derived from amino acids at positions 14-56 of the surface antigen Pre-S1 region of the human HBV genotype C, or a variant thereof; R1 is myristic acid, palmitic acid, stearic acid, cholesterol, and polyethylene glycol groups; the flanking sequence described by R2 is derived from amino acids 58-59 of the surface antigen Pre-S1 region of human HBV genotype C, with its C-terminus amidated; preferably, the amino acid sequence of the delivery vehicle is as shown in SEQ ID NO: 234.

**48.** The delivery vehicle according to claim 46, wherein, the core sequence of the delivery vehicle is derived from amino acids at positions 14-48 of the surface antigen Pre-S1 region of the human HBV genotype C, or a variant thereof; R1 is myristic acid groups; the flanking sequence described by R2 is derived from amino acids 50-59 of the surface antigen Pre-S1 region of human HBV genotype C, with its C-terminus amidated; and R3 is a small molecule pharmacophore

group; preferably, the amino acid sequence of the delivery vehicle is shown in SEQ ID NO: 228.

**49.** Polypeptides used to optimize the pharmacokinetics of drugs transported through OATP, such as statins, sartans, tinibs, anti-tumor drugs such as platinum or paclitaxel drugs, or use of polypeptides in the manufacture of reagents for optimizing the pharmacokinetic of statins, sartans, tinibs, anti-cancer drugs tumor drugs such as platinum or paclitaxel drugs, wherein the polypeptide binds OATP and inhibits its transport function, is derived from the hepadnavirus surface antigen, and retains at least the biological activity of binding to OATP and inhibiting transport function of OATP.

**50.** The polypeptide or the use according to claim 49, wherein, the polypeptide is derived from surface antigen Pre-S1 region of the human HBV, and its N-terminus is modified with a hydrophobic group, its C-terminus is modified or unmodified, and has the activity of binding and inhibiting the transport function of human OATP1B3 and/or human OATP1B1.

**51.** The polypeptide or the use according to claim 49, wherein, the polypeptide is derived from the surface antigen Pre-S1 region of human HBV genotype A, B, C, D, E, F, G, H or I.

**52.** The polypeptide or the use according to claim 49, wherein, the N-terminus of the polypeptide is modified with fatty acid.

**53.** The polypeptide or the use according to claim 52, wherein, the N-terminus of the polypeptide is modified with myristic acid.

**54.** The polypeptide or the use according to any one of the claims 49-53, wherein, the polypeptide is selected from: the amino acids derived from positions 2-119, 2-69, 2-59, 13-119, 13-88, 13-72, 13-67, 13-59, 13-52, 13-47, 13-42, 13-37, or 13-32 of the surface antigen Pre-S1 region of the human HBV genotype C, or variants thereof.

**55.** The polypeptide or the use according to any one of the claims 49-53, the polypeptide derived from amino acids at positions 13-59, 13-59, 2-48, 12-58, 13-59, 12-58, 13-59 or 13-59 of the surface antigen Pre-S1 region of the human HBV genotype A, B, D, E, F, G, H, I, or variants thereof.

**56.** The polypeptide or the use according to any one of claims 54 and 55, wherein, the N-terminus of the polypeptide is modified with a fatty acid, and the C-terminus is modified or not modified.

**57.** The polypeptide or the use according to claim 56, wherein, the N-terminus of the polypeptide has a fatty acid modification and the C-terminus has an amidation modification, and the sequence is as shown in SEQ ID NO: 99-110.

**58.** The polypeptide or the use according to claims 54, 56 or 57, wherein the polypeptide is derived from the surface antigen Pre-S1 region of human HBV genotype C, and its amino acid sequence has a substitution mutation selected from the group consisting of: N15D, F25L, G35K, N39E, N46K, F45L, N48H, N48Y, N48K, D50A, H51Q, H51N, E54K, E54D, A55S, N56K, N56D, Q57K, or combination thereof.

**59.** The polypeptide or the use according to any one of claims 49-58, wherein the polypeptide binds to OATP1B3 and/or OATP1B1 and inhibits their transportation of statins, sartans, tinibs, and anti-tumor drugs such as platinum or paclitaxel drugs.

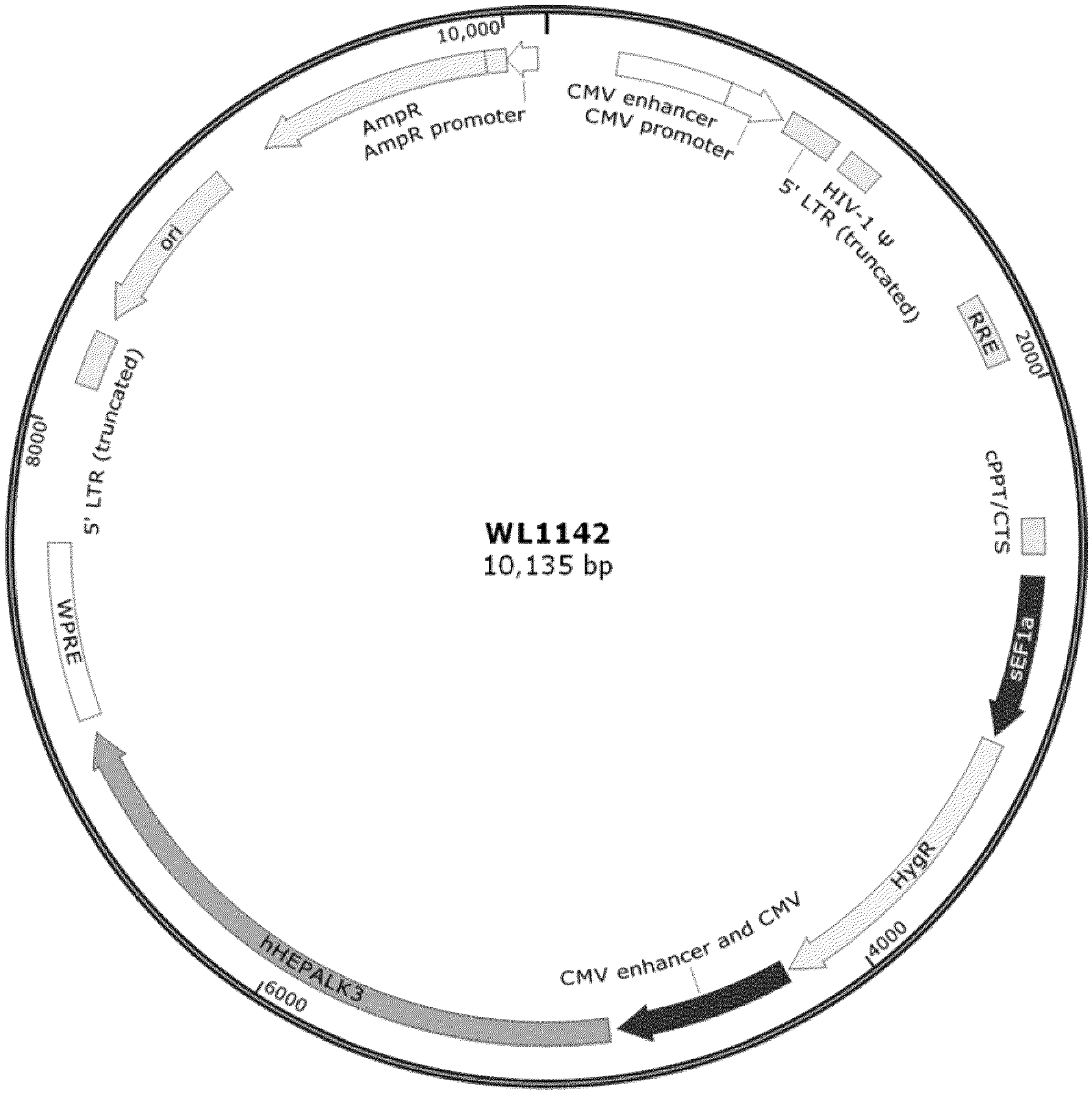
WL1142
10,135 bp
10,000
2000
4000
6000
8000
CMV enhancer
CMV promoter
HIV-1 ψ
5' LTR (truncated)
RRE
cPPT/CTS
sEF1a
HygR
CMV enhancer and CMV
hHEPALK3
WPRE
5' LTR (truncated)
ori
AmpR
AmpR promoter

Fig. 1

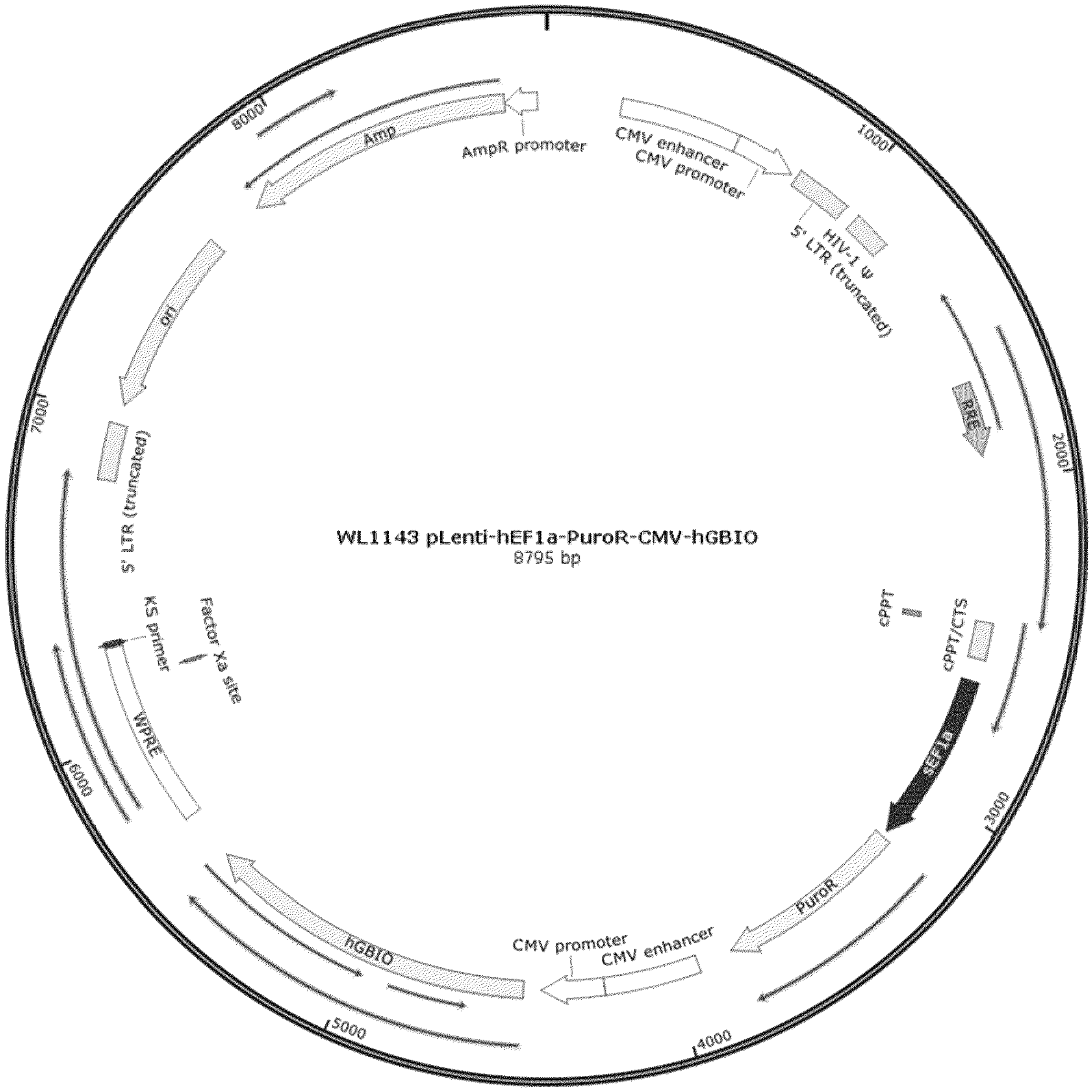
WL1143 pLenti-hEF1a-PuroR-CMV-hGBIO
8795 bp
1000
2000
3000
4000
5000
6000
7000
8000
CMV enhancer
CMV promoter
HIV-1 ψ
5' LTR (truncated)
RRE
cPPT
cPPT/CTS
sEF1a
PuroR
CMV enhancer
CMV promoter
hGBIO
WPRE
KS primer
Factor Xa site
5' LTR (truncated)
ori
Amp
AmpR promoter

Fig. 2

A

Myr-G-TNLSVPNPLGFFPDHQLDPAFGANSNNPDWDFNPN-K-DHWPEANQVG-NH2

|

FITC

B

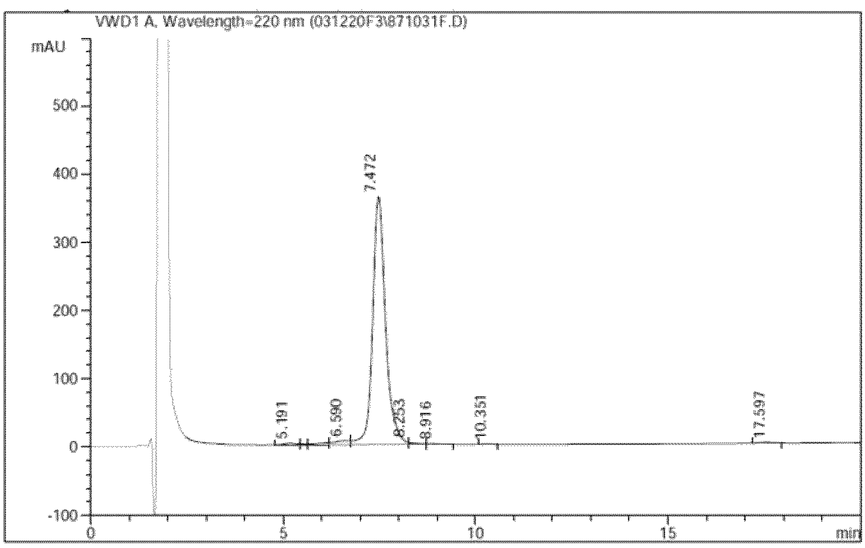


Signal 1:VWD1 A, Wavelength=220 nm

| Peak # | RT [min] | Type | Width [min] | Height | Area | Area % |
|---|---|---|---|---|---|---|
| 1 | 5.191 | BF | 0.279 | 2.440 | 49.316 | 0.558 |
| 2 | 5.428 | VV | 0.196 | 1.205 | 14.142 | 0.160 |
| 3 | 6.186 | VV | 0.374 | 2.995 | 67.161 | 0.759 |
| 4 | 6.590 | VV | 0.380 | 5.799 | 159.963 | 1.809 |
| 5 | 7.472 | VF | 0.353 | 363.441 | 8449.862 | 95.550 |
| 6 | 8.253 | VV | 0.253 | 2.971 | 45.168 | 0.511 |
| 7 | 8.916 | VBA | 0.280 | 1.228 | 27.049 | 0.306 |
| 8 | 10.351 | BBA | 0.199 | 0.635 | 7.520 | 0.085 |
| 9 | 17.597 | BBA | 0.339 | 0.869 | 23.259 | 0.263 |

C

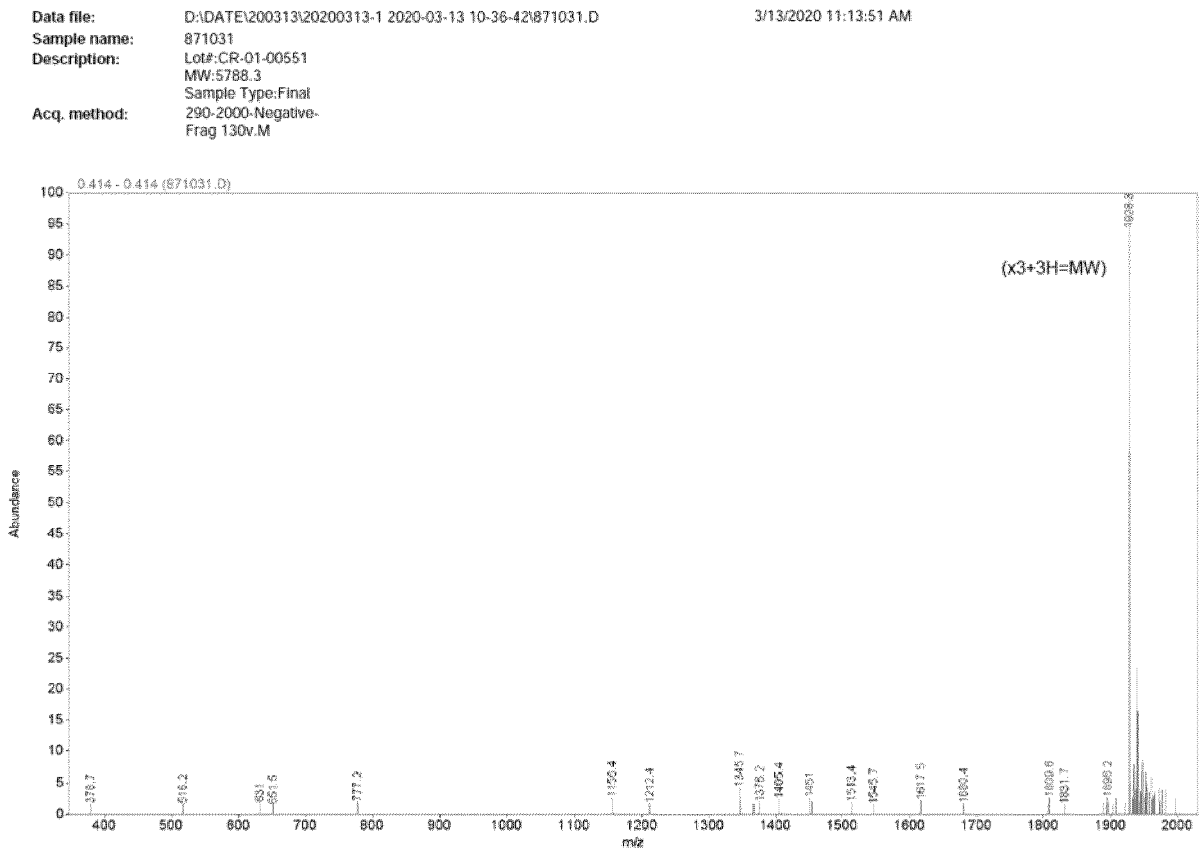
Data file: D:\DATE\200313\20200313-1 2020-03-13 10-36-42\871031.D
3/13/2020 11:13:51 AM
Sample name: 871031
Description: Lot#:CR-01-00551
MW:5788.3
Sample Type:Final
Acq. method: 290-2000-Negative-
Frag 130v.M
0.414 - 0.414 (871031.D)
(x3+3H=MW)
Abundance
m/z

Fig. 3

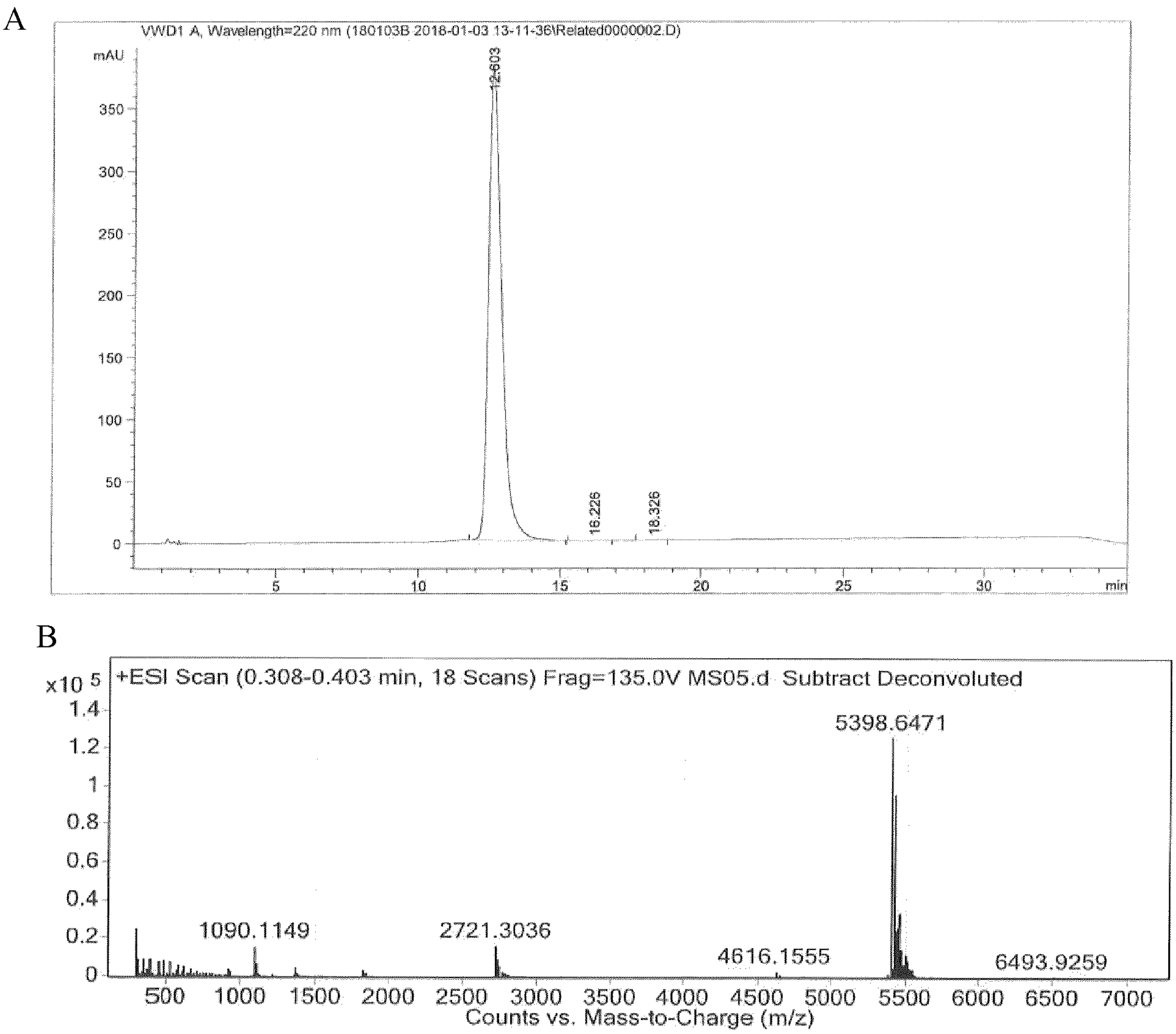
A
VWD1 A, Wavelength=220 nm (180103B 2018-01-03 13-11-36\Related0000002.D)
mAU
350
300
250
200
150
100
50
0
12.603
16.226
18.326
5
10
15
20
25
30
min
B
x10 5
+ESI Scan (0.308-0.403 min, 18 Scans) Frag=135.0V MS05.d Subtract Deconvoluted
1.4
1.2
1
0.8
0.6
0.4
0.2
0
5398.6471
1090.1149
2721.3036
4616.1555
6493.9259
500 1000 1500 2000 2500 3000 3500 4000 4500 5000 5500 6000 6500 7000
Counts vs. Mass-to-Charge (m/z)

Fig. 4

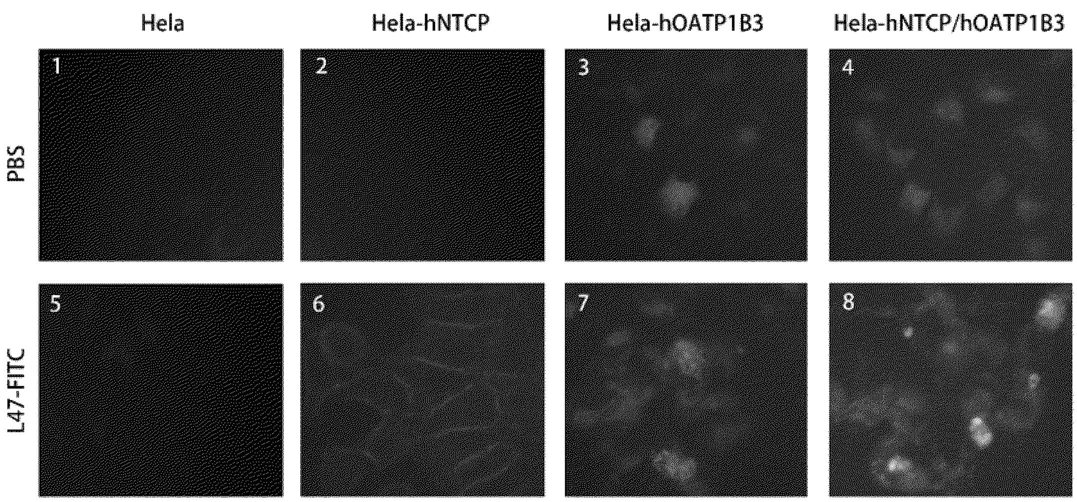
Hela
Hela-hNTCP
Hela-hOATP1B3
Hela-hNTCP/hOATP1B3
PBS
L47-FITC
1
2
3
4
5
6
7
8

Fig. 5

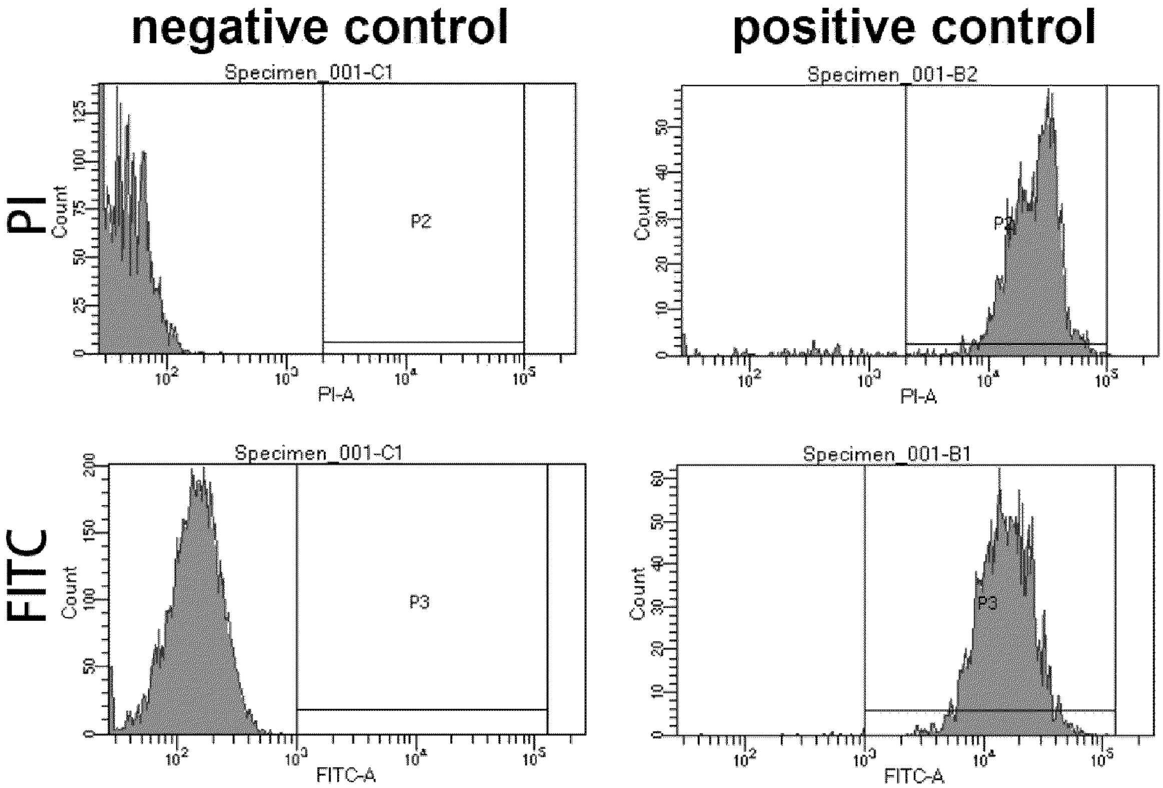
negative control
positive control
PI
FITC
Specimen_001-C1
Specimen_001-B2
Specimen_001-C1
Specimen_001-B1
Count
PI-A
FITC-A
P2
P3

Fig. 6

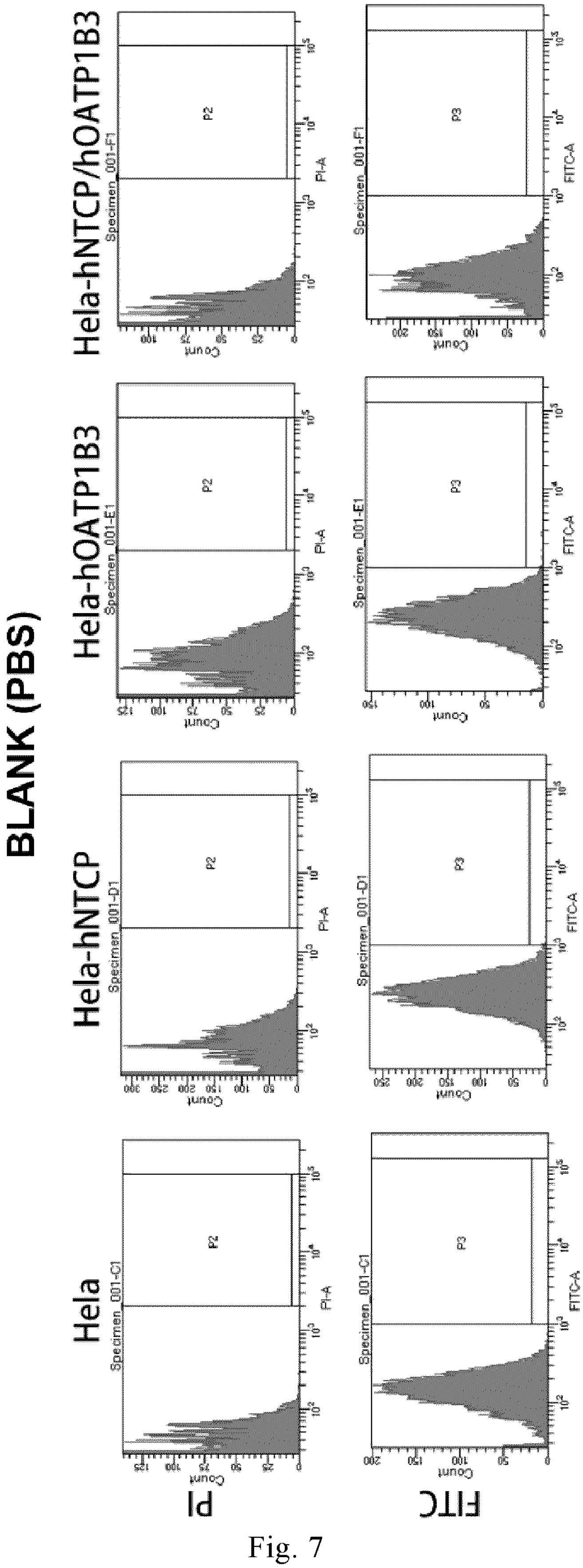
BLANK (PBS)
Hela
Hela-hNTCP
Hela-hOATP1B3
Hela-hNTCP/hOATP1B3
PI
FITC
Specimen_001-C1
Specimen_001-D1
Specimen_001-E1
Specimen_001-F1
P2
P3
PI-A
FITC-A
Count

Fig. 7

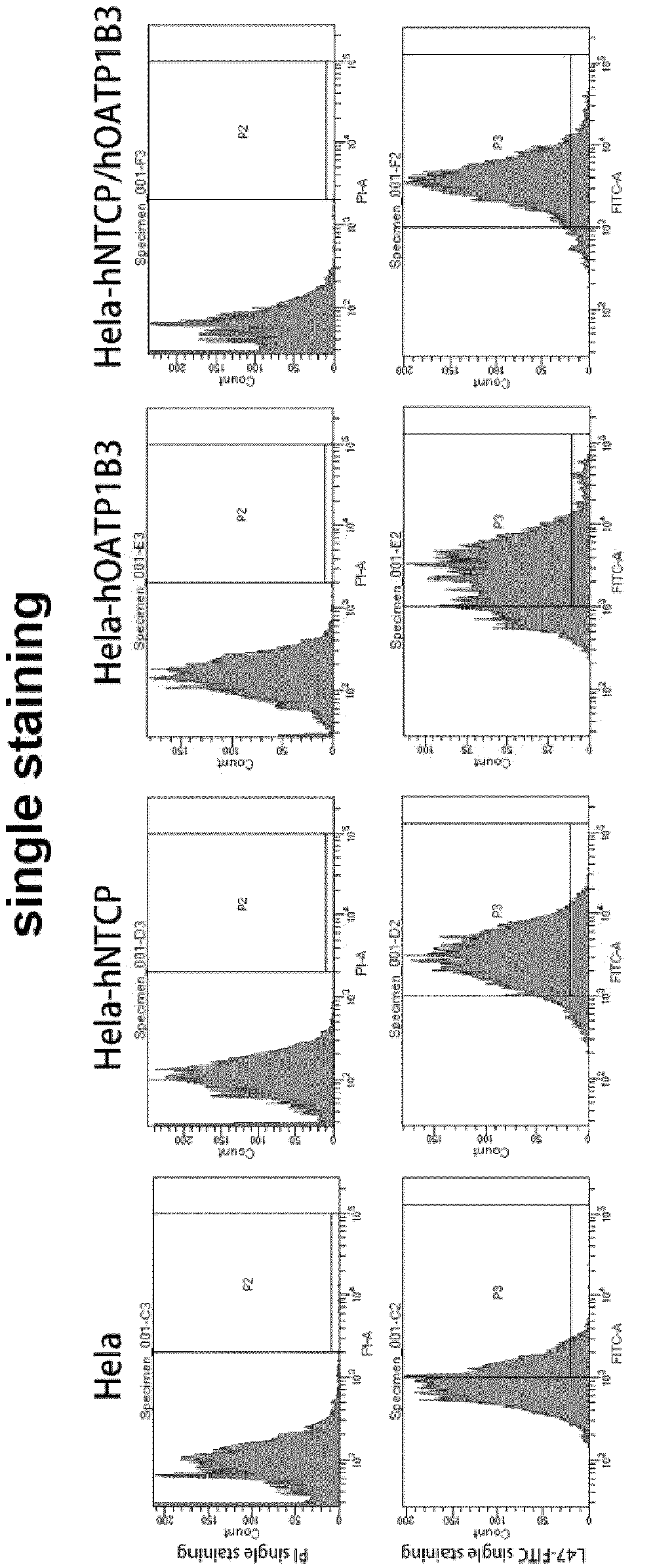
single staining
Hela
Hela-hNTCP
Hela-hOATP1B3
Hela-hNTCP/hOATP1B3
PI single staining
L47-FITC single staining

Fig. 8

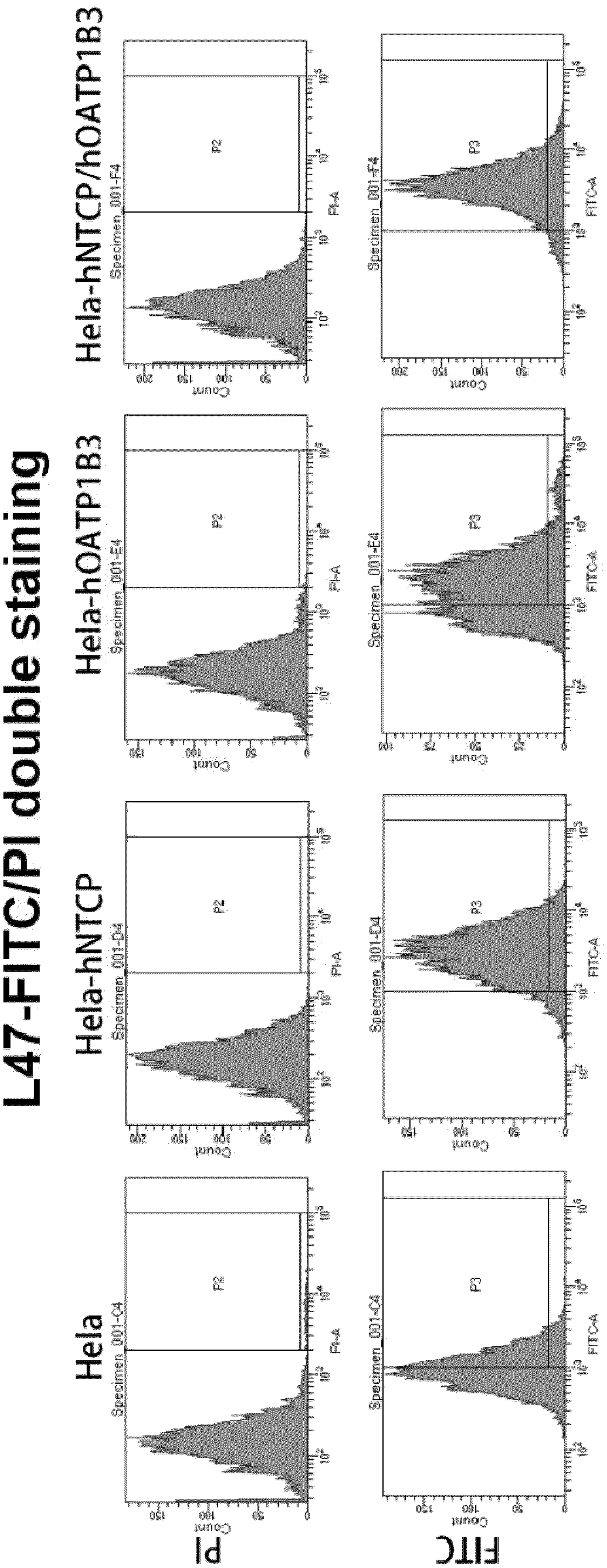
L47-FITC/PI double staining
Hela
Hela-hNTCP
Hela-hOATP1B3
Hela-hNTCP/hOATP1B3
PI
FITC
P2
P3
Count
PI-A
FITC-A

Fig. 9

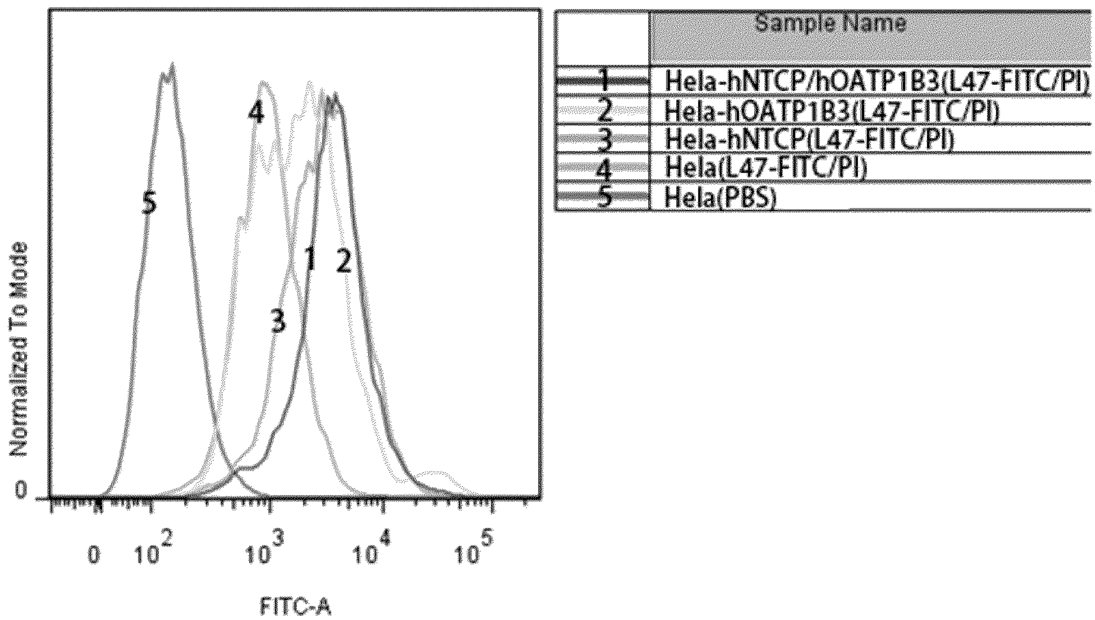
Normalized To Mode
0
0
10^2
10^3
10^4
10^5
FITC-A
5
4
1
2
3
Sample Name
1 Hela-hNTCP/hOATP1B3(L47-FITC/PI)
2 Hela-hOATP1B3(L47-FITC/PI)
3 Hela-hNTCP(L47-FITC/PI)
4 Hela(L47-FITC/PI)
5 Hela(PBS)

Fig. 10

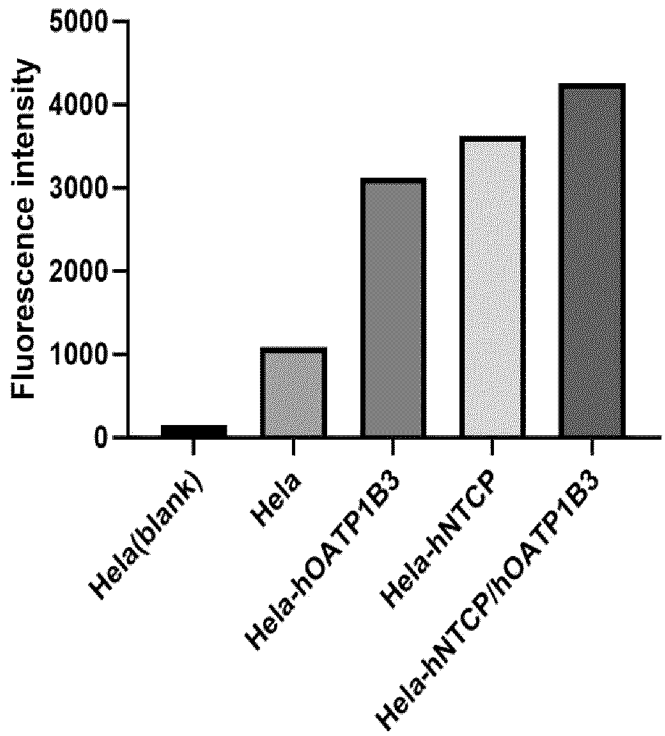
Fluorescence intensity
5000
4000
3000
2000
1000
0
Hela(blank)
Hela
Hela-hOATP1B3
Hela-hNTCP
Hela-hNTCP/hOATP1B3

Fig. 11

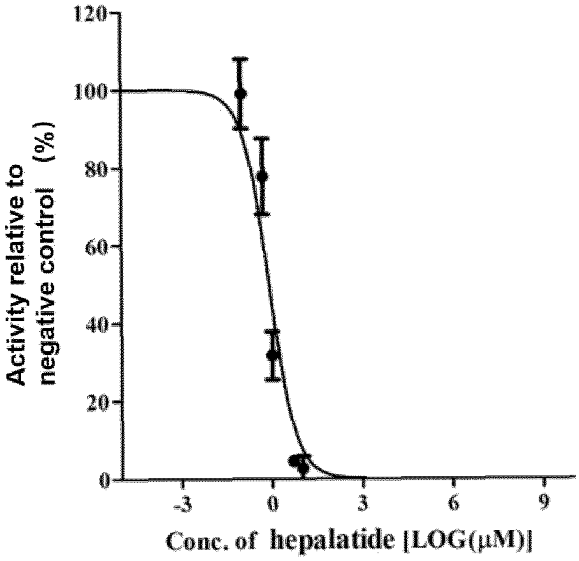
Activity relative to negative control (%)
120
100
80
60
40
20
0
-3
0
3
6
9
Conc. of hepalatide [LOG(μM)]

Fig. 12

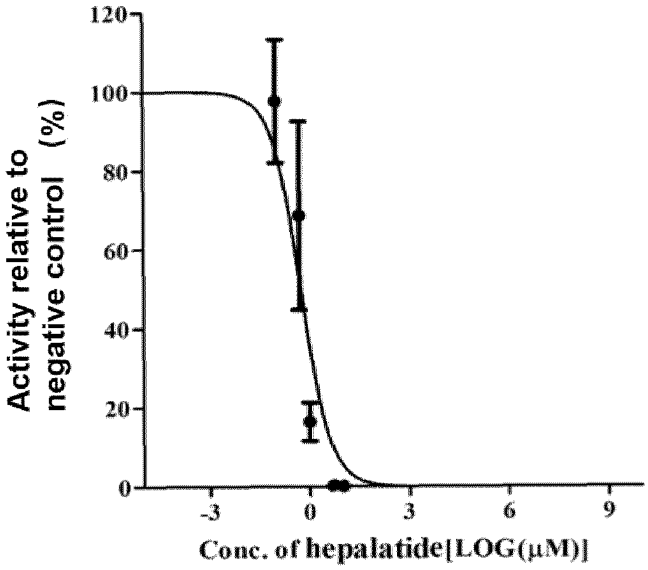
Activity relative to negative control (%)
120
100
80
60
40
20
0
-3
0
3
6
9
Conc. of hepalatide[LOG(μM)]

Fig. 13

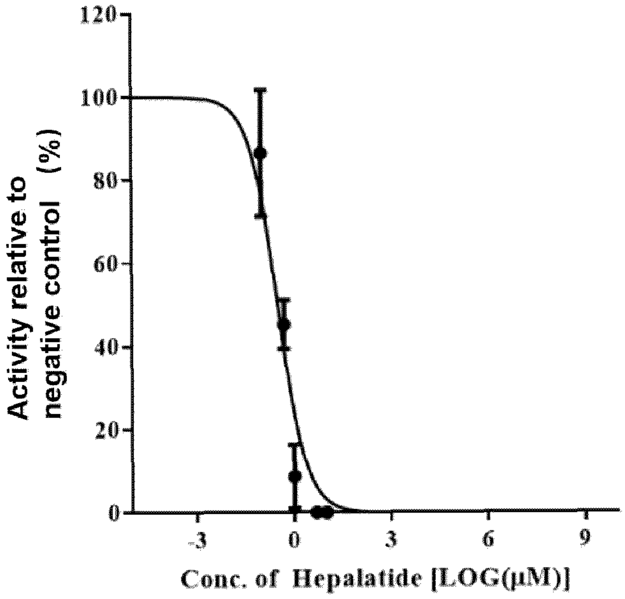
120
100
80
60
40
20
0
-3
0
3
6
9
Activity relative to negative control (%)
Conc. of Hepalatide [LOG(μM)]

Fig. 14

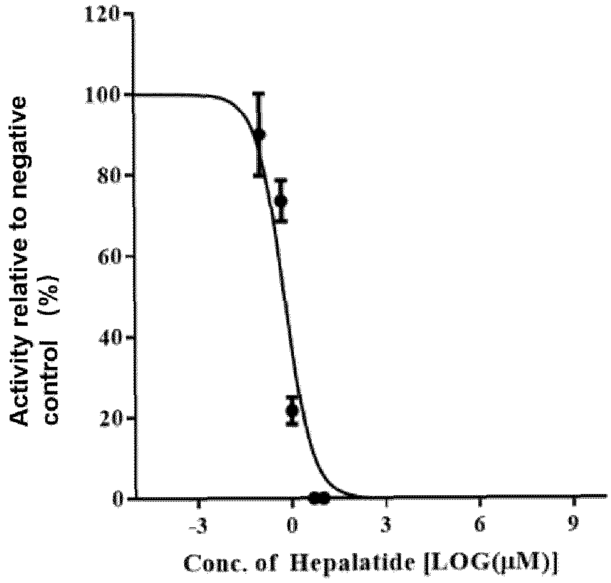
120
100
80
60
40
20
0
-3
0
3
6
9
Activity relative to negative control (%)
Conc. of Hepalatide [LOG(μM)]

Fig. 15

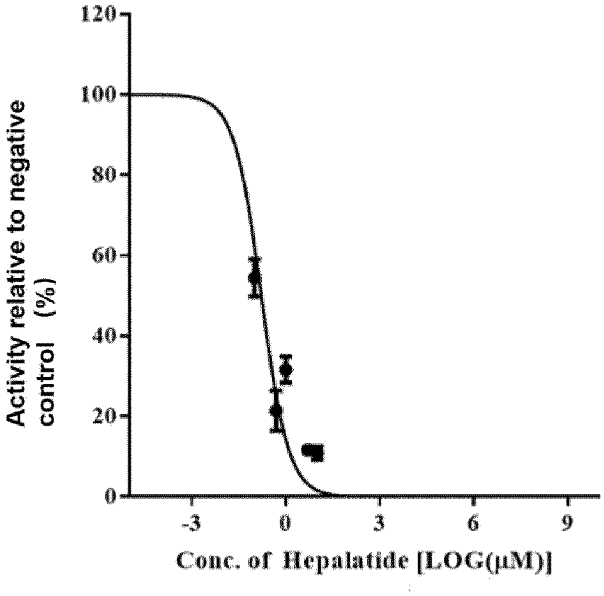
Activity relative to negative control (%)
120
100
80
60
40
20
0
-3
0
3
6
9
Conc. of Hepalatide [LOG(μM)]

Fig. 16

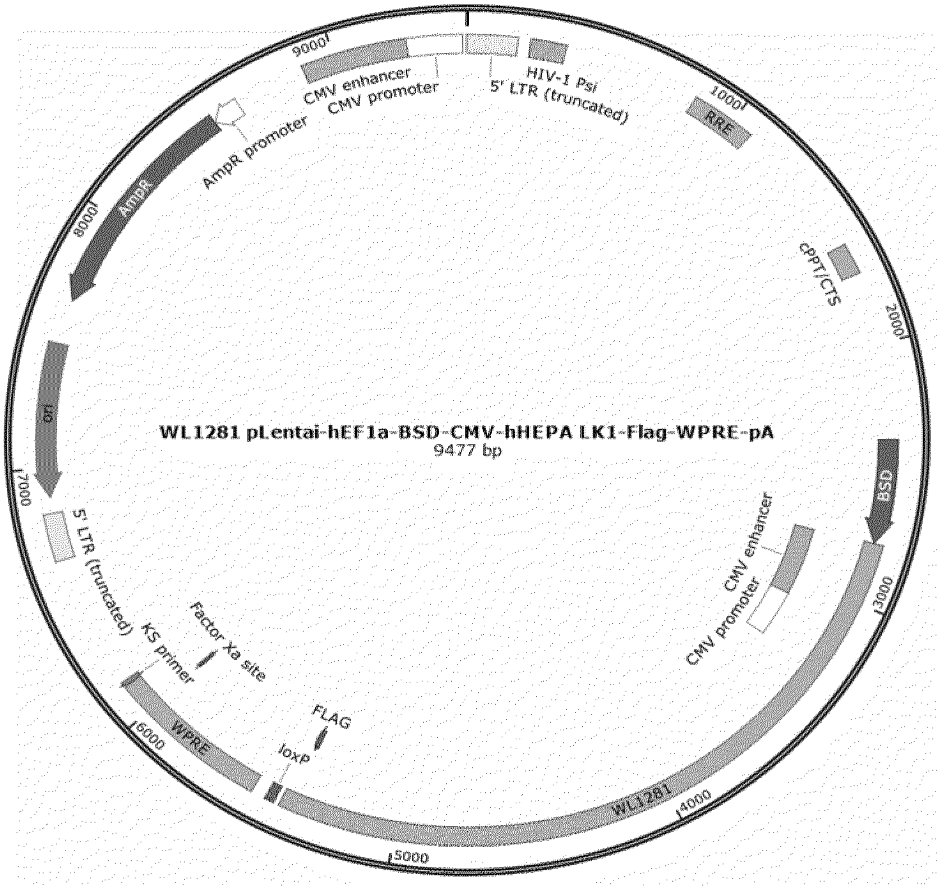
WL1281 pLentai-hEF1a-BSD-CMV-hHEPA LK1-Flag-WPRE-pA
9477 bp
CMV enhancer
CMV promoter
HIV-1 Psi
5' LTR (truncated)
1000
RRE
cPPT/CTS
2000
BSD
CMV enhancer
CMV promoter
3000
WL1281
4000
5000
FLAG
loxP
WPRE
6000
KS primer
Factor Xa site
5' LTR (truncated)
7000
ori
8000
AmpR
AmpR promoter
9000

Fig. 17

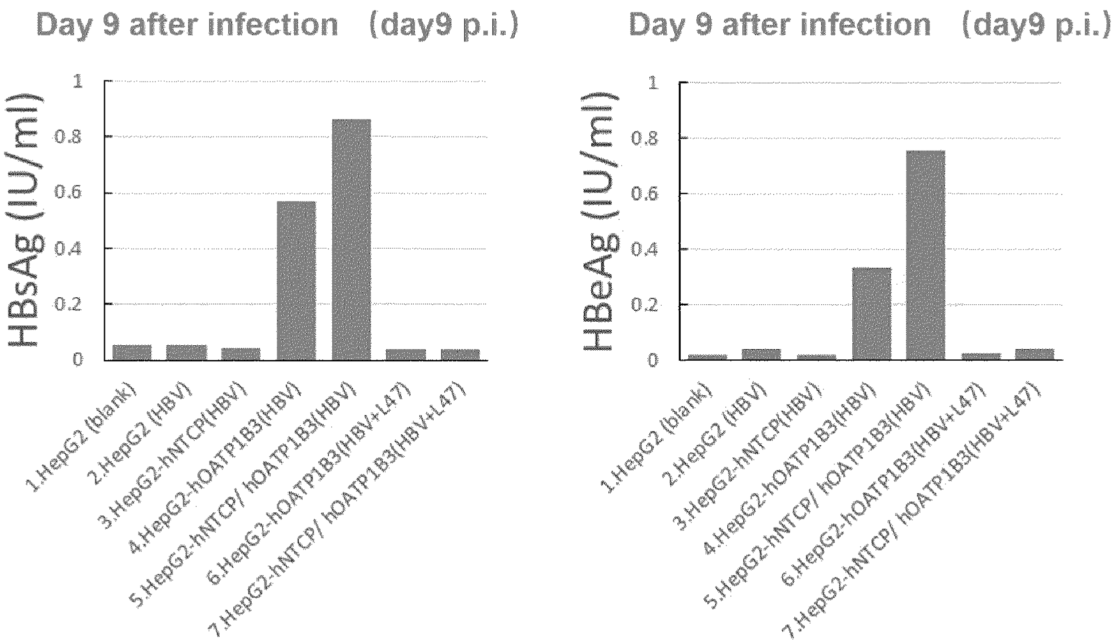
Day 9 after infection (day9 p.i.)
HBsAg (IU/ml)
1
0.8
0.6
0.4
0.2
0
1.HepG2 (blank)
2.HepG2 (HBV)
3.HepG2-hNTCP(HBV)
4.HepG2-hOATP1B3(HBV)
5.HepG2-hNTCP/ hOATP1B3(HBV)
6.HepG2-hOATP1B3(HBV+L47)
7.HepG2-hNTCP/ hOATP1B3(HBV+L47)
Day 9 after infection (day9 p.i.)
HBeAg (IU/ml)
1
0.8
0.6
0.4
0.2
0
1.HepG2 (blank)
2.HepG2 (HBV)
3.HepG2-hNTCP(HBV)
4.HepG2-hOATP1B3(HBV)
5.HepG2-hNTCP/ hOATP1B3(HBV)
6.HepG2-hOATP1B3(HBV+L47)
7.HepG2-hNTCP/ hOATP1B3(HBV+L47)

Fig. 18

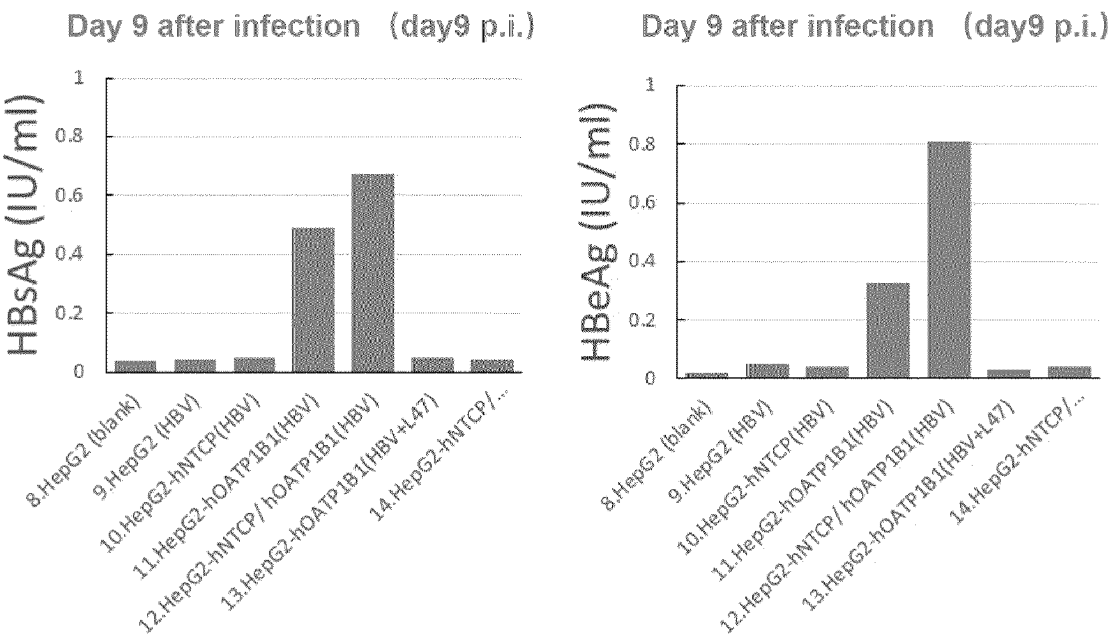
Day 9 after infection (day9 p.i.)
HBsAg (IU/ml)
1
0.8
0.6
0.4
0.2
0
8.HepG2 (blank)
9.HepG2 (HBV)
10.HepG2-hNTCP(HBV)
11.HepG2-hOATP1B1(HBV)
12.HepG2-hNTCP/ hOATP1B1(HBV)
13.HepG2-hOATP1B1(HBV+L47)
14.HepG2-hNTCP/...
Day 9 after infection (day9 p.i.)
HBeAg (IU/ml)
1
0.8
0.6
0.4
0.2
0
8.HepG2 (blank)
9.HepG2 (HBV)
10.HepG2-hNTCP(HBV)
11.HepG2-hOATP1B1(HBV)
12.HepG2-hNTCP/ hOATP1B1(HBV)
13.HepG2-hOATP1B1(HBV+L47)
14.HepG2-hNTCP/...

Fig. 19

## INTERNATIONAL SEARCH REPORT

International application No.
**PCT/CN2023/106678**

**A. CLASSIFICATION OF SUBJECT MATTER**

A61K38/16(2006.01)i; A61P1/16(2006.01)i; A61P31/20(2006.01)i; C07K14/705(2006.01)i

According to International Patent Classification (IPC) or to both national classification and IPC

**B. FIELDS SEARCHED**

Minimum documentation searched (classification system followed by classification symbols)

A61K A61P C07K

Documentation searched other than minimum documentation to the extent that such documents are included in the fields searched

Electronic data base consulted during the international search (name of data base and, where practicable, search terms used)

CNTXT, DWPI, VEN, ENTXTC, CNKI, 万方数据资源系统, WANFANG DATA, PubMed, ISI web of knowledge and keywords: 上海贺普药业, 乙型肝炎, 丁型肝炎, 乙肝, 丁肝, hbv, hdv, OATP, Pre-S1, Hepadnaviridae; GenBank, 中国专利生物序列检索, China Patent Biological Sequence Search System, EBI-EMBL, STN和序列: SEQ ID NO: 67-78, STN and sequences: SEQ ID NOs: 67-78.

**C. DOCUMENTS CONSIDERED TO BE RELEVANT**

| Category* | Citation of document, with indication, where appropriate, of the relevant passages | Relevant to claim No. |
|---|---|---|
| X | CN 109718364 A (SHANGHAI HEP PHARMACEUTICAL CO., LTD.) 07 May 2019 (2019-05-07)<br>description, paragraphs [0005]-[0191] | 1-16 |
| A | CN 104130316 A (SHANGHAI HEP PHARMACEUTICAL CO., LTD.) 05 November 2014 (2014-11-05)<br>description, paragraphs [0026]-[0040] | 1-16 |
| A | CN 108064238 A (GENEONE LIFE SCIENCE, INC.) 22 May 2018 (2018-05-22)<br>description, paragraphs [0005]-[0019] | 1-16 |
| A | CN 111542340 A (UNIVERSITY OF WASHINGTON) 14 August 2020 (2020-08-14)<br>description, paragraphs [0012]-[0233] | 1-16 |
| A | CN 114174332 A (HUAHUI HEALTH LTD.) 11 March 2022 (2022-03-11)<br>description, paragraphs [0005]-[0057] | 1-16 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 October 2023** | **26 October 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/106678**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/106678**

**Box No. III Observations where unity of invention is lacking (Continuation of item 3 of first sheet)**

This International Searching Authority found multiple inventions in this international application, as follows:

Invention 1: Claims 1-16 relate to the use of a substance in the preparation of a medicament for the prevention and/or treatment of infections with hepatitis B virus (HBV) and/or hepatitis D virus (HDV) in animals or diseases associated with the infections.

Invention 2: Claims 17-24 relate to a method for establishing a cell or animal model susceptible to HBV and/or HDV.

Invention 3: Claims 25-59 relate to a delivery vector for a drug or a group, a polypeptide for optimizing pharmacokinetics of a drug transported via an OATP, and uses thereof.

The above three inventions do not have a same or corresponding technical feature, and therefore do not have a special technical feature that makes a contribution over the prior art. Therefore, inventions 1-3 do not fall within a single general inventive concept, lack unity of invention, and do not comply with PCT Rule 13.1.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-16 (invention 1)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2023/106678**

| Patent document cited in search report | Publication date (day/month/year) | Patent family member(s) | Publication date (day/month/year) |
|---|---|---|---|
| CN 109718364 A | 07 May 2019 | WO 2019080919 A1 | 02 May 2019 |
| | | JP 2021500411 A | 07 January 2021 |
| | | US 2020316166 A1 | 08 October 2020 |
| | | EP 3701966 A1 | 02 September 2020 |
| | | EP 3701966 A4 | 30 June 2021 |
| CN 104130316 A | 05 November 2014 | None | |
| CN 108064238 A | 22 May 2018 | JP 2017538445 A | 28 December 2017 |
| | | US 2017260258 A1 | 14 September 2017 |
| | | US 10196437 B2 | 05 February 2019 |
| | | KR 20160063725 A | 07 June 2016 |
| | | KR 101864693 B1 | 07 June 2018 |
| | | WO 2016085289 A1 | 02 June 2016 |
| CN 111542340 A | 14 August 2020 | US 2020345838 A1 | 05 November 2020 |
| | | US 11389532 B2 | 19 July 2022 |
| | | WO 2019099624 A1 | 23 May 2019 |
| | | US 2023053634 A1 | 23 February 2023 |
| | | EP 3710049 A1 | 23 September 2020 |
| | | EP 3710049 A4 | 05 January 2022 |
| CN 114174332 A | 11 March 2022 | WO 2021013135 A1 | 28 January 2021 |
| | | KR 20220035483 A | 22 March 2022 |
| | | CA 3145057 A1 | 28 January 2021 |
| | | US 2022259292 A1 | 18 August 2022 |
| | | EP 3999533 A1 | 25 May 2022 |
| | | EP 3999533 A4 | 07 September 2022 |
| | | JP 2022542035 A | 29 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 1396309 A


### Non-patent literature cited in the description


- **JACQUET S et al.** *J Virol.*, 2019, vol. 93 (5), e01738-18
- **HUGHES et al.** *Lancet*, 2011, vol. 378, 73
- **SUREAU**. *Curr Top Microbiol Immunol*, 2006, vol. 307, 113
- **BARRERA et al.** *J Virol*, 2004, vol. 78, 5233
- **LE SEYEC J et al.** *J Virol.*, 1999, vol. 73 (3), 2052-7
- **BRUSS V et al.** *Virology*, 1996, vol. 218, 396-399
- **YAN H et al.** *Elife*, 2012, vol. 1, e00049
- **NI Y et al.** *Gastroenterology*, 2014, vol. 146 (4), 1070-83
- **HERRSCHER C et al.** *Cells*, 18 June 2020, vol. 9 (6), 1486
- **LEMPP FA et al.** *Hepatology*, 2017, vol. 66 (3), 703-716
- **HE W et al.** *J Virol*, 12 September 2016, vol. 90 (19), 8866-74
- **HAGENBUCH B et al.** *Mol Aspects Med.*, 06 April 2013, vol. 34 (2-3), 396-412
- **KÖNIG et al.** *J Biol Chem.*, 2000, vol. 275 (30), 23161-8
- **ZHANG Y et al.** *PLoS One*, 2017, vol. 12 (6), e0180257
- **LEMPP FA**. *Hepatology*, 2017, vol. 66 (3), 703-716
- **YAN H** ; **ZHONG G** ; **XU G** ; **HE W** ; **JING Z** ; **GAO Z** ; **HUANG Y** ; **QI Y** ; **PENG B** ; **WANG H**. Sodium taurocholate cotransporting polypeptide is a functional receptor for human hepatitis B and D virus. *Elife*, 13 November 2012, vol. 3
- **NI Y** ; **LEMPP FA** ; **MEHRLE S** ; **NKONGOLO S** ; **KAUFMAN C** ; **FÄLTH M** ; **STINDT J** ; **KÖNIGER C** ; **NASSAL M** ; **KUBITZ R**. Hepatitis B and D viruses exploit sodium taurocholate co-transporting polypeptide for species-specific entry into hepatocytes. *Gastroenterology*, April 2014, vol. 146 (4), 1070-83
- **LI J** ; **SHI TD** ; **HAN JF** ; **ZENG XG** ; **FAN CL** ; **HAN C** ; **LIU HL** ; **WU YZ**. A systematic study of Tupaia as a model for human acute hepatitis B infection. *J Vet Med Sci.*, 10 July 2021, vol. 83 (6), 1004-1011